# EUROPEAN PATENT SPECIFICATION

(11) **EP 3 201 630 B1**
(45) Date of publication and mention of the grant of the patent: **28.11.2018**
(21) Application number: 15771573.1
(22) Date of filing: 28.09.2015
(51) Int. Cl.: G01N 33/574, C12Q 1/68

(54) **MULTIPLEX TP53/CEN17/B CELL GENE-PROTEIN CO-DETECTION ASSAY AND UNIQUELY SPECIFIC PROBES FOR 19Q12, INSR, ATM, DLEU2, TP53, AND 13Q12**
DETEKTIONSASSAY VON MULTIPLEXEM TP53/CEN17/B-ZELLGENPROTEIN UND EINDEUTIG SPEZIFISCHE SONDEN FÜR 19Q12, INSR, ATM, DLEU2, TP53 UND 13Q12
ESSAI MULTIPLEX DE CO-DÉTECTION DE GÈNE-PROTÉINE DE TP53/CEN17/LYMPHOCYTE B ET SONDES UNIQUEMENT SPÉCIFIQUES POUR 19Q12, INSR, ATM, DLEU2, TP53 ET 13Q12

(30) Priority: 29.09.2014 US 201462057164 P
(43) Date of publication of application: 09.08.2017
(73) Proprietor: Ventana Medical Systems, Inc., Tucson, Arizona 85755 (US)
(72) Inventor: ALEXANDER, Nelson, Marana, Arizona 85658 (US); GROGAN, Thomas M., Tucson, Arizona 85718 (US); HENRICKSEN, Leigh A., Oro Valley, Arizona 85755 (US); KELLY, Brian D., Tucson, Arizona 85741 (US); NITTA, Hiro, Tucson, Arizona 85718 (US); STANISLAW, Stacey, Tucson, Arizona 85742 (US); TUBBS, Alisa, Phoenix, Arizona 85044 (US)
(74) Representative: Burger, Alexander
(86) International application number: PCT/EP2015/072231
(87) International publication number: WO 2016/050681

(56) References cited:
- EP-A1- 2 636 757
- US-A1- 2008 299 555
- HAIPENG SHAO ET AL: "Clonally related histiocytic/dendritic cell sarcoma and chronic lymphocytic leukemia/small lymphocytic lymphoma: a study of seven cases", MODERN PATHOLOGY, vol. 24, no. 11, 10 June 2011 (2011-06-10) , pages 1421-1432, XP055233883, GB ISSN: 0893-3952, DOI: 10.1038/modpathol.2011.102
- "Chromoprobe Multiprobe-System CLL Panel", , 8 July 2005 (2005-07-08), pages 1-95, XP055232748, Retrieved from the Internet: URL:http://www.genycell.es/images/producto s/protocolos/pmp__.pdf [retrieved on 2015-12-01]
- ALMOND CLAIRE ET AL: "An evaluation of the Cytocell Chromoprobe Multiprobe-CLL System", JOURNAL OF MEDICAL GENETICS, vol. 42, no. Suppl. 1, September 2005 (2005-09), page S82, XP008178334, & BRITISH HUMAN GENETICS CONFERENCE; YORK, ENGLAND; SEPTEMBER 12 -14, 2005
- LIU HONGXIANG ET AL: "Richter transformation: clonal identity does not indicate a linear disease progression", BRITISH JOURNAL OF HAEMATOLOGY 1, vol. 157, no. 1, 3 November 2011 (2011-11-03), pages 136-139, XP055233501,

### SEQUENCE LISTING

<110> Ventana Medical Systems, Inc.
<120> MULTIPLEX TP53/CEN17/B CELL GENE-PROTEIN CO-DETECTION ASSAY AND UNIQUELY SPECIFIC PROBES FOR 19q12, INSR, ATM, DLEU2, TP53, and 13q12
<130> P31819-WO
<150> US 62/057,164
   <151> 2014-09-29
<160> 74
<170> PatentIn version 3.5
<210> 1
   <211> 5000
   <212> DNA
   <213> Artificial Sequence
<220>
   <223> nucleic acid probe
<400> 1
   agtctgtcca cggcagagga aattctagct ccaggtttcc agtgtgaagg caaaagggtg        60
   aaattgtcca cgtgttacca atctcaagtc tccctgcttt ttcaaaataa aaccctgcag       120
   aaagcaatgg ctccatctaa aagaatgact ctccattgtt tgaatgtcaa gcactgtggt       180
   tcactggagc agggcctccc ttgggggacc agcatttatg ataccctgga atgacactgc       240
   ttaggaagcc acctttttgg tgcctttcag ttgatttcag gcagtcattc atgcactgtt      300
   tatatggaaa aggacacttg tcacatgaaa ttcatgagct catgtctata cctcactgcc       360
   cagtgttgta ttatttactc tgcggtatga gtgtctcttt tagagaaatg ccctcagctt       420
   ctggatgtct gcagtttcta gtcacaacgg ctgtgattct ttgaagtggc cagtaaacca       480
   gatttttgac aggtgtggag agccccggcc cctgctgctc tgcagataag tgccaggtat       540
   gaaatgaggg ccggtcctcg gaattgtgca ttgacaggca gggttgaagc ctcaggaatg       600
   gattcaggca ccacaaagcc agatctctgt gcttccctgc tgtgccccaa ttccagtttg       660
   ggtgatttta catcaggtcc tttctctctt actttcccat atacgaatat aaacacatag       720
   cccctaggtc tggagtcaat ggctgctttc ccagagttaa gcacaggtca aaataggtgg       780
   caggaggtgc tagctacatg cttcaagtgt ctcagcaaac ctgagaaatg cagtgaagaa       840
   gctctcttag gacacagaga gccctacgtt tatccagaca gacactccat tagccgaaac       900
   gaccagacac acgaagtttt aagagtcccc gctgtcagaa ttccaaatgg ttagtcttaa       960
   actgacttag ggcgcagcgc tgcctcttta caaagagctg agcagaagtg tccagtgtta      1020
   gcgctggcac cttaccgcct atctctgtgg cactattagg cctgaggaat gattgagggc      1080
   tgatgtcgct gtgcccaggc cacctgcaat gattccatca aacattgact tctcgtggaa      1140
   ctttcacaaa gccactactg ggaaaacaca tgtgtatcca tgtttgaaag tttatcattt 1200
   tgaaatgggt ccgggctacc tgttgctgcg gagtcctaac agaaccgcta caggaatggg 1260
   cccaccctac ctaagaaaaa ctgagggctg gagcacgggt accccaccac catcagggaa 1320
   ttcgagggga gactggcagt tatccaggga tcccagaagg agtcaactgg gagttaagag 1380
   tcctaacaac cctagaaaaa ctgctccttt ctgcgagctg gtttcacact cttaacctga 1440
   tgcaaagcgc cgagagctgc tggggaatga tatcactaac ccaaagcttt ggtccttagg 1500
   ctaagcacct gtaacagatg aagcaaagtt atcaggcccc gcaagttgct ctctacagtt 1560
   tataatcccc atcttccatt tgagggagaa ctagatgcta tcatttcttt tttttaatcc 1620
   cccaggtgtg atctaccatg cattgtctca gaaagaggcg aatgactccg atgtccaggt 1680
   cagttcttgg cagggagtcc ggctggatca aagtctgtag ttctcattac ctgttcccac 1740
   gtgccagcct ccttttctgt tgtgccaaag tcaagtttgg taaaatgagg ctttccttaa 1800
   accaggctgt gcccacgtgg tgggcagcat cattcaacct tgtcactgtg ccctctgcac 1860
   ttctgcagaa ggtgtgcaat cacatatcat aagtgaaatt atagtcacaa tgacgagagt 1920
   gaattaattt ttagttagca tctacaggtg caaagctgaa gcagagttcc caccatcata 1980
   ctgtgagctc cctcgaaggc catttttccc agaagtcctc tctaagagcc tgtgctcatt 2040
   catggtaagt tgacgccctg ctctcagtga cacccttcag agagagcgag cctggggcct 2100
   cctagttttg ctgttttctg tagtgttagg atcacttcct ccatagctta gtgctactct 2160
   gagctcactt tcatctggtc atgcctggtt gaatatattt gaagtgagcg tttgacacgc 2220
   tgttgtgtgt gacaaacggt catgaattgg tgtgtgagtg tgacttacat ctcggcagct 2280
   gtgggtagca gatgcatggc ggtaacccga gctccccacg tctttctgcc cgcggtgctt 2340
   acccttcttg gtgtgtgaac ttggctgagt ggctggggag gcagcctggc tccacctaag 2400
   accttcagga gtaagacccg tgcaagtaca tctgttgccc atggcaacag gctgtttgtc 2460
   cttcggcttt gttagacaga aaagaggaac tgatatgttt ggagatcaac ggccaaatag 2520
   cctcccacgc tccagaagac gcttaaagta tgccatgctg ttcccggccc cactcaggag 2580
   atcagctggg cctcaggtgg tgctctttgg ggtggatctc aagcctctga tcattgtaaa 2640
   tgcggcttca ggaaccagcg ttggtattgc ctggctgcag acggttaaag acctagttgc 2700
   ctgtttaccc tgcaggcagc cacagatgat tcaggccaag ctcttaaagg cagatcttca 2760
   cggggctatt atttcaggta atttacctaa gagcgtgtag ctcgctggct gtcttagcca 2820
   gtccggccat ttgctggaca caccctaagt ggtctctgtg taccttctcc acacagggac 2880
   ccctgttggt accctaaagg gtgctgggca catatgtgac ctttaattcc agaatcctgc 2940
   ccaagacccc tgtggccaca gaccctccga aggacacctc ataggaacag tccagaggct 3000
   gcacattttc aaaattatca ccaaagaaga ccgcctgaaa ggtatgtagg tgtttctgag 3060
   ggcattgctt gcgggctgtg gctcccagct actgcgttag agcttggttt ttcttgttgc 3120
   ttcccttatt ttcctacata tccttctacc tcacattgta aacctcggaa agtggtgtaa 3180
   tagtttctcc acagagcata tgacccctca agtcagcgca gattgcagat acttgctgaa 3240
   cctgcattaa agcctgcaga gaccatcccc tgcacccgtg gctcccccaa gaggtgtgtg 3300
   tgtgactaga ttgacctgtg gcagaataac cacaggcaac cctaatgccc ctccccttgt 3360
   gggtttcccg tttcctatta gcttattttt ttcattcaat tgtggaaacc gatggcttta 3420
   tttcctacat ttacgggagc aaattccagc ttcggtcaag tgaacggtct gcgaagaagt 3480
   tcaaagcgaa gggaacgatt gcgaaaggca ggccacaaat ctgttctccc ccaggagaag 3540
   tagcagtgcc agggctttgc gtttctggta acttccaccc aaaccacacc agagagaaac 3600
   ctccccaaag ctcagtgcac ccagagtctc acagggacca acacggtgcc tcggtcctgc 3660
   ttccatccat gcaagtgtcc tccgctcacc agggtgttct cggatgcagt tcaaagtgca 3720
   ggatgtgctg cctttattac ctgctttccc gctgagcaaa tccctgtctt gagtgggaag 3780
   tggcatctat tcccccagcc atggagggta atgtggtgct ttttggcccg atctctccca 3840
   tgtccactgt ccaaggagca ggaccctggc agagctgagg ccttggccat gtggtcccac 3900
   gactgatctc ccaggttcca aagagtaaac aggaacatcc ctccacaaag ccgtaaattc 3960
   tggtaaaaaa gctcgctttc tggacaatgg ctttagcttt gaggaggata taggacctca 4020
   cccagagact cacaaaacgc cagatagatg cacagactgg gtgaaatgca acagactggg 4080
   atagggaagg atttcctggg gcccaggatg tgcctctaat aatgttgaat aagggttggc 4140
   acacagcaac tgcaagtggt gctggtcagt gacccctgtc tgggccagat gagcccgtgt 4200
   ccagctatgc agaccgagga ggttatgctc catcaacagc tgtgacgtgc agcaagtgtg 4260
   gcccgcgctt ctctgcccct ttcagtttcc cccatgaagc caggaaaccc aagttcaagg 4320
   cccaagttcc ccgagacctt cggttccaac cacagagaac gtgaagaaag ggagtgtggt 4380
   gtttattgca ggcacattta ttttaacagc tccctccttt caaaatacgg gctcttccgc 4440
   taccccagaa ctccagggac caagtgctct gcagccctgt cactagggcg tggttgccac 4500
   gctgaggaca ctgagatgaa ttatccccgg atcacatggc cagtgggtga gtcacatggc 4560
   aagtcccctc tcaccaaatc agctattctt ccagaacttg actgttagct cctcaggggg 4620
   cagaccgatg ttttaggcct tcccagacca ccctaagtcc accagtcacc atctccacag 4680
   tgctggagag agaagctgac cagccccatt ctggctccat tgagtaaaag gtaagttgtg 4740
   gccctaaata atgaagacag aagagctaat gggctcggtg aagaagggca gctcaggcgc 4800
   gccatttgct gtcctggtgc tttctgttgt tgaatggaca gttcagaacc tccagaaatc 4860
   accctgagaa tctcaggctt tgtaacagca gtgtctcacc cagacaccag gagcctggat 4920
   ttgccaccac tttctatgct gtgttataaa cacaaacatg tgtctgcctc cttgactata      4980
   agctctgatg aattcgcccc                                                  5000
<210> 2
   <211> 5000
   <212> DNA
   <213> Artificial Sequence
<220>
   <223> Nucleic acid probe
<400> 2
   gctcggagaa tgagtaagtg aatcagtgaa tgtgcccgca gctggtgtga tcaggaactt      60
   ttgggtcatt tcagggccgt ctctggggca ggatggtgct accagcagtg cctcctacag     120
   agcactgatt aagtcactac tgagtgctct acaggtaacc ccttcaaatc ttagcaacag     180
   cttcaagagg caattaggaa gctgcctttt tttgaagtgg tttaagatct ctgctcttga     240
   ttgcaatctt ccaagagctg attttggcca cttggctctc atttctgaag tgatgatggg     300
   cctaacttgc aattcagagc caggaatcac acttatggcc accctgtgtc tgaccttaat     360
   gagagaggct gtggctttag gtccttagca gctcatctca tcagagcttt ggtgactgtc     420
   acccaggccc ctgtccaagt tctggcctca tggcactaat cccccacgta gccagtggct     480
   agctgtgcaa cctagagcca ccttccagaa cattagccct gccccctgtg gagtccctga     540
   gaagagaaat gccccacgag tgacaaagaa ggcaaagtat ctcctcactg tgtgctggag     600
   cggccggcac agcgactggg gccatacatc tcagcttcca ctcctgagtt ctggacttct     660
   cccacaccct ttgtgtggct ggaggtttta tgagaaggcc caaaaaatgg gtccccgtac     720
   acagcgaatg ggatgtttca gcagagacac aaacaagaag gatgcgaggg acccttactt     780
   ggagatattt tctcctgtcc atttgggaaa ctgggagaca agaatggcga agaaaaggag     840
   tctcgaagat ccgtcaccat cagctaggag gatggaacaa gcagatggag aggaaggagt     900
   tccagacaaa gacagaacat gaatgaatga ccatatagga acttgccaga gagaagcaga     960
   cgtgccccag gtaaggtgag gcctggttca tgcacatgaa acaggatata gatattccaa    1020
   aatcacaccc tggtcacaac acaggaaaca gtaataagag taactgatcc ttacgcatgg    1080
   ttgctatgtg cgtatgctgc catttgctcc caagacagaa gtttgctctc tttatccatt    1140
   ttctcagaat aaaagacctc cccgcttctg tcttccaagg attataagca aggaggtttt    1200
   aacatcatga gagggcacct cctgcaggaa ctgcaaatgc aaggcaaact tgtcagcctc    1260
   cagaggcctc agggagggca gccaaggtgg gtgacagcag tctggaagtc aggcctctcc    1320
   tccacttctc tgaatactca ggtgcagtcc ctccagaggg cagggctcat tgcccttgca    1380
   gtggatccca tctctccagc aaaataccac aagtgtatac agtaaatggg aatctccctc    1440
   ccacccattt tcccttccag aggcaaaccc atgaccagtt tattgtgaat tctgccagca 1500
   ccagacccca aaggctgaca ctgaaatgcc aaatggttct ccaagatgtg tcatccaacc 1560
   aagacagctt ctcctgcaca gacccctgcc ctctactcgg caaaagcctc ttggatgtac 1620
   tccattgaca ttaggtgggt ctaatactct caggttatat tggcctgcaa catttatttc 1680
   acatctcgaa ggcatttttc tcctttcccc ctttggcctg gttggagcct caaggggaaa 1740
   atccatgctg ggaaacaaaa acaataatgc ccgtgaacat gaagtggatg tgcagacagc 1800
   gcatgtagta gtttgggaat cagacaaatt tagggtcagt ttgaggatct acccaagcta 1860
   aacagtagga cctctgtttc ctttgttttt ctttaatttg ggcattgagc acagaggtgg 1920
   ttgccagaat ccctaacctg aaaatgccga gccccactgc ttttctgaaa cccgggttcc 1980
   cgcagtctgg aagccagctt tccacagcta tgtggcttgg ctgactcaac ttctgcctgc 2040
   ccctgagtca cgagtttttc ctctcttatt tggaggagcg aggccctcca ccctccaccc 2100
   gagaccagtg cgtgctgcct ccagggcgtt tcttggagcg ccattcagat ccatagttgg 2160
   aaggaaggac agaagaggag actgaggtcc aagtgggccc gcaggacaag gctaggagga 2220
   acatcctccc tctagtttcc cagtcatgaa aaggcttcct tctcccgagg cctcctctgt 2280
   acggacaagg tgttagatgc tccttcctcc gcagcctgac ttcacccatt ccaccttgag 2340
   ctgctcccca ggaccggagc tctgccccta gatctttgca cagctgactt caggctggag 2400
   ttttcttcac agtgtttagc actggctctc agaaaagacc ctgtgaatgt acttctagtc 2460
   tcactggcaa gaagtgtgca ttcctgaagg gcctggtctc ttgccaagaa aaacctacag 2520
   gcaaggtcaa accaggatgt gaagttgtgg tcatgtagag aaggtgagca tgccggggca 2580
   gctcccagga ggatgttgtc accttataga cgaaagtgga atccaatctg agccctccat 2640
   tcctggtcac atctatgtcc tctccccagg ctacttactc ctgactttct ccttcctatc 2700
   gttgtttgcc gtcagaccac attgatctgt atctctcatt gcgtgagtag ccccgggttt 2760
   ttatcctttt ccctgctggt aggcattttg gttgtctctg gtggaatgca gttctggttt 2820
   tgtcagattg tccctcagcc aggtactcgg tgatcagtca tggtgggaca ggtcagttct 2880
   gtcactggta atcccctagg aggctcagaa ctgcagctca gtgacccaga aacactatgg 2940
   gtgtgatcag ctcagcccca ggcgtgccgt gtgtaccgga atgcatgtgt gcacgtggtg 3000
   ttctggactc ccagtgcctt tttgctggac actgtgtcct tatggcttca ctgcaggtaa 3060
   tgcctttccc ttcaggaagc cccagaacac ccacaggtct cagccgggtg aggactctgt 3120
   cctgtgtcac ctctctccag gtgtccagct gtctcatgcc tttttgtcct gtcctcagct 3180
   ctccgtgtgg tcagcgaaac caaggacacg ggaaccaact gtcctgccag ccgagtgctg 3240
   ctgtccacct ctcaaatctt cctcccagct gtgcctggac tccatgtccc tgccagaagc 3300
   ggtggcaaaa atctaccact gtcccccaca ctgggagttc ttgtgtatgc tggctgcctg 3360
   aaggagacag acgctcatgc tgtgccttgg ctgggctccc ctttagagct ctgcaggatg      3420
   cagtgttccc acgggaccca gaggctgcag acactaagga gactttcaca aaggtcaagt      3480
   agcccctacc tgtgggcttt cagagggttt ccgctggcct ggccacttcc ctgcccagcc      3540
   tgttctttga acaaggccag aaagaggcct aggagaaggc aaagccgctc ctgggctcag      3600
   tgtgcccccc aaacctggcc ttggcatctg agctccttga aagcacaggt gtttctcatt      3660
   tgtttttggc ctccatggcc cccaacccat ggtcctgcac aatgacagca ttggctttgc      3720
   ccaacccgct tgtcctgcct ctgccaacac ctacacctgg gcaacccagc caaaccctaa      3780
   ggtgtctctc gaccctttct aatccttgga gcatcagctc agggttcctg ccagacattc      3840
   tgaccctgaa acacaggctt tctggagaag agggcctgcc cactgtggtc ccaggctggg      3900
   gcagaggcct ttgaacctcg cctgccccag ccataaccac ttgctggcac aacccaatcc      3960
   ctgagggttt ctttccccca cgccagagct ggagttggta acggttttaa atcagttaca      4020
   gagaaaacca cccagcaatg agcaactcca agatgtgaag gggagcattc agtagcacaa      4080
   tggacacagt gactggcgcc ggagcaagcg gttagggaaa tcagtaagtc ctcaaaggag      4140
   tactgggaga aattagcatg ggggccggac acacagttca gagagtgggg acagccagcc      4200
   caaagagggg ccacactgtt tggaaataca cgtgcctgcc ctttttagtt cagctctgct      4260
   gaggggcttg tgtgtttgct gaaaagatta tctcaacatt gcacagccgg ccctcctgcc      4320
   ttggtcagtg ggaacaagca catcagatca agctctcatg ggtctcagga tctgggatgg      4380
   tgctttggaa ctgaatgttg atacataaat aactcacatt ttaaacacta gaactcaggc      4440
   atgagacgcc catgcctgcc tgtctgttcc ctctgagcca tgccttcatc gggtgggaga      4500
   aactgaaggc acatcaggca atgccctctt ctgcatggct tagatgagtc ctgcatttct      4560
   taattcccat atgttcgttg ttgggattga gagacattct aggacccagc tagttccagt      4620
   gtcttcactc agcaaggcct gctccatcgg acacaaaact gatcaggagt tggaccatct      4680
   cctccctggg catttttccc cagaaggatt tgctgtaagc aaccggagaa gttggcattt      4740
   gttcaacatg gaaaaagtgt cccccaactt tgaagcactg aaccaaacat ctttgttgag      4800
   tctgagctgg gaccacctgt gtcccctggg cctgacacag accaggacct gatgcacgag      4860
   taagaatgaa tggggcccaa tcgcctaaca aaggcaactc cacgatatct gcaaatcaac      4920
   gttttttcct tcacactaaa aacgggcaac tttagagccc actaaagcta tacaaaattg      4980
   ggagggggca tgctctgatt                                                  5000
<210> 3
   <211> 5000
   <212> DNA
   <213> Artificial Sequence
<220>
   <223> Nucleic acid probe
<400> 3
   gtgagactga atttcctcca ccaggcagct gatatgctcc ttgcccctga acttagaaaa     60
   tcttcagtga gcccatgtgg ttaaaggacc tggggacaga ttctgggact taaagacctt    120
   gtatccacgg aaagttctac ctgaagaggc cacgtgcaga atatggtgca gagaagcgga    180
   aggtgcccta gggaaggggt agccttgaat cgcatggacc ttccgaggcc caggacacag    240
   aagagctgga cagcttggtt cctatggagt ggcagtggct acttcacgcc agttcattgc    300
   ctaatttaca agttttactg aacttttccc catgacacca gtgaaatgct ccaagtcgac    360
   aagagttgca ctcatgtgca ctaaaccttt atgagaacat atgctcccgc ccatgaccag    420
   ggactggaag tagtgaatcc cccgggctct cttagtggac atcctgttct ctaccagctt    480
   ttactcaatc tgaataaaat agatgcacag tgtcatcgag gaagaaggaa acagccttgt    540
   ctcccccatc tccggttcct tcccggatgg caagatgctg aagtcaggcc tcactaattc    600
   gaaggtctgg ggccacccac acatttattt ttctcctcag tgtgagcctc agcttaacga    660
   aatgtccttg aatgtcaatc ccactacaga agggaagaac ctctctgact ccatctatgg    720
   tttgggccac agccccaggc caggtagacc ttctgaggct caggacacag gaattcttcc    780
   ttccccaagc caaagaaagt ttttcaggaa agctcagcag ctccaagcgc ctgtatgaca    840
   gacagcatcc agacggtgga aagttcccac agacaggaca ggcagcagtt agtgaataag    900
   tcacatgaaa tggccacacc atatgtgctg agaaggtgca tggctcctcc cagcctcctc    960
   cctggtttcc tttctgcagg gacctcacag tcatgaaggc cttctttgcc ctctgattaa   1020
   cttctgactt acgacagatt aaggagcaac ccggccacat gttctgctgg ttcatcacga   1080
   gggaggtggt ctgacccttc cctgcaggct cccgacccag gatggctctg aacgaacact   1140
   gttactaaca cacacactgg gctgccttaa gcaactgttc ttaaaagtca acacagttgt   1200
   ttccctttct caccagcact tcccatttca ttctaggtgg aaatattgaa acaggtgcta   1260
   cagcaattca actgaggaag ttatggatag attctattgc ccctactgta aaattgctct   1320
   cccaatcctg ggatataatt ttgagcccag agaacaaacc catgaatcca tgagaggacc   1380
   gcaaagccag gtcctcagaa aaaacctagc ttccgacctg ctccgaatga cttgaggtga   1440
   aaatctacag aggctaaaga gatgtccttg caaaaatgct acttccagct ctacctgctc   1500
   agaggaagtc actgtgtttt aatagtccag caccacgggt agagatgttg tggctgactc   1560
   catccaaggt tcaatttctt tgtagtaatc atttcagaag tcctgagccc ttgctaaaaa   1620
   gctcagcttc ctgaattctc caggctcacc agattgagag ggaagcccac cctgctcctt   1680
   ctcccaccgc aaggctgcct cacacctatt tcacactcag atttatccac ataccaggcc   1740
   ttgatcacag gaagcccatc tgccagccac agggaaggca aatgccaact ggaagccccg   1800
   aggtacagag agacggagac aagcccatct caccgagagc ggcctagctt tctgagtttg 1860
   gaccagattg taagagataa gggagaggaa ggcttcagat aacgctctta ataccaagct 1920
   tccccaggcc ttggctggga taaaacaacc ccatttattc ctgaatcgga gcttttgctt 1980
   gagtttgggt gactttcaag ggcccaacaa tgccctaggg tgctaggtgg tgttcacacg 2040
   gccagccaga gtcaaaactc agacggggcc agcgttctat gccctcactt tctcccaaag 2100
   aacttccatg gctccttcag gctaaagcca cccacctaga ccagggctgg atgatccgca 2160
   atgtgggcca cacccaaaaa ccctatagct tcttttgttg taaaaaaaat ccaccttgtc 2220
   ttgcatttca gatctcactg aagagttctt ctgtgcctgg aagacttatt ttcagtctga 2280
   gaagaatgat ttttcaatgg ttgtcttttc ttctattgga gctttggtct gtttgaggtt 2340
   ttgttctata gcagaatgtt ctctttcgct ccagtgtaca taaatgaagt cacccatggt 2400
   gtgatcatga aaacagagga gctgaaactt ctaggcacat tcaggctagg tcacatccta 2460
   tttcaggtgg tagctccacc ttgaaaagca gggggctgga agagctggga ttcaggaatg 2520
   ggcttgctga ctccaacagt gaaggtcttc ccctccaaga gaccccaccc gtggggaagg 2580
   gtgaccctca aatcttggac agggctctga gaagtcccag aatatctgcc cacagcaatc 2640
   tagcatcatc agaacccacc ttcacgtgta gccaccgtgt ctccatgttc ctggtaatgc 2700
   taacacaatc agaacagaaa tctagggctc ttggccttca agcctctgtt cttcgcttaa 2760
   cacacggctg gcagagatgg ggagacctga gagggccctg gaaaatcaga gaggaggaaa 2820
   tgtcagggac tcctccagca cggaaaacat cctgatagtt tggagcgccg ggaagagtgg 2880
   gcaccttgga atctgcaagg ttacccatct tatagccggg ccgtccctgc aaaattccag 2940
   ctgcatgggg gaaccacact tctgacttca gaatgaaacc agtgttttgc acacattcat 3000
   gcggccctgc gtgtgccaga ggcttgcaga gtgatctgag cacgccaaga gattgctgtc 3060
   aagtaagctc ctccacaaat ctgcagaaac ctcaaagatt tgtatgtgga tttgagacca 3120
   acgggaaacg cgctcccaaa caatgctcgt tgtttgcacc gagccgcttt tctgctaagt 3180
   ttcatcaagt aatttgcatg tggactcagg gccttcccag tgccagctcc cccttcagac 3240
   tggggaatag aacagtagcc aagagagata aggtcccctt ctggcatccc tgacttttca 3300
   ggaagggaat attgagacct cctggttctt gagagttact tgggcccagc caggctctca 3360
   gcatcttaca gggtctcctg ctactctctt atctgtggac acaatttgtt gtccatgcgg 3420
   taactttagg ggctgtctga gatggtctgt tcctaaaaga ctgtggatgg gagaggtgac 3480
   agtaaatcct ggagagtgga cctcacttaa aatcaaattc tcgcccttaa tacttccttt 3540
   cagaactgca ccgaaaatga cgatgtcttc tcatgcatat gaattatcca aagtgtagga 3600
   ccactggagt acgctgaagc ctttaaccca agtacattta atgctgcgaa gccccgagtg 3660
   aggcaaaggt gtctttttat tttagaagac atttaggaca taaagagagc ttatgctccc 3720
   caaatctgtt tccgagccag gtaggatgat gaattctgag gtgggactga gggggaacgg 3780
   tggcagctct ctcccctgca ccgtttggac attggcgtga caccctcctt ccctgttcat 3840
   ctgtgaaaat gtgcagcgtt ttcatttaaa acctcctgct ccaacatggg gccttctgca 3900
   acctgatgac ttctccttag agaaaaagca ataaaaggag cctcttctgt cacaaatttc 3960
   cacttagcat ggtttgtgca gaggcagaat ttcaggcttt tggactcaga gccccacatg 4020
   gagtctgctg acctgtcttg ggattgaagg gtgataggtg agaaggatgg agaatttgta 4080
   aagggccggc agtctccctg gccttcagtc attggaggtt ggatttccct gtttccaact 4140
   cttagggagt ctctactggg gccagtgtgt cagcatcacc ttggcccctt ctatacctta 4200
   tgaagggtaa gctgggatcc ctttggcccc acgaagcatt aaagccttca tgtcctctca 4260
   tcaacacacc ccattccagg gcttctgtgc ctccccagcc cttgggacat ttcagtgtcc 4320
   tccaccttta taccaaagac tgtgcccctg tcactatatc catgtcagcc tctaagcccc 4380
   cggctcccag gaccacatgc gccccaggtt tgctgcccag gcccaagcta cccacgggca 4440
   gtgagaaagg taccatctcc cagaatggga ccctcttgcc tgtgtggtca gtgccctttc 4500
   cctggaggcc cccccttgga actggaaata gtcctggata ccatgattga agcaacttca 4560
   ggctgtactg agcccacgcc tggctctggg gttgccctcc gctcctgcta cccactgtgc 4620
   acttgttcca ggtgcattcc caagggccga cttccttggt tcattttgac aaactttgag 4680
   gttgctcgtc atagccctta gatggagtgg tacagtcatc ccctggcacc aagcagccca 4740
   ttagcccaga atggttatct gatcagatgc ctgtctccta tggacctatg ggagctggac 4800
   agcgaaggct ctggcatctg tagccatgag aggaatgttt gccgagtgac cccatgagcg 4860
   agtgatgcca gcataaaggc cagtgcaccc aaggctggcc ttgttggcat ctcaggctga 4920
   tttgcgtccc ctggggccac tctgataact tggctaaaca aatacaggag gtgctgtagg 4980
   gacaggctca cagggccaga 5000
<210> 4
   <211> 5000
   <212> DNA
   <213> Artificial Sequence
<220>
   <223> Nucleic acid probe
<400> 4
   ggaaacttgg cgtaactgca gagaccacgg cttcttcaga gcctcacatc tcctgtgttg      60
   gtgccatcgc agctgcacag cccccagccc taacacccag agggaaaaga gaaggaaagg     120
   accaactgcc ttggcccatt cagtgatctg gccatgaaac caggggctgg ctgaaatgtc     180
   attgtccaag aaaaggtgct ggactggctc acctgagttc cacaggaaag acagactgtc     240
   ctgagaacag acactcgctc attttcatca ctgttcctca aacaagtaga ttttgtttta 300
   ataaacagcg ttgtcaagtt ggtcaagttt gttgtttctt acccaaactt cccctttaaa 360
   tcttcggcag gacagaggag gaccctgggc ttcttgtcaa atttgtaatt gtcacttgtc 420
   tagtgttttg gtgcgcacat ttacagaacg aagcctgctg aaaagcttta aagtacagca 480
   cactaatgat tcctgagata agacctctag catccacccc caggcttttc cactgcactg 540
   tcgacactga aggtcaagtt atgccccaga ctcctactgc tggaggggct tgggccatat 600
   gtggcctttc atagcaccta gaatgcccct gctcactcat ctgtgaagca aggacgccac 660
   tctcactctg cctgtcttgc tgtcactcaa cactcaacag ttcctgatgg cccaaccttg 720
   cctgttcaca ccagctctgg gcctgtcccc catggaccaa gctctggatt ccttgaaggc 780
   caccagtctc cagcctccag ccagctgggc ctgtctgctt ttactccaac accatcagga 840
   gctacaaaca agatttacaa aagccagcaa gtttacacgg gcctgaatag cagattctct 900
   tcacttcttc tgctccttta gtctagtatt taggaaaact ttcgtccaaa tgcaagcttc 960
   caagatgttc tccctctggt ctgccacacc tccttttctt gcgtttgcaa acccatttcg 1020
   gaagacagga aatgcttgat ttacctaggg cacccatcag gttctgtatt tcagatcaaa 1080
   gtagataaat ggtaaggcaa aaagatggac gctatttaag gtggttggac attgttttca 1140
   tggaggctac atggagctac gggccctagt caatgtcatg tctgagcctt tgttgaacac 1200
   cacaaatctt gcgttcctaa atgcaaccct cccttcctga attaactaag ccatcttagc 1260
   aactatatcc tctgtgggtt ttacctgttt ggacacaggg aaggtgctct cataacagcc 1320
   aacatgtggc ccactctgcc accattagga gcctagcttg tccaactggt ccagcctcca 1380
   ctgagcagtc aggtcctgct gctcctttct cgcaggggac ccagacttta aaacgcaacc 1440
   catgcaaatg aagcaagaag gcagaagtca agcccctgga ctcggctgtt ttcattggtc 1500
   agattagatc acatgcactc tagccatgac ccactcagag aggccagaga atgccgtttg 1560
   ctgattggct gagcgtggga gcgaggaccc cgctttgttc ggtcagagac caacagtagg 1620
   ccctgcttca gtttcctcat cctctagaat caaggaggac ccacgactgc ctccagttca 1680
   cccttgaggc ttctggggct cagaactctg agcgggcctc ccgggctgct cagctatcag 1740
   taaaggagtt agttcccggt gcccatgtgc tctgtacccc actgcatagg ggagagggca 1800
   ggcgccaggc atccttccat ccttgtcctt gatgtcactt taacaccatg ggaaccttga 1860
   catccttcac acatccttct atagacagtg ctggggcttc gaatcaccta tcctcatcct 1920
   aagaccgtga agtcacagtc ctgatgcgct actcttttct gagtgcatgt gcgcacctct 1980
   tggaactgtt aaaataagca tgctgccccc tcaagagaac cgtgaccaga aacttagtcc 2040
   ttgggaaata tgcgctactg cagaacttgt caaatggcaa agaatcaagg aaggaataaa 2100
   tcaagcactt tctatacttc atctggtggt tttaaagctt ccgtggttca gaggaaaatt 2160
   tgcatctgaa aatgcggctt ttcccctagc tgcctgatgt ggatgagaaa atatcagggt 2220
   tgtagtcctt agaagtctcc ttccagatta gtaatgggaa cagaattatc ccaagtgaat 2280
   gactctggtg gtgcctctcc tagtatgtgg cagtgaagaa gagtggggta cacctatctg 2340
   tacaacgtaa ctgttgggct ggaagagaat gccatgcacg ttccaacacc ttatttttgt 2400
   atctggaagg caacaagtcc agttgatgac agaggctacc tctgcggagg gtactgggtg 2460
   atgctgatca aagagaactt taaacccatg ggtaatgtct cgggcctgga cagaattctc 2520
   caattaactg agacctggtg acatttgtgg tactatgcga tgctcacagc acagatgcac 2580
   gctccctctt ctgattccag cagtatgcgt cagcattgcc tgtgcaccct ggtgacacct 2640
   ggacctcctt ctaccaaaca aagacactgc agtaaggtgg aagcctcctt tgacccctct 2700
   tggtgccccc atacctggta ctgaatcaca catgttcttc cttttcttgc cttttccccc 2760
   aaggcgctca ctggagctct ttctttgccc attacattct cctgagctct cccaggcttg 2820
   ggaaaatctc ccttcctctc cctgctctgt tattccaatt ccccaggacg ccagaaactc 2880
   agacaccttt gtgcaagggc ccttagggca aatccagaat cccaggcccc cgtgctctac 2940
   acaaaccatc tctgagacaa gggagggcct gcttcatacc ctgtttcact ccccaccaaa 3000
   taatcaaccg tgatttgtta tcctgcaggg ttgctcatac caagagtgca gttcatgacc 3060
   acgcttcctg cacacgtttc gctgaaggag tggttccatg cattttagtg gatcctgctc 3120
   acttgggttt ctatggcagt catagcttca ctcagttaag attgaggcat gccctgcctc 3180
   ctacctggac cccatggaag catcttctcc tccagtagtc taagcagttc tcagcccagg 3240
   gattccagca agtgtctcaa agccacacac tcggagagac gacggccaca tgacctggct 3300
   aatggcctga aagctgcgag aatggcttaa cacacctgaa gaagggacat gtatatgctg 3360
   ggggctggca ctctcccaca ttagctcttt tgcaccattg ctcactgaga cggtgatgct 3420
   taactcaggt atgtgtctaa tgacagggtc ttgcagaagg gaccagcctg agggactagg 3480
   actgcctcac ctttgcaagt aacaggggta ttttctttta ctgcgcagaa ccgaggggca 3540
   taaaaccctg attcgtcttc tgtggctggg tttgctctct gcgtgctcat atcctttaat 3600
   tccgtttccc ctcggctgct cccagaagtt tccataaaaa cagatgtaag gtaatcaaag 3660
   acttcccagc cccgggcctc tctgctcctg aaggcttcca taataatagt aataacacct 3720
   ttgctgagct tggtgccttt ttcaatgact ctcagagcca tgccgtgaga tcccggtggc 3780
   atcgcaatgg ctgatgacag acagcctaga ttgcacatgt gtgatattgg tgggtctcca 3840
   gcccctgcct ccatcctgag aagcatcctt tttggggaat ttcctgatgg gtagagtctt 3900
   agccatctgt ccccagagcc tccatacttg aggagtaagc tggtcttgct tgagagagca 3960
   ctgtggtttc ttggatctaa gagtggaagt gggatggaag ccagaaaatg tccagcaccc 4020
   atgatcgggg gcctttctta cttccccaaa gtgctctctg ctcacgccag aaatcatccc 4080
   aggaggacca gccccagccc tgtaatgagc ggaaatggta ttccatagta atccatccca 4140
   actgactcgc tattgtacac gcacctgcta acatttttta agctgggact ttatggaaag 4200
   ttgcttctga agtttgctga gcaattcccc ctctctctca ggcatacggt ctttgtgctg 4260
   gaaatttccc tcgtggctcc atgcgcggac tggcagacgg gagatcacgg agccgtgagt 4320
   ctttctttcc actgcaggac ctgttgtggt ccacgccatc aggaaagtgg tagtcagggc 4380
   agtctataac ccagcaacga aatgctagga ggctggcttc tgccctcacg tacctgatca 4440
   cagtgatatg ggacggtcca ctcaacccgg accttcatga aatcctcctg attatttctg 4500
   tgccatgttt gcctggtggg ttacctagcc cacttcactt cctgccagca cttgggtttg 4560
   taggccccgc ttcatagtac acctggatgt gcatttgtct ccaaataaaa tagagggaat 4620
   aaactgttac tcaggtgtta agcccctatg caccaggaat cttgaggatg cagggagatg 4680
   atgagaaaga gttggagtgg tgtcaagggg atcaccaacc ctgttggaac ttcccccggc 4740
   ctagtaccag ccccctaaac ctgtgagcca tgacaattct tctcaaagat agctcaaata 4800
   ttttaagccc agaataattc tgaccaaatc aataaagagg actcgggtca tgccccccag 4860
   gagaaccctg tgtatttgct agcatcagaa gtcatggacc caagtgggaa atagctctag 4920
   gaatggaggc ttctgtgggt cctttgctct tggaaacacc tccttcctgc caccttcccc 4980
   agtgccacac gtggcacctt 5000
<210> 5
   <211> 5000
   <212> DNA
   <213> Artificial Sequence
<220>
   <223> Nucleic acid probe
<400> 5
   cctctctgag gacttagttt ccccaactta acagactgcc ctggtgcctc ctggggatgt       60
   ttttggacac tgagtaaaat ggtgatagaa gactacaggg caagtaagga cctgacaaca      120
   acctgaccag ctgcacgtta caaagggcag atacaaagat aagaaacgag ggccaactac      180
   ggcagcattt gcatccttct gacatcagtt tccgttgttg aggatggtgt gtgaatcaca      240
   tcttgcgcta gagaatggtg cgtctaaggc tcagcacaca gcgatacctc gggaaatgtt      300
   aggcagtgca catttttaag gaaaatgaaa gacatggagt tggatgaaca tagggctgtc      360
   tctgtgttca gggggccggc atgacggtgc aggagcagtg cagacccacc tggctatggt      420
   ctgcaacctt tggctgttct tgcttaagga acaggaagat gtgactttag catgacccaa      480
   actgcaggtt tacacgttgg caaagaagct ctcgctccta aagcagaaag ttccacagct      540
   tctaggtatg catgaacgca tgtccttgat ggtgaggaca gggcccagcc ttcatatgcc 600
   tccctgccca agtccttgtc agcctctact ggtcccaaga ccatgggcct gcatgctcat 660
   taggccatgg gcagcccgcc ttctctctgc ttgcaaacct gaggaagcat ttattccagc 720
   atgtgagctg tatttcaagg caaccaaata gccctgattg atatggaaaa tttctttccg 780
   gaggactcta gtgaatcaac agcaaaagaa gatgctgaga ggaaattggt gagaggtgaa 840
   acataaaata acagcactga gctggagaca atggaattta gagattgtgg ccaggaaagg 900
   tttgggggtt aggattttaa atgtacagat gtggtggaag cttcggtgac ttcgctctct 960
   gcttctctcc tcttcctgag ctgcttaggg acccctgctg acccggtatg atggcactat 1020
   aactgatttc cctgttaccc accagcatga agggcatgaa gacagggaac ttcattagct 1080
   gtccccaaac acaggaaatt aaaattaatc agggtaaact taagaaggga ctccttgtgg 1140
   gaaccaagga acagaccctg aaaatataga aaatatagct atcttctccc taaaggccag 1200
   ctcctgctgt tgctctgcac acgtgtgggc tcaactgtgc caaatcatcc tattgtcccc 1260
   tgagccagag gccctcccgt atccaggaat tcacagcttt gagaggccag ggcctttgct 1320
   atcccagccc gtgctctgtc ttgcagctgt gcctgtcttc agaaatccca aaatgagatt 1380
   taaatgaaag ctgagggtat agcctccatc tgaagaagcg atggctaggc ttgctcccca 1440
   atgtaagagg aggttaggcc gggacagaag caccctttca ctggttttca tccttcccac 1500
   tctagtattt ccatgtgtag cctcacaaga taactggctc atccaggtgg ccacagatgt 1560
   ccagcttggc cccacagcaa ggccttagcc agctccactg ttggtcttcc ttctccttaa 1620
   cctttatgtc ctggtgcccg tcctccatca tcagagcctg aaccatatag gaagctaagc 1680
   aaataacccc caaatctctt gttgccctcc ctacacagca gctggttcac cacctctgat 1740
   gcaatccaca gtctccctca aggctgggcc aatgctcggg agctctggac ttgaggcaag 1800
   gtttataagc aacaactttc cccctatcta cacctatttt tcttgtcatg gtgtttaagg 1860
   acctcctccg tcttctgtag gtcagatttc aaattcttct ccctccagca attacctgca 1920
   ttgatcccca gctcagcctg acctcagcaa gagtatcagt ggaacattca gtgtaatgat 1980
   aggtggtgtc tcaatacatt gctgaacaga aacagtgttc gacctgagtg cagggatccc 2040
   agctaggtta gcagagaagc ccctcacttt gcagagcagc agccagggta gttcatctgg 2100
   tttcccagct gtgttttgaa tagaaaagaa acttccagct gaagatgaag cagggccgtc 2160
   cctgttgcat tcttggagtt ttagaagcat gtctgttctc ggatagctgt gctgccggaa 2220
   tagccatacc atttcccact agaaggcatt ttcgtggtta tccagagatt aatggcgaca 2280
   gatttctata cgttttgtag tttttccctg ctcacataca gaatttttct ctggagttat 2340
   cataattccc cccagtacga cagtcttgag ctacttggag ccattgtgcc aaactcttgt 2400
   tttatctggc tttgctcttc taactgaaag tgcttgggct tcctctgaag gcggccaatg 2460
   tgttgtggtc gccaccatgt gctgtgtctt cgaggtagca tatttgaatt accctgcaag 2520
   actcaggaga ttcagaggtc ttaaggagtg tctaggctag cttcctgtag acttcatagt 2580
   atgacctgtg accatcactt ttatcaaagg gtatatgggc ccaactttca aatgtgttaa 2640
   aggtactgaa aaggggatat ttcaagagta cccacaatga gtcaaagttc catccattta 2700
   acaaccattg gtgtggcttc tgtgttaatt ccatcagtgc gccctctctc tttccatgcc 2760
   agggtactga gaagtcattt tggatgcatt ataaattgag tctccgtagg taagttggct 2820
   ttccagtgca tgcacaaaca aggtgtacta agcttacatc cagttgtcag cctcagatta 2880
   agcccacgat gtcttcactg gtcatatgtc agctgagcgc atgatgcagc ctgttcgctg 2940
   agcactgatg gagtcaccaa gtgccttgct aatcattgct gcccacttac aggcctcgtg 3000
   ctcacatgac tttttactga acattcttca aatggtggtc ttttggagtc ttgtgtttga 3060
   ctaacagtgc ttcatcacgt gtgctagtgc atacaaggaa tgctgtgttt ctggaagtca 3120
   aagcttttta agtggcagtg ttgtcacagg cagctcttaa ttattggtgc taataaaggc 3180
   taagtcctga atgggtaaat ttaatcaatc atgtctatac tcaggaaccc taagagctgc 3240
   tagtaagtag aaatcaaaat tcccctgtaa gaggagcttt ttccacttgc tagtaatccc 3300
   taccccttga ccaagtcgct tatgttctgc tattcagcta aagccagatg aggccacaag 3360
   agacactggg gtgaaaatgg cagctcagtc aacttgggtg agagatggag aaaaagcgag 3420
   tgattcacca accctggaga ccctgaactg tggttgagca gaccatatcc tcatatcccg 3480
   cctcagctca cagaattcac aaatacatga tgcagaggtt cacactgact tttagtttct 3540
   aaggcaggta gagccatgga gccctttaag attcagagca tggcacccaa tacgtgtcca 3600
   cgcctgcgac aatagagtct taaatataat ttgctgcaga tacttcagtt cctgcaccga 3660
   cagacaattg cagtctgtat ccaatgagca cagatttttt agttgcatag gcttaccagg 3720
   tactctttcc tgggagcctc acatgggagg gcaacactat cctttattca ataaacagca 3780
   tcccccacct tttaaatcat ccaaaagttg tgtggtatct gattatgcag tgcggttcaa 3840
   gtcaaatgct ggcttccttg gggttggtga gtgaggtgcc ctcgatggag tccctgctaa 3900
   aaatcagaat actacaaaca cgtacaacat agaaactgat gattccccag agatgccgtt 3960
   cttaagagtt ggagtgttaa aacagtgtac attgctctag taagattaga aagcgagaca 4020
   ttaacagaaa ttaatcaagc ccctgtccag gttctagtcc ttttttgagc aaaggtgcca 4080
   tacaccacgg aatcccagtt cctggcgggg ttaccggggg catggcatcc aacagcccag 4140
   tctgcaatgc accattagac acacagacaa atccatcccc tctactttgg ggcagcactg 4200
   ttctattccc taggcctcct ggtgatccct gaggtgacct ttggccctca gtccctctaa 4260
   ggcacatcaa ggccggaggt gaggcaggtg tgattccagg cactgaggcg gactttgctc 4320
   ttccttgaag ggaaccctgc actgtgctcc cgggaaaccc cctctctgat tccaccactg 4380
   tgatggagaa aactgaggct ctgaaaagga tggctcatct gcggcttgag atagatgcag 4440
   acgtcaataa ggaattcacg gaaacacaaa aggcgggctg ggtggctttt attaattata 4500
   aaaaggtctg tctgcccaag ctagtcagcc ctcgcatgag cctggagatc agcaaagccc 4560
   tagaagccct taccagggcc ccctcactgc catttttgca cctgcgttct tctttgctat 4620
   agtcccacgt agaggagttc tgacctcagt attgagtcct ctctttgggt ggaggtagag 4680
   gcagggtatg tgtgtattgg agcaacggct gaaagactca cagattattt gcccgcttct 4740
   gtcatcactc tccaccccca gatcctaggg tgaactgtca gggatggcat cgaacagttt 4800
   acctgcagct ggcgacattg cgggagccac ttacccgcag gtaggtttca cctttgatga 4860
   gccatctgac ctggagccac caaggctcat ccagaaatgg acttttcgca gttgaggtct 4920
   gcctcacccc taaggcccct agtttccctc tctggtgtct cttagatctc cctgtgtttg 4980
   gatttccatt ttcctcttcc 5000
<210> 6
   <211> 5000
   <212> DNA
   <213> Artificial Sequence
<220>
   <223> Nucleic acid probe
<400> 6
   caggtctggg cttctcatgc acaaagcctc tgtctgccac ccacaaaccc attcggggta        60
   gagttgccct cttgtagtgg aataaaggac aagtgtggcc gtcgctgctc tgatgtgtct       120
   cagtgagtgc accgggcaag acgctgtttg aaggagcttc cgtggaaaca tccataaaac       180
   taaccttgag ggaagaactg acaaaatgac tttgttccag cttctcagaa gtgagaccag       240
   agatgacatt tgcatctgcc ttggagcgtt ttgtagattt ctgagccttt gtcactgatc       300
   ctcctgcctt gcccccaata aatgcctttg aatttaggta gaaagaaagt tggaagacgc       360
   ccgcagggtg tctaagaagg gttaggcata ttttcactat caagtggcaa atgaagtcat       420
   cagctccagg tagttcaagt cccagcccca cctggctttg aggtgaggtt gtgctttgag       480
   ccggtaccat tttggatgtt gtgtggtctg gtaactgcga ctcaggtgga agcatggctt       540
   tctcagctga tgcctacaag tgtagatgtc tcaactgtaa ccagtggatt tccacatcag       600
   gagggaagtc tagaagattc caaagtctgt tctcgctcgc acacttacac taagtgcttt       660
   ttacgaactt ctgaggccac aaacttctgc cctgacaaga ttttctcaaa gttccggatc       720
   caggattcac gagtgttgct tgccctgtgc caggcacttc ctttaccttt tgtatctagt       780
   tattttatcc caagagatca gtctgcactg tgagcttaaa atactctagt gagcggatgc       840
   tttcatgcag cagatagcca ggtggagctc tccctgccaa tcttacaacg ttaattaatc       900
   gtttgtcatt gtggttaatt tttcctcaaa aagaggcccc ctatctctct ggcccaatgc      960
   atatttataa agggcaggga agtcaggcag aggatgctgg tcccagggtc ccggagcact     1020
   aaacaaaaag gagctgaagg tcaccacact ttggccattt gcagggagcc cttccccaca     1080
   caggggcggc atgttccctt gaagcatgag gaatgtgtgt gttccacagt ctagagaagc     1140
   accagggcta tcactgctta tccttcccct tgtggcagag agtgggtggg agggtatttc     1200
   accattcctc ccagttcact gttagcattt atcctggaca aatgcatcag catttttgat     1260
   ggtcagcctc tcaataatcg tagaaatacc ccatagtaca tagctgaagc catcaattac     1320
   cttttgcaaa acacagtagt attgcagaag tcagatgtga agtgaagggt gtcttgtcat     1380
   gatatcacac aattttatta agagccccag gcccaggcaa gagctcgaag agtattagct     1440
   tttgctgttg taaaccccat ggggtggtct gcaaactgct tcccatactc gctccgtcat     1500
   tggattagcg tgtcttgtct attccgggag aaagttgtcc tacattctaa aaatagagct     1560
   gggagtctgg acatgggcat gcacagtcag gggaggcttt ccaaaagagg ccgtgctcca     1620
   ctccagggga tgctccgtga cctctgaagt gaggacaccc tctcaaatca gaggcacggg     1680
   cctctctcct ccatagaggg ctcattgact ctcctctctg ttttcctctt gatatatcct     1740
   ccccacttct ctgctgcaga agaggcgagg tgagtaccat tcacctctcc caagtggtcc     1800
   cagcccctct ggccagcatc aagcccttat tgagagctcc aactgtgcac ccagcgtcag     1860
   aaatgatttg gggtgcctgt gtccggagca tacagtcctg cctcctctga actactctaa      1920
   gtgaagccaa tcaggattac tttcatttca tcacttcaag gacttcctcc tcggagtctt     1980
   ttcatccttc tcctgccttg tgacattttt cttccctcat taagaaagat gcccctggtg     2040
   agatacctgt gctgggctca gagcagaaaa taaatatcgc tggcatccac tttcctatac     2100
   gccctaacac ttccctttgg ggacttttcc tttccattgc ggttttgctt ttcccccttc    2160
   acacgtctct tatcccagct gtagttggct gtcttgtttg ggtcacagtt actgtagccc     2220
   agggggattc tgtgagagac agatgggcta atttggctcg ttcgtcattt ttaatgccct     2280
   aatttcctgt tttacacgca gccgttcagg agctggctgg ccgactgtcc cagtacctcc     2340
   ttggtcacct tcatacggag ctttggtcct tcaactttgg aaagggtttt gattaccgaa     2400
   ctgtgactaa tcagcgctca cccataagaa atggtgtgca tctgcaaatg tcgtggtgtc     2460
   ccttgcaatg ccctatttca ggtctgcagt gggtgaggga aagcctgggt gcaaggcccc     2520
   tctgtcacct ctggagtact tgcctccatc aaagaaggga gcagctgcct cctgccggtg     2580
   ttagccaggc ataggctctc tcatcagcac ttatggaaat agcacaccaa gttgtcaact     2640
   gcccgtaaac cctttgccat ccctccggtg gcagcatgtt ccccgctggt ggggagaaag     2700
   agaaactggt cccgtctgcc gtctttccag atagttcctt ctggagcaga agtagtccaa     2760
   attccaaacc tctgattact gacatttgcg gttagttttt ctgaataatg caactttaca 2820
   cagacactct caccactggg cctcaccaag tcctgatttt caaaacactc agagcccttc 2880
   ctgagctcac ctgagagatg ttttaaacct gctcactggc tggctctctt ctcgacttct 2940
   tgggacctga gttgtccttc atctctgtga cctacctgac aggtgtttct ggggtccagc 3000
   ccagctcctg agcctgtcaa ccctggctat gcacagccct cgcctgagaa gcgtcttcaa 3060
   aattctagtg ccttctcctc ccttcaattc caggataatg gctgtgtgtc aaattcacca 3120
   agatttagct ccagacccag ctgatctgca gccaggatgc tattgggagc atcactgctt 3180
   ccgtctttga cccatctttc ggaggatatc aatgaacatg aacacatgcg agagttcctg 3240
   tacggagctg tggaccataa accacgcatg ctgtcccggt ggcaagtcag ggggctttct 3300
   tgtgaataga gctgaccaca gtagcacagg tctcaccgta ggcctccagg aacaccctgc 3360
   aacctcagtc tgcccctcca cattcatttc caggaaactg tccctgaaac atgcctgcaa 3420
   gaaccacccc ttggctcagc agctttgcag tgatgaatcc aacctctgca gaaaaggcac 3480
   cctggtgttc taattcctgg cttttctggc ccttaccctg ttctgctgtc cactcctgat 3540
   cccccatttc ctccctccag acttctttcc aggatgctgc attcctacca cgtgtgctgg 3600
   agatacagag ttaagcacaa gatcttagcg gaagatgaaa acctcagcca aatataagtg 3660
   ggggactctg cagtgaggca agtggacacc tcagaggaca ggtagtgctc ttggccttgg 3720
   tgcatatctg tgacccaggt cccctatcca accatgagcc tccaacgcag gcaagaagcc 3780
   ggcctcttcc ttccagcctc ctggcaccat caggaagggt tgcttggcag gccacctccg 3840
   gactttgatg cagaatgtcc ccaggttcta taagcctcca ccctcgagtt cccaaaggct 3900
   tgcaagccag ctttttttta gccttgtagg gtccagaccc gatgggtgaa ccatagtctg 3960
   tctctttgca agcagtcagg cctggacctg tgctctcatt attttgtgtg gctgcagaat 4020
   ttgtgcacag ccctggtggc atcatagtta ctcctgccac ccacctggca ggcctccatt 4080
   gcttctaaat gcggtccagg gaaaccagaa tctatttcaa gtcaagcaga acttctccgg 4140
   aagctggggt agcatcccat gtccccacaa tttgctgcag ggggagctgg ggcaccccaa 4200
   aggccccccg gcccacgtgc tcaaccactc tgcttcactc tagaaagaag agaaccattt 4260
   agatgactga ctgaattttc ctggaaatct ggccctccat tgtaagctgt tgaggcacca 4320
   tcaaatccca catcacaaag agatgcaccc gttttaaatg aacttagaga agatttggcg 4380
   aatccatact ccagtctact caatgaactg gtgatgggga gtaagtgcta tttgaatcca 4440
   atctccaccc ttgttattaa caacgaaagc tcaccagcgt gcccccaccc cccaccatcc 4500
   accaggtcaa taatatatgc aaccggcaaa agaaaacatc gacctcattt cctccagaga 4560
   gcagacaact gtaaccctgg agtcctggtt tgcatgtaaa ccggggccca ttaaatcctg 4620
   accaaagtgc tgaagagttg ccccacaaat gatttggcta cactgggaat cacccggaag 4680
   gaggccaagg gtgtgtaatt gcagaggctg attcctagct attattctct gaccttccat      4740
   gtcgcagaaa ttatgatggt aatcagaact ggatcatgtt tcctctctag tttggaggag      4800
   cctcgagagc cactctgcac ctgctgtctg cactagctac agaagggtcc agatgagtgt      4860
   ctgcacaggt aggtcttgcc gctgaagact aagccttgca tctggatctt tggaacaggt      4920
   ctgccctgaa gtggtgtttt atgggcctgg gcactctggc acaagccctt tctgggcaca      4980
   aggcgaagtt gatgagactc                                                  5000
<210> 7
   <211> 5000
   <212> DNA
   <213> Artificial Sequence
<220>
   <223> Nucleic acid probe
<400> 7
   gactcttctc ccaactcctc tgtgcatggc ctgaacttgc ccttgcaacc tggcatggat      60
   caagctggct tcctagtttc tgccccccag ctccattcac ccagggagca ataggcagct     120
   gtcacccaca aacgacatgg ctcccacaat ctacttccca gatcatagtc ctgcctagct    180
   gggactggag gtaggataca cccttcaagg aagccacgtt tctaagaggg ctctggcata    240
   tgcattggtg agttgaagct tgacagtgtt atagcttctc tgctgttagt gtgaggtttg    300
   ctttcacaaa cacctgggtg cttgcggact caccttttac aatggtttta ccagcctcct     360
   ccacaagcac aggcaaagtc agtttcaagg acaggagccc tcatttttta gccacctcgc    420
   tttcatccct ggtcctcatg accccttgat ttctttctga ggaattccct ccctcccact    480
   gtgagaacag aggggccagg ggttgggaag ttttgtggct ccctaccaag ggatgacgtg    540
   cctgtctggc aggagctagg ctcatgcatg gctcgggaga gcacctgctt gtactgagtc    600
   catttaagtg gcagggactt tgaaatcagt gtgtcagtcc ctgatgcttg tttattttca    660
   aagcaacata ggagaatgga tgtggagggg aagcggggag tcaaggtgac acccagaact    720
   catttaggga catttgcaga ctcccaagtt ccttcaccag caaagtagga aacctctcag    780
   gaatcctcag gctcttcctc aatctcctga tggatgctgc cataggaagc acaagagggc    840
   tgaaaccaat acaggagtct gtgctaatcc ctcaggactc aaaaatgccc caatttttgg    900
   tctgatacac atggggctac ctgctttgca cttcctggcc aaggggatag actgattcct    960
   tatgggagca cttttgaaga atgaggaaga accttctcag accttcccag aagcctccag   1020
   gaagtctctg tttggacgac atcgcacacc cattcttgaa ccaactattt agaagggaga   1080
   tgcatcaccc cctttgcctg tgtggaacga acacgaccca tctttactgc tagcgactgc   1140
   agcagataag gcaaggccac tttcacgcag ttaggttaat aggaggctgt acaaagggct   1200
   ggttttgata gaacttaaaa aaccctcgtt tgcttggaaa ccacctgttg ggatgaactg     1260
   cgggaatctt tctttgttct ggttggttta aattattttg attctttcag agaatgtgac     1320
   acagaccctc agaggatggt gtttctattg cctttcctct gctgcagtgg gttaatttca     1380
   cctccttcag tgtataatac ttttattctg atgttagtgt gactgaaatg caaaggatac     1440
   tttttggggt gctctgaaca gctttctggt atcgtgttga gttacattgg aagagagagt     1500
   gttaatgtta attcggcaca caaaataacc acgtatatct gtcatgggtg tgtaaaatta     1560
   tccccagttc aggaaaaagc gcgtcagtat ttaatctatc ttttgtgaag caatttattt     1620
   aggctttaaa gctggcccag ttgtgggagt acagtgagtt ctagtaaagt agaagcgtag     1680
   tgacatcctg agtgtagaag tggaatgtta ctttaattgc aaaacacttt ctgagcctga     1740
   aaaatggctc ttggctggtt tgaatgccgg ttaggcaggt ttatttcttg gttacttcag     1800
   aaatggaagc ttaacctgtc tagactgtga cctataccat aaaatgtgtg gtctaggact     1860
   gttgtggagt gtcttgggct gttcagggag gtttccttgt atttcttgtg ataaactaaa     1920
   ctgcacatag tttaaagaac tgtcagccac gtaggccata taacagctat tctttccaag     1980
   cctagaatcc atgccataga taagaaaggc acagttgtaa gaatatgttt gcttgtatag     2040
   ccgaaggcac attccgtttt gtaggtaggt gagttttcag aactcattac ttgtaagttt     2100
   aaccattaca gaatgcctgc tttaagaacc tatttctggt gtcttcaggg tttttttctc     2160
   ctagcctctg acctcaagaa tttaaaaaac ttacaggagt gttttcttgt acatttatgt     2220
   tgtttcctgt accagctgct ttagatggtt ggttagcttg tgcagatcct atagtggtct     2280
   ttcttttagg accagaatct tgtccaaagt aaatgaggtt ggtccagtta gcagcaggta     2340
   tctatcctct ccctttattg tggctacaga cggagaaatg ggattcttta cccattcttt     2400
   ggcagggttt ctacgaggaa ggcatgtgaa tggagaagaa atgattgata atctctgata     2460
   aaacagtggg catgcaaggt ggaattcagg cagtatttca ctttcagcca gaacagttag     2520
   agttcctgga tctctagttc aagtcacccc caaaaccctc caaacatgta aacgtcttca     2580
   ttcttagaag tattactgaa ccgcctccat tgaactcttt gcagttcgtt ttgtcacatg     2640
   cagtgttggc atatactcct gttgttattg ctcataggtt aaagacagtt ttgtgttgca     2700
   tacaagagac aatattggta tttagagcag tgaactagag cagcgttttg caaaccagat     2760
   aatgctgctc tctttttctc atggcctagt cttaagcatt tatatccatg ctgtgcttac     2820
   gaaggtttta ctgagagaac aacaggagct gtcttgatta ttgtctgcag ggttattgca     2880
   aaaatcatgg taactgcccc gacttcatta attttcaata aatgcatcgt tgtagttgct     2940
   tccactggtg gtgtaagtgt gttagcattt ccataatgga ccattcgcct ttcattatac     3000
   tagtgagcag tgacagaaat gtgacattta cttcatggca ggttcatgtg accctcaatt     3060
   tcacttttca gttttgtaaa accctctaaa ctgtagaagc gagatatgaa gcaactacag     3120
   gagttcagag gctggagccg ttcttagggt gtctcacaca ctacttttaa aagcatgttg 3180
   ctttaattcc tacttaaccc taggtcctac acacacaaaa aggaagtctc cgtcctttat 3240
   gttgactggt gagaatgaaa tgaaatagta catgtgatgc ctggcagttc atctttatgt 3300
   aactcttaag cacagagtcc tgtttgagga tgaagatgtg aatattgcaa caaccacagt 3360
   gcttgtctga gaagtgcatc acttggtgat aatcctgagt aaaagattag taatgagtga 3420
   agaaacaggc acctgtgata actgacaagt cttcaaacac atgtgtatgg ttgttgtaga 3480
   gagaacagta gaacagattt tttatgcaaa tgagctgttt cccataggtt ctgtcttgat 3540
   cttaatcctt taagggtaag ggctaacttg tggttgtgtt aatggtcttc atcgctgctg 3600
   agagttgaag gcggaacctg atctataagg atccattatg ttgaagccaa agaagcaggt 3660
   ctagctattc taagtaagta ggctggagag gtgtttctta agagatacta tttggagatc 3720
   actgtttgac taagccctga acatttccac gaatgtagcc tttagagaga gagggttcct 3780
   caagctgtag tttcttccag gaattgtagg tagtttgagt agaaccaaca tttgagaatg 3840
   agctggagaa tggagcatcc gggttggaaa tgtcacctac attggcatgt tttcagaagt 3900
   tgtgaaaagg tgctacctaa aacagcaccc ttatcctttt tcagttgttt gagttctctg 3960
   catcccttta ttagggcagc tcttgagttt tgtttatccg agtcaggaca cggaatagga 4020
   tacaagatac tgctctcagg actcagctcc tatcaaaacc tccctcacac tgccccttgg 4080
   tggtcacacc ctacggctgc aatgacttga aattctagta tctaggcacc atgtcttaga 4140
   atttttggct gtgacaacag gcatttggcc acatttctag tcattagtcc agtgattaat 4200
   atcgctgtta tcagtatctt actcattatt ccagggatat tctgtgcatt tacagccata 4260
   gacttatgca ttcctacccc accctataca cctccactca gcttaccatt ctgctgtatt 4320
   ttatgttatt gtgagcacca ggttcgtttg ccttgcggtt gcccagcttt cttttgtctt 4380
   gtcttaaatt ttctgtatag ctgactgagt ttactgaagc aaaaacattc cataacctta 4440
   accttttccc tgaccccctg cttttaacaa ctgccgacat agttttatgc tgttatgtga 4500
   tttgctgccg tgagaaaagg tctaattgtt gcagaaattt aatgatctag agcgacatca 4560
   gggctgtgag attttatgaa ctgtttacac cgtaattttc agtgattgtg ttttatttaa 4620
   aatagcattg catagcatga gagccgtatc ggagaacatg tgccactctc gagaagccct 4680
   tcatatgtgg tgatatttca cattcatcat tggtagagaa agaactatgt acttttctcc 4740
   tccatcctct tctgatctgc ttactgattg agtgcctgtt gtaaaacagt aggaaatcac 4800
   ttgtaagctg tttttggaag aatgaatgaa tgtgggtgta tttcagccat tcctcttatg 4860
   gtgcctgcag tggctttccc tgcgcaggtt gttcccatct ttgtttgggg tctggaaggg 4920
   gatcagattt gagtggcaac tactgtaact tcacctgtcc ttcattcaga gagttttctt 4980
   aagaatcctg gagttaatta                                              5000
<210> 8
   <211> 5000
   <212> DNA
   <213> Artificial Sequence
<220>
   <223> Nucleic acid probe
<400> 8
   cactgggaat tcgtctaatt cgtctttctc agggaaattt aaggcctaga agacatagat      60
   ctggttggta acaaaacagg aactctttcg cctctgagtg tattgtggaa agtatgctct     120
   aagtgctgac tagggataga aaaataacat gttaacccca tgtgaaccct gaacaggctt     180
   ggtttttatt tttttactcc catttttgtt tccagtgggc taggtcccta cgtttctacc     240
   acagccgtaa catgcttggc atgcagttta ccttttcaga gtagaaatta tactgttgca     300
   tttcatgttc acagacttga ttttgagcct gtctatgtta catctttcag ataggttttc     360
   cctcatagaa caagatggta gtagaagcag ataaagtgtt aataccagaa gtgttccagg     420
   agctgtacac tgagagttat aacttgggag tcatagtatc ttctttgagg taagaaaact     480
   ggcaactgct tcacgtcaga agatattttc ttgggctcca agattttctt ggtaatgtct     540
   atttgggatc ttggaagcta aactgagggt aagaatggac tgtcagtgat gatttgttgg     600
   atgttgtggc ttagggcaga cttggctatt ctaagataca gagcggtgtc taggctagat     660
   ccacctgtag tcacaaagac gtaagtttaa tttttttaca tgcaggtttt ccacacggcc     720
   aggactcaga cctgtgtgtg tcttctgtgc ggttgttgca cactccaaat ctggtctgtg     780
   tgggttcctt aataagaaag gctctctcaa ctttgaaatg tactgaccag gccacactag     840
   taagttgtgg agcctgaata tatggcagct gatgaataca atgatgatgg tagacatagc     900
   cggaaaccat cagtcagtcg acacctttct ggtaacctgg gcgtggttca ttacccaggt     960
   ggcattctct tgctttggaa atttaaaaaa tggcagattt gctggtggtt tttttctctg    1020
   cataccccgc tgtttgcttg taactaattt tgcatgccag taggtagcag tggtgagcag    1080
   aggcacagac agaaagctgc ttctgaggtt tgttgccccc ctgctgctcc aggtttgagg    1140
   ttagttacag catcataaga cgctctcaag catagagaac tcaggctggg cttaaaaagc    1200
   cgtggagatg tgaaatgttt atccatttac aaggcagaag tcacaaaccc agacacaagg    1260
   catccgatct cgtcctctgc tagatgatta cagacatgtt ccagcgctag gcatgaccga    1320
   tgagttcctt gtctgtgaac catgactggg ctcttggact tctggagctg cttatacttc    1380
   ctgcaggtgc ctgctgtgta gccactgcta acaactctat gcctttagcc ttccctgggt    1440
   ttctggagga aagctgcctt ctgccctgga cccagctcca tcctgctccg gaagtggccc    1500
   tccagtttct tgaaaacatc tcaaaggtac taaacataag tgctcagtca ccctgttaga    1560
   tgtgggctta tggagtgatt cataaaaatg aaatatggac taaagacatg caatgaagtc     1620
   gttatttact tatggacaac tacccttgca aaacaaagtc aggaaaagtg ggtgatgttc     1680
   aaatgaggcc atttgaactg aaagactacg ttcaatacat agaagatgat acatggccag 1740
   ccatgtcatc tgcctgtgat tccagaactc atctatctgg gcccttttca caatgacatt 1800
   gcgattacat ttttgtttaa atgtggagca catttcaatg acggtagagt tgccggccgt 1860
   tatttacgca tctgctaagg gaaaggagac tatttgtcag tgaaatgtca acagcaaacc 1920
   tcatcagcag gcctcactgc cgtgcaatca agacagcgcc atagctccat ataaacaaat 1980
   gtatataatg tgcctgtgtt aaacagtccc ttcgacagcc tttaaatgtt aaggaatcag 2040
   tgctgtgtat attacccaga gagtctcagg gagcgtggac tcaaatgtga ctggcagctg 2100
   tttggggagt ttcatgtttt ggagccatga accagccatt gcactgaact tgaaagtttt 2160
   tcctcagaac cctgatctgg gtgttcatcc acagccatct acccagaaga cctgcaagtg 2220
   aatggaggct cggatccaaa agctaccccg agaccccagg gtcttcactc ctttatggca 2280
   gtgtgagtgg ggcagacagg atggagaggc tcagaactga ggcccctgtg tttgtgctgg 2340
   ccacagctct cctttcctcc cagctcattg caacctcaga aggcctgtcg agtgcctgtc 2400
   cccatctgag gaaactgtcc tttcaatatc tgcaaaaggg ccatccttgt ttgtatcaat     2460
   tgctcctctc cataccctag ccatggtatc gtttctttta ttggacctga gtacatagaa     2520
   tggctttatc acaatacttg gttcacatac agtcttcctt gctctatgca tggcccatac 2580
   acagtataat tatttaaatg gccaggtagc tcttctagct gcttctgttt attctccttt 2640
   tctcttagca gagtattgga atgaggctgt tgagagctgg gctgaaggaa tgttttggga 2700
   acagaatgct catgacaaat gaacaaccac agaggcaaag ccaggctaaa aatcgtcaca 2760
   aaacagaaca gatgttccca ccctctgaag acagagttgt atgctaagga gaggagccgt 2820
   cattgcaatg tgaatagagg caactgggaa ttgcattaaa gggacttcaa ggatctccca 2880
   gcttcattct gcggacagcc cctgcctcag tcccaatcta tgtaacattc atctctaacc 2940
   ctttcttggc ctcgtggtag ctttcctcca atactccaat tggggccttt gccatgatga 3000
   acagtgaaaa caaccttcca caagctcccg gaaactccct gatgcctttc tctgaggtaa 3060
   aggtctttag atggtaccta gttttgtgtt agaagctttc catccagaga ggtgatgaca 3120
   gtcgtctcca tgaccattga ttaatgaaaa cagggctagt tggacccaga gtagatggtt 3180
   ataaatggtg ctgtcagaga attcattatg gtgattgaat cctcttcaat agagaacggt 3240
   ttaaagagag cacaggcccc gtgcgtctca ctcagagtta aggcgcatag gttaaaagct 3300
   gttggaaaga aaagctcatt tttataggtg gtgccacctc cacaatttat actttgccca 3360
   tgaaatacca aaaccaggga gtcaattttc caaggaagaa aacactttta ttgatggaag 3420
   agatctgcag agacggtcgc catctgcaag gaagcgaagg ctcttgcagg tgcgggttga 3480
   tccagggctg cccaagcgtc ggaagtgaat atttgaaacc tacctggtct tcccaaggtg 3540
   gcccagttat tactgtgtga cgacttcttc ctggcttccc tggtgaaaag ctgttccgtc 3600
   gttaaaaaca taatcaccca taaaagcctg ttgctcccca gagtggtgag gtatctaagg 3660
   gatgccccgg ggtgctctta gtagaaagcg gggggtctgc acaccatccc tgcactgggg 3720
   ttgcctcttg cttacagccc ccatccaaat cctaccctct atggaggccc aacagaacca 3780
   ccctcctcca ggcagccttc acatgggcac aacggacaat ctcatcagga gagaggggac 3840
   ttgcagtggc acctgcatgg aaatcaaaag agcaccagct gtgtgctgca gaggaggggg 3900
   gccgtgggca gcttctcctc actcacattc cacgagggcg tctgtgtcca cagaagctcc 3960
   tgcaaaacca atcaggagct gtgacatccc tcaagcttgc taccacgggg cagtgtagga 4020
   caaaccacgc ctctgtagcg aagtgcatgg ccgtccccag aaaagcatgg ggtagcagga 4080
   ggtatgtgga gggaggaggg tccttctagg gtcttctagc ctcatgcagc caatcccagt 4140
   gaagccataa gcatcacata ccctgtcccc aagccaggct gagcctgggg aatctggatg 4200
   tgcaggagct ggtgagccca ggacaaggaa gaaactgagg tgaggctatg cagccatgct 4260
   ggctaatgcc tgcagtgatc aggataggct tcctggtgga tcccattttc aaccttcccc 4320
   aggagagtct cttgcacaac ctggaaagtg attgggctca gaccttcacc tggaacccag 4380
   gtgcagccat ggggctgagg gccttgtcac cacaacccct gcactctgcc agagagtcta 4440
   ttcctggaaa tgaaatttca aatctgcact cttaggcttc ccatttctcg agcacccctg 4500
   cccagcttgg cctcagagct gcaggaaaag cctgcccggg ccttgtttat ttttggagag 4560
   ttttaactgg ttgggcgagg aagtgagcct tgttaaaagg gcggccctgt tggtagcttc 4620
   tccatcacgc cgcacaagcg gctgagtccc aagatgacct tttaccagag cgggtagagt 4680
   tcacagaatc ggatcccatc agatggtggg tatttccagg ccacaaagtc ctctgacctt 4740
   tgaacagttg ccgccgaatt tcaataatga aagggccttt tttgaatatg tacaaatgag 4800
   tccatacatt ttatttccag cctcatctgc gaatctaata ttgacccgga gttatctgtg 4860
   atggggaagt tattaattct gacagatata agggagaagc tgtatgtggg gcacacccaa 4920
   acaacctgcc tcagcacgtc ttgatggaat attattttta tttcctgcag accgtgggtg 4980
   ctaggagttg ggtgagatgg 5000
<210> 9
   <211> 5000
   <212> DNA
   <213> Artificial Sequence
<220>
   <223> Nucleic acid probe
<400> 9
   cactcctagc ctctggcatt cctggaaaac tgggctctca gcaggaactt ccaacaggtt      60
   ctaccccaga gagctggccc cgactccacc tggatccaag ttcgtgagcc aaggctgagt     120
   ttattgtcag aaagatgact aagaaatccc cgacacagtg tatcatcagc attatgattg     180
   gagaaacaac acctacaaaa gtcccaatgc ccacctgtgg taacaccttc ctttctctca     240
   acctggcctt tctcacccac aaggtgggaa tggtgatttc cacctctcgg gagaagagag     300
   cgtctctggg aagggctcac caaggtaagg tggtcaggac ccagtgagcc tttcccagag     360
   agggttcaaa agaatgtctg gggccaagtt caggtctggg catgaaaaca gagaggtact     420
   tgcatggcga ctcttcccct tgtacccaga agaacacgac tgctggatag ctctgtgcca     480
   gtcccttcct gtcctttgga gctcttctaa ggtctccgga aaccttcata gactctgcag     540
   aatgaagcac cacagcttgg tccagggcat ctgcagattg agacggccct ctcttcctcc     600
   cccttgtccg gtgtgacctg gcaggtgagt ccctgccaag gccttagggt gatggggtac     660
   tgtagaaaga tgggtttgtg ctcccctgtt aatgctcctg aaaaatggtg atttagaggc     720
   cgcccccact cgacgggctt tcttagccga agacgttcag ctaatgaacc tcaagcagat     780
   tcatggccgg gtgtgaagtc aaaaacttca ggatcgcctg accctgtaaa ctgaaaattc     840
   atgtctctcg cccggggaca aatgcattct ttgtaaaggc ggcccgtggc cagccttggg     900
   aggtgtctgg accatggcga ggtttgcgtt tgccgttatc tgggttgcat tggcgaagct     960
   gccagtcagg gtctgcctgc ctgtcgttaa actttccaca ttttgtttct aggaaatgga    1020
   tcaattcttg ccctcgtgtt cactgaatga agtaacttac agccccagct ttgcagccag    1080
   acagagccct gggcaagtta ctcagctgta acgtgggttg cagacacccg tgttgccaat    1140
   gttattacca tgattgttgt tttcttccac tggcagatgc acaggaaatg gctctctctg    1200
   cagtcataaa acagcataac gatgagatgc ttttcccccc acagccctgt gatgccactt    1260
   tattgcttag gaagtgatgg tacatgaaat gttcagtggc ttgcccatgg cctcaagaga    1320
   gcaaagctgg ccttcccacc tcaccacatg acactggggt aagatggaca gcgggatctg    1380
   ttgcctgtgc tcctgtgagc gttccttggt ttgtcaaaca aactctccgg cttgaactaa    1440
   gtgcctttct ttcagatttc agaatggatg ggcctcagcc atggcaaatc tgagagtctt    1500
   ccgggtgatg gcacctgttt tcatgaatga cacagcaatc tccacttcag taagggccga    1560
   ccatactgtt gattcctgcc ctgagaccct tctcccctgc agtgccaacc agcccccagg    1620
   gaaccgtact gccttggcag catgtcactc actctctccc agggctgcat cgcttcacac    1680
   ttccagtcct ctgtatgtct tccaacaggc tgtcgtctgg cttcctgggc cctgcatcca    1740
   gccctgaatt agccacgtgt catgctacac tcaggcctag ccataatttg gaaggggaca    1800
   cccatgatgt gcagggctaa tgttaaaagg taaaattgtg tgcgggttcc caggcaatca    1860
   taattagagt aattgtcctg tgttgcaggc acccctcttt cctcctccca gctcctgcag 1920
   acctttgaag gtactgacga gcctaaataa accttcctgt agataacagg tggtggtggc 1980
   caagagaact aaccaaacca aattgtttaa gtagctctct tgctggtgtg gttttatggc 2040
   tagcctctgc tgaactatag acaccccaac ccacggggca gatgggcagt gggaccccca 2100
   gagcaggagt gtccttagca aatgttgtac ccaaccagga gaagggacgg gccaagccct 2160
   gtaaggtgca tcaacattta cacactcatc acaacacagg gtaggagagg gttaaaccca 2220
   aatggcacgg cccttccggc tggtccctgg ttactaagct gtgcctggat tacagcacct 2280
   ggggccatgg ggcccccacc tgcggccagc atgattacat cgtccgtccc tcgcagcctc 2340
   ccaaagacca cggtccacct tgactgcgtc tgctgccaag gatgaggcct tttgagcatc 2400
   ggaggtctca ggctcctggt gaagccccct gagcttcagg aaggtaagtg cagccatttg 2460
   aacagccctc attggccgcc ttcccattgt ggagaattag catcaaaata tattctctag 2520
   ctgtagggaa cgtctcagaa ttgttaacct tctccccagg tattagaggt aacacactgt 2580
   gagattagat gtatattttc ctcactccca tggcctccct aactcccgtc ctggctgagc 2640
   ccattgtggt ccacacaatc ctgtcctctg tggctcctga gagtcttgga ccctcaattc 2700
   tcagcttgcc tgggcctctc tgcctgctaa gaagcttcca ccagtggcca gtgcccctct 2760
   cccaccctgc agtttctggt atttggaggg caagtgcttg tgttgagtgg aacaaatgtt 2820
   ctggctataa ccaagctgca aagctgttgg aaaagcacag gtcaggaatg gacacggctt 2880
   ccagggaagc cagggctcag gaagaggcag tcgccttcct ttacttcctc cagatcagtc 2940
   ttcgagctcc ggcacatggg gagatcacgt tggctgttaa tggggtgtct cttttgtgtt 3000
   tcaggctctg tctcctgggt caggctgggt ccttggcaat ctctgcctgt ccccttcagt 3060
   aggtttgaaa aaccaagcct ggagggatgc ggtggtgata gcagcatacc aacactgttt 3120
   ttgttctttt caacacctca ttttgacgca agagcttctc acacaccttg gaaggggctt 3180
   cagcccagat gaggggtttt gacgccgcag ctgggctctg aaatggctta gaattttagg 3240
   ttttcagcta aatggtgacc agggcggtca gtgtgtggcc aaactcattg gaggagaagc 3300
   cagtgagcat gcatctctgg tctgtgcctc tgtgggttgg ctgcagtagc tctgctccat 3360
   gcacctcctt ctggggccag ttacatgggc ttgatcttct gtgccagcaa ctcccaaata 3420
   tgtttctagg ccaggctgtg accttcagac tcactccaca cctcccttga cttctacact 3480
   tgactctcct gaaggctaca actcagtgca tctggtcttc ccttgaagac tcagtttccc 3540
   tgtttgtcat gtgagcaggt tgattagatg agctactagt tctgagagca agtgtgggac 3600
   cgtcactcta gactttcttt tggctttatg cccccttggg atttacctga tggttgagaa 3660
   catgaacatt tagccaatga cccatatggg gagatggaag gagttcagag agacaggact 3720
   gaacttggag gctctaagct cagaaagtag gatctgtggg gccacacttt gggcaatgtc 3780
   tggccctgca gttccaccgc cctctgtcct gaaacaggct tcccggagcc ctgcgcaccc 3840
   caaacaaaag ctgcctctga ctcctcagcc tttgtgcaca tctgcattat tagcaatgct 3900
   agttcacaaa ggcccagaat cccagcatgt tttacagggg ggaacactga gtataagatg 3960
   ggtagtggtt agagagtcaa ggtcaccccc aggccagctc acttctttga aaaaacattt 4020
   tcagcacagc tgaagaaggg aggttctaaa ttgagaatct gatgaggtac ccctggcccc 4080
   agtttaaaat agtcaatgat ggtgaatccc tctcaccttc tgtttctccc aggtccacct 4140
   catccttcct gaccaagttc ttcacctctc ctgaggagcc ttccctccct accccagggc 4200
   tcctcgaaac tctgactctg gagtaaggtt tggtggacag agctctgttg cctcctcagg 4260
   cccattgtga gttaggatct gttttcttgc ctgccatctt cagctgccga aagcacctgc 4320
   ctggggcctg gaccctcgcc aaatgtcaac atgatgttgg ggaccaacag gaacactgga 4380
   agcacagtgt tgaggagact tggctcttag caacatgttt cttgcattct gctcattctg 4440
   tttcacattt cacacttccc caggctgagc gagccatcca tctagtgcaa acgcttctga 4500
   gctggcgatt gctctgaaca gaccttcctc ctcttttcat tccagtctct ctgatcacct 4560
   ggggagatac agaccttgca ccttctccct cacagcccag gagggcactt atttggacct 4620
   gcactggggc tctaagcgct tggaggaagc catgacttgt gtcttccatt ttgcaatagt 4680
   tctcattagc gcttggtgcc tggagagacc aagctcctgc cctctagtgt gctgaagacg 4740
   agggcttcca ggaacccaaa tttgtgtatg gggcagtata ggctcagaaa acagctttga 4800
   acttcccctt tataggggca tcctctcaga gctgagagac cagaactgca gacagcctgg 4860
   gcatcccctg gttcttaagc tccccctata atttccccac actagcatcc tcttttgcag 4920
   aaggttctag cagatgttgt ggttgcaaca attggcatca gtgacttgga gctgaaacct 4980
   ggcttgtggg gtgctagggg 5000
<210> 10
   <211> 5000
   <212> DNA
   <213> Artificial Sequence
<220>
   <223> Nucleic acid probe
<400> 10
   acaatgggaa atgcaagttg ctgggtcccc agagctcaga taatgagcat gagaactgtg        60
   ggatgcatta agagggacct cctaaaaata agaacctgaa ttattctccc atgccaggaa       120
   taaattgcac taaaattttc acacagtgcc ccctccactt tcactgcctt ggctctcggg       180
   ggagctgttt tctcaggctc aggagctctt ctgagaagcc cctcctggcg agacgttgtt       240
   gtgagcctgc taatgtcttc cacttaagca gcagccaccg aggtgtgccg cttcctggtg       300
   ggagaagggt tctaggtagc cttggtgtga tcttcaaagt gttaggtaga gagtgctcag 360
   tttcccactc tggtgaaaga caaggaggca cagaagggct cttgcggcaa gttctctttg 420
   gaagaaaagc caacctccag tttggtgaaa gtgtcagcgt tttggggtgc agagcatgtg 480
   accctaatgt aagccagcac ctggccaaaa ttcaaatccc cactttcctg atgcagaacg 540
   gactgccaga gctggatttt caacacaggg ccgccaagac tggggccagc tcccgtccca 600
   gatgcagaga aatgtcactg ctgctacaca aaagtgcggc attatcagcc ccttgcaaag 660
   gcaagtggat taaaatgcca ttaacacagc aaattaagct gaatgacacc cagtcgcaga 720
   tgcttttctt gaggctctga ttataggttc tgacctgcgc ttggggcact tttagggggc 780
   catggcttcc ctgtggggat ataggcaagg ggttcgcttg ctaaaatacc cttctatttc 840
   cctagcttgg ctccaactgt ctggtctgtg gccaccaggg tctctggagg cctgacaccc 900
   aacgggtcca actctgaaga gttctgcatt cctctgttga tttttcccct ctccctccct 960
   caggttccaa atccaattct agctcctggc caactggtag caccgagagg ctcatagttc 1020
   agcatccaaa gccgaggaaa tcgctctccg cctgccctgg tattgttttg caagcctcgc 1080
   tggaaccacc ttttgagctc gtgcaagctc agatgccaga atcccctggc acctctcatc 1140
   ggttcatcca cctacaactg acaaatggag ctcccttccc cactgcacaa cagggaagga 1200
   tggctgcaac cctcttctgc cctccggaca tgtggctcca ccccacccat ctctctggat 1260
   gcctgagctc ctgagtctgg gctctgacag ccactttcca ctggaggcct ggccctcttg 1320
   ctgcctggtt aggaccctct ttccaaggca aacacagtcc ccaccctctg atctagatga 1380
   ctgaccatca gccaagggca ctggggacct agaatgaaaa ttttaacaac tcagaagctg 1440
   tgcactgaag gcgcccctgc tgcaggttct cagaagggta ttccaactaa attgcagaaa 1500
   catcttcctc gcttttgatg agctatcact gcgcagagcc cgaatcccca agtggcaagg 1560
   tgtttttcca ctcaatggga gaacagccat cttcggttgg atgaagatgc ccggcagaaa 1620
   aaatagggtt ttacaggata agtcattaca gtgctatcca tccacagggc ctacacacac 1680
   cagctaactc ttaagcttgg gtaaaacctt cagcatcaga gccaggcaca atgtggagga 1740
   gacccaatcc agggctctat gggatgtata cctttccctc tccaccaagg ctgtgaagtc 1800
   accgtcccct catggagctg catttctccg gccttgggcc tgagcctggt ttcacagcct 1860
   tcttcagata cctgcggaga cacttccaag acagcaccgt acgattttct gatacctgtg 1920
   tgctgccagg catgatgttt gaggcataaa cccagacctt tgccgaggac agggagactc 1980
   ggcttgagca agacaggagt cttcttgaag ggtctttctt tgtctactta tcagaaatat 2040
   gtagccttcc tactaccagc gtttttgata cacatgtgat acaggcataa taagatggct 2100
   agctgggaca gcccttccat cgatatctgt ggaatgagaa cttggtttgc tctagagaaa 2160
   ggggcttccc aacatcgcct gaacatacac agcaagctct gagccaggga ctgaccacgg 2220
   gcttggaggt catgttctat cttccctgtt gcttcccctc ctccttgtac caacagggcc 2280
   ggctaattag gcaaggcctg ttgcactggg ttctatcact caaaagggca ctctggatgg 2340
   gcctattcag aggccattgt ctggccagca aagctgagca tgtggctgga gggcattagc 2400
   agtgacaagt ccaggtcaag ctgagaattc ccaggacatg gctctgtcta agcattgggc 2460
   cttccccttt ctggacccag tcatgagagg cctacgaagt aaggcctggg agcgtccaga 2520
   gctgggtctc ggccatcccg cagttgtagt atcaggaggg gaagataaat taagatgagt 2580
   gcattgcaca gttagacatt ttctgttaat gacaattttg ccccatctgt tccaacatga 2640
   aaatgaggct ccttgcagca gacaagatag atctctaaga gtttaagaag gagaaatctt 2700
   ttgaatcgtc atattacagt aagtcatttc cgtaaggcct ggtgagcttc tgtctgctga 2760
   ggggccaaaa ctcagtctgg gttttcagct ctggaagaaa attgcctctc acacaggctg 2820
   acattaagtt gagttactgt tctgccgtag acttctccag gaagtcaaaa agacagttga 2880
   gagaatcagg ctgaaaatcc tcatttagtg tgcaaatgcc tcctctgcag ctgtgcagag 2940
   ctgaggaacc accatttcct ccaatagccc agctgaggac gaagccgcgg aggggctagt 3000
   gccctgtcta gatacatcta gaaggatgtg cacataggca tacatgcagg gtagatgcca 3060
   ggccaggaga acccaccagc agtcgactgg aggatctcca agtaaatcac aggtaaccca 3120
   aaggttattt gaagataaat tccctaggag ccaggatact ttgaatttgc ctgcttctgg 3180
   gcttggggca gaggactcca ttttatcaaa ccttagcaca atgtcgtatc tgccccagat 3240
   ttttttcagg aactgagacg ttaccgcagc agtgtgccct ggtgtccctc ttgcccaggg 3300
   ccccaggaag ggaaagaagg aaggttccaa atgaaggaca cacatttgac ccgaaggccc 3360
   agcaagaatt gcatgttgca atgtgcactg tgatctgcag cccctcgtgt gccttttcca 3420
   aatggctgaa aagtacctca gttgtctcct ggaattcgtt ttagcagcaa ttcatttctg 3480
   accacgcagg acttgggatt gttaatagtg agtgtattac ctcttagcca tcgggccgta 3540
   tttatgggct gtttcttccc ttccaatcat ggctgactcc tgcaggaata tcatactttt 3600
   tttacttttc ccttcagatg ggaccctata tttggaggcc ccatggagag agaaccccag 3660
   gaacccttgc tcgaccttcc cagatggcca ggttggggac ttttcaacct ggagcgagca 3720
   gtcacagtaa cttaaatgtg gaaatctgtc agagcgtcca tggggctgtg gcgcgattgg 3780
   ttttggcctc ctgccatttc ctagctgggg gtgttggaat cgcaagtcac aggtggctgt 3840
   ctcaagaacc cataccctga tagggtagtg gaccccatgg ggtacagctg ccctggagtg 3900
   ctctgagcag cctccataca tcggttgtgg agtgaggatc catcttcacc ctttgatatg 3960
   ctctggcccc accttgagct caatcagtgc gggtgaccct tctgctccac attccttctt 4020
   gagtgatggc tcaggacctc agcatagctc tctagactcc caacgcccac cacacaaggg 4080
   cgccagcatt cagaaacacg atgtgggctt agaaatcaca aagtgataag tgcctgtgct 4140
   gcgtgccagc tttgtcatcc acagtcccct aagaagctcc ccactgggtg gaaggggaca 4200
   tcgcctgtca ctgctggctc gtccggggtt aggacataat cggaaaatga gaattccaag 4260
   tcggatcagg aggagtggaa ggaggaggct gtgactgtga ggcctcccaa ggtcagcctc 4320
   gcccctatgg tgccaggccc attctttcct gctccccgtt tccacagctc tctaggtgta 4380
   acccagccct ggctcggagc ctccccgggg tccctgcccc tccacatctc aggagagctc 4440
   agatgctttg ccagccccgg gagggtggca ttctgcaccc agtagtttcc atggtgaaaa 4500
   tgtgggatcc cagtgggctt tggctcaggg atttgtgaaa gtgtggctct tgcaatgggc 4560
   acgtgggata ggagggtggt aatagaataa tgatcacagt gttcctgcaa gcatccttcc 4620
   agtgcctgcc aggcattctc ccagcaactc tgccttgatc cccaaatccc agtccattgc 4680
   tgatcggcct cagactctgt gcagcaatca gcctcatctc tgacggatgt actttagcct 4740
   ggaagcctct atgcatcctg tttcacagac aagcatctaa cctcaaaaaa ggcagggatt 4800
   cccccataaa taccgcagcg cctttggttt agagatggtt tgaatgggga gaacagctcg 4860
   aatgcactgt tttttcctcc aagtaaggta ctataaaact ctgaactgcc tggcgcctgc 4920
   caagagatgg tgactccaag ctgtggattt ggtctgcgcc cctccgagtg ggtttccagc 4980
   atgcgtctga ttctctcctt 5000
<210> 11
   <211> 5000
   <212> DNA
   <213> Artificial Sequence
<220>
   <223> Nucleic acid probe
<400> 11
   aagcaaagga gatatttcag acagaatttg ttaggaccta atgtggaaaa gctgaagtag       60
   gacaagagcc tctctaagat ggcatatata tggcttactt aggagcctga gaggacctga      120
   ttacaacaca ccatgatgtg ttcctgggtc tgtttctgca ttctgcccaa cagcctcaca      180
   gcagcagcca tactttacat cagaggagtg tctttcccac attattcttc tgagctgaga      240
   gggaacatgc atccgccctc aggtagccta gtgggggttc ttccacgctg tctatgtcac      300
   ccttgtatat aagtgggcca ttgtgtctgt agcttctaag gacccttctg gttgctcctg      360
   ttcttgaagt tccattgcca tacccatggc tttagagagt taacaacttg gacttgtaca      420
   gattaaacct cagaatacca caatacagtc catgccttgc ttcctaacac cacaagatag      480
   gattcttgtt agtgaaaaaa gcagcataat tttctgtatg tttaaacaca ctctagcctc      540
   caggtagcag cccatccatt ccgtattgat gtcccatcta cagactgtgt gcagacccca      600
   gcaattactt gaacccctaa caactgaatg gtaaaggctg ccgtctctct aggtgtcaag      660
   cagttcaaaa gtgaggccca ggcaggacat cccaggctgt aatttgactc catgtggtgt 720
   attgaccttg aatagagtaa ggggtcaggc caagagaaaa gaagagatct gataataatg 780
   gctcagattt aggtagaagc tgtgaaggat gacttgggag cagttcctaa aaccatcaga 840
   gctctgccta tcacgctttt gggtagagac aatttaggga acggaccatc cagggcaagt 900
   tcccatccca ttctcacatc ctgaagaaca tggctttgtg gctcttagga gatgccaaca 960
   tggcgtgagg gcaccatcac tagtgggagc agtgatgctt aatgtattga agccactctt 1020
   gcttcaaaaa tcaatgacaa agggaatgaa gaagaggggg caagtttgca aacatactta 1080
   ggaaggcaaa taatcgaaag ctgctctcac aaaaagacga aggaggaatg gagctctcac 1140
   tcccttcacg cttgccaccc gaaggacctc cctttccttc ctcccttctc acctgtgtca 1200
   cccaacccag tctgcagccc tccttagggc tgggttgcag ctgcaaacag tgtttttgtc 1260
   tacagtgccc tctctgaaaa tagttgtctt tgttgtgact taacggcccc cccctcaaat 1320
   ctaatttcac tttcctctcc ttgtgtcttt ctttgggctg aatgactaca ctgtatcatt 1380
   gctgaaatga ctgtctttaa tacctttgtc atttcctttc tgtctgcaaa aattctcctg 1440
   ttcttagtgt ccagtgtgac ttctggctct ccttaaacta ttttgtctcc attagtttag 1500
   atgctgacct ttgctgttgg tcaaggtcct ccagggcgta ctgcattctc agggctttgt 1560
   ctctcttgag tcaatgaggc ccctaaaacg tgggtttaag aggtcagtta ttgccatccc 1620
   acaaagcaca aactatacaa tctattggca acactgccca tccagtgtgg ccctgacttt 1680
   ggttacttac atttctgtac cacaggacag aagctgaagc caggagttca tggccaaagc 1740
   atccatgagc tctctggcta tggctggcat atcgctgaac accaattttt gctctacttg 1800
   tgagggctta caccagacag ctgaaggagg catgcagaag gcggcgatga gctcaggcac 1860
   caaaagaaag gatttctaga aaggacaata tttgataagg tgttgggaaa cagaagggtt 1920
   aggaaaatgt taggctgtga cttgctcctt gagtgaacgt ggttctcatt aactcacagg 1980
   aatcagaaag taaagaacac cacatgttca gtgggactgg ctcttttaga gcccaaagaa 2040
   ggtggaatag gcatttgagc aacagacagg ttttgaacaa taactattcc cagtagttga 2100
   ctatggggga aatgttacag agtgggactc tgaggagcac aagcccagag ccagagaggc 2160
   catttggggg gctattgcaa tggagataag agttgtaagg atgggataca atgaaatggc 2220
   aagaatgcaa gagagttcaa tgagaaagca tccaaccacc agctgtactc agtctagaag 2280
   gaagaaagtg aatgaggctc gtgggggagc agagggagta ccatggccaa gagtcagagt 2340
   gtgcacgtca tgttcaaaag cttccagagc tgcactctgg cctcagcacg gagaacaagg 2400
   ctctcagttg agctcaccat ttctttgctt tcccaggcac catactcttg tcttcccgga 2460
   tgcctctctc ccaggccttc cctcctagct tagcaaactt actaaaatat agagggtgct     2520
   aacatacctt cctgccagaa accagaggaa tcatgctgcc agaatgtagg ctgtcagccc     2580
   catagttcac aaatgtgaga tttgtagcag gactcgctga tgcaaaacga gcacaagagt 2640
   caaggaatca accctttcag tggggtagta atgaggctgc ttgaggtgtt ggtgcctcac 2700
   tgaggttaat tgaagaccca gcaacaataa tgagctgaga gaagccagtg atgtagcaaa 2760
   tggtgcaggg catttttgat gttttaccac atggatggta ttaccacaaa gcagatcaag 2820
   ctggcaaatg taccaacagg cccctcggtc cttctgcgca gtgtcctctc acagctgctc 2880
   ccacaaacac acttccccag tttgctccac accccagttc tgtttccaac ttatcgccct 2940
   caccttattt catcttctcc cttagctgag ctaattatga gcttgatcaa ggacagatgt 3000
   taatatagag agatttatgc ctcgggaaac tccttctttc agatgtgtgt aattgggata 3060
   gcaggtacat ggtgttacat ttggtgtgat gtgtacttgg tgtactgatg gactcagcct 3120
   cacagttctt tgctcactca ggccctgctt gttaagaaca ttgttgcctg ctgtcagttg 3180
   gccaggtcat taccaatact aactcacgct acagaaagca aaaccctaat ggggctcttc 3240
   agatctgtcc cataataagc tccaactgac cctggagagt tccaggcctt aatatgccta 3300
   ccctcaaagc tgtgcacccc aggcttccac agagacctta cattctgttg ccatcacgca 3360
   tctttatgga gccttttgat ttgggcataa aagttcttta tcaaatgaat ccagttccct 3420
   ctgtagtact cctaaaacct ggaagcctca tttctggatt ctgtttgaac aacaatgcct 3480
   ctgcacataa cctcaatgtc agacactaga ggtctcaata atgtggatgg ccagtgtcca 3540
   ccactgggct ttgtgaccag caggaaatct aagagttcaa ggaagaacaa gacaagcaag 3600
   agaaaacaag tgggaggtca tagataacag gtgccattgg gtattagaag ggaaatgtgg 3660
   aatggtcaat gacttgagac aattaagaag acaaccacca ataaaccaaa tcttatgaga 3720
   aacagatcaa agcaaaaggc agaagaagtg ggtaacccca agtctagtat gggaaggaga 3780
   cagtgtagaa gtgctgtggg agctgagtgt attttcagga acagccattt gttatatcct 3840
   ccaggttcaa gccagctctc tctgtgacac cagggatgtt tcttccaata actccacagg 3900
   caaaccactg actctgggta agaagaaaac tccatagctc caacctccta cctggacagg 3960
   aaaacattct attttattct agagaaatag ccactcaggt cgtgcatgtt tttctctgtc 4020
   gtccctgttc aatgcgtgac ttcactttgc tcctccatgc caatttaccc ttctctctcc 4080
   tatgtttaat gtcacaacta tgtcaactgt agcatggaaa gaagaacttc tcaacctcct 4140
   cttcaatgtt ttaaggctgg catcttttgt tgagagagta gaaaaggtag ctataggaat 4200
   gtaaagatga ggtacgtttc cagggtgaag tcaatatttc accaccctta cccaacagca 4260
   ggaataactc tgccagactt catacagttg tgtatctaaa ttttgattag tatcagtaat 4320
   tattaccact gaaggctagg caaacacccc tcttgagaga aaaaggttga ttgaaagcaa 4380
   ggtagacgca tttctagaaa caggttttat gctcctagcc atccaatgag gaggcccaac 4440
   caccaagact aagaccacag cagctgcagg cattattctt caaaaagcta ttcatctcat 4500
   gaaactccag atacaaagaa ctaggcgaac ccaggtgtag gcaacagagc agagcctgga 4560
   ctcggctgcc cttggttagg ctgggacaaa gaacacattt agaagagaaa gaactgacga 4620
   cgccccttga gtgccttctc ctgcaggccc ttgactgttg aggataccca gttgtttcag 4680
   cttcagcaga caccctcctt aatagaagac cattggagac ctctgataag cagtgaaaga 4740
   aaccagggtt gtggttcagt gataagcaaa tgtggagagt ggcaccgtga aaatatgatt 4800
   gctagaccca tcacaacaaa gcatggttgt agctgcctag taaggggaag taaaggaaga 4860
   gccaaccagg tgaggagaag acctctgtga gtgtgagtca ccaaatgcaa acctgtagtg 4920
   aactcatctt aattggaaag tgggagagga aagaggactc agagatgaag acaaataatt 4980
   gatgtcagag tcagagagag 5000
<210> 12
   <211> 5000
   <212> DNA
   <213> Artificial Sequence
<220>
   <223> Nucleic acid probe
<400> 12
   agtgcctgtc atggtgctgt ccaccgtaca gatccccagg acacatcctc atatttgaac      60
   tgaactgcct tattccacac atgacggttt ccattgcctg tcttaactat gtcctcccaa     120
   tctctctccc caagcgcact tcagagccat cactaactga ctcatggaaa aaggaggaga     180
   aacagaacat ggagaaaaca gacagactgg caaggcctga atccttgctc ctttggcttt     240
   ctgggatctc cctagaattt aacatgggat ggactagagt cttgaaataa agctggggtc     300
   atgctatttc caaacacagg tggcatttta gcaaagcttc ttcatctggt cccatgatac     360
   aacaaaacca acgaaaggca gctaacccct ttaaaaatag tgttgccata gagtgagcca     420
   actgggaaaa tgataggaat ggcagccaat gtctacatca aagagatagt gcctcaaact     480
   tgtgcaagaa aactacttga gagctcagat gcatgaaaat gtctctcccg tgctagcatg     540
   gtcctgggag agggaactcg tgcacttctc atgggctgat catgccttgt caagctctct     600
   tcacagtgct atctggccat ctgagaagag ggatttccat catgcagcga ctaccagaag     660
   ctcatagccc tttgaggcac cttctgtctt tgagcaatct ggcacttaaa atacaaataa     720
   caggagacca aagtcaaaga tgtgatccct gaggtttggg catttaacct ggaaaagaaa     780
   agagtttgac ttcagaagca gaatcataaa acactatgga aaattccagg agcctccatt     840
   cctgaccagt ctggaaaacc aggttcctgc cccggcccct tgccttcacc atttgacaat     900
   gaatagttgc cttcctgttg gcaaagaaat cataagggaa acaacatgcg aagaccctgc     960
   tgtttctccc acccatgact gtttttcttt tgattcgttt ctatcatgtc tgtggtgtgt 1020
   tttcctgcta ctcctagaag gttgcttgca aatgagtagt aaatgaagca gcagttgatc 1080
   acagtacccc caagagcagc agcatgagga attggatgtg catggaagat gagcgactag 1140
   agatgaataa tggacatagg gaagcagaaa ggaagacaca ggaagtccaa actactaaaa 1200
   cagtctggct gtggcatgag tgccagagag cagggcccta caaatgcata gcattctgca 1260
   atctcccctg tgctctaggt gagctgcccc cctgccttga tatacccata ggctacgttg 1320
   gcaaggctgt gtcttaacag ggttgctggc acagatggcc caaaagatgg aaatttaagt 1380
   aaccctgttc cagcaggggg tgcatgcgtt tcagccatga gagtgacaga ggccagaaca 1440
   cgtggctaga ctctcgccaa gtccatcaga ccgttgttaa gagggctgga agagccaaac 1500
   agcaagtggg gccacaggcc tcagagctga tgtccacgtg attcctttat tctctttcca 1560
   ccactgatct caaattacag ttcctgtgca gatatttaat atcagaatag ctcatccgtt 1620
   cacctattaa tcacaggagg accactgaat gtccagattg ctgcaggcca cctcctacga 1680
   cacagaagat ccttctgtga ggtgaaagtt ccaagacgct gcatcttcag gtcactctgg 1740
   ggtcaaagat agtttcccat gaggcttagt ggccaattgc agttcagaaa taaataaaaa 1800
   gccacagaaa atgagtttaa tctgtgaatg tgatttatgg ctgtcggggc agcaaacttc 1860
   atcatgcagc catagcacac aataaaatat gttgcaatac tgtatccatg tgcatcctca 1920
   ttcctacata caggatctat tccccacacc ctagtagact tggaagcccc atgttcaatg 1980
   tgttaagatt ccctgctcag ggggagactg gtcttgggca gaaacagaga ctaaagagca 2040
   gaggtttgct gagcagacag gcaaataata aggaaggcaa ggaattgttt catagtttat 2100
   atactccccc tcggaaaccc cctttctcct gcatcattca ggccactaca acagcaggtg 2160
   gatcttcatg atgttttttg ttagtctctg atggagaaga catgcatggg atggcttatt 2220
   ggtgaatttg agctgtttca ggccgatttt cttataatcc cacatgtgct caccagctga 2280
   cccgtatctt gatatcatct cctagtaagt ccttcacgtg tgtggcccta agcgttctgt 2340
   ctattaaagg cagctggatt tgaaaccccg taggacccaa cttgcaaaga taaggaaact 2400
   aggaaagaaa atgaagcgag cactggtcaa aacaaatgtc attctactca gcagcaaagc 2460
   atgtaaggaa ggcattaagc ctcatcttcc aacatcatag aatgggaagg cctgggaaat 2520
   gaaagaacag aagggagagg caagtaaaac actgaaacac gaagcccacc tttctccata 2580
   aggctctgcc ttcaggagcc aacacaggct ttggagaacg caaatgtgtg ccgaacctgt 2640
   tttgggctaa ccatgcctgt ggtatttgga aagttacatg aactctaaat cagagatgtt 2700
   ttccccaaag ctgagaacat cactgtccag agttaacacg caatgaagac gtgagcaatg 2760
   aatgaatggc tgtttccttt ctcttaagta tcatttttca caatctttaa aacaccactc 2820
   tgctgttttc tttattcctc acacttccat tcctgttgtc tcagacttgg agtattatga 2880
   tggattcccc acccacaccc aaggaggact aatttggtca taaagctggc tcagagggca 2940
   atagacaatg gccaggatac ccaaagggaa attatgatag gagatctgat tttggtgaga 3000
   aggtgactct gaaagtctga ctcaacacag aggttacagg tgatcagtta attggccagt 3060
   cttggacatt agcgtggggc agggagataa gggtgaactg aggtcttgcc aaagttacta 3120
   ctacactgcg aaatataatg tactagcagc aacagagcct acagatctgc ttcatacact 3180
   tacagattct aagagggcat ctcctccaag gacaccagag aactgcagcc acgggcctgg 3240
   ctttgactgt gaactggcag gaaggtctac agatagcctt tctgatttcc tcagactcta 3300
   tcaaaaccac ctcaacccag aaaggagctt ctgtgagaat cacaaggctt ggaaattcag 3360
   gttggagggt gggatttgcg ggtgagggat gaagtggaac gatgtgttcc ctttcaattg 3420
   tcaacagcta aaacatagag caaccatcac acccggtgta acttccatcc attatcagtg 3480
   ggctgagacc aaaactgttt agtcatgcta cgtatacagg cttatatcca aattttccct 3540
   ctccacaatg gcacatgagc atttcctatc actctcacat ggtcagcagt ggagtcctga 3600
   tgttaggtga gatgactgct ccagcagctc cagcgagaca gggaagccca gaaacggaag 3660
   gccatggtgc tattggcatt ttgatgctac atgactttga gcataaagcc aaatcctgag 3720
   gccatttagt ttagggaaca agagtcctct gaagctgact gcgtcatagt gcaatcgatt 3780
   ttaataatga gtagttcaga ctaaagaagg ctggctgacc acatttgagg cagcccggga 3840
   gaggaataat tatattctag tgtcagcaca tcctctttcc acaatggcca tacacataga 3900
   gaaagtaaca tcccagtggg agaaaaacaa tttgagcaga aattaggtca cccacaaagc 3960
   atagccttga ccaaatcaaa aggaaatcta tctcttaacc tccatcatca tgtgacctga 4020
   cagagatgga atctcccaca tctggggaca gcatccccca acatcagttc acaggaagcc 4080
   tgtcccgcaa agcatacagt tgtgtttgtg tgtccacact cccaaaggag taatggcagc 4140
   acatccagga gacaatcaga aggtacatca tccattagtt taagttccac tgagaaatca 4200
   tattccaggg ctcagactgg agagaaagta gaatgagagg tggctctcaa aggtttgcaa 4260
   agtcttctca ggctgagcaa tgaagtgtgc cttcttcctg cataaaaatg ttggtgtaac 4320
   aaatggctat ttaaagtcct ccttcgggag cagatgcctc tttgccctac aggctccttg 4380
   aaagtaggca cctgtctgag taggtgctga atttggctca ttcccactgg gacaaggatt 4440
   caagggcagc ctttcagagc acatggcttt ctctttatgt cgcataggtt acaatgcaga 4500
   gtgacccata aggaagtgga agccatgggg cttacagact caccatacca ggccaagaat 4560
   tggcgtggct tgcagagctc aaatcagatc cacatccatt taattttcat agagaaactg 4620
   ccacagagga tgccacatac tctgcctact tgatttctat ttggagggca gcactttgta 4680
   atgaaaatca tgttgggcct ctgaaatccg ccttctcccc acagaacagg actgctacct 4740
   cccctactct gggtactgca tgcctatccg tgaggctaca tcacatcttg gaaagacaga 4800
   tgagtagagc gcccagctca gctgctgggc cctgacagta gctgcttaat tcaggttcac 4860
   cctctcccct tctcctcttt cactcttgac tcatttaccc agaatactgt tttcaacttt 4920
   accctgaact cccagatccc acaaaacatg gaaacagtgc ttggtaccag ccaggaatgt 4980
   cagtctcatc tgccaggata 5000
<210> 13
   <211> 5000
   <212> DNA
   <213> Artificial Sequence
<220>
   <223> Nucleic acid probe
<400> 13
   ggcacagcag tgaagaattt gcccttctct ttgtaagcac aaaatagggc tcagaaaaga      60
   ttcagggttt ttccagccca tttcccccat cattgtatct ctctttaagg tctgtggttc     120
   tccaagattc catcctgcac ctcatttccc agttgtcatc ctacaattgt catccttccc     180
   tgacgttcat catctaggga gctacttccc atggtcagtt tatctgccca ggtcctgaaa     240
   gccttgaagg ttaggactcc tttttgtctc agtcactttg tcccactgtc tgcccaaacc     300
   agcatacttt tttccaatcc caaagcatct cttctctgta gccaaactga cagggacaac     360
   agtcatgata taatcttaca cagaaaacag gatgctggca ccttagctgc tatgcacata     420
   aagaatatgc acgcagaact aggcctcagg agaaaagtcg tgctggatct taattcatgg     480
   tatgtggtta aacatgtttt gctaatagcc ccagatgtgg cctagtccac acaatccaaa     540
   cacgcgggtc tgccctggac ccagctcggt tcaggaaatc acagaattaa atggcgattt     600
   gaagctttta gtttttgcac aactctgtca tccctacagt gtgcactcac ctaaaggggc     660
   actggctctg aaacgactct cacagcaggt gcggctccat ggcagcatgc ggaaattcca     720
   aataaaaatc agtgagaaag gcttcctccc accagaaggt cttgcatagg agaccaagca     780
   gggaagggag ccgcagacac aggatatgca gaaactcacc ctgaggcctt tgctaaatcc     840
   tcagacacaa agaaagctaa atgagagaca aagtttggcg ggaggatcag agaagcccgg     900
   tccaatgccc tgccatctct tcctcattcc cctatttcca ccctcaccaa tggtctcacc     960
   ttctctttat aagatggttg tttccaaaat ggcagctgca ccttcccagg catccccgca    1020
   tggagagccc tgcttcacct tgatgcacca tttgaatgac accctttagg aagccttcct    1080
   ggattctttc aaacccctcc tatcctgaag gcaagaagag ccacagaagg gtttgatgga    1140
   ggtaagggat gtgcctggct ttacattttg gatcatttgg aagggaatga gaatgccata    1200
   gaactggcta ttgttctgca tatcacatgc ttccccatta tttctctttc catgtccaca    1260
   ctgaaatttg gtcgcagcga gtgggcaggt aaagggaccc aaacaaagga aaaccagaaa    1320
   acacaatgca cctatgtcta ctgccaaaga cctgggataa tctagtagag cctcaatcac 1380
   cccttttcct cccatgatat agaaaaggga atgttttgca gacatcctca tctttgcaaa 1440
   tgccctctct agtcatcaag tcccctacaa cattaggatc tccagaaaag gaagataaac 1500
   aacacccagg aacagttctg ggaactcctg gtacaaccgt atgcaaatca tttcacaggc 1560
   gtgctggcag gctgagttcc cagagtaaat gattttcatt ttgataaatt gcagccattt 1620
   cctttttctg atttggatca aaccgtcttg ctcactgatc tcctctggct ttctcatggg 1680
   cagatctact ctcatttttc ttcctcttgt tcatgcttat ttctacctgt agctgcagtt 1740
   ccccaagtcg aagtcaatcc attcactgtg gtcccttgct caaagcaata ggctcaggag 1800
   cagtagctcg tttaggcaac acttcctgag gcttgtggtg ggccacactc tatactaggt 1860
   actgtcccca gttgagtgct gaattggaga aacaagcttt agatcaacaa aatacgattc 1920
   cgtacctaat gagcaccact caaaatacag ttgcaaatct gggcacaatg tctgaattat 1980
   tcaccatgag ccatgtaaat gcttcagctc tctaggaggc tgccacgtcc tctctctgtc 2040
   tttggggaca gccctggagc ctgacttgtc tcccaaatga ccaatcaagg catttgtagt 2100
   gaaagtgccc agaggacctg aacgcatggg cttgtgaatg ttctcccatg gacgtcagct 2160
   gccatcaggt cttcagctga aataggctgg agggaaattc caactcctgg ctcctttgct 2220
   taactcttcc atcctctcca atatctgggc agacggccaa gatctttgcc attttagtgg 2280
   ctcaaggagt ctaccagata ctcttaagcc ctgatcatac cccaggctga ttttatagga 2340
   gagctggata agacagcaag gactatgatc ccaaggtagg aatacatgtg aaaaacccag 2400
   ggtggcccag acaagtgata aagttccaaa tctaccttct ctgcccttcc tcgtcctgag 2460
   gggtccagag gtcagaaatc tgtagctgca ttactaataa gtttcacttg tctgactcca 2520
   cttcccttca gcaacccctc cagggtttgt ttgtttctga tctggttcat tactcttaag 2580
   tcttgccaca gccacattcc caaatgctca ggcctcatgt gtgctctctg gcagcgagct 2640
   gtgcttttgt ggaaaattct ccccttctcg cacgtccttt ctttgacata ccaaatgttg 2700
   ttttcaaatc taggatttat caaatgtgac agcctgacaa tcccagagac aagaattcca 2760
   caggcacgtg cggcacagga caggaatccc tgccactgtg acctcagttg ccaccttcat 2820
   cacagttgtt atcaattccc aactgactga cctgttctca ccatgactat ctggaggcac 2880
   tctttggatg ctctaattgc cacaaatcca atccatatgt tcccaaacaa tgcaatgaga 2940
   tccacgggcc cagaaagctc gtaccttcat tcccaaagaa atccttggtg taaaatccat 3000
   ggtccgcata agcagtgttt gagtttgctt ctcaggacat ctgtaagttg gctgttgtga 3060
   ctggttatgg caaaccgaac tacctctttg tgtaaagtgt caaggttctc tggtcatctc 3120
   tattactatg atcacagtgc ttcattgtgg ggtggggaag ggggtgaatg cagtagggga 3180
   ggttgagtta gtttttgttt gaagacagca ttcattcctc aaacacctac tctgttcctt    3240
   agtttagatc ctggctctgg gcttgactta cctttgtgaa attacttcgt aaatattttg    3300
   tgtgttcact ggatcacaca gctgagcaac tgcacaatcc cctctgcgca gcagcccagg    3360
   ttcctgcagc tgtgcacatt cctgagtgga gtcgccacta aagattattc cctaagctgg    3420
   gactgcaagt gttttgtcta ggacagtttc gaatgactca gatgagaagg gctccacgtc    3480
   attctgtgga aaatgaggct gactttacct gtgccctgat ccccataagg gcctgcaacc    3540
   accaggagag gcacctacat gatgtctggg tatagagtgg aaggatctgc atgcaaggcc    3600
   gcaaaaagtg ttactatgag tattttgatg ttaaaggggg gctttttgtg cacagagacc    3660
   ataaaccgaa gtcaccagat gtttttaact aattcaaatt ccgctgaggc aaactcctcc    3720
   tccaacctca tagacacagg aggtattgac cagccctggt ggtgaaacta gattttagca    3780
   actggaattt atcttacact tctacatcat tagcgccaca gagtttgcag tacacagcaa    3840
   gtgccctgga taaatgatgt gaggctgatg gaggaacaat gggaaactag tgtaatggaa    3900
   catcccctgt gagcatgtat ttcctgaggg ccttctgtgc ctctgaaggt cacgtgtacc    3960
   tagaggaagg cgatctgggc tgctgtttgt gcacttctga ctaacctatc ttgcatccat    4020
   gggccctgtc cttcttctgc tttgggctgc tttttatccc ccatacacca gggctcagtt    4080
   gtcaagttcc caacattacc ccagcatgga ctgatacgat cactggagcc ttggaggtga    4140
   gtttgaaaga tgtggatgag atttgttggg gagaaggagt ggtatgcaag gaaaatttac    4200
   caaagcaaac atagtgaggg cccctgcatt tgaaggctca cccactgcaa atccaaattt    4260
   ccccagtgca ccagttcatt aaagcctatt tcctctggct ctctgaactc attcccccta    4320
   cagttctctg aggcttttct cctcccaaaa tgccagagca tgagcttatg cgtctctact    4380
   tattattttg tagctcagaa tgtggccatc aattcaaatc agccatacaa gacagcttga    4440
   gaggccccag ggaaaccaga ggacagtgga tgagaatagc tggaagtcaa aagaaggtag    4500
   gtccaaaatc gacaaggtta tcatgagaga caggagtagc cagtggccac agtccacaca    4560
   acagagagaa gatatcctct ggaggatcaa agagaaacag gcccggctct aaccaggaag    4620
   gcagcctcag gcacgtaggt gccttcacgc atcccaggcc tgaactgttt catctgtaaa    4680
   atagagttga tagcagactc caatcctggt ccttgagtct aaattcctgt ccatctctca    4740
   ccagtgcagc cttccaccaa aagtgtcctg ctcttttcta actccttgct attgatcatc    4800
   ggtgtgaaca ctgctgctgc cagatgctca gcaccggaag gaaagccaag tagagctcgt    4860
   ctggcacatc tgctacagac acagaaaatt aatccattgt atagaaatga tatcaccaca    4920
   tcaggacttc tgctccaggg gcaccagggt atgggcaaag taagagctgt gtgtgtttcc    4980
   atcttcctac agacctacta                                                5000
<210> 14
   <211> 5000
   <212> DNA
   <213> Artificial Sequence
<220>
   <223> Nucleic acid probe
<400> 14
   atgcactcac agcctttaaa cattctccaa atgtctgtca cactagctat gcctgcctca 60
   tgcgcaccaa gagtaaatat tatcctcgcg ttaaaataaa ttaagctccc tttcatgaca 120
   aacacatttt gtcgggagag gagactggaa cacagagagg tccacttcca ttgtttaatc 180
   attgtccttc aggacttggg attaaaaata ctgacaaatt attcagagga aggaagatgg 240
   tgaagttggg ggagtgctta ccagaattag agtaataagg ggaagttgag agagagacag 300
   agctaagtga cagtgatttt tgtacccaca cataaacaac aaccactact tgaatcatca 360
   gctccttggg gaaaatctca ttgttacaaa tgtctttcaa aaacagcaca gagagtctcc 420
   agagcacaga gaattgtaaa ctgctgggat tcagcttgca ggccaatagt cacgcatgcc 480
   cccttcactg gtttacccag aaagatacca tcgaaaccac tgagcttcag atacagtact 540
   taggcagtct gcagccttgt gggcacattg aagcaaggct gatactccca caggtgtccc 600
   agcaccaaag tgaagagcca gccccttcat cagagagaat cttccctttg ggctgttcag 660
   gtcacttaca cagactcaag taaatttaga acctatttat cgggagctca caggggacct 720
   taagtaatac agctaggcag agggctgtgc ccaggccatg tttcccagct gatcactcag 780
   agcttcccaa gttcaaggcc agataagggt gtgctctcct tggctctcct ctactaacac 840
   atccttttac cactgaagat cctgcctccc cattcctccg ccatctgcaa agggatgtgg 900
   aatgctaact tctggggtca atgctcagtg acccgcaggg cttctgtggg acttggctag 960
   aaatggacaa gagagttgtg tgatatccta aaggtaaagt acagataaca gatactactg 1020
   atacaaagat caccagggca agccaccaaa aagaaaatgt cagacacaaa aaatgataag 1080
   ggtgttgcaa ggctgtcatc acctcaacta tgttaaaaaa tcacagggct ctgagtttac 1140
   acatcaaaag cagaggggct ccgtatctcc cttttgttcc ttgataaaaa tagccaaatg 1200
   tgtttctttt tctgtaacaa gtctgacttt gttaaacgtg gctcattcag tcctttctca 1260
   aacttaaagt gtcatgtaac ttctgtgact tctactcttg tggacatgca cttggaggtt 1320
   agatcttagt gcttgctcct tcgctgcttg tgcaggggtg acctacgtcc aatgttagga 1380
   aggtgccaaa gaccaggggt ccttgtggcc acacatacat ccttatccat gcggggtcct 1440
   tgtcagcaat tcctggctga tccactacag agagatgcta aggtgcatgc acggcgattg 1500
   ggagaatcac tgctttttat gagtagagga tggctttcaa gaacaatgct ctcctgggtg 1560
   agataacaca ttgacctgac atcaagtgag ggggcaggga aaatgtggat taggtaagaa 1620
   agacacccag agaaagggga cccttggtag tccctaaggc tagcccaggc gtgaaacact 1680
   gtgtatccac agccaggcag gcctcccagg aagggtcttg gatgctccac tacactccgt 1740
   catccaagac aacactgaaa agactcccag atctttctgt gagtgagaaa agagaccagc 1800
   gctgagcaga atggagccta ctttcctaaa cagacgtcaa actaatgtcc tggcctcctg 1860
   cagttacaaa agctccctta cagcacaact aactcagccc gccaaatact gggggcttaa 1920
   atctgcaccc catttacaaa tgcaatgaaa ggcagtaggg aaaacacaac agtgcaaaaa 1980
   cacaattttc catgcaattt atgacagcac tgaagcagct taccccttgg cctggattct 2040
   tgccttcttc gtctaaaggg aacaggctgt tgctttgcac agggtgggca ctccacgcat 2100
   cgctgcctcc tttggggctc catccactgg ctgacccact catcatctct tgccagatgc 2160
   attccaacct gtgttctcct tcttaaattg cctttcccca agccgcctat gccctgagca 2220
   gcctctatgc tcttcagatg accatggctc acaggcgtct ttccctccac cagtatcttg 2280
   cacttaatag acattaagtg tctctgcctc ccttgtacaa caactacgct caatgacaga 2340
   agcaccttga tattagcact accatggatt cactctacca aatcactgtt aaacacaatc 2400
   gccggagagc cctgttggtg ctgtagctgc aggaaggcta acgaggaaaa gttagcagag 2460
   ctagcagggc tattgtcacc catggttgag cctgcaccag ggcttttcct tcctgtcaca 2520
   gtgcacaact gggaacccac agagaagtag tcaaaacagc cggtgtgcat gcatctctgc 2580
   cggtcacggc cccccagagg ctcccctttt cctgtccgaa gcactctcca ccaagcaata 2640
   cactccacag acttgcccag gagtgggctc tgagtgctct gtgtggatgg ctcatactgc 2700
   gctagaacct tgctcttgaa agaagcccca acattggaca gtcctgggca gatctgcact 2760
   tacacatctg caaagacaga aatccacaga ctggaggggc accccactgt ctggcctgga 2820
   tcctggactc ccaggaatgg gcaagggctc aagtcaaaaa acatgtatgg ggcacttctt 2880
   acaaggacta tattgaggaa gctttcggga tgtgtggcac cttttctctt aagtgccaag 2940
   gggctccttg catgtcttaa atgactctat ccacctgccc accttccctc cagcaccatc 3000
   agcctagatc tcaagttaat acccagccat aatcattttt ctgacatgcc ttcataacat 3060
   ggggcctctc tcgcacactc aaacaccaac ttttgaattg gcatgaagaa aaaggagaca 3120
   aaaaaatctc aaaccgtgtc cagttgggaa agagccatcc agccttagag tcatgctatc 3180
   gaatattctg gaaataaatt ttttttatga agccacctgt tcttctcctg ctcacttggt 3240
   gtaattcttc acttctgttt ctactccacc tagtacctgt tgccacaact caatatgtat 3300
   catattgctc tcatcttgaa tcatttgttt tacatggggg aaacaggatg agttggcaaa 3360
   aggaaaaaga ctgaaaaaat gtctgaggct aacatgccct cctgtaaaca tagggctgtt 3420
   cctccgtgac agcgaaggcc agtcatctcc tgggaagaga agaagctgaa agcagaagct 3480
   gactcttcca gggtggtttt gccctggttg tcaggaaaca gtaggaagag tctaggtcct 3540
   taatatcatc actcttgcct gagtccaagt ctttacccac catctgtcaa tggattattg 3600
   caccacatag aaactgatgc ataaaatatc ttccattctc ctttccaggg cctctgtgct 3660
   gtctactgtg gaaccaggta tacaatgggt gtcccattca catccccgca gttgttccat 3720
   tgaagatatt ccacttgggt catgacctcc ctctccgaat ccacttggac aatccagaca 3780
   gcattccaga ttcttgcact gtaaatgaat aaaactagta aaggtttcac taaacacagc 3840
   taaggacacc gccaatctat aacaggtgaa acgcactcgc acacacaccc ccagaagtct 3900
   ggcaaggatg agaagatgct tagtgcagag ccaacagacc caagcggaac tgtggctcct 3960
   accatcaact gcagtcacca acatgaaatg gcaagcaatg cacatttctc ctatagttgt 4020
   tttccaacct cctcccagga caatcttcct ccaaataatc caggcccttc aaagtgtcct 4080
   ggaacccttg ttacacaatc agatatgaga acgataaaat gaacccaaaa gtcagaggca 4140
   cccaatagcc aggtggaaag ctcctctggt cctttgaaga aggtggtagg ggaagaacag 4200
   gcaacccgac aagcagagag cggcctaaca ccactgcagt caatcagggc ttccttggca 4260
   ggatttttct gaagtgctgg tgcatatcac ataacatctc ttcaaaaaat gcaaaatgac 4320
   acacacaaga ctggaatatc gagtacggtt gtgcttggca tctgcagggc actggtttca 4380
   ggagctctga ggccaagctt atgttgtggt ctgttggttt gtttgcttag gtagcgaatg 4440
   cagcagctga ctgcaatgct catttgcaat ctgcagacgc aggtccctcc aggagggaac 4500
   gaaggataga aagtttccac actcacctct actagaggac atgccagccc cccatgggag 4560
   gggcaagacc agtctttgga ctttgctgta aaattaaggt aactatggga aaacccactg 4620
   gaaaccttca tgctgagagt gagataagca gaatgcagac ctgtcctctc tagccactgt 4680
   ccctggggct gcccttgctg cagcccttga atcccttctt aactaaaaga catgtgatcc 4740
   ctgcctgtac ccttccccaa tgttttcttg gagctctatg ccacttattt aactctacgt 4800
   cacaggacgc cagggccctc cctgtccaaa ggccccactg cagtggagat ttgtggtggc 4860
   acatgctgtt tgaagtatga gagcccccaa gtggggctcg atgccaggaa gccaggtctt 4920
   ttaaagcact gtcacctcag atgagccgct caggagcgct cacacctcag gacccagccc 4980
   tattcttcac aagtcttcaa 5000
<210> 15
   <211> 5000
   <212> DNA
   <213> Artificial Sequence
<220>
   <223> Nucleic acid probe
<400> 15
   cgcggtcagc atgtgagcct ccctgcacag gcagattctg tgacatagga acaaataagt 60
   ggctggggag tgagtcataa ataaccgatg tcgaggtaca agggcgtttc accgaggccc 120
   aacttcccat cctcgtgcct gtcgatggct caaatggtaa gtgggcaaac cctgagtgcc 180
   acgtgccctc atctgggagc gcctctgcca agtccaggcc agtccacatt tgcccggaaa 240
   ataggccagg ttgcaaatgt gagtcatgat agcaaagctg atagccacag atgacagaag 300
   ttcaactctt caccagactc gcagtaatta tagtcaacag gaaagtcgaa ctcagccaaa 360
   gaggcaaggc aacaggaaat tgtggggaaa ttttaaaaac gttcagcttc tcaatctcac 420
   tctggaattt tataaatgcc cctggggggt tggcagaagg aaaagggctt ctgtgaaatg 480
   gtgaggcatt tcccagagcc caccttaaac tcaatgggaa gcccaatggc cttgatcatg 540
   gtagagaata ccaccagagg tggcaaggct gctgcaggga gaagacgctg ccagggatgt 600
   atagaaaata atagatgtcc gttgtggcta ctactacaac acggttggcc tttcatctcc 660
   cctttgtctc cccaaagaca catacttctg ccatccaaca atccactgat tcctctgtct 720
   cccttccaag cacagtgcag cattcctgac attcttcctg gttctcctct ttcataatgc 780
   ttctattccc cgtttgacat tagtgaaaat gtccctttgg tttttcctca gcctgtccaa 840
   gctcccatga ttcactgagg aaacggcctt attaagggat aataggttat gtggtcccct 900
   caacacagag agcccctgga aattacagtg cattcaggat ttcccaattt accacaggcc 960
   ttcagagcca catttggcaa cagcctattt ccaatgtatt attctttcct tcaaggcatc 1020
   tactgaatgt taatgtgcac gtttgaaagc tatctgtggt aggctgtaca ctatttcctc 1080
   acaattttcc cttctctctc agccaatgca gagtacatct tcctcaagca gcccatcaaa 1140
   tgaccgcagg ctctgaggac acccctgaca aagcctccaa gccagaagag gtaaccagcc 1200
   ccagcaggag agccacagtt cagagggcaa ccacttcaga cacatgccct cgctgtaagt 1260
   aggcctcggt ctatatctca gtggctgatg gtgatatcaa gaaagctaag ttgtctgctg 1320
   gcaaactttc aagctccatt tcttcatgct tgatagacca caaaccagct ccatgagccg 1380
   gagagaagct gcaattaata cagagaaaaa tcgcaaaacc acagagaagt gcagcactaa 1440
   agacaaacag acaacgtggg actccactct ctggatatga gaagtttaca gtgtaggaga 1500
   gaagtttggt gagctagatt ttaagtggcc cccaacccag ggaaacatac accatgtgct 1560
   ccaggcttgg tggggatggt gcgtggatga gagcagacag actgatgtcc cctcgttcat 1620
   tctaaatcac cccacacatt tatctcccta tcaagcttcc cagaatcagg atctcttacc 1680
   tatgtcatgt aaacctccaa aacacaattt cattatgggt ttaggggcca gaagaaaaat 1740
   tagaagggca caacagaggt ggaatctaat ccaagaccac ctatataaaa acagaactct 1800
   agtcttccaa gtatctggga gatgcacaat ggcccatcag ttggctccag ttgtcaccac 1860
   tggcacattc actcacctgt atcacactcc ccactgtgat gtggcatttt tcttggcatt 1920
   gagactggtt attccaaaca cactcttatc agccttgcaa ctgagccaag ccgtttatac 1980
   atgtcctcaa ccattattcg tgccctttat tcacagttcc tcacacctgc tgcagcaagc 2040
   tagcctgtgg gaatccaaat cccaccaatc agctgaatgc acctgtgttg tcttattctt 2100
   gacccacaca gtgggccaca tttccatccc agccctgaca gggagcatct ccctctccct 2160
   tctctacttg ttccagtaga gcctgttctt taaaaccagg acacagctaa aacccatctt 2220
   aagaagtcat ttaatcatgc cctggcgcaa aaaagagaag ctgctataat atttggtcag 2280
   accaactctg aataagctgc tctgaccaat ctgctcccat acccaagctt tggcttcagc 2340
   ctgatccaaa cctccaagta ccacccgctt ccctgacagt gttccctcct cacattgaca 2400
   agccctactc agggttcacg gtcattgctc catccaggac tggtctgccg tcacttaaga 2460
   ggcgactaat aaggatttgg ggggcaacag agctgccacc tccctctttg aaatagtgct 2520
   cctgaaacgg tggggccacc tcaggatttt cgtcacgtca gtgtcaacca catcattccc 2580
   cgtcttgatt gactttctta ataacttaaa atattaggca gctgagacaa cattagacaa 2640
   ccccagactg ccattagtgg ctccgctgat cccaggaagc atatgtaaca gttcttcttt 2700
   ttggaattac agatccatgc ccaaaattag ggcaatggac taagaagaaa gatacctggg 2760
   aaactgtagc acgctcataa gagttattct ggagatctgg ctcagtataa caagcatcac 2820
   aggtgagaga aactgccctg ggaagcccca caggagaaat gggtttgcaa ggggaaatga 2880
   cactgggtga ctatacttga aggacctctc ttgctcacag gaataatgca gaactcagcg 2940
   gcccaaaatg gaaagagagt agagcagaga aaagtcctaa ccaaaccaca gagcatggag 3000
   gtgagcacaa aagctcagac ttggggatca ttaaaaaata gatagtggtc aaaagtgcaa 3060
   tagcagcccc caccacgaaa gacaggtgtg gagagagagc aaaacaaaaa tttggcctta 3120
   ggaacttgcg agtcatctct gtgatccttc cagctgtgct ttgcactggg ggtaataaat 3180
   cacgcgtaaa taataatcac agtgtagagt cccacatatc tttgcacagc tgctcacatc 3240
   agctacgttt ttcaaccaaa tacggcaagt ccactcaaag cagagccgac gtgaatcatc 3300
   accatggtcc tccctagtga tgggggacaa agtggacaca accataaatt gtgtgggcta 3360
   aaggcaggaa gtaaggcaga tcaatagcag aggtacagcc agccagaatg agaccaccgc 3420
   tcgctttctc attcacaaaa tccatttcct gtctcaggtc cagaacttcc agcgtttggt 3480
   tctgttcatt tccccctgga tactaggaga gggggtgcaa aaagaagcac aagtcagaat 3540
   aaccctggag aaggtcctat ccatttcaga gaagacatgt gagaccacag tgagcattca 3600
   agagcccaat tcacatcaaa caggagaaca ctgcaaccca acggtgtatt ttcccttgcg 3660
   cactttttcc tttcaaaagc attcatttca ggaatgatgc ggttttacta tagtgcatgc 3720
   tcgttacaga aaacatggag gacactgaga agaggggccg actgcccacc tccctatcaa 3780
   ccagtgctgt tagtgtgagt tgccatggaa acaatgggcc atgccttctc tacagagctg 3840
   aggattaaga ggcctgaggc ctcgacacta tggctcactg tattcaggct acggggagaa 3900
   gatttatcca aggagaaacg gaaacccgtt gctaccagat ttcctcccag tcaggcctgc 3960
   accctccctc ttttgggtcc catggagctc tctggtccac atcagatacc gaagtatccc 4020
   aggcgctcaa aagctgtctc tcacatcagg gaggtgttca agttaggcac aaaatgttca 4080
   gcctgcctcc ctttgcggag accgtgacta cccagaacca gaattagaaa tcaaagtgag 4140
   gtccaggacg tactctggaa tgcagactcc tccctccctg ctccccaggc tcagtataga 4200
   gattgttcat tgctaccgta agtagcacaa accttagtaa atcctaggcc tccctgagca 4260
   atgtacacac actttctcat taaaatttca ctcccacgcc aagggctggg ggtcatgagg 4320
   cttggagacc tcgctcactc aagttgtgct attccaagct gctccctgga aagccagcca 4380
   ggggttccag caatgcccac ggcacagaag ctaaaatcca catagacaaa ggccgagaga 4440
   tgatagggcc aggaggtatc aagaggaagc catcgggtag cagagggcaa gactggagga 4500
   cctggagaca tttgtaatag atgagaaagg agcaggaaga tgaaaggggg cttgctgctg 4560
   tagtcaggaa ctgcatctat taaagggtta acagcagttc tcagtacaac tgagtaccag 4620
   ccatgaaagc agcatcagac atttataatc tagctggatg acacagccga gcagaaggaa 4680
   tagtgtagtg taatacaaga ggatggggct cacccactag cctcagagtg gatgacagga 4740
   aggcttttct gatccaaatg tgagaaggag accttgcctg ccttgccctg ccctaacctc 4800
   tctgcttctg gttttcatga ccttcccatg actacagtca cttggcattc tccagatccc 4860
   tccacagtat tgaactgtca agattcacct aaaattaagt cttttatact ccagtttacc 4920
   tggtattgtt gaaacagcac tggacgcaaa gttagaaaag ctcgtttcaa gtctcactaa 4980
   tgtcagtctt cagctaaacc 5000
<210> 16
   <211> 5000
   <212> DNA
   <213> Artificial Sequence
<220>
   <223> Nucleic acid probe
<400> 16
   ggagccggtc tccagccctc agttctgaca gatgggtcaa gttggcctcc ttcccaagca      60
   ctggtttgga ttctttcagc ttctactttt tcagttggat tctcacacct ccctttaaaa     120
   atagggggtc ttagctcagg gagacggacg tccacagagc gaggctgcca ctcactgctc     180
   tgtctctagg ctcacgtcca ctctcagagc tgcctgatga gatcccattg cagtgttttc     240
   cgaaggaagg agctcaactc ccctttctca cagcgtctgt cgcttactct cccaggcagc     300
   aatctttcaa gattttgctc accttaactc agatctgctg tgcctggaat tgtgcaactc     360
   agtggtccta cgagagtgtg cttggtcaaa gacaccccca catgccctat ccagattcca     420
   agccattctt gtcccataga gacctccttg ggtgacccaa gatctggagc cacagctccc    480
   tatggagggg tgtggcccct cgtgtctgaa tgggagggag ggcatggaaa cgtcagggag    540
   aaattcacat gggtccttct gcttctttat ctccctcctg acacagctct gaatggatca    600
   agctcacccc actgggtctc cttgtcaaag tgcagatcag tctgctgcct tgcttgttag    660
   gccatatgct ccccctttgt ctggggaaga tgactcacta ggataccagc gcttctcctg    720
   gaagcccttt gtgaaatctt tggaggtcca agtgataaag tttggaagcc acatgccatc    780
   tttggctgtg caaatgcaag tacgcaggtt tgcacagagc ctctagcact gcgccaagca    840
   tcacgggtac caaggtggat ccagcacaat ctccatcctt ggcttgctcc agcggcatcc    900
   tgcaatgtcc ttgtgcccct tgccatctgc caaatctact ctcactcttc agggctctgc    960
   taacctcctc agaggctctc ctatgcagtt tatccctctg tatgttttta gtctatctca   1020
   agcaccagga tgaaggctcc caggctctgt cccatttgtc ttggtatatt ccgcctcccc   1080
   caaattctgg catgtcagta aggcagagaa accaccattg agtctcagtg gaccacttct   1140
   tatggaccca gttcattggc tctgataacc tcgtcttcca aagttctgct ttcttagaaa   1200
   aatttcccca cagccatttt tggcatggcc atccctgcta tgagatggct ggcatgtctt   1260
   cggatgccgc cccatctacc tccctagcct cactgtctcc acgtatgcta tatttctcct   1320
   gttttggaat caaggttaag actaagagac tcctttacct gctgacccac gtggtggtaa   1380
   cttgaaatta cagaggaaaa aattgtataa ggcctcctgc cttcatgaca aaacacacag   1440
   gcttttcaca ggagcccagg ttgcaacatt ctgacactag aggcaaggtg cactctctga   1500
   ctggggcaaa ttacaacatg tcccacaggt gtccaagtca tccatccgga gaagctccaa   1560
   aaagggcaaa tgatgcctga aatcactttg gtctcaaaca aatggaacgt ccacttgtat   1620
   tttattgctt gtcacaaacc ctcaaaccac agcaacagtc acagtttatg ttagaaacag   1680
   caaaatcgat gtgaattctg ccgctgtctg tgcagtgtga ctcgcgggag ctcctcctct   1740
   cattcagcag gaagtctttg caaagtgcct gcgtgcttta gaggctttgc agggccctag   1800
   tgtcatccaa ggagtgaccg ttggctgcga tgcttgctag cagaggaggg tggacagccg   1860
   aggatcccta cactcccgtt ctcttctgcc ctccaccggg atcaagcact ggaaggcctg   1920
   tgagtcctcc aaatgaccct ggtcatcact ggcccagtct gaggggtagg ggataccaca   1980
   gtttacttgg aatgctcaaa ggctgactcc agtacagcca ccaggagctc gacaggtttt   2040
   tgagtagcag gaggaagcac tccaaagcta tagaccagca aagtgggttt acatcccctg   2100
   tatccaaaaa ttgttctggt gaatgataaa ttgcagccag tctctaccca atgccaaaac   2160
   agcaagaaga aaatgcatta cttgtctagt gttctgaatc ggaatttccc tgaaagttct   2220
   tatttcaagt atgacacagg cagggagggt tggttgcctt aatggcagga gattccagta   2280
   aaataaactc caggaggcct aagatgaaac aaaaagagtc aagtgcaact gctgagatat 2340
   cttcagaacg tggccaagag ccgtggagtc catcctcttc cctagccaca ctggtttcct 2400
   atcaaaactc ggcttcaact gctggatgcc caagtgagta gggctgctcc aggagctgca 2460
   atgcttccta ataaacagag aaccatgata ggcaaatgat tccaggcttt ttgttccacc 2520
   ctcagacccc cttgtaccta aaaatggcac catcgcgttc acccagaatg gcagggccac 2580
   attctctccc ttcctcccca ggatccatcc tcccctcatc gtttcatttg taggcttgat 2640
   atatgctggt ccatggactt ctggcttgaa tatcttctta ggtagcacac actcttccat 2700
   gacaaactgt aagttagata agattcaagt tctacctcaa ctctgacttg ggttgctctg 2760
   tggccttcca tgactgattg acattagatt cagcttcttc tctcacgctc ctcaaggttc 2820
   tatggtctgc agacaatgag tgcttagagc atgctatggc gcagttagga attcttagcc 2880
   aacccctctg tgcattgtct agcagatcac agaatatacc acatccacca ggctcaacag 2940
   aacacaccat tatccctctg gagttaaact gggagtggca gccaagacag gtggtcatac 3000
   atggatgccc agctcagaat ggctgactca ggaaagaaca gcccgcagct ctggaggtta 3060
   gtaagccctc tgccagccaa gttcccaggg aactccaagc gcaatttcct tctccaaagc 3120
   ctattgcaaa atccactatc cttgcctcaa ccatgcaggg aaaatagccc ttctgtgtta 3180
   tgaataatag gttccagtaa ccatgtaggg atgaggcctg aacacctcca gatttgtcct 3240
   tctatctggt tgaggttttt tgaaagatcc ttcctgtgaa attctgtcct tccacgtgtc 3300
   atcagaagcc aggcacaagg ccacgccctt tccattaact ggatcagtta gctgccagag 3360
   ccacagggag ctgctctgag gactttgtaa atcagatgga tagctaatgc tggcaagaag 3420
   acgggggtaa aggggcatgt aaaaccttca aacctgggtc ctacaaccag ataagatgga 3480
   aatattctct gagctgttaa atgagccaga gcctggactg ggagctgatt tacttctgtc 3540
   cgtttcctct caccccatgt gcttgatcct catctttggt ggagtgggga gggtggacgg 3600
   tttctccttt ctccctggct gaaagtaaag attatcgcaa agctccaagc aacccttcca 3660
   gtggcagcag gtagaaacag gaggataggc aggaatggag tctcaagggc cttcatggcc 3720
   tggatctgca tgataattga ggaggatgtg acttggatag actgtcttgg gactctcaaa 3780
   gctgcatgtc cttgaggtga gaccagcagg gaataaagca gagctcaaaa gaggatggaa 3840
   tccccaggac ccaggaggtg ggtaggaaag agaccagaag gacagagtcc cagggactat 3900
   cccaggtgtg tcagtcactt cagttggata tgctcttacc tgcggcgtca gacatgctct 3960
   cactgtttac gtgtcagata cctgtttctt gagactctgt aggccgcaga ggacggatga 4020
   agtcagcttc ctttgcagtt cctgggttct gagcagcgac ttttccctct tctcagataa 4080
   ccgcagaatg tccgcataga gctgaggcag ctcccagttt ctacttcact cccagcaaac 4140
   acgggcaaaa gccctatcac agtgtatttt cttcttcccc tgcctcccat atgctcaaca 4200
   gaagcaggaa ggatatgtga ccaggattta cactctccac aggaagaccc actcctgtgt 4260
   tcagagttcc cttccaagcc tttaatgatc aattttactt acttctgcag accctagcca 4320
   cagtgatgtg gctatgagag cggggagggc aagaagggga caggacccag aatgtttagg 4380
   aagcacggat caggggacac gagaacagaa ggaagggatg ccaaagtgga catacgaagg 4440
   ctgtcgagag agcactgagg tgagcccaaa cggagacaag cctgcttgca tgggcagcca 4500
   tgagttccca ttagcagact tacaaattta ctccaagcag gtgaattagg agcagcacag 4560
   tcgctggcct tggagaagtc cctccatttc ctgtgacttc agggatggta aagaagcgag 4620
   atcgtgtgag tgcgaagact ttattctcac acttctgcta ccatgatctg ggctctaggg 4680
   aggaacacat gaacgttcct ctggggttgc tacaggcttg cagttggagt ctggacctac 4740
   cttaaaagcc cccaaaccag gggcatgagt ccaagagagc agatacctta ataagtcatg 4800
   aggagtccca gagccaggga aggcactgct tcgaagagtt ctgttggcag caattcatgc 4860
   aaaggtgcca gcaagaaagc atggggactg tgcatgccat ctggtctctc cttcctccac 4920
   agctctctgg gggcaccgca ttgcctgccc tgaaaagtcg atgcttcact gtggtgagaa 4980
   acgggacttg taaggctgtg 5000
<210> 17
   <211> 5000
   <212> DNA
   <213> Artificial Sequence
<220>
   <223> Nucleic acid probe
<400> 17
   cttcgcattc aaagactatg agaccaccaa ttagattcca aacagtagac aaaggcacag      60
   tatcaaacgt gggtaggctg ttctgccaga atctggcttt tttccccaaa actgaaatac     120
   cagactctgt tgccttcttt ggaaagggtt agggaataga gtaggaaggt gtgttgagtg     180
   attgtggagg atgcaaacag cctgccctga gcagaaagaa atcatctcta agctcctgtc     240
   ttgggtgaca tgttccctga cacagggttg cttttcctca cctgtctaaa ctaagtaaag     300
   caagctaaaa ttaaagctgg ccgtctacat aaaccgtcca tggagcggca aggatttgcg     360
   ctctgccgcc tgcccaagag ttccaccgga gaccagatac attgtcagcc tgtcctctta     420
   agcaaaagtt ctgcaggaga gccagcggag aagcttgaga aggagccagg aagggagtga     480
   gttccacatg ccccatgctg aaaatgatca atgctctgga ataaagaggt gtcttttcca     540
   gccatgcccc aatctactca tgagaagcag gggctgggga acaaatctcc aataggaaga     600
   cctcaaaagt cctttgggtg gacacagagg ccagttgggg cacttgccac agcttcgggg     660
   tgcatcttgt gtcaagacct tggcacgctg aagaagccag tctgcactgc agaggatgct     720
   tcagccagag gatcaattct gtactctctg caaagagctc tggagctggg ggtgtgcctg      780
   gagacgccag cacttttctg tctcctgctt cagtccctta tcctggcaga ttaaaaaaaa      840
   aagagtcaac agaacgatgc tgcccactgc tcaggcaaag gccccttgga gtaccttggg      900
   gggaggccct ggcttcgaga acactgttct agtgtgcgct gaaaagatgt ccctatttag      960
   atcgtgcctt tcacgtggct attcttctct aaagttattt aaaaagagtg ctctgggggt     1020
   atgagaagag cagaggacga gagcagaaac agttttgtaa tgaggaaggt ctgggcattt     1080
   gagctccagc catggataag agcagtgctg agttccaggc ctgccacagc tgagtcacag     1140
   gtcactctcc agtccccagt cctcttggca gtgaaacagt cccaactcca caactcctga     1200
   aggataggcc tgaagttgtt atcatgaaca ggaaccagaa tgttccacga cattttgcaa     1260
   acaatatcgc taaaaaagag gctagtgcta cagacagtag gtggtccact attttccttg     1320
   atccaactcc ttctacagca tgctcctagc tcctgggact tttttgagcc ttttggacaa     1380
   aactagaaat tttagtaaga cgcccataaa caaccagtaa cacactggaa acactggctg     1440
   tgttggagaa agtgcttccc agagaggtga gaagtctaca gggctttgaa tgccatacat     1500
   cgaaaagtta gtttcccaat gtgctttgtg cagccttgat tcaggaagcc ggcactatag     1560
   gcagacttta tacatttctg ctaatgtctc tcaacatttt ggtgttactg atagcttcat     1620
   aggaagatgc aactgagaag accaacaagc aaagactctt gcccacacgt cctgccagct     1680
   agcacaatcc cttgggttaa caattaggga atgttggcaa aatttattca gctccttatt     1740
   tctaccctaa gccccaattc cttcctcaaa ttcacagtaa gatgtaagta tacttgccag     1800
   ttgctgcctg tccactccat ggactatcca gcatgtgaac gaataatgag gatggtagca     1860
   ggtgccgaaa ctcctcgcgt gctatggagt caaaccatcc agaaattcca tctgcacaga     1920
   ctgggctagg taaacctttc tgattccaag ccaccaaaag tagcccctat ggatgaattc     1980
   ttcatctttt ctcagtccca tgcagttctc ttatatatgc tgatctaggc ttattgaagg     2040
   caggtgcgct ctttaatata tgtgggcata gggaccccct tccatcccat aaaagaatac     2100
   ccatcaaagc aggacgtgcg tatggccctt cagttgtcca ggaaaatgaa gcagctccga     2160
   cagatgcatt gttgtgtgta ttagactgag ctatgaacaa tgtcttaacc tttgatttct     2220
   gtgccccaag aagcatctac ctggatgtcc caaattcaac gtgaacagaa ttgaacccat     2280
   tactccttcc aaagccacaa aaattccaaa tttatttttt tctccccatc agggtcccac     2340
   ttcacccctt gtgagccagc tgggttcagg agtaaaggag ggtccattgc gcagtgtgac     2400
   tctaagccca gtgatgatcc tgtgtgtcag gaagaaatcg ttttcatcct aaggaacagc     2460
   cgcatgaagt gcacacactc cctgcacatg acaggttctc cacattgcta cttaattcca     2520
   actaagactc acatttgcta tcactaccag atgcccctac ttgtccagtt aaagcatttg     2580
   tgtgtgtgga gaatgttcat tcatctaagg gactagaaga aatgggatat cctgtgaggt     2640
   tcagaaaagc ccagcaaggg tcaggggaca ccactgcaga agaatcattg tcaaatcatc 2700
   gggagtcacc atgctgggag gtctggagta agatctgtgc catctgcctt gcccaatcct 2760
   gtaccagtgc ctgtgtttca aaatctcaaa ataaaagtga atcattttac caggggcaga 2820
   tgtgtatctg tcctctccgt ttcactagtg caggctggct ttactttggt ccatcaacat 2880
   acttttgtca ttctactaca gggactgggc ccttattact gtgctgctca accaatgaat 2940
   attaaaagtc acgtccatga accatgagat ctcaattgca cagctttgat gagttctatt 3000
   cagtaagcat cctggatggt ttccccagta gagccagggt cccagaaact cctgggaatg 3060
   ccatcttaaa accatagatg agctaggagt ctgcaggggg cgtaaaattc ataccaagga 3120
   gaggagttag tccttgaaag attacagagg aggggggaca taccagtcag gtgaaacact 3180
   cactgaattt atgtagtaac gattttcagt aagtggcaaa atcacaaggg attaacattt 3240
   gaaaggccac tgactttcca acctcctccc cagaaagtat cgaatcttgg aattttattc 3300
   ttgccaagca ggagtcctca atgctggaag caccccaata gtcgtaggag gtctcttcta 3360
   cctgatttgg ggagcaaagg tgtctgccgg tagccctttc ctatttgcaa agttgtacta 3420
   acaggacact ggagcatagg aggagatttg gggaagcctt tgcacgtggt aatgagccat 3480
   tccctctact ggctgagcta gttcttccta agtgtagcct acaaattcat aatgtctaca 3540
   tggatctcct tttaggcgaa agcccggcaa aatatagttc tttaaacctc tggacaaggc 3600
   tcaacatttg cccagattct aatttgagac aatgtaaaac ttacatctgc gaccacccaa 3660
   agctgttcca agtatattca tgatgttcag attctatgtt tgccaatgga gaatgcatgg 3720
   aaatctggac acagctatgg gctcttcact tcttggggcc tttaactcac aaggcccatt 3780
   tctcaccata acaggaagag catttctcaa cttccctaga aatcattcag tcacacgttc 3840
   aacaaatgcc tactagcagc cagtgagaga aaggggcagg gggaagggct agaaagcata 3900
   gtccattcaa actattcaga ccaaatggta cacttctcta ggatcccctg acacgggcat 3960
   caaggaaatt ctggcggaat attgtgagtg gtgtctgaat ccaaagcaga gaggagacca 4020
   gtaggctaac cacaactctg ctgcattttc tgcccccggc ttctggaatt tactgcgacc 4080
   aaaaccagat ttttcacctt ccttagttta gcttctgccc agctaaacct ccacgtcaac 4140
   taggaacatt ggctgctttc cctgaccctt cgagttactc cctggcctcc ccacttctga 4200
   cccgtttcaa tatggcgcca gcatcatcac tcactttgtc tgttgcacag ctcttcaccc 4260
   attcgccagg gcaatagagt caatactttg taagcaaagg ggcagagtcc acgtttgatt 4320
   gaactttcat tcatctcatt gctgtctgca gttttgccct ttttcgtgaa aacagctgct 4380
   catgcagccg gagtaagcct aaaatcgttt aggagtccat agcaaaaggc taggacaaga 4440
   cgataaggat tgaataaagg taatcccaag gaggtgtggg aagggaatct gtacaagaat 4500
   cccagcattc caggactact ccagcttgtg cacaggaaag cagagtattc agttggcagt 4560
   ccaggctcag aagaaggctg ccttattttg tcatgtcatg gctaatgata gagaaatcag 4620
   agctacattc cattggcaga aagggctcat ccttgctgca tgtggaatta cagaaagata 4680
   tttcttttca cacatgcaca gtcccgttac ctggaggctg ccatctgagc agaatttggc 4740
   acaagcttag cacaatgcct cacacctaaa agtctgaaag tgttgcttga ctgactgatg 4800
   caggaccttc cagcagggca aacgtcctga agaaacgcag gagctatcag caccgttccg 4860
   tgttgggctt ggctgtgaga aaagacagcc tgcgtgctgc atctacacag taacagacct 4920
   gccctcgttc actcactgac cccaggaaag ctgtagactg gacagcatcg aacgtcaggg 4980
   gagtctactc atcataccat 5000
<210> 18
   <211> 5000
   <212> DNA
   <213> Artificial Sequence
<220>
   <223> Nucleic acid probe
<400> 18
   attttgggag ctcatgaaac cagttagtag actatggagt gggatctcga gcactggagc      60
   aaggtagatt attcagtaag acacagaaaa catatttcaa agcatctcag caatcagacc     120
   agatttagag ccttacgtcg agcaacgaac tccacatttt gagatgaggc tgtgagtcca     180
   ggcgggctca gaaaattacc ggtgtggttt gtcgctcaca cgtgtccctc tactgttccg     240
   ttggttaatt ccagtgagca ttcacttgca ctcctgaagt gatgacctta acgtttcacc     300
   ttcgcaagcc accactgtgt cccaagaaac accagctctc tacacagggc agcccctgat     360
   ctaatgtcac cagggggcca cccatgggat gtaatcacaa ttaacactgg tccagaaaac     420
   tgggtttgga atccagtaat ctgctcatcc tgatctctct caattccaac ctccagtagt     480
   ttccaaggaa tagccactct ccctggaatg atcctgggat ctggaagaaa gaagcaggtt     540
   aggacaactg tcaaatgaaa acaagtaact ctcttgatgg caggaatgat ggacaagcta     600
   aaacaatcgg gcctttgccc actgaacagt ggcataattg atactttcta ttctagggac     660
   aaggaggtgt tgaggaaata tgggaagagc agaaatcagc cccctcccca gaggtcctgg     720
   gctcagatat ttgcacacat ctgcgtggga gctccttgca gtggatagtg gaaggatggg     780
   acaaaagaga aacagaaaaa accctgtgtt ctccactgtg ccctaaagag gacagagatc     840
   catggaaact aacatggaca caaaacacta attgtcatgg aaggagtggc caagtggagt     900
   acaagaggga tgttgctatg caatttctgt ctgcgcaact tgcattcatt attggggatg     960
   agattgatag ggctgaactg taaagctagc aaatggaaga ttcgtttcag gatcatcctt    1020
   gctggtaagt agtaagtaga gaatcaaacc caccagatcc taacttgtga ggccagaggc    1080
   accccccccc cgacactgag gaagatggga ttgcaacaga tgaaccgggt caaggagaaa 1140
   gggctgttca gcagggaccg cacgatgttc aggtgcctgg gacagtacag ttggctggag 1200
   gagctgactc agcgcggaga ttgtgaattc tgctattgat gctgtgtcga gttgaatgaa 1260
   ggagctgccg caggctgcac agcatggaat ggagtgaaga cacaaatata aaacaggaga 1320
   aaagttatga aaatcctcca ggccagtgat ctgcatagaa cctcatggtc attgtcccca 1380
   ttcagtttac ctttttataa agaaacagat ttgtatgtag aagaattggc cattgctaag 1440
   aaatcctgag tcagaactcc ctaaagaacc aaagaggtag gaaagtcctc tgtgagccac 1500
   aagcaagatg ggcaaaacac agccatgtga acagcaggtc acttagcaac aagatgggaa 1560
   gctctctgtc agtcacttga ctcaaatatt tcgtaaagaa gggacccacc aagggaagat 1620
   tctaggttct actacagaga aatggaaagt agaacaccgt ttaaccaatc ctagtcttag 1680
   gggatgctaa atagttttgc acggcaaatg ttccattcac atctcctgaa tgaagtctct 1740
   tacactatct caaggcaggc tgtcccaact gcaaacaagt tgtccaaatc agcaaaggag 1800
   agggacctaa tgtggactca tctccaatca tctggactca gctccgtcac aaacaagctc 1860
   tgatgagttt tgtgctgcca cgtggtacct cgggattcaa accttagaac agcactcctg 1920
   gtatcctgtc tctcaccaga cgtgggctaa tgctaacacg tccctccatg ctccacctga 1980
   atgcaaattt gtatgtcacc agtttgcagg aggcattcaa ttcacaaact caatgtcact 2040
   tccaccagaa gaatcccagc aatctggtca gtgtgccttg tggggaagtc tgccatgacc 2100
   tgacagaggt catcaccagg cccaggggac caccctgcac cagggaacct aattgttggc 2160
   gaacgtgggc acttcctctt taatccacac tgtgaatatt ttttacagac atatcagaat 2220
   tcatggagta accttcagag aagcaatagg taacaccaaa ggcagcccta aaaaagcaaa 2280
   gacacaggaa ggttgccttg aatttcagtg gagagagttg atgaaggtca ccacggtgac 2340
   cacgcatttt ggtctccatg gtgacacagc tgataaagac ccttaaattt cattttcttg 2400
   aatttgtatt agtcttttct cctcccattc cctggacagg aactcacaaa ggctccatta 2460
   gccacacaat taccagggaa atgtaagcaa atgagagaag ttcccacatg gaaagggggt 2520
   tgactcacct gcgggaggaa acccatgcag gttctgcctt cacctgtcac agagcagagg 2580
   gccagagcct aggagcaggc agtgcgcaag gcatggctgg gtacacagag aagccgcctg 2640
   cagaaggagt tccctggtca gcttggccag tgaacacact ggtgattaag agtctttaaa 2700
   gtttgtaata tggggaacac aacagagcac acttgaggag ctgttttgtg tgacagacgc 2760
   ccaggctgag atgcttggaa agtgagtggg tggtgggcag catttttgtc cttggctttc 2820
   caggcaacac taaaataacc caaactggat gtgtgaagca aggtgtagct gcggagtggg 2880
   ccactaagat gaacaacgga tcctacctta atatgagcct attcaatcca cctcaaagtc 2940
   acagtgtcag acgtgtagaa gacactgtat cagcccatga gggttaaata aaaagaaagt 3000
   acaagggatc agtaggaggt ttaaggaaat caactgggac tctgaccaat ccactgcctt 3060
   ccatggattt tttccactgc ctaatgcaga cacaagagta caaacacgat cattatagac 3120
   acgtaaatag tttcagccag ccgactctag aaggaaagca tgggacatgg gagaatagaa 3180
   gaaatgaatt cagatggaaa aataagcaaa acctcctgag aggagctacc accgtttcag 3240
   gccagcttca aagttggaag tgttaggatt aatctacatt tcaacagcta gggggcatgg 3300
   tttaaaatgt gtatcagagc aaagtgactg cgactgggtg atcgtttcgg cagcgactgt 3360
   catagtggta gatgcgctcc atgcatttag cacaggccca cccacatatc ccataatcag 3420
   aacccagatg aagcttccat ctacttaaag tccttaaacg taactccgac caagcccttt 3480
   tgccagagtg cccgtcaact acctaccatt cagggtggtg aaacaggggc atgaggaaca 3540
   cgatcagatc ggaaccatcc ctcgggccac aagcggagaa tgggtgtttg agatgagcag 3600
   tgctagatct ctgagagggt cagtctagca cttggaagcc aggcctgggt gaaggattac 3660
   gtttggtgtg ctagcgcatc gctagtacca ggctcgtggc ccatgtaaat ttaagatttg 3720
   tactcaagga acagccccct gttgctgaag tcctggtgtt ggcaggagaa tgcccccaga 3780
   cccgacagcc accctcccat ggcactcccc actgttgcac gtggaggtct acagagaaat 3840
   tcacaataaa ccgaggcacg acctgctacc aaaggaagat gggggaggga aggcccactc 3900
   taaaaagcca gatgaagaaa gaattgtgat gtcaatgtac aacccagctc tgctgatctg 3960
   attgctaaga ctgctaagca cctattacct aggtcacttc ccaatttctc ttccaatctg 4020
   tcccctttca aaaagtgtca attcctcctc atgaccccgc ggtatctgtc ctttccatga 4080
   catctgctga gggctgtgga tgctcctggc ccttcttgct ttggcggata cacatgggcc 4140
   agatgcttgg gccaagtcct aacccaggtg attagaatct gactctttcg cttctggtcc 4200
   tagcaccagt gacacagagg agtcaacatc ggacaaatct gcagtggctc ttgcagaagg 4260
   atttggaatt ggtatcacct gctcaaaggc ctttggggac aaatttgtaa tcatctattt 4320
   tcagagatgg agttcactgc tgcccctgag ggaatctgca aatgtggcat cctatgtgct 4380
   gtcaggaatc tagtcactcc tgaatcccaa gtgtctgcaa tagccctaga cagaagtatg 4440
   tggccatctc atagcaaagg acatcatgaa tgtctgtatt caaagtgagg gctaggaccc 4500
   aacctaagca caagagctga gagaaaagaa gttaacctct gaggcttacc caggggccag 4560
   aactgaagac taagtggtgg agctgagaac atttctttca agataaagaa ggtaataaag 4620
   ataagacggg gtgtgaacct gaatgcaggc ctcggagccc agacaggtgt gcaagataca 4680
   agaacgctta tctaagacac aaaaagagat gactcctagg gaattctctc ttctccccaa 4740
   acccaaaata atccaatcag cacattccca ggtcaaaaaa atagaagccg ggttgaaaga 4800
   atctcggtcc ttttaacatt gcagtccacg tcacagcaac gtggcctctg ctatcagaac 4860
   aacacgagag gacctaaatc atctttttgc aaacagagga ggcaccaggc aaagcaaacg 4920
   gggtctatgt gtcctctgca ctttaggggc ttataataga atgctgcata tactcacata 4980
   cacagtcagc tgccaggcat 5000
<210> 19
   <211> 5000
   <212> DNA
   <213> Artificial Sequence
<220>
   <223> Nucleic acid probe
<400> 19
   ttatcatgga cttgggtaaa tgctgtgcta aatcttgggt tagatttgga gcttgatgct 60
   gtgaacagca gagggattgg aatatggggg tcctagcttc agaagccatc tgcattgagg 120
   agctgtgcag caatctgggc ccaatccaac ctggcaaagc cattcttaga attaaagaga 180
   tggaagagcc cgctaaacaa ccccctggac aggaaattgg atcattacct cattgattct 240
   ggctcctggt gggacagctg atttcacggt gggcacacag tgtcatggct aagaataaat 300
   ctctacctag ggctctcaac aggagagtga gaagtttcga gcaaggccag tcacaggcgg 360
   cccctaccct cctgtatacc ccaatgtctc tacatagatg gaacttcaaa tttcacatta 420
   ttcaaaccca ccataatctt ccctaggcca gctcttctta ctcatgccta taattcagat 480
   attgccaaac ccattccccc tctttgctca cgtctgagtc tcttagtaac cgtatttttt 540
   ggtcttcaat aatcacccag aagccctgct agtcagactg tggactgaac ctttctcatt 600
   cttcttagat accattttca catcgctgtt gtactccgaa acccttaaca gctttctagt 660
   gcctcacaat taccatctaa acatttctgc ctgacttgta cctgttctct gaatgtctac 720
   agcatttcca gcctgagccc acgcaatctg gcacttgttt gtaacatctt ctaaaagtct 780
   ctgacattgc atatgcttta tcagtggtcc tctgaggatc tacgttttct caggatgaag 840
   gaatgaggcc cagacagtgc ttgctggggc tcctcggtga gcaaacgccg aagctgaacc 900
   tgagatatga gttagtcaag ccgatacagc agcagaaggg ctaatgcagt tgtgggcttt 960
   gttcccaacg gtttcatcag cggagaacag gggtgagagg gcagatggga tgctctatta 1020
   gaatttgtcc cgcaggctaa cgggaagttc ctaatgcaaa tagggtggca caggcacagt 1080
   gaaatttaac tgagggtaga gaggctcgcc cagggccatt gcatcacctc tctggaatgt 1140
   ggtgaaggta agtgagattg tgcctgccca ctgctttgca ttccaagagg caccacggct 1200
   aggagaatgt ccttgatggg aaaacatcag tctatagttg caaggaaagg gggctgactt 1260
   agatttggac tccctgacat gccaggatca caacagcatc catttaggac cacatggaat 1320
   acagaatggg gccaaaacac agaaatctgc ttttgggctc atggatatgg attagaggtg 1380
   actagactat attttcagtc aggtgtccta atttctttgg atgtgggcat cccagtccta 1440
   cagtttcttg agcaggtacc atgtgtctat agattcattg acatagaact cagcttggta 1500
   tgaccacaaa cagtggatct aaatttggca catttcaagg tcaagtgatg cagcaaacag 1560
   aatctgacgt caaacaatat gactttgtcc attcacccgt tccttaatgc acatcagcag 1620
   gagggcattg ccatgtactc caacttggac cacagaaagg caagacatcg tcacaataag 1680
   tcatgcgctt ctagctcata gtccactggt ctaggtaaaa aatgtgtgaa tttctggtgc 1740
   cgtttagtcc tagtcccttc tctcggggtc ccatctttat ttaatgcaac atagtatcgc 1800
   tgttcccatt tttcctagag cagctatgtg gtgaccacac tgggagggag agcctgtgcc 1860
   tgtggcaagg ctcagatata acagctttaa caccagtata acacgcccca ggcaaaaatg 1920
   tgtgcattga agctggcccc tagagaaata aggtgacagg acttactggc ctttttgctc 1980
   ttctcactgt tcaactgaaa actgaccttt tcactgccag ttaacactgc cctatttccc 2040
   tagcaggcta ttagagcatc gttcacgcac acacataact cagaaaataa agccaattat 2100
   gctcaccaac catgtggtac ccagtctgct gccaaggaca gaacctgcca aatataccac 2160
   aggacaaact agacaattga gaggtcttcc ttggaagctg ttatgctgac accacctttt 2220
   gatagattaa tagaaatggc ccttcccctc ctcaacactg tggtgggcta tgtctaattc 2280
   tctcaaggat aaggtgacct acttagcacc ataacctttt agtcctcggg ggaagccctc 2340
   tgcaacgggg gtgtcaggga tgcagacatt tttctcagcc ggttgtcaca gtgccactga 2400
   agaggaaaga ccacagaatc cggagacatc cagaagcctc catgtttcta aaccctgcag 2460
   caaagttgcc ttcttggctg aaaaggtttc tctactgaga agccactgtc acctggttat 2520
   ccttgcagac cctaacttac aagctgatca gacttctcca gtgtgcctca aggctcagga 2580
   tgaggggaag caggagtcag attcctgagc atgttcctgg gaacccacga caatattcct 2640
   ccaaattcgc atcccagacg ggaaatgtct ctgctgagca actaataatt taaaataatc 2700
   accctgccca gagcttcgtg cctatgcagt gttctcccac actggcaccg cagataccta 2760
   gaaggcaaag ggcataatct gcggggccac aagttccctc ggggaacaag tcatcctgag 2820
   gtgctctgca gaacaggcac cctgagctca caggaagctg caccaagcgc caaggctgga 2880
   tttacagtga ggcagcatct acctgcccca caatatggaa caccatgcaa gaaacttctt 2940
   tgactcatgt gtgtctgccc agcagaaaaa actgccccaa atgaggtatt tactaaagga 3000
   tacaattctg ggcaaaataa tgtaagtgct acaaagggat acaaacgaga aatgttagca 3060
   cctcccccat ctctgcgcca tcctccagtg tcacttttgc ccaagtgtgt aatctaacat 3120
   cctctcgaaa tttcaacctg gcagcgtatt ttaattgtga catttgaagg gtcacgtgac 3180
   ctaacctgcc tgcccttcct actcctcatt ctcactgcca cacgaggcat gataaccaca 3240
   aacccagtgg gaaatatgag attccagccc ctgaggaaca agactttcac tctcaccttc 3300
   acacaggcca attatgcata gaaatggaag atggtgattc ctccttcaac tggagaccta 3360
   agtacacagc agccatacaa gcgagatact gtgaggccac acagctgggc ttcccagaaa 3420
   aggggaaaga tggtgctctc cagctgtcta cctggctgcc gtgtcccaca gtattgtcac 3480
   tggcactccg tgggtctaca aaacatggag ataaagctgg atgaagtccc tccagtactt 3540
   actgatccat acagctcccc cttctcattc atcccgaggt agagtccact gtccacgcct 3600
   ctcacccaga gaagctcgaa ccagggctgc cagatcaacc atctcagatc ctgcaccggg 3660
   gtaagttcgg atctgtagca acctgagcac atctcataga cagtttgggt gctcatctga 3720
   ggtcctgatg aagaccggcc tttcagagct acctaacccc agccaaaaag ggaacagact 3780
   taggtttcct aaacaagtta agcagcgagt ggagacggcc ggagcaattt cacactacta 3840
   ggctcagctg tgggcttagg ctcaggggtg agcatgagga gcaaatcctg ggcctcgtgg 3900
   aaaatatgac tttagtcctc ccaacaggac aatccgtcag caaccttcgc ctatggggct 3960
   ttcagaccct cacaggggag aataaacaat gcacttaata gagtcggagg gaaggggagt 4020
   tgatttaatg gactcggatg gccatggcag tttctcttcc attcttcggc cacacacaca 4080
   gaagcgcaaa ctatgtttca tgatccacct ttcatccgca tccctaactc cagctgcaag 4140
   aagcttttgc tgcttcctcg ccacttcacc atcagatttc tcttgcttgg ttttgttggt 4200
   aaacagaaca aatgctaagc agtgttgaat aacaatcatg aaatcctctg cctggaactt 4260
   gagtaactct cattagaatc acagtcttta cctttcccaa aggctaaaat tgtaccctgt 4320
   atgaaccttc tggcaggtta actccgtgac ttatgctggt gtgtaggctc acctcctaac 4380
   atctatcctg gcttctcaat ataacgtaaa attagcaaaa agaggcagta cagcaaggta 4440
   gaaggagcgt attcttcaga ggttctccca tccggcagat tgagggagct ctttatttgt 4500
   gtaccttgag atataactca tcccagccta taaatttgaa cccagttaga gtgctcaggt 4560
   cctcacttac taaacccata aaatagaatc atatggccag cttactcctg gctgtacctc 4620
   aggactatgt gtggagtgac ataaaattac agttgcatgg gacccatctc agacttactg 4680
   ctgagatttc cctccccttc tctgatattg acaaaaatat tcttcccgta ccatgtggta 4740
   tgcatgaact gcaacaaact ccaaagaaac actggttctt attgccagga tatgactttt 4800
   agaccctaac ttcaggggac aacaaaatca cagagattac agaccttcat gctaaacaga 4860
   gagtggccct tctgaactga agcctcccca tcctggacac cagctcttgc cgctggagtg 4920
   aatccagcct tccttatgga ctagcgtcag gtgaacgccc ttctgtcttt tgcccctcca 4980
   ttttcccttc cgaagtgctg 5000
<210> 20
   <211> 5000
   <212> DNA
   <213> Artificial Sequence
<220>
   <223> Nucleic acid probe
<400> 20
   ttaactcagc tgacgcacct gtgagttacc ataaccccat gacctcgcca agggcccata 60
   tacatagtgc ctcctttgct ggaacttccc tcaatgcctg ttcaataatt gaccctagca 120
   tgtcgctggc cctgagcagg tgccctataa atattagttt ccctctcttc tccctgctcc 180
   tctgctatca aagctgttgg tcttacaaca cagcccagag aagcgatgcc tctggtgaat 240
   aaattgacac agaagtgtga ggtacagact ggcccccaaa atattgttac caggccatca 300
   gctacaagaa taggtaaagg gcagcattgc cccagggata aatgttaaca ttaacctagc 360
   ccgcacaaag ggaggggagt cgacttcaga tacaatggtt gtccatagtt tgaagcgcct 420
   acccactgga gcacaactct tatttcacgt ggctccccaa catatctgtt catcagaatt 480
   gaattcgttg ccttcgagca ctcttcatct ctgagcctaa gaacctaacc ctgactgctc 540
   ttcaaaattc caaaaatgca ttttcactac ctgctggact tccctacatc attattacaa 600
   tggtgtctag tgtgctcatc actctgccct ctggtcccag actgtctttg atcttcctca 660
   cggctcatcc catctgctgc ctggaggagc tggaaggacc atagaatgtt ttcctgagcg 720
   gctgggttct aggaaggatt ggcggggtaa ggtggtgtgt acccactgct catagaaaat 780
   gtgagtcaaa aatatgggtt taagttgcct ttcaatagac agaagatgta ttaattgtca 840
   gaggctctga ggtactgagc cattcattat ccctggccaa ccatgagaat ctctccctcc 900
   ttaccaggga ttagttgtca gctccgggcc agaccctcca ggaatggtac acaggctact 960
   tgtcggggtt gtcatgaaag gaggaagtaa aatatgacaa acagcacctt ctgtatggac 1020
   tggtggcctt taggatgcag agctgccttc ccatccaata ctggcagcct taagataggg 1080
   ctaatgtcat gtccttatct aaggtagaaa actagaataa tgcagatcag aaagcccaca 1140
   caattactag acatgagcca cagagtcaga actgaggaaa acatgatcac aaccaagatg 1200
   ctttgcctct gccaaacacg tcctggcccc taggtctttg cccccatctg aagcactccc 1260
   aggaaactct caacacccaa aggttgccat gaagtcctca aagtgcaaaa gaaactcagg 1320
   gcaacccagt caattggaga aagaagctct ggagaaaagc cagatcctgc tgccagatgc 1380
   ccagcctcct gccctgctga gacgaagcaa aggcacatgt taagagatgg tcacaataat 1440
   cacagggcaa ggtaatggga gctgcactgc gaggaaggga aggtaataag atgcagacga 1500
   acaaatgtgc agagggtcaa gggggctgac aactgttcca cagactggag atcattgtgt 1560
   ggcccctgcc tggccctcag gacactccaa gccagtcccc gggggttggt ttataaactg 1620
   catggttgaa aatacatccc agcccaggac tttacagctg atgatgaaga aatgaacaaa 1680
   gctggagccc aaaaagacca caggttaaga tgggctagtg atctggaatc atggacattc 1740
   agtcaagctc atttgtgttc acagggcaga agtaaaccaa accttgacca cagtaaacgc 1800
   atgcaacagc caggactgga ggtatcgttc taaatctgca ctggggaaaa cctctgggtc 1860
   cctgatgata gagacatggg gctggtgtgc tgctgtctct gcctcgcctc ttgctacctc 1920
   tctggtcagt gaagtggcat tcacatccgc aggtggtttg cgtccagtta cccctgtggg 1980
   atataccttt gttaagcttt ttcagagcct tttatagtca cactctgacc tgacactact 2040
   caggcacctt cccactaaag tgattcactc actatcccta gaacaagact taaataccgc 2100
   cgagacagtg gttctcatcc ttttgccacc acggtggaca ttcctttcag catcagtggc 2160
   tgaatacagg aaggattgag aggacctata ggttgaaaac cccctggagc tgccatggaa 2220
   gctgttcatt aaagctgttt ggaagccacc caccagtgtg tcctgaaggt gcagctgaag 2280
   tcactatgcc tgcctaccag agggatctta gtctgctact ggagcgggta acccagatag 2340
   ttagggggag aggggataat ttacaaaact gtgaaactga accatgagag ctgagatgca 2400
   tgatggtagt agtactgctt tgactgttgc tttagcggga gccccatgca tggagctgtg 2460
   actgccctac tgcaaaggaa aaacaggaaa cagctgagca gcaaattttg tatagggtta 2520
   ggggtggggc atgagagatt tggtgataga gaggcaaact gctatcagtc agtattcagc 2580
   catgagcttt aagaatcctc atatctcttt atcctcccca tccaaactga tattctaact 2640
   gtgcttactt aagtctacaa gccgttttct ttgccttagc tcagtttacc aagcctaacc 2700
   ggtgatatgt ccaggaagga tactgtggtc caggttagag cagtgtctca ggaaagcaat 2760
   ccagaatgaa agcctgctat tggatttcac agataatatg agggatgaaa ttgaaaaaca 2820
   gataaggtct ggattatcca gaagacttca gtggccctct ccaaccacca gatgaatggt 2880
   atttggcagg gatgaatggc gtttccttgg taggcttgag ggaaatcagt gtcatcctca 2940
   gtcctccatt gtgcactcta aaaaccatca aatgtgctgc tttgagacct agcttatcta 3000
   ttgctgcccg agaacttgcc tacatgcttc ctggcctttt aaactggaag tagtgctctt 3060
   gccctgctaa ggctgattcc tccgccccct tctctagttc ccatgatttt agcttaagtt 3120
   ccctgaaggt ttttttcata catccacgag cctgaggtca cagcttaaac ctctgttaac 3180
   ttcttaataa ttagccaact gtgtagttct tgtctttgcc accatatact gtagcttaat 3240
   ctgatttggg gctcatcttc ctgctctgta aaactgagta tcaattatct aatgtgataa 3300
   gtattcaaag gacatcgagg atacactgtc agggatccaa acagctggct gtggatcaac 3360
   ttttttcaga gaccttggcg ataaaggtaa ttttggtttg cccttttcat gataaactcc 3420
   aggaatagaa aaaagcctag agagaaagct gccagtgagg ttgaaaacag atgtagtgga 3480
   tgtccaagag cagaaaacaa tggtagtgcc tcaagtgctg cctatgacct ttatatggaa 3540
   ttaccatact cctgctccct gctaaacagc ttccacttct cagctaccct actctatgaa 3600
   cttagacttt tctatactta gacttctctg tacttttatc ccacgctgat tggggtctca 3660
   accctgatcc ttccccttct ccatccctcc aacacatcta taaagagaag aaaagcaagg 3720
   cctccttctg gcatcaagcc cagtggagtc tagaggtttg gccatttttt tcacattgcc 3780
   gtattccttt gaaaatgaga ggcaattaaa gattggctac attctcgtgc tctaaatact 3840
   ggcttctggc agttccaggc actctctcca ttgcctcaca ttcctgagaa taacattctc 3900
   gaaggaaatg tgtttgcaca agtgggacct gaaataccag tgccataagt cacaaaatac 3960
   catccagcta cttcacttta cctcagggac aagaaattga aggcaataga aggcatatcg 4020
   ttgttgttgt tttcacagag ctacactgat gcgcctttta ccatacagtc cttattgtac 4080
   ctacagcctc tactcctgaa gtcccaccaa gctccagaga ggagcttgct gaagagtttg 4140
   ccatgacttg tctcagggtt gtcttctcac cctgttaatc tacttattgc agaattgaag 4200
   cctgaagaag gccacgtgaa gggtatttgg tcagatgtgc ccatcactct ggcagcagag 4260
   taaaggatag gttttatgag agagaagacc attcagatat ctacgtattg ttctggggga 4320
   aagcattgac tcttgggcta ctcaggcagc tcctgatggc atttacccgc cctgcatcag 4380
   tgccttctga ttttgttgtg ataggatatt tctggatgga gacacaagga attggtaact 4440
   catgtctggg gaatggaact gggaatcaga gtggaagaac tcttcatcat ttatccttca 4500
   gaatgaatga atcctccacc attttctctg tcaaccacaa aaatgctgac tcctcacctg 4560
   tctttccaga tgcccttatg ggtcaagatt aggggcacaa ttctgatacg gttagaaagc 4620
   agaacaggtt ctgggaatgg aaacaaattg aaatgtcctc ccaaagtttg gaaacaaggt 4680
   taccctccag gtgttaaatc tgaggacctg aagccctcgg aactctcacc aggcttttct 4740
   ctacttcgat ctgggtagca atttcattct cttttactgc atgtacacca gagaaattct 4800
   acagtggatt gttactctaa gctgtgccat tctgtaggct aagaccagct taggagactc 4860
   caggggaccg cagggcaaat tacactcaac actagaggat gagggagtac aagacagact 4920
   tcaaagtcag agttccatgc agataagcac acagatccgt agacagaggc ggtagaggaa 4980
   aagtgagtca ctaagttttc 5000
<210> 21
   <211> 5100
   <212> DNA
   <213> Artificial Sequence
<220>
   <223> Nucleic acid probe
<400> 21
   aggttataac tttctgagtt atttaccagc cctaggatta cagcatagtc ccagtgtttg      60
   ttagcaaaac ccatcttaga gtggatattt cctgaggctg agtattgaaa aactatagtg     120
   gcacaaagaa ttgaaaaggc aaatacagcg ccatcctgta tcaattgcat atgccttgtt     180
   ccttaatgtt tcttacaatt tgatcatgaa atttgggtgg gttccccgtg agtcatgagt 240
   acttgaagtt caaatggcag tcctgtgtac agacaaattt tcacacacca tatgtcccaa 300
   gccaggtaaa aagtggagta taacatgaaa gaataggcaa aacggcctgg ctgaaagagg 360
   ggatttcatg ataggaaaca ggaaaagaga aggaaagaaa aagtcaagaa gtttccattt 420
   ccttctatga aacagtggac agtcattaag aggctttttg agtagtagag ttctaagaag 480
   gttgctcatg tagaataagt ttagaaggta attattacag caccacaagt ggtaacaggg 540
   tccactttag tagtgatgat agcagtacca aagacgggac caaaacaaag gaaatagcta 600
   gtttgatttt caagcatgtt gtagttcaag gagcagctga gctttcaggg agtcaagtct 660
   gggcctgtta catggaaaag acaatagcag agagaaagat gagaagaaca gaaccaaacc 720
   tagaaaatac cctatttaca ggatgcaaga ggaagagagg ctattgtgga ggccaaaaca 780
   cttaatggat ctcagtgcag gacagccaag gagatagaaa ggacattaca ggcaaaaagg 840
   agaggaactt caaacacatg ggatatggct ggctcacaga gggcacatgg agatgcagtg 900
   atagatttaa cagcttgcac cagtggacat aatcatgcaa ctaatccaca acagatcctg 960
   gtggtgaatg ctaggctgac tccaaatctc agactccatt taggcatatt cttccaaaac 1020
   cacaagtcaa atgtctgtat gccttccacc agtcatcatt tggagaaggt gtttttctca 1080
   aaagcaaaat cctaagtgag tttttggaag gtctgtggag gagaaagagc attcatcaca 1140
   ggtttcccag agagaagaga taagtcagtg tactgctgac aacatgagcc agggttaagg 1200
   aagctttaga ggacaagagg agctagctgc aagggtgacc acacaagact tcaaagaact 1260
   agtggccaga taccatcatt tgaagagacc tgaagaaaga tcaagccatc ccattacata 1320
   gatgagacta gcttatcagt tcaaatcaca actttggttc cagtttagtc agccccggat 1380
   tgtgagaata gagggattaa gaaaaatatc actgaggtaa aagcagcaat gtttagtgat 1440
   tggctgaata cggcaaagga gaaaaaggag tccaagatgg atgaagctct tattctacct 1500
   atgtggtgtc aaaactatct tggtgtcctc attagattgt tcttgaggcc agggacctta 1560
   taaaaattcc catgcctttt cccctggcta aatttaaaac gctatattcc gctccttcta 1620
   cccagtccct ttaagaggtt tcagttgacc tgccagcttt ccattccgtt ttattagttt 1680
   tgccttggat gctgtctatg tccatctctc tgctatacac aacaaaaacc cccaccctcc 1740
   agctcagatt cagtacaagc cctcacttgt ctagggagct agcatagata ctaaaagcca 1800
   aaaatagatt gacctactgc atatgcctgt cccactgtat ctctttggta caaacacctg 1860
   ttccaaactg agacaactag gttgtcttta cagagaagtg taacaggaaa ctgcagcagc 1920
   tagtcttcca catggactaa gcagcagaga aagtgggcct gcagaaagtc aagaattagc 1980
   agatgtcaga aggaagtgga atgtccctga cagccttatc aaaagcagtg gtatattagg 2040
   cacagagagt atgttagggg tatataagcc tccttgaaga ctaactggta gatgagaaat 2100
   agttctgctt tgtttactta catcattaat cagacatgag ctaagagacc acctgttcgc 2160
   ttttttctga cttaaacaat ggagaccaag gagagctgaa tgctagaagc acccagaact 2220
   ggctgtgcaa atttatttca atcctgccca aaactccttc accttaaaat gccttggagg 2280
   tggctctcat caagacaaga gctctggccc ttccctctcg aaagtgcaaa cactccattc 2340
   tcgcttcccc tttgcccctc ctcttccagg gacgacttcc tcctccgcaa tttctcactt 2400
   ttgggcacct cacagcactg aatgtggtca tcagaaatac tgggtcatga cccgtgggaa 2460
   tgggtcttgt aggttaacta gatgcaacat cagggacact cgttccaggg tgataatatt 2520
   tctaaagaca gaaggaggga tccaaatgaa tagtctcctc ctaaatatcc gctggggaca 2580
   tgaagggttt cacaaaggcg ttagtcagca agtactctgc ttgtgaaatg cagtgttcca 2640
   tgaggggact tcccaaacat ggcttcccct cgcccccagc cagcatcact gcgaatgaga 2700
   tgttaccatc atctacatga gcaaagctgg gttataagca cactgtgtgt cagttcaggg 2760
   gaaggagaaa gagcagctct ataccaagca aagtttcttt tagttacaag gagacactta 2820
   gactgggcca ttatgtgcct aggatgtagg aaagatgcct ttggcactgt aggtgttagg 2880
   ggaagctagt aatcactttt gccaggtttg gccttttatc tgacgttacc tggcctttgg 2940
   ggtattaatg tcctcctcgt gcagtgctgt caaggagtgc tctgcagaac actctttcct 3000
   tagctccctt gataagttgc cttagcatat tctttgagca gaaccactaa atacttgagg 3060
   tttgtagcaa tggaagacct gcagacaaac ttaaccaatt ctcttccatt tccttttagc 3120
   ttatcttccc ttatcccact ggcttatctt ctcttctccc actggctacc gcatgtgtct 3180
   gttttctctt ctgcctcttc ggttttgttt ctttttaaaa agtccctcat ttgattctca 3240
   gctccctcac tgtggttcat ggccttcaaa agaaaacgat tttttgttcc ttcagtagaa 3300
   tatctgtaaa ggtccacttt tttggaaaga gaaattcaca gagaagggaa atgagcagtc 3360
   acccccaaag tgtctatttt atggtgtttg agagtaaaat gtgttggaat tttctctcgt 3420
   catgccacag cagtgaccac ctattctgac tcactggctt catgttgagc tttcaaaaca 3480
   agggctagaa aaagttaaag gtccaagtga agaagaaagg acacatgaga aaactacaga 3540
   ggtcaaccac gctgtggtca acattttggc cattttgata ggtaagtaac tacagccatt 3600
   aatttctgtc ctgaaaagta ttttggtttg catacaactc aacttcccat ccctgtagac 3660
   ccaagttcat tattatgttc acccaaacca ctattttatt ctgaagtgga gaaacttctg 3720
   tgaaatggtt agccttgtta gataagaaga agtgttgggt attgggcaaa gctgggcaca 3780
   tactgaatgt gatgttctaa aaaagatggt aagtacttaa ataagtttca gtgacagaac 3840
   aggaggagga attgtcatct gtgcctaggg tgatgaccag tttcagggga tgtatcccaa 3900
   tattgagaag ctggtcattg tctcattatt gttctgcaaa aggataccag aaagcactgg 3960
   aataagtgcc gaagtctttt atacactttc tggtaccata agcatcaaca caatgactgt     4020
   tcccagaagg gcagtaggaa tgtaatgtca tgctgaaaaa caggcaccca caaggctaat     4080
   gcattcatag gctacttgat aagtactttt gtcctctaaa tgcctatttt acagttggca     4140
   aagccacaag aagggcattt ccagggtggg aacatctggt tcattttacc taaggaagtc     4200
   tctgaatata taatttatcc accccctcca acactgataa tgctcatagc aactcaatga     4260
   cctcagtgtg agaacataag accacactag taatagatat acctttcttt ttttgcactg     4320
   ctagtatctc tttccctcaa acttcctcac tctctctgat atttctccta ttttcacaac     4380
   agctatgtaa gtggcttaga agggaaaagg acgctataca agagtactga ccttgaacac     4440
   acaatgatcc tgcccacagc ctaagaaaca tactctagcc aaacagagca gccatttgtc     4500
   gactgctagg agcttataac cccgaaccct ctcctccaga attcaccttc cctatcatca     4560
   catcaccctc acactgtatt taggggtttt gtatctgctt gtgtctcaac ttatgggagg     4620
   aaggtcaaga gaaagatagc tgctccctca acacctctgt aagacctgcc tttcagttca     4680
   agttttccat ctgaagctct tcagcaatga ggacaagtag gaaagtatga cggtaacaaa     4740
   taacccactg gccattcaca aagatcttgg tgcctcaggc aagtagtcag atgagtttgc     4800
   aggacgtaaa tagcagttcc ttatataaag taccaggttt tttccccatt ctctggaaat     4860
   gtttgtcctc ccttaatttt gcctcaacac aagaaaataa taccctgagc tattgttcct     4920
   gcaaagtgga aataatagag agggacgttc ctgcagactt aacccaatca aaagtcacgt     4980
   ttgtgctttt tgtgcatctt tattatatga caatttccca tcagtccaaa atggcataaa     5040
   ccccaacact gaaaatttag cagttgaaag tacagtcacc tgtgcctact tctaaagtta     5100
<210> 22
   <211> 5100
   <212> DNA
   <213> Artificial Sequence
<220>
   <223> Nucleic acid probe
<400> 22
   ttcaaggcat gaacagaagg aagcctagaa ctcgtctcct agcaccttgt tcaccacact        60
   gtgcttggtg tgtgtatgtt gggtggaggg aggggaaggg acagtccaat tcttatcgta       120
   aacttgtccc tgatggaaac tgctcatctt gagctgcatt cacactccca gttcatgtag       180
   tgatgtgata gtaagtataa ctgctgcatg acctggtgtt tctatactgc taatgactga       240
   tgcaagtaga cacatgagtg atgagctgtg acaatctata tcaatcatta cacaatctag       300
   agtgtcccaa cagcagtact gcaaattcac cagcaggaag agttagtgca tgtacccagt       360
   gtggtaagga ctgtgggtca gagaactagg cttttagttt aaacacatcc agtaaaaaca       420
   tcagaaaacc tagaaccatc tcaggttaac cacctgcccc atgtgaagag tcagggtaca 480
   ttgtgggagc gagtccacag cacaggaaag gtgaagtgaa ggttcagaag tgaagggtcc 540
   acctcacagg acaagagaag catgccctga gcattttggt cgctgttttc tttgatttta 600
   ttacatccat caacaacact agctaataca gtggaagata aaccggccct aagcaaatcc 660
   acagggtgca agttggtcag cccatcgggt tggttaaaat aattgaggca taagctttag 720
   gcactggtta acgattggtt aacaaacgaa gactcttgaa agggtaagtg actatacaag 780
   agttagagaa gaatagctcc ttccccagaa ttctcagtac cccagataaa cccattctct 840
   ccctgggttc cctgcctctt ctagcaagga ttaggagcat tcccaaaaat cctgtggact 900
   gcccatccct cttctcttag tccagagctc ttacttattt ctacgcatta tggccgtatg 960
   aaggctgttt gtaaacctgc aaaatacaat accaggtatc atgcacgagt tattgaagac 1020
   agctgttgta agcatttcag gacaatgcac ctcccttaac accgagtccg ggatttgggg 1080
   cggggtgccg gaaaacgcca tggagtcgat gcttcctctt ccaagtcagg tcggctcccg 1140
   ttaccttctc agcattcgcc gttccggtct tcctgagcgc gtgcatgagg tctttcgcgt 1200
   cgacgtctaa tctgttaaag gtaagaccct cactccagct tgggttttac tgtgtggccg 1260
   ccgggttcgg ctctttggga aaggcacggc tcaggttcag caaaggaaaa accaagaaaa 1320
   tgctgcctaa caggtttcag tgactcgacc ccccacttct tccatttctg ttctcgttct 1380
   gtctgcacga aattccgagt tgagttcatc tttttcctta ggaaactgct ttctaacccc 1440
   cacccagtta caggacacaa ccagatgaca actgatgaag cgataccatc gttttagggc 1500
   actactcccc ctctttcttt aagaaaacta attcggggaa ttgagtaacc catcccactc 1560
   tgcttttctc ttttataatg gtatagcaaa tttctcatgc agtttattgt tccaccccaa 1620
   acagcaattg tttaaacctt ttcttgctga gcctcacccc tcttaattct tagtacatta 1680
   ctttacccag ctatttgaag gttcttctct gttccatgct acctcccaaa ttgtctcatt 1740
   ctgcttgttc tttttcagtc ctcatcttcc ccccaggact tggtggtatg tcccctctta 1800
   acattccctc cttggtcttc tctctctcaa atcacgtttg tttttcactg acttccctcc 1860
   cctgtcccca atagaattat ttctcaggat tttatcttca acttagctgg ttacttgttg 1920
   gctcactagc atgccagagg tcgtcatctc cctaataact aactcttaac tttcattctc 1980
   tctccttctg attggatcta tgatttatga taaggcaaaa gtttcagatg tgttaccaac 2040
   tgctctatgc tccctcacag attgaggaaa taatttctgg tgggatgatc agtgaaaatt 2100
   tattctatat gaatagtgtt ccttgggatt gttgcagcat agaaacccag aatacaggga 2160
   aacaacaaga gggataggag tgaaagggaa gtcgggtcag agagcggcct tcccctcctt 2220
   cagtgtactg tcctagagag atggttgcat tcgacacagc ctagggagga aacaaatttc 2280
   aagggtgtct gtgttggtca taatttgatg tggtatgttt tgtcccaggc gttatgtagg 2340
   gatttctggg tgttttcggg gcgaaccagt aggtcaacat ttgtattatt ttgttgtaaa 2400
   gaaagatcta tgtaacatct tatatttctg gctgataaca cttaccccca gcccaaacac 2460
   attttctttt tcctaagcca attaacacag gacccaaacg taatgaaaga attcaagagg 2520
   ctttgtagca agttaataga gatgaggcag ccttcagtca gtcagtgaaa acgtacataa 2580
   atcaaggttc atagtctgaa tctattagga gatttcagag cctaaccagt gtttaattta 2640
   tctccagtac ctgtctactc tggtactctg caccttttat ttttaattgc aggacttaca 2700
   gttttgaagc ttttacgcca ggaatctgtt acaaaacagc agtggtttga tctgttttcg 2760
   taagtacccc actaattctg tttgctagca taaaggaaat tgagaatagt gcttgttctc 2820
   tgagtactcc tgacttagaa aaggagcata gccctactaa aggggacttc aaagtagaaa 2880
   tcgtcaataa ccttttactt aggaaatcaa aacctctgga atagttttta cttcttgctg 2940
   cttttcctgt cgcttctgtc tgcctcctta agacttttga gaagcttcta tttcctccct 3000
   tgtttgttta cctactttat attcacaggg atgtgcatgc agtctgtctt tgggatgata 3060
   aaggcccagc aaaaattcat caggctttaa aagaagatat ctgcatttat ttcaaattgg 3120
   tacacctctt tttttcagcg agtactgagc catcaagatg atacggcttt gctaaaagca 3180
   tatattgttg aatggcgaaa gatggttaca gtaaaacact caggcattac aatagtgtaa 3240
   cctgggcatt gttgccccat cccagcctta aaatttgatc tgatgttgac agggatactg 3300
   ttcaaacata aaaaacagac tccaagatag tgcaatgaag ctggtacatg ctgagagatt 3360
   gggagaagct tttgattctc agctggttat tggagtaaga agttcttttt gcctatgagc 3420
   catagcacaa ctttgtagga ggtatcttgt ttagaacaca ttgtcctttg gagctggtat 3480
   gcaagatcaa atctatttgc acagtaccat ggtaggtgtt ttcttttacg tggaggactg 3540
   accttagggt gcttcagtat aaggaaagtt agaatctagt tgggacagtc ttggaattgt 3600
   tgggccacag agttgtccct taggaagatt aatctggtga caggaaagat ggaagactgg 3660
   aatggggagg agctgggaat aagaagtaga aagcatttgc aatggaaaag acaagggaag 3720
   agagaaactt cttagaggtg gaacattgta tcacatggat ggggctagat atcacagaga 3780
   atcaagccaa taatgttctg agattgctat ggatttagaa gttgtgctca tctcaggcaa 3840
   acatacagga gttgatggca tatggcaaaa agtttttttc ttcacggaat ctagcaaaag 3900
   ttgttagatt tacctatgct caatctggtt tcactgctta aggtggaagt gtttagttgg 3960
   gcctattcca gtgatgttat agaataagat tactcagcag tgactaattt ttgatatatt 4020
   tcagctcatg gaatgctgtg taaatgccct ggtgacatca tttaaagaga ctatcttagc 4080
   tgagtgccaa ggcatgatca aagttcctaa tggtatagag ccaatgttga aagacttgga 4140
   ggaacatatc attagtgctg gcctggcaga tatggtagca gctgctgaaa ctattactac 4200
   cctcttgata ggttgaatga aggagggagg tagatcactt tgattgttac agtagttcac 4260
   acctgagcca ggcattgcaa ggagagatgg atggattctc gttatagtct cagttttgct 4320
   aacctttaaa gtagtgtatc aggactgggt atttctcaga gatggataat tcagtaggcc 4380
   tctatctgta tatgctcata gacccagaga gaaaatcagc tttgaaaatc ttaagcttgc 4440
   aactgaactc cctgatgctg aacttaggag gactttatgg gttggtttat gtttttttgt 4500
   tctaaaaact ttggtctgtt tgaggacttg tagattgtac tcattagccc cctgctcaca 4560
   actacatttt attactactc catagacctc ttaaaagttc agggatttgc ttgactggta 4620
   atgacagtaa agggagtgtc atatctatgc agttatgaca gtgtcctgga ttcttgcttt 4680
   tggcagctat gaagtagttg gcctcttccc tgctctcttt cccctgctct tactgagctc 4740
   ttgaagcctc tgagactcca ctacatcttc tgtgtggctc cttggcctac atggcacact 4800
   agtgtccctc tgctctcttt tcctcttagg ttctctaact accaccttca cctctatcta 4860
   aggagaaact gggatgttta caaatgcctc tgcttttcct cttcttcctc tctgaacccc 4920
   tgacataaac cccaatttat gatgatcagg agcctggtat ctgaatacca tcaagggtga 4980
   gaggaattca cttgaccttc cattagttag catgatggca ttcccttcat gttgcacact 5040
   ctttgacagc atgctgtttt gtggagaaac ttgcattcat gaagagccca ttatcaactt 5100
<210> 23
   <211> 5100
   <212> DNA
   <213> Artificial Sequence
<220>
   <223> Nucleic acid probe
<400> 23
   aaaactagaa atagcactct tggtttcttt tgatgaaaag agtgagctgg tcaatgcaac      60
   attgggaaac ggcagatgta ggtgagaagt taagtattta ggtattttaa ggtctcatga     120
   ttagggattt gtagcctcag gtttgaatca gtcacctagg ggtaagagaa aaaccacaac     180
   ataagacctg aaaaacagaa aatcacagtt ggcatccttt attcaggtct cttctctcca     240
   gcgtacactt aaaattggtg ccgagcaggg atataacctg cagttaagtg aaaagaaaat     300
   agacttcaaa ttctaattca aaatgagagc agtcaggaat gtagcttttc cactgatgat     360
   agcatgtggc tatatgcctg gaagaggtaa taaaaaaaaa agaatatgga ctttttatat     420
   cacggaactc ctgttttgac agtgtacaac ccagagcagg gtgacaattt agattaacaa     480
   ttgatggtga gagttgagtt accacacttg attccacttc tgtcttttct ccagaagctg     540
   gcctttgttt acctctgctt ctcctgtgtt cactgaacac acacactctg taacatgctt     600
   ctgggctggc ctacattttt gaagtggatt acctaaatgt acagagtggt taagccgtgg     660
   tcagtaagat tatgtttgct tttggcattt ttgaaccatt aacatttctg tgtgcccact     720
   gaagaagctc tgttgacact ttgtacctgt ctcaggattt cagtgaagga agtgtcttag 780
   ctatgtgcat ccctccagga aaaccacttt ttgagtagct cttcattaat acatctgtcc 840
   agacactatt gcctgctatg acaggaatga taatccaaag ccacaaattc agatatgcaa 900
   agaaataaat gtactattct tctcccctca atgctcctaa atgtctctga acttctctag 960
   aagctgtcct gatgaacata gtggcacttt ttcctggtag atcttttact gtgtgatacc 1020
   ctagtcatca gctttggcct ctcacaactg gtaatctcta tgaggccaga cctttagttt 1080
   tctgagtcat ttctttaaca taggccttgg tgagctgttg aatagagagg ttcttcggtg 1140
   caggaatcac aggggacttt aagggacaaa cctggagtga tggggaaatt aaagacaggg 1200
   ccagcagcgt gtcaggtgct cgttgacccc agcgccagtg tctccatcca cgtccttcac 1260
   tcgtcaacct ttcctggccc aggctgcggt gtcccagcgc ccaaaaaccg cctaagtgct 1320
   ccgataacac ccagggactc gcctattttt acagcgcgtt tctacttcca aggtctcggc 1380
   gtaatcatct taatgagcgt aggggcagag tctcaaagaa tttattctcc acatgcgtaa 1440
   gcaagggagt acgcaccaca gattgtcagg caaagcctca ggaataggta gagaccttca 1500
   atgtttgttt ttgttgcaat ggtaggtcac tctagttctt acgtctgggc gtagaataac 1560
   attacttgca caagggtgct ggcggtagga gctttgcaag agtctaaaat tgatcctggg 1620
   tgactgagat aatagaaaag tcacaaggcg taggtttcca gggaagatga gtttttagcc 1680
   tatttgttag tctcatggga cattcttttg cttctcagtg tattcatggc ttttctcttt 1740
   gttctaaccg ctgcttttct gtggcattgg ctggtcagct ccagcccctt ttgacttttc 1800
   atcctagaag agagggagtg gaccagttgg gaattagaca aggagggcca ttacagcatc 1860
   tcctaggacc ttctgaagag tctctgtttt gtaagggtca aagtggtatt gccccaccta 1920
   ggatacaggt cttgattcct agccagagtt cctttcagta agggaccctc ttagagttgt 1980
   ttattatgca gttaacaaga gctgccagcc actaagcttg gttccattgc aattcaggga 2040
   gccattgaaa aggcaggtca gtagttactt ggctttttgt gttaagggag caaaaagatt 2100
   tgagattctt tctcataagt tagtatgttt tctcacatgc tcaagagtgt ctggatttgc 2160
   taagtccatt tgaagtatgg tcaaaatgat agcgtaggct ttttcattat aaaggagaac 2220
   acggtgctta accctgggaa tcaaatcatt gcttgagccc aggcttgatg actaatttgt 2280
   agtcattaag ttaagttcta gagagcatgt ccaatgttcc atatgtaatg aacagaggat 2340
   caacaccata tggtggggta aagcttgaag atttgattgt aaaagacggg ctaactgatg 2400
   aaaatatttc aactttttat cagggcagct gtgctgagaa tacagcaaag aagctgaata 2460
   ttgcacgaaa tgaacaggac gcttatgcta ttaattctta ttttaaattg aaaagggatt 2520
   tactagaagg ttaccgggag tgcctccaag aactgaagga tgaacagtag caaccattca 2580
   gctctgggaa cttcagggat tgttcactac tactaagaca tgctctcttg gcttctcaca 2640
   cctgcaggct tttgtgcttt attctctcag cctttctaaa gttgctgcct ctacctctag 2700
   ccaatgccag tacactgaat gatggagcag ctgctctggt tctcatgacg gcagatgcag 2760
   cgaagaggct caatgttaca ccactggcaa gaatagtagg cccccccctt tttttaaaca 2820
   gcatttgctg acgctgctgt agaacctatt gattttccaa ttgctcctgt atatgctgca 2880
   tctatggtga gaacaaagtg ctggctgatg gttccagatc tagaatggaa agcaactagt 2940
   cactccccag gagaggactc cattctgaaa gggaacagct ggcagcaggt cttcagagta 3000
   tattccaaat tgtcagtaag ggctatccaa gtctcagtgg gaaattgcta agcagtcaca 3060
   ggaaaagaaa gttttagggg ttcagggttc ttagaaatct ttggaacagt ggaagctgtt 3120
   tgagctgtat aggctagaaa gctaccctta gtccactagt agaagagagg aagatggagg 3180
   aagagttcca cgttaatttt cagtaagagt tggctattct ggacaaacta gcagtgcttc 3240
   atataatcac tcaaaccaca gtgtgtgcag cagtactaga aacaagacag aagcccatgt 3300
   tataaaaaag ggaacagatc accacttcca atacacgaca atgcctgttc ttaagcagga 3360
   cagactgtaa cagaagtatc tcgcattgca ttttatctgg ctttcaataa ttatctgccc 3420
   cgacttacaa aaacttctaa ctgaaccagg agctggataa gatataggag aaaaggaaac 3480
   ccaaaacatg agaatacttc cttgataata tctataggtt cttctttatg tttttcccct 3540
   gcccctgagc caggtgtccg gggcacaggt aaatcactac tatcagcaag cctacttact 3600
   cttgtttttt ggtcatttct tgcagtgaag ctatagattt ctcacttcgc aaacccccat 3660
   tttgctgccc aatagatagt tttgaatttt ctggattttt cttttctgat tgagtttctc 3720
   ttatggtggt atgccggcta atggcacttt ccgatttaga taaaatctta ggcagaggag 3780
   gcttctcttt ctctcttttg tgggtgagtc aagattagga aaagagactg cattcctttc 3840
   tttgttttgg gacaccatct tatggtttgg cccctgcgta tttgccacag gagcagtagt 3900
   cggtacctat catgtttaca agcctatgtc actcagagca catgctctgg agttctaata 3960
   gccatatttt aatttccacg agcttcagca tttatgtggg ggtgagaaat atgtagtttg 4020
   aaggccttga gttacacaaa attagcaagg gtggagaata gtttagttat ggctccagaa 4080
   gccaccatag tatcaacagt agtggtaggg aatggtagca cttctaaggc aatgctaaga 4140
   agcccaggtg taaaagttga gatggctggc tggacagatc aagggctata gagtagctag 4200
   gtgggctcta ggctgtttag taaaggaagt gaatccggaa agaagacaca agttcaggta 4260
   aaaggtgagg attcaagtaa gaaaagctat cactggttga caccattttc agttcataaa 4320
   tcttacctgc cgaggaggag tagaaaagtt atttccattc tgtccaacca cagatactgg 4380
   gatcatccct accattcctt atattaaggt ggtcttaatg ttattcagtc agtgaaccat 4440
   gctgatcaag cataagactc tacagccaag acatcatatt acaatcatat ctggattcct 4500
   taagcacgta actggtcaaa actatggagg gaaaattaca atgtctacat ggtcctcaga 4560
   agtcaacaac atagtttgtg gctactgtaa tttagtcctg taatacacta ctgtcaggtt 4620
   gatcccctca ttttcatgca acctactacg gtctctctgt ctatgctggt gctgcttctc 4680
   tttataactt acagatttta gctttaactc caaatcctac cttcattctt taagctttag 4740
   ctcaagcccc acattctcta ttaattctga cctctccttt atgtaatttg taacagcacg 4800
   aagttacctt gtgaaaagtt tggtgacaca gcttggttga cagcaaatac actgtttgac 4860
   cttggtggtg tctgtaactg aggtggtaga ggttgagcag ctgacttaca gatgttccta 4920
   acgctgttgg atcagtcaca caggtagcta tcagaatgtt atttgaattg ccaaaagctg 4980
   tgcttgtggc tggcatcaac tgaatgttaa aagactctgt tccattgagg ctgaatcccc 5040
   tacttcggca tatacaacag cacctacagt ttcttctgaa gataggcatt taactagttc 5100
<210> 24
   <211> 5100
   <212> DNA
   <213> Artificial Sequence
<220>
   <223> Nucleic acid probe
<400> 24
   agacaagatt atagttggca ggttttctcc attaatacta gaaacagcac tggtaagttc 60
   agtatctgag gaaacaaatg gatcatcact aatgataact gttaaagtca ttcaaattag 120
   gcacggactc aaaggtaatg tcaatgtcac acttctgttc agtgggtaca gctgttttaa 180
   aggccttttt ctgtatgctg tactgcaaaa aacacaaccc actataattc agcctaagaa 240
   gaaatggaga attggacttg attgttggca gaggaattaa aggaggcaca cagtctgaga 300
   gctcactctc ttttgtattt actaccaata gcaacagtga ccattgtccc catctttctt 360
   ctcttagtat taagcctcac aggcaagttg tcatttggtt ttgctgtttg ttattttata 420
   tggtgaagtg agaaaggctc caataaagga ggctaactaa ggtcagtctt gaatgaaaga 480
   caaccaaaag taaaaactct taggatttct aacatgtaaa gatagagaag tagaaaggca 540
   ttcctcattc caggaggaca gactgacatc aacaaaggca aggaagtaga aaatataatg 600
   tggttgacca ctacaaaata tgacctcgat ggatctgaaa tttggccact tggtcgagta 660
   acctgtgtac ctgtggaagg aggagtggta aactgtcctg gaagtaattg tgaacattgt 720
   ttaaagttgg ataccaacct ctctgactgc cagcaaacct tggggaactt tgcatgctcc 780
   taacaaagaa aacaaagtag gcatttggag ccattttagc aggttaagca gaggaagaaa 840
   atgaaaccca tgctgaagtt aggtggggta gtcacttata gctctgaaag aaaggttaag 900
   gtcaggaaga aaaaaaaagg cttggagaca gcagcagcga aactaatcag aaacgagggt 960
   gtcattttga aactaactct cggagtcaaa gttgtaattc tattgaaaat gctgtagcat 1020
   gtactgatgt cacaattgaa accttaggtg gaatgctcta gtgtttggca gccttgaaca 1080
   gaaacaccag gttgtgctgc actttataac gttctactct cttcacctgg aactgtatcg 1140
   ttaaccaggt ccaacaggtg aaaaacctaa taaggttgcc tacaaagtca ttatacacta 1200
   gtcatctcgt tacaggcaat gccctagctg tgatttggga atcaactcac cactctctac 1260
   ttgttgatga atctgttttc caacctcttg ctaactaata tacttccttt tcatggtgga 1320
   gtttcaagta cttatcaccc tagatgcctt cagtcacttg gggacttctg atagacggtg 1380
   gtattaaaat ctactataga agctgacaga aagtatatat gcaatcatat ttggggaaca 1440
   tgtccagttt ggatgcagca caaggtttat gaagggtaac agttggagat acaacagaaa 1500
   acttcaattc gtttggcatg tctgcagcat aaaacagatg ttaggaagtt gagatcaagt 1560
   tggagaggta ggtaggatca gttccacaga gcaccttgaa gcaataaggg ctacttaagt 1620
   ggagtagtga gatgcaaaag tcagagtgca gtatcctgaa ggataaaagg taggtgaaga 1680
   acagcaacta agtggaattc ggagatttgt aacaacagta aataaaatga attgcgagga 1740
   caactgtctg ggaagccatg acagaagtcc aaaagagaag taatggcagg cacattgcgg 1800
   gaaaaccatg gctggcgttt tttgtccatt aaatgaaaag caggaagtca gcagagtagc 1860
   tggtttgtgg agaaagaata ctcacacatc gatgtgaatt tggaaccatt tccgtttaac 1920
   gtttcctgca aaatgtgctt atcaagagcc ctgcgcaaat aacatttttg tggacagcgc 1980
   tgggcattta aaagaggcac aggatggaga aggaagatca cccctactcc caaattcgta 2040
   gttataaggg cgggttatta gaagggcgcc aggaaggtct ctctacaaag atggggggcc 2100
   tattttggtc ccaacactga accaaggaaa ttgcgcccaa ggttgaagca cacgcaatcc 2160
   gtccgtcatg cagacaaatg ctctttcagg ggtcctaatt gcagcctctg aagagaggag 2220
   catctacata caaagaggct taaactgccc agaacctccg aatgacgaag aatcaccgcc 2280
   agtctcaact cgtaagctgg ggaaaacctt tggcctcaaa ggtccttctg tccagcatag 2340
   ccgggtccaa taaccctcca tcccgcgtcc gcgcttaccc aatacaagcc gggctacgtc 2400
   ctcctgcgcc gcatctcctg gttccagtgg cggcactgaa ctcgcggcaa tttgtcccgc 2460
   ctctttcgct tcacggcagc caatcgcttc cgccagagaa gcggacttgg ccaataacct 2520
   cctcctttta aacgccctga attgaaccct gcctcctgcg catcctcttt ttgtgtcacc 2580
   ttagggttca gatttaacta gacctgtggt gagcactcgt gagcgttagc tgctgtattt 2640
   accaggtaca gataagacaa ctacagtgga tgataatgta tgtggtgata ggggagtact 2700
   aaaatgcgtg taaaggcaca aagtgtagaa ggaccctgaa gctccagcgt gaggcctggc 2760
   attgaatgaa atatattttg tgggttttca gctgctgaag agaatggcat gccaagggca 2820
   gagttatttc taggaagatg ggatataagc gtcattgtca agttgtgcaa aggggtcaac 2880
   ttggttgaga tctaaaaagg tcattaagct gaaggtaaga aaaacaagtt caaggaagtc 2940
   acaccatggg gaaaaaaaag tcaagtttag ggctgcctct tccgggaagt gtcaaatctt 3000
   gtgaatgaga gaaagataac atgtcaattt tgtagcccac cagaaaagtt tgcaatgtgt 3060
   acagtagttt tgccttgcag tgaacaaacc ttttaattgc atttttcctc atgaaatgaa 3120
   acttcaccaa agaaagcact ctgtctgtat ctgtctatat cccccaagtg acctgacagt 3180
   ttaacagtac tttagtaaaa ctagggagga atctggtgtt gtttcctgtt tatagctggg 3240
   aggtactgag agaccacaaa ggaaggaagt tatggtcaga gattgaaata cttcagtttg 3300
   cttaggagtc atttgtagcc tttacgttac tctgaacaca agataggctg tgtctgtaac 3360
   agggccctca acaagcctag agacagcctt tgggaatagg cattttacat tatcacttac 3420
   aaccatggct gtcactgaaa caaaggcagt ctttccccta gtctgcttac tatattctgt 3480
   catctagttt gttctctgaa tgggaaatac aaaaaaatct gagacattgt tccctctagg 3540
   agctacttat gcatcaatac ttagatacta aggggtctga cacagactgt agggcaaaag 3600
   aagagagtat tgcccagaag gtatagtgct tttctgtagt taggaacttt attggctgga 3660
   actggagttt cctcttgctc tgtctatgat tgcctttggg atattgtcat agcaagttaa 3720
   gaaggaggac tggctctctg ataggcaatt agaagtgtgc acctcatgga attcactgaa 3780
   ttgtacgctt caacaataga aacgaggaag acaaaacagg atgtaagcac taggataaag 3840
   gaatggagtc cctcgtccac ttaacctttc cacagattgg taattggaac caaatgattg 3900
   attgtattgc tgtgactgaa aatgactcac tggcacaaac attccttatc tccctcaaga 3960
   gggcaaaagg atgcgtaact gcttacctat accagatgtt aatgattaaa taaagcctac 4020
   aatccattca aaggagtgaa ttcggtagcc agggaaaatg acttgaggac atgagagggg 4080
   acagtgtgcc tctttatgat atctaaccat gtgggccatt ttcttagccc tagccccagt 4140
   gtatgtggag gtagagcctc tggcctagcg agtagcaagt tgggtcctag gtcatgtccc 4200
   acatgcctgg tgagtcccta attagaacct ctgatcacag taaattgtag gctgagtcat 4260
   tggtggaatc cctggtcatg tttcccaccc ttggtgaaaa cagagtgatt aaatgttaga 4320
   gcaggttgac attggaatct gtggactcaa ctaattggtg ttcggccaga gaacagttta 4380
   tcttttctgg ctagtttgag tcattgtact aacaagagtg aatgacttgt ccaccagact 4440
   gaccaggctt ttggaaatag tatttattga gggaaacaca tggagacaag agcagcatag 4500
   cattgggatc cagcagggcc agaagtcaaa ttttagagct atcaggcttt taagttcagt 4560
   tatcagggga gaaggactag ggtccaagcc ttataattac tctaccagca tagtttccat 4620
   ggtgctttgg agtaagccag cctataatca gttagccttt tacctctcag tataagactt 4680
   gttcggtgtt tcttaaaagt accttcactc cctcttcaat ctcaccaagc tttccacttt 4740
   tcaagaaaaa caaccgtccc tcattcctat agttctcata ccctgctgtt tgtttttttg    4800
   tggaacagaa ataggcactc cagaggttat ggaatttgag agagatactc agcctctagc    4860
   cactcccatt caatctccca gcttagtctt ctgagcattc cactcaagta gaagtctggg    4920
   aagaagttac gcagggaaca aagatgacat tgttcgcagt ccctgggaat tcttaacagt    4980
   aaagttctgt aattgagaca ataaagtcaa gagtaaaatt cagatagagg agcagttata    5040
   catcagtggg aaatttggtt ggcagaaaaa ttaccaggtc atacaactta atgatagacc    5100
<210> 25
   <211> 5100
   <212> DNA
   <213> Artificial Sequence
<220>
   <223> Nucleic acid probe
<400> 25
   aggtaattac ctatcctctt ttaccatagt ctccccacag taattactct ttctaaattg      60
   accacttggt tggccaaccc aataaccttt tcttagtctt gtctaaactc taaatgttag     120
   atgttaaaaa gccaagcagg ctcccaaagt cccttctagc tttagtgttg aatgattctt     180
   gtgcttctgt ttgtgatttt atctctagtt taggtgttta gatttagtga ccacgggatg     240
   gagaaatcac ctggttaaag tcagcttgaa gctctcgtga tggtttttgt ttctaattag     300
   caacatactt acctccttgt ccatagtggc actgctttta tggacagacc tgatttacaa     360
   cagaagccct aagtggcttt cctgtgtagc ataaaattaa acagaccttg tttttttaga     420
   aatagaaagc gctactttgc tcccatgaaa gctgttagct ctgggtatgg tttatagtta     480
   ttgtgatcca caagtgttta gacagtgaat aaaacatcac cttctctgca atactacacc     540
   gcactcatac caattcagca gagtggtacc ctgaacacac atacagctta tttctcatta     600
   gagattttta acagtgcata cagaaattgc tagaatggat ggtctgactg atgagtactg     660
   cttgagagtt gaccagaact ctgaagaaat agtgtagtag agctattctg tgtatcctat     720
   tcttagtctg tggtacatgg atttcttggt agagagcaga tatgaacccc aattttgtat     780
   caggtgatag tctacaggtt gcggttatac ccatttgtgt acagaaatgt agttgataag     840
   gaagtggatc ggtaggaagc cttatatttg aagaagggtg ccaaagtata actgttccta     900
   gaaaagggtt ttcgaaatga gtatcatctc gcatagtttg ttggactgag gcatgccaag     960
   aaaagtatag gcattcctct ttctacttcc tgctacaaac cctccttcaa aacaaattgt    1020
   ttctgggatt ccaggttcat tctttaccct gacccttcaa gaaagttttg gtgattccct    1080
   ctttgttctc ttcctataca attacctgtt ggtttttgca aacttatgtt gctacttcag    1140
   ctgtaaaccc agcatacaca ctggataaag tagaggcatt ggtgtcccca aggtttgtct    1200
   cttttttttt cccacccacc aaggaaaaac atttttaacc tgcttttttc cccgtacatg      1260
   aagggcagtt gggtacagtc atggtaatgc attatatttt aatggggcat tagattggag 1320
   gatttagaaa gcagttaaag atgatgtgat cacaggtggg ttttccccct gctgcagaaa 1380
   tatgggattt ttacaataaa ctcacatcac ataagttact cattttctct ctctaattcc 1440
   tcataggcct ctgccttttt ctcacacatg caggcataca cgctctaccc actgcagtat 1500
   accactgttg agctttgact ctgagctgca tagtggccaa agcccagagt cttcatttct 1560
   caatcagagc ctgaaccaca gattagcaac aagttggggc tccttgaata gtgcaagttg 1620
   acgtcctttg catcagtata ccgggtctcg tactgtgcca agtgctattt aatgtaccaa 1680
   aaagggagag cattcttccc agaatggcag taggtattta attatttggg agactgtcaa 1740
   gaggtgcaca gatgctcaga ttggtttgag tgccctttgc tattctcaga tgactctgtg 1800
   attcctttct gtatcaatct ctcaacaaaa aataagggtt tgattccccc tccccgcccc 1860
   agagctagat attgatagag agtaatcaat gtaaaagaga atcaagcatc aggggtcaca 1920
   gctaactcaa agacagcagg ggagagggca tgtagaatac ttattaggca ggaagggaaa 1980
   tcagaaaggc catgagatca gaggaagtgc ttagttctgc ctggggtggg gagaatagac 2040
   agaagtagat ccaagaaatg cttcacagag gaaaaggtgt gtgagccgag ttttgtaaat 2100
   agatggagtg ttgctttatg aaatgcaaag gagtttgcac cttatgctat aggcctcata 2160
   agaccctaaa ggatagtaat atcagacttg cattttagaa ctaaggaaaa gaccctgggt 2220
   aaaagcaata atagttcctc ttccttccac ccacctggga cctaagccca tgtaacatac 2280
   atactgttat tccaaacctc ttccttgtag ttttcccagc ataggtctgt gtactcaact 2340
   tggattgggg cagattgcag tcagtgtaaa ctaataattg ccacaataat caaagactga 2400
   ttttttttct taccaggtag actgtgtatc tcatcaggaa gtcactgatg tgaagagcac 2460
   tgcttcattt taacataggg ggatgtggct gggcagcaga cttaggaatg agggccagac 2520
   tagtttactg ctggtgctac ctcagtacct ctctgctgtc ttaactttgg gacagctcac 2580
   ctgaataggg tttgggcctg ccagtcattc accaatctgg taagggtatg tgagacaaga 2640
   aatcaggagt gtggcctccc cagggaagca tggcaggtag agtgcagtat gggcttgcca 2700
   acctagtttc agtcagaagg gatttttatg ctaaggtgga tttgggagtt tcttccagta 2760
   cttgtatcct acctaacact caccagaaga aggtagttct accctccaat gctttgtgta 2820
   ttttacactc atctacctaa tcttccctcc gtaaatctgt ccgaatttcc ccaaatcatt 2880
   tggtgtgatt gttccaatct gatcttacca cagtaagacc ctttctgcct tcagctttag 2940
   gagggtgctt ctcaattagc atcctggttc ccagtcctct gggacatcat ttctttcctc 3000
   tttcattgag gcacagtcac tatttctgaa ctgatctccc cgcttcctaa gcccctcctt 3060
   cagagcactt taacctgggt gtatttccct tttcttttca gcttggagca ttatgctagg 3120
   atagtttcct caaggaaaca tgaagtgtgc atgatgtttg ttcccctccc ccatcaacta 3180
   ccatgtgact ggcttatttg gcacaaccag tctgataatg ctgcatctca gtctaaatat 3240
   cctagtagtg tttcttagtc gcctcctgac gaaaaggcaa atatgaatgt tctttctttg 3300
   atgggaatcc aaagagttgt attgcatgca gagtagtgaa ggcaaatcgt aggccccaaa 3360
   aaaactgaga ttaggggaat gatctaggaa ggaaaatgaa gcaaggcaca ctaggatatg 3420
   ggataaaggg accgagagct ctgccttcca tgaatgcact aaatgcctca agtaaacatg 3480
   caaaataatc agtctgaagc cagtgttcta tttctgctca atcttcctta cttaactgag 3540
   ctagattctg tgcccagaat atatacacaa gttattccag tcagaataca ctatctcttg 3600
   accattctta aagcaaatat cttcacctga aaccagaaac accccaaatt tctgagttca 3660
   tcccactttc actcccacat atcactgtcc atccctacaa accttctctt gcttcttgaa 3720
   gaagtgacac acattatcct cttctccaat gccaacatct tcaatgtatc accttcactg 3780
   gcttcagtta tgcaaatctc ttctggggtc tgatgacact cttcttcaac atgctggctt 3840
   ttacttttag catttcagta ctgctaacta ctggcagaac aatagtggcc tagaacctaa 3900
   ctgctaaaaa tcacctacct ttgaagttag ccgaagagtt gcttgtagca atttccttcc 3960
   agctggcaat gtacctaagc acattatatg aggattcatt gctaagaacc ttcaggacaa 4020
   ggagttgaag gaggtataaa aatggtgccc accatctgcc atacttagtg tcctttcacc 4080
   tccctctagc atgccccctc cagcgctgtc tttgcagtgt ccagaacacg ttaagagata 4140
   catttaggta tggcaaatgc agctgtcacc tcagctcctg aacagagact gaaatagaga 4200
   gtctgaattt ggctccttct tactactgct accccttggt gctctgcgaa ctttcttcaa 4260
   tggccttcaa taggatactg ctgggtgact taaatgaaag caccttcaag aaaccaagat 4320
   aaaagacctt gatcgatagt gtgctgagcc ctgacaatct ggggcacaaa cacttaccat 4380
   agctaggccc catcactgtc tgatcctgga agaggaacat ctgaatatcc attaggatga 4440
   atgccagcaa aagccacggt agctgtcctg tggcattgtg cataactccg tgatggtgat 4500
   gctctggata caggaatttg caacagtact cattagtgtg tttgagtcca ctagtgaaac 4560
   tggctctatc cattcttcca aatgataata acaatgttcg cactaaatat cagtgttcaa 4620
   ggcatttatg ccattctaac tgtccaactg tagaaaaatg tccatagacg ttgaaggata 4680
   ctttccaagg aattaggggt tctgcagtta gctaggggaa ggcctttgca aagtttggtc 4740
   ttactgagat aatcagtgct tgtggccaag tagtgctact gaaatatacc cagataatca 4800
   taagacttca cctactatca cttagcggat gatggcgggg aaagtcttat gtggaccgag 4860
   atgcctattt tttcttccct ttgaactcaa tggttttatt tatcaatttt ttgtacttgt 4920
   ttgccggccc ttagcttgaa tcggccaatg gtacaaggga aaggcagagc aggatttcaa 4980
   aagctcacct gagaaaattc catcattatt accatctgca tttgttcagt gtcccaggaa 5040
   ctacagacat gctgtctata gggaatctca agactgaggt ctgcacaaaa tattggggtt      5100
<210> 26
   <211> 5100
   <212> DNA
   <213> Artificial Sequence
<220>
   <223> Nucleic acid probe
<400> 26
   agcaacatgt acttactcat caaagttcag tgtatttgtc cagttcagca cttcatccac 60
   ttcccattcc atcacagaat ctatcccccc atcttcaaaa caacaaagac ctaggaagaa 120
   tcaaagctta agttgcaaag gaggttgagg aaagctaaga tctataggac acaggaaggg 180
   actagattta aaagccagaa aggcattgta accctgtgac ccttgcaatg gaacccaggg 240
   ttgccctcaa cccatgctgt gactatgaga taaaggacca cttcagagct gccagggagg 300
   ctaattaagt gatgggtgag tagtacactc agccttcaga agcacatgct acagatagat 360
   caacaaagta ctagtgtact gaggtgctta ataagtgttg aacccatagt accaatataa 420
   caatctaaag ccccactaca attgcctatg gaagatggta cagcccatgt ctatactaat 480
   agtattcttt ctgaaaggag tttctaaatc cagcctctgt catactttct attgcatggg 540
   gtaccatttt cctcccatat ataaccctct tagaggcact caatatcctg atctaattta 600
   gtctgtgtca gtttttctag gcgctagtac gagtatcttt tgttagtatt ctgtctaccc 660
   agtgttctca ctcaggagtg ggaaaattgc ctgggggctt taattaccag gctttctcta 720
   atgggtatcg tcaagtagcc agctattaag cagggtgtct ctcaaatacc aaaattaaga 780
   ggcaacacaa caaaattaag ctggagacaa ctggcacagc tgccccatat acagaccttc 840
   aattaacccc tgtcttcagt ccttctcctt ccagctcttt taaattgtta actccaaatt 900
   tatctgctca tgaaccccct tctctttgtt atggattaac cttctacaca gtgtcgtttc 960
   aatggggttc tggagagata gaaggtgaaa atgtgtcctc tctttattta catggcttct 1020
   ctgctgaagc tccttcaagg gcaaggatgt attctgaaat gcctagcaca atgtcaggaa 1080
   cacaacagct agtcaaaggt gaaaagagtg aggtcatttt acctcttggc tctcacccaa 1140
   ccagataatt caacttggaa tattccttac acctataaga ccagctctac ccttataaaa 1200
   tcaaataaac ctttcttaac gtgcatagag acagagttgc atccacaaaa ttgcgcaggt 1260
   ccctaaccta aaattagacc ttatgtggtc aaatataaaa atgattcgcg tggcctttat 1320
   aagtttcgta atttcgcttt tcctttttac ctgtggtcct tgatgctagg ggcaggagaa 1380
   gaaaaactaa cctattcttt aagaggggcg gatggggaat ggtatcaagg gtatgggttc 1440
   tttagggcct tgaaacaacg aaggctcaaa ccagtgggca caaaggctac cggaacagta 1500
   cctcctagcc ccgccttcta ggccccgcct ttcctctatt cgcgctctga caaagtgcag 1560
   gggatataga ccaaccgctt gtgaaggctg ctggttctgt gcacaggtcc tgcagtggtc 1620
   ctaaaggagg ccagagggac ctccaaccag gcatcaatcc tcttgttgag gcttagcagt 1680
   ttgaagaggc tagaggaagg tttcaaacca gaaagccatt aacactaact ctttaaaggc 1740
   tgcggctgaa ttggcgagat gaagaattat gtagattaag gctgctgtct ccctatagaa 1800
   ttttttaaag cacatccttc ctgttccaca cactgaatct gcctggtttg tctcaacttg 1860
   cttttaacta ttaggcctgt ttggcagagg caatataaat aatgtacaat atttaagaca 1920
   caaattcatt aggtatgggc tcaggacagc ttgctcttat ttaatacttg cgtgcttgtg 1980
   gttttgcaca atggatgggt ttgcctttgt tcttggccta ttattagtga atcccaagat 2040
   agcaggcttc ctgaagattg ttgtagaggt tttcagtgtt ttcaaggatc cccttttcag 2100
   catatataaa gccaccggga gaactagtcc acttggtgca gtcttctata ctgtccttca 2160
   cagcttagat tcaatctttc cttaaagtgt agccgggata ctgactcaaa gttcttctct 2220
   tgaaggcttt ttcctgtggc actggcagat ggctgtgcta tcttctggct gaggaacaag 2280
   ttccattcca tcacgtactt taaccatctg aaaagttggc agtctcacac attctttctt 2340
   ttaggcctat agtcacacgc atttttgaat ggcaacaggt ctgtgtaatg ttcctccact 2400
   aataaaagag agcactgaga agatgggagg agttgctaat ctatatttgt gagggctccc 2460
   agcgtcgttt tacataatat ggaataaaga tgaaaatatg tgaggacacc cgacccagca 2520
   gtgtggacgg acactctgaa ctgacctgca gtacttggta atagtagcag taaagcctgt 2580
   gtggctgtag taggtcccta gaggctacag gaagagacag agtattttcc tagacatctc 2640
   cgttaaagag aaacgatcac atgtttggcc cctcttccac agtactagga agccactcct 2700
   gtaccttcct ggccacccag gaagtactct tcagcttccg catctgtccc accagcctat 2760
   ttcctctctg cataacagga gaatgaatcc gttttcacgt agttaatctt ctacaatgct 2820
   gcccgcaggt aagccatttg caagcaactg aggctcttat gaagggtcac aaaagtgatt 2880
   ctgcctcagg cgcccaatta aacataatgc ttccaaggtt ctagtgccat gtagaaatgt 2940
   tcataatatg gcgagtcctg ctttaaaacc agactgtcgc ccagcatgag atatggatat 3000
   tataaacctc attctctgag tccaaatccc ctgttctact cattgtatca ctctgggatg 3060
   aagaggacac actcccagat ttggtaaata aatttttctt catcaggttt tgtcacattg 3120
   ttcacatctc tcctctgggc ctcgtgataa tgctctgaag gagtaggcag gcatcacacg 3180
   ggtttaaaga ccaaggcacc aaatacctga aataatgtcg gaccgtgttc aaagcaaaaa 3240
   agagattcag tgactgggtc cactcaccaa ggcattagct tcttccattt tctgtgtttc 3300
   cgttggaagg acaacatctc ttgaatccag agagccacac catgaaatga taggtatggg 3360
   gctaggggtt ccattgactt ttcgatgctc caagggccaa atctccggta aacatggtta 3420
   ttgagaatcg tgtaatgaac aatggcacct ctctcatccc caaacctttt ggggacttct 3480
   tttagcattt gctggagatt ttactttcct tttcacacca ccttggataa acagtttagt 3540
   atatgccctc tcccaatcta acctactctc ggtcacttag ctcataaatt catctctaat 3600
   ttactgcaca tgactttgag gtcatcaggt ttaggccatt ctgaaaggca tgggacctac 3660
   acacaaggct gactctggag tatataataa tcacttacct ctcatcaaaa atgttccatc 3720
   attcctgtcc aaaggtttag ggacatgtat ccggaccaac tctttaggtg aaagagattt 3780
   gactacgact ttgaatggtg cagaaaggca acatggcaca gcagatcgaa atctgaagag 3840
   gaaagtctgg gaattggaaa gccattttca ttgctccctc cccaaactgg aaatgtacaa 3900
   cagttggagg aatacttctg gcacttcgga tacaatggag tcatctcctc ctctgttgtt 3960
   taggaagctc attaaggccc tctacagctg cagcaaaagt gtaccatgag gctaaatgga 4020
   aatcctccaa aaatatctgg ctagctgcca acgacgaaca ccctccaaga tcttattgta 4080
   aactgacgta cgacaatcat attctacact gtcgaatggg agtgataata agggaaactt 4140
   tctaagttgt gtacaacagg ctacagcttg agaagatttt tcctcttttc caattagttc 4200
   agctattcag ttggttcttg aagagggaaa gacttcagga cttggaagtg aatgttggtg 4260
   gcagaaatgt aagaaactag ttgggtgagt tttcagaact acttcaggag tggtgttctg 4320
   aatcaaatgt agaaaacaag ccactgaggt agttaaggga gaggcagatt cttgcctcag 4380
   ctcaaatttc tccagtggct ctaaaagtga attatgtttg gttcctaaat tctctgcttt 4440
   gcccagctgt caagaccccc ttaccccttt cccaagttgg gacacacttc tggcagctat 4500
   tctaattgtc tcttcgctga tacaacaagg tgctagttgg ccagagcccc aaattataca 4560
   ttagcaaatc ctgactgaca acagcgtgaa accagcagga cgacgcgatc cgtcggcttg 4620
   atgactcacc actggaaagc gctccctgca atccctcaca gttcagacaa ctaagtccga 4680
   gatggaaggc aacgtgtgac taaaggacgt agacccggtg gtgattgttg tgggacgatg 4740
   aaagggtccg gccccaagat cgatgtagtt tagggaaata tcttttccca aggaatgtct 4800
   ctcttggggg aacttaatgc ccaaaacatc agagccaggc actggacaag cctttgtccg 4860
   cctcacggct ttaaccgtgc tactcagcat tcttgctgtg gcgtttagca atactaagcc 4920
   tgcaccaacc tgttttcttt ctcatcttaa cctgtctcca aagagtatat ctcttcagag 4980
   caaccagtgc tttgaccaga tcagttcata acatttccac cttttcggta actcttcttt 5040
   ttttactcca cagtccgcct ggttaacagt gaggctttgt aacagcataa ctttaactat 5100
<210> 27
   <211> 5100
   <212> DNA
   <213> Artificial Sequence
<220>
   <223> Nucleic acid probe
<400> 27
   tattttggaa actgactcat gctgaactaa tccccttccc ctgggagttt aaaatcaagg      60
   ttaggagatt tttaacgtac cgactgctct ccaacagcag ggcatgcatt atgcaaggta     120
   ttgggtatat aaactcagtc cctgcccttc catgtattac agtctatagg gtacatttta     180
   gactgtgaga ctattggttt ttgtgcccag attcttaggg ttgtataaga acattgattc     240
   ttccacagtc aacatatggc ctgaagggag gggatgtcag tttccagagc aaagaaaaag     300
   ccagactgga aagatgacat aatctagaaa ggatgattgg ggaaaagaac atgggcctag     360
   agggaaactt agtgctggca tttatattat taaaacacaa ttataaatgt gtgaatctcc     420
   agaaagaaat gtcagacctt cttcattcct ctcctattat gcaacaaagc acattgattt     480
   cctgatgttg gttcgttgct taaaaaccac ctatatggca gatctcattc aacagccata     540
   agagagactg gtttgaaaac ttatgcctcc cctgagcccc ctacatttta tatttttcct     600
   aaaagtgctt acagcttatt tgtttcagat atacagaact gccagtcaca aagagccact     660
   aagtggcaat acagccacaa acttgcctgg gaagtaaaat taacctaaat ggatttttac     720
   cccctccagt acacatattt acctcctaca caaagtagct gttattagct tatcaccaac     780
   caaacctttg ttagtagcag ctattttcat gaattttgtg ttttcctgat acttcagcca     840
   gaccaacatg caaacgttac aaatagagca actgaacact ggccatcaat tacctttgaa     900
   ttagttaccg catgttcatt agctttgagt tgcatgggaa ccatgtaaag taacccacgc     960
   attttgcgtc tatgtaaggg aatgattaga aggataacaa cactgccatc tattggtctt    1020
   tatcctgaat taggagagaa atcccccgag gttctgcatg gcaaataaag agttaggaaa    1080
   gccaaaatat cagtgggtat atcctgcagc aaagaatgaa ttccagggtg tcagatctga    1140
   cttgatggca ttttagggac ttggccaagg ttcccacttg atatttggga ctaaaatgcc    1200
   tttcagagga atttctagga ctgctaacct tctattggct tactagggat tcaggtccta    1260
   atctataacc tcaacatgtg ctttgaccat gtgcctccca aagatgaaaa atgttaggag    1320
   ggaagtaaag cagaaagccc aaatcagaca gtgaatcagg gattaagtgg gagtggtgga    1380
   aaggcaggct aagaacattt tgggacattt cagagcagac actgcccaga ctagatcttt    1440
   tatccttcca tgcacatgca catgtacacc ttagttccat tataagcttt tggtgaaaaa    1500
   gtgtctgtgt cacaatttga gtccactggg tcatcctcat agatattaag cggttcactt    1560
   ggagataagc catcttctgg cacctgacta ctgggagaat gagttcagaa aaagatctga    1620
   atcttcgact gtacccattg ttgacagaga attcattccc aagggtcagt cgactcaggg    1680
   cattgtcaat agaagtgctt gatgaagcac tcctttgttg ctgcagaaaa ctggccagtg    1740
   gactgcttac tctagatttc tgtgcaggct ccagtggaaa aggagggctc agcctcctct    1800
   tgtagaaggt tctctgttgg gtagtacagt aatggggaga aggttctcgg atttcttcac      1860
   atgctttccg ttctcactca acctgctttc gccaatggac gccaagagat aaatggtcaa      1920
   agccagatct gcttccactt tgggggaaga tagtagctac atgtcttctg gggcttacat      1980
   ctttagctgg gaattttctg ccttcaggaa gtgaaggttg atcccaagct gacttctgca      2040
   tttcatcttc agccttcctg ctctctgtca tatttgcctc ctgacttctt tgaggtaatt      2100
   actctctgag tgagaaagtg ttttattagt cattttttgg cagttggttc caatgtcccc       2160
   tctgccttcc ctaggctgtg atccactgga gccatcacct aaggcagtca tattttcagt      2220
   ggtgagcgtc tgatcagagt tgacggacat ctttagattt tcacatgaag gtgttcctga      2280
   ctgctctcgt gtagaataat tgttttccca cgttcttttc ctgacaagtc tgagcaaaaa      2340
   ggtaaagggt cttcatttga aagagcaaat acacttctcc tggaggcagc cattttaccc      2400
   tccagttgag gcatgtggct cccctgaggg acatgacata gccctgttga tgaggaatgg      2460
   aagtggtcct tttctaacgg aagtagatcc actgcttttt ttctctctgt ggcttcaggt      2520
   gcttccaggg ctgagtctga aaggactttg gaacattcat tcgctgactc aggagaactg      2580
   ggactaaatt tgtttaacat atgtcattgg atttatctgt gtgcggtata tacattttgg      2640
   aggtatcttt cctggaaaag actctgggtg acttttttga acttattatg gtagatgcat       2700
   aaatcaggta aagagtttga gatccctgtc tgtgaaacag gttggggtat ttcacctgcc      2760
   ttgtttgtct ggtccatctt gcttagctgt ttgtcttctt gattctgcaa gttgggactc      2820
   tgaggattcc ttctgtcatc agaaatctgg gaaaaggaag ttttgaatga ggatgctaga      2880
   gtctgagcaa ttccaaatga ctatccagtg tggatctctg aaaatcaaac tgccaaggat      2940
   gtggctcttc ttggcctctg gaaacatctg ttccataacc agaagaaaat gattcccagt      3000
   tgttctccgc ttcgtccaaa ggtattgctg aagaaagatc tggtaaatgt gttgctttga      3060
   tggtagaatg gcatgttctc tgagttctca aaagtgtcgg ttctcctgaa aacccctggg      3120
   atacacatac ttgtcagcgg gatcaatttc cattggtgat ggtgccctca ggaactcttc      3180
   ctggttctcc ctgtctctag aagcttcttg ctgtgaaatg ccctgtggct ggtaaggccg      3240
   accgttgcct gctgtcaaaa cacagcgaag tactgccaaa agtattcttt tgcacataat      3300
   gatattacca tgtctgttac cactggaata aaagtgaccg aactgtgttc ttgatccata      3360
   gttgaggggt gttctggtat tcactgagct tgcagactgt tcaaagtcag tgcccagggg      3420
   cccccttacg ataactgaag acctagacca agcccagacc cagggctcat gctctctagg      3480
   accacagcct ctgtgatctt taaagccact tagaatccca gattaacaat gctgtttact      3540
   catcactcct gacatcagaa aaattcctgt tctgcttctc tacattagtt tcagaaaggc      3600
   attgcctttg caaactaaat gaagttcctc aaagtgaagc tggtggcgac ttcagagtta      3660
   acttttcaaa tggccgggct tatatagaat aacctttgta agtgggggtt ccttgcagag      3720
   aggaaagaaa tcgacccctc ttgagtttgg agtgctgtct ccatggctcc aatggcttgg      3780
   attttggatt tcctaacgtt tcataacctt gcaaacatta tccaccccat gccttcactt      3840
   tcttgtgcca aaactccatt tgcgtttcct ctgtcaaact ggctctgtca ctcatgactc      3900
   tcatccactt ccctttccag tctttatttt cagctcactc tgctacccag tccttgcctc      3960
   ttgaatccct ctccccacct caaaatttcc ctttctattt acttcttaat ttctccataa      4020
   caggtctgtc tgattccacg gcgctggttc tcttaacccc tacattcctt acgaccttgt      4080
   gttttcactc ctgcttgttt gttctatgag caagccacaa ttgactactc acaaaggaga      4140
   tttattaata cggaaggagt gctcccatga taacttattg gtcactgaat atgatttagc      4200
   acaccaaaca atcttttctc tatgtatgta gaccagtaca tttcttgtgg cccccaggat      4260
   cacattcctt tgtaatgata atatacagtg aaatgaaaag cctctagaga ccctgagtat      4320
   aatttcctct ggttttattt ctgcccagac cctctacttc aagacctatc ctactcaacg      4380
   tgttcaggat gctttcaaca agacactgac tgatcctaat tagggctcag ttttggactc      4440
   agaagcccct caaaataatg ctccgtgctt tggagttgcc ataaaaggtg aagcctattt      4500
   tgttgctgct actattcttt tgtggttata gagaatttcc tggaagactc ctgttgatgc      4560
   tggtagccaa atgaggtaga aaagtatccc acaggatgag caactccaat tcactccact      4620
   gatactgtct gtgattctga cctgactaga caaacaaacc tctgtcatag agtagcctta      4680
   gagtacttga gaacttccta caatctggac aaaaagccca gtgtaagaag tgccacattg      4740
   gaggtgtgtt cctaagggta cagagctggc agtatcactg gacagtagcc agagtttgtt      4800
   taatatagta caacttccca tgaaatagtg gaatctgctt gtaccattcc tgcctggctg      4860
   ccaaccaagt gatgagaagt tcatcacctc aaatcttggg cttttcataa tagctattca      4920
   tggggagcaa cagtcactgg gttccatttc ccttatttgc ccagctcttg ccttttagac      4980
   tgacatgctc caagcagtga tctttcaggc atttgccttt ctttttacat tccttttctg      5040
   cctcagaccc cgtggctctc aaacggcaga ggatggtcag tctgaatact tcccctagga      5100
<210> 28
   <211> 5100
   <212> DNA
   <213> Artificial Sequence
<220>
   <223> Nucleic acid probe
<400> 28
   ctctcagagg gcaaagctgg atggggaggt catgccactt cagtccactg cttagttaaa      60
   gcagagtctc cgcaggtggc gagcaactgg ggtccatgtg ctatgagagg cttcctcttt     120
   gagtacaaat gataaccaca ctcagaaagg atctggtgca aacccatcct ggctggcaaa     180
   cgtggctgtg gctgcctctg ctgcccaaca cctcagggct gtacaggggc aacacgtggc 240
   aaaatatcag agatgccact gatggcataa gtggaaacat gatggcctca ctccttcctg 300
   gagctaaaga aactgggtct ctagatcaga tcaattaaaa gtggcaaggc acagttgtcc 360
   attcaaactt ccagcagctt catggttcat aaaagttgaa cttgggcaca gcaagggagg 420
   ccggggactg gcaagtaatc caatatgcct ggagccagga ctacatgcca gactgtgaag 480
   gttctttctg gaatcctgct gaaaactatg ctggtaaaag gcccctggac ttggtcattt 540
   caagggatac taagttacct catcttttgc atttacccac aagttctatt gatgctgtat 600
   ccacctccag tcattctgcc tgacagtcaa ctttcacaat ttctcttctg gactcctctc 660
   catccttcac atcaccaata gaaagatatg tctaaaatgc aaacttccct ggataatcca 720
   gaacccttta agtctcatct gctcacattc cactcttcca caggtactct agacttgtca 780
   aattaattcc acctccatta tgtacacaca tggaaatatg ccaaagggta gtgtttgtcc 840
   agatgaactg gctttgggtg attttatctg tcatgtgtat aaaacaagga gaagagtggt 900
   cctggctttt atcccccatg ccaaaaattc tgacagccct taccagagtg ggaggaagct 960
   aagctgcagc agttccgaaa tttgaatgag cacccaaata aagccaccca ggataaaact 1020
   taaaaccact cccctcgtct tgtttctccc tcttgccacc acaatgctgc cttttatctg 1080
   tggacaccat gcctggaatc caatctaggt gtcttagatt cccatgtctt ccaactcaga 1140
   cataggcact caactagcaa cgagcaagag tacacagtgg ggcatttgga aggaggaggg 1200
   gcatctgaag ggcaacgcat atcacttatt catagtaggg agggcatcac tggaataact 1260
   gggggtctgg aacaagaaag gaacaactat agattctgct tgtataagct gatgacgtac 1320
   accattggat gccctgtgga aggcatttgt gagattacga agttactgaa attctatgtt 1380
   aggggctgag cacatgttac agtgtgacag tcaagccctt gacggcttca gcaagtagaa 1440
   gcctgactgt tgcctgcaga ggatgactgt gtgtccctgg gccattagtc agtccctccc 1500
   aaagaaagtt aggttctacc tgagattata gagcccttca gaatgtatga cctgatttat 1560
   atcacctgtc gtaatacaat gcccccttta ataaacactt tacagtcaca taaatctaca 1620
   ttgtggtctg ctctttcttt catcagacaa aaacagcctc gggtgaaagc tggaagtgaa 1680
   aaaccctcca ttcacccaag tagtttttat ttaaatatat cttcctggac aggacatagg 1740
   ccagactctt ggagcagtgg aaaaactggc aatcattatt tcccaatatt taacccatca 1800
   atgggccgta acatgacctg agtttaactc accctttctg tcccagtgat ggaagctttg 1860
   aagtctcctt tccagatttc ctgaatgaga acagggaagc ttaaggcagt cagagcaacc 1920
   tctctcctgc gcctctgatg gttctgctgc atagagaaaa ctcaaattag tagggaatct 1980
   ttccatgtgt actgtgaatc gttaatcaat ttagtgttta tgagggccca ggtaggcaat 2040
   ggattcaaca gtgaatgtaa aaaataggat acttgcttgt ccatccagtg gtcaaagata 2100
   actagacagc aatcaacatg ttcaacttac agaatttgca ttggtgcctc cttttttgcc 2160
   atttccaccc tccgatgcgt ggtactcctg tctttgttgc cacaacctcc acttccttgg 2220
   agccctcgct aattgtcagt tccccaaaag caggcaccac ccttttctgt ttctgaaaac 2280
   atcggtctca tttcaggaga aaaataatca cagttcactt aatcgtacct caagctttcc 2340
   ttccacggtt gtgtacatat ccaccactgc ctgcttcttt tctgtcttct ggccaatgaa 2400
   aaccaacttc ataaccagcc tgcacaacgc tcttcctctt ccatgtgggc ttgttatatg 2460
   ttcagcacca actgggatgt accatccact ttatgccaat gaatccgtct gtgaaatgct 2520
   gccttggatt tcatcctagc acctctggaa cacaccactc ctcttatgtt ccgattcctg 2580
   agttaaactt atttttcctt tccccctcct cttctttgtc tttcccaact tagtccttgc 2640
   tgacctttgc ttgtcacttt tgtttgactt tattctgagc aaggctttgt gcccatcttt 2700
   aaagtggatg aacaggtaca gtcaacagct ggaaaattca aggttcgggt agtcagtgat 2760
   ggcattgtgt ttactattac caaggaagca aaacagaaat cattcctaca tcacatccaa 2820
   atcattggga aatcttataa agccgaaaga ggtctccaga tataatctgg ccaaacaccc 2880
   tgtcttcggg tgaaatcata ctgagctctt gccatctgcc ggattttgca gttcttattg 2940
   ccaatcctca ccctaacact gctccagcat cgtcctcatt tacagagagg acatggaagt 3000
   gaatagttcc ctttatcagc tcctcttgcc aaaggcatcc attcctcttt ttgatgttct 3060
   cacttctctg tttatctcct ctctgacatc tctgacattt gtcttgttac acctcgttct 3120
   ccttccaggc agctaagcac cccaatctct gtcaaccctc cactccttaa ggacatcttg 3180
   ccaaatagtc ccttgatctc tctggggaaa gtgtggcctt ttgttctcct actttagaaa 3240
   ggcgatactt ccaggtaata ggtttcttat cactcaccct ccagggaaag ctttaaaaca 3300
   cctgtggttc agtggctgca ccagtagctt tatcatccgg ggcctggtct ctctccctgt 3360
   tgatcttctg aaaagattgt tatttgagag cctttgtcgc cctgtaaaga aactcaaaag 3420
   catcagctat ttggcaaacg cccaacagat ccactcccag atttctatgt cgtacttaaa 3480
   tcacagcatg atcatgagaa ttcacaaaca cagcttagat ggcaagctca agacaagagg 3540
   agtgcactgg gcaaggctcc gcctgtcatc acccggctag ctctcacact gcactgaatg 3600
   atatctcttg tagcccatta aaacataaac tacaagcatg aggacgttgc ctgtgttgtt 3660
   ttctggaaag atgtctgact tagggtatgg actcagttag ggagcctgat ttattcaacc 3720
   catgcctaga ataaacctcc tctcttgagt ctctgtactt tttttgcatg ctggttgtct 3780
   catcactgga ttgtcagctc aactagattt tagcttttgc gtccctctta ttccccttta 3840
   tacagatgct cagacatctt ccactgaaac ttgtaggaca agggttaatg gcaagttgag 3900
   ctcggagctg cagaatgggg acataaatgc ctcttgcaag tctggtatac tagcacagct 3960
   gcaaagcagg ttagaaccat gaaggttttc atccatagac tgttcatggc ttctttccaa 4020
   agacaactac atccttaatt ctgaccacat cctctgcact gctagttatg tagagttagg 4080
   taacaagata gaatggtaca aaatagctat tcaatctttg tgttaggatg cagaaagctg 4140
   aggaaatgcc tggaggtttt ctaaatgtca gcaccatgaa tctactgctc ttggttccca 4200
   gagagtccat gactctcaaa ccacatctgc ttcaggattc cctcactggg tcaaaggaag 4260
   cttgttgttg acatcagtta aagctcattg gtttggatta cttttccaca tgattgctgc 4320
   cttttccaat tcctctctgg caaacaaacc cgccttacaa agatgagtgt ggcattgaaa 4380
   ccctttttat ccatctactg tgtcctactc atagtaaagt ggtcatgggt agttcctgtg 4440
   ctttgcctcg tgggatccaa cattttttgc attcctaccc taatcaccca aaagctaaac 4500
   aggtattgag cagtaccatg aagagaagcc atcaggcacc agagtcttga acaggaggaa 4560
   gctatggggc agagaggaaa cttgcaagaa gaaaatcagc gaacagagaa gctggaccac 4620
   aagcctgtgg ttactagcct ggttgattga ttggcaccag aatgctgcag tattctgatc 4680
   aacattccgg ttcctgagga ttgaggaaac tgctctatgt tccttgtcac ctgaaagaac 4740
   ataatgaaaa catccagcca taagcagcat gtaggaattt gttcacagag gaattctcca 4800
   aaggtaaaga gccaagcaaa tggcaaaatt ggcaactctt ggcatttgct tcagagtgta 4860
   attcacaggc tagtctattt cacactaact ggggactcag gctgaatgtt gtcttcaaca 4920
   cacctaagct tcttggttgt ttgcttccta ttgctcttgc ctttcttcaa gttgcacgat 4980
   cactacaaag agaggaataa tagacactct ctgtcactct cttcatcttt tcccagccaa 5040
   ttgccctggg aattggcatg gtagcataaa atcactgtcc gagcttacgt gagggtgctg 5100
<210> 29
   <211> 5100
   <212> DNA
   <213> Artificial Sequence
<220>
   <223> Nucleic acid probe
<400> 29
   cagttctttg tcaccagccc catgcttacc tatctggact tctttcctat gactccgtca        60
   tttaaaccca gggtgccaac cattccaagc actttgccat ttgtttacaa tgccctttct       120
   acatattctt tgtcacacga tctaacttgt gctggcactt agctaaaggg ttgaaaaacg       180
   ccttttcaag tgaggaaaaa aaggctagga aagaattcat gaagacaggc cttaaaagac       240
   aatcggaact agacttgctg gggaataaac tccactaagg ctgggtctga atccattatt       300
   ttcctatgct ctcatgtaaa gaacaagact tggaggggaa actaaagatt tccttttgtg       360
   gtcttttgcc ggtcactcaa cacagatagc accaacacac tgggagtcat tctcctttac       420
   ctacctccca cttatccacc tgccacttca atgccagcct ctatgaagtt ttgcctaata      480
   attttagctc caatccatct gtatctcttt cctttaccct tgtgcctgct gaagatctta      540
   ttctcagcct tgctgtttta aactcagggg tgtgtgtaca acatatttaa gcaaattctg      600
   gtagaatggc cgctttgtct ctcttgagtg actctggaca agcttcaaaa ccaaaatcca      660
   cagtgcccta tcctggtggg cagaatacac tgaagacagg acaaaacacc cttctgctga      720
   ccccgtacca aaggggaggg tgccggtgaa agccagcttg ttactgcttc agggaggaga      780
   agcagttact gaaataaagt gctgctctca gcttctctcc cagaagtcct tcaccaggcc      840
   atctggagcc gaggaacaag ttccaagttc tcttggccag aattctgctt tgaaatctgt      900
   ttgccttctc ttcctcccta atcgaggccc ctaaacacaa ttcttcccta atccaggctt      960
   cactcaccac ccatggccat ccacctaatt tgacctggtc ccaatgctta atgttgatgg     1020
   ttgacctgca atcaaaaact ccaaatgact gtctaccttc cagaaaggaa agcagatctg     1080
   agccaagaga gtaactaggg aaaggaagtc taaggaagaa gctggagcct cagctcggtg     1140
   gaactgggaa tacaaagagc tgggtagagc tgtctgaggg tgaggcctgg tgtggctttg     1200
   tggatggtag aggcaaagca attacacaac cgtggataga catgacctaa tttattggcc     1260
   ttcccagtta cctactgtgg agccagtgct ataccataac agaggtggac agactctcct     1320
   tgatgcactc cccatggaga aggccattgt cagaattccg cctgcttttt ctactctgag     1380
   gagtgaagag ctttttttac gctcccaagg gcaaactcga gtgcaaatgt agtaattcag     1440
   ccagcaactg agacctcccc tcaaatatgt acaatcccaa aagtcatttt cagacacctt     1500
   gagttcaaag aagccagttc acaaggccag gatctaacca gcttgtgggc acgacagcat     1560
   ttgtgtttat gcataaccta caccaagtgc gcagtgaaac aaacaaccag atctaaatga     1620
   caatcagtcc acttgatgga gacctcatga tgaatggtct acccttggtt gggtttgcca     1680
   cctgacgtag aaaatgggaa tgtatgaaag ctgtgttttc ccctgtgatt tccttctgag     1740
   attttataca aactggtgct atagcatttc gcctcatcaa ctctttgtgg ttatttggtt     1800
   tccctcaact cccctccttt ctgattttgt tgtcaacatc agagtgttca atctcagggg     1860
   atggatcatg tgtggtcaaa gtctgatatt tcaatcccat catttcctct ttcatctaga     1920
   aagcctaaca caccatggaa tgaggaagct ggctcctgtg atctgttaat atttgaagta     1980
   tggcctttta atagggccat ttggggaata actcactaca gaaaaaccga cctgaatgaa     2040
   ggtgaaggag agaatccacc cacgtttacg aaagaccatg ccttagctag cacttcttag     2100
   aaagtatttt ctgactaacc ttcacctgac tttctttcgc ccttcacctg gtttcctcag     2160
   atctttcttt cccttagttt gtattgccta tagcagtaag cattggaaag tgcagacagc     2220
   tcagagatcc gtggtgtgtt aatctggtac tttgttatgt ttcagaactt gctcaagcct     2280
   cctgagcaca cttgtgactt aggtctggta cagatgaggg gagatcagga gaaagttcta     2340
   tgtgttcagg aacttttctg tgggtggggc tgctgccttc tgcatgatga actcaactac 2400
   tgcatgccaa tatctgatac tgaaattaat acatgtgagc ctgaagtggg taagcagaac 2460
   aatcccatgg agcctgcaag ccagacatgg gtatttgata acacacactg agaataaccg 2520
   cagtttctta gtgcctttgg aatatgcact gaaattccgt gcagttctga aattcttctt 2580
   ctgaaagttg ggtgtgcctt ataaagccac tttttctcaa ggcagatggt atgaaatcca 2640
   tattccccaa cctcaaatat tccttcatcc ctcaaagagt catgtttagg ggagaagggt 2700
   acaaaatgac ttgctaaatt ccaatcaatg gcacacagtg tggtctgaga gtgatgtcct 2760
   gaattccctt ccacagttat ctagagaagg acttagatgg atattggcga gagagtccct 2820
   ttaattcatt ccagcactta tcccacactt ccccagcaga aaaactggtt aatgtacatc 2880
   ctgggtaatt atctgtgtct ctgcgggacc caggcccagg acaaaagaaa tgaatttgct 2940
   ctgtacctga tcctgtcctt ctcggccctc tgtaactcct cattcctttc cagcacctga 3000
   gtcatttaag aaactgaaat caaacgccgg aggaactttc gtcattttct ttactgtgtg 3060
   tgagttacac ttaagctcct tggcgcctcc tgttaggaag aataataagt ccgccccttt 3120
   gaaagtctaa aaccacaaac ctagggaacg cccacacctg aagggctcat attgacaata 3180
   ccttaatgac atgtttctct gctaagtgtt ggcgccaaag tatttgcttt tgctgttgct 3240
   gtttgctcgc agaacttgag cttgattagt aaaggagtga aaaaggagat tcatgactaa 3300
   ttacttatct gaaagaccag gaagaactaa actcaattac actactgact gtggatgaac 3360
   ctggctgaag ggcggaaatt agtgtatcct tggaaacgaa ggctagtgct cagagcacag 3420
   tgagctgaaa ggtaggctga gctctgacta tgaatgccat gtgatgaaat ctagacttca 3480
   ttgtgtaagc actgaggagc gagcagaagc tgagttgaga actttgagtg tccaacctca 3540
   tcagacttca gcactgaggc atgcttcagc cctgggttgt ggcacaatgg ggcatactat 3600
   ctttagaaat ctctcatagt cccctaccct ctctagtctg ttggccaaat ccccaccaca 3660
   gggcttagtt ttttccatcc ttgacaatca ccaagccaag cagctaggtg gtcaatggga 3720
   ttctgcccag tttcctcctc acacctggct ggcttctggg ataataaaat gaatttccag 3780
   atgttacctc ttctctccct tgcctttctg tttagaagaa gtgccctttc aataacaatc 3840
   cttgtgcaga catcaagtaa cacctggaaa agaaaccttt ttgtttgttg ctgggcagcc 3900
   tgaaaggagt gagattgaaa gttaagttgt cctttctcca tcaacagcct aaatactggt 3960
   atgctgcagt ctctttgcaa ccttttatta gatggatcat ttttaaattc attatcagga 4020
   cctactagtg tctgggtgga agactttaag cagagctaac tgagttaact caccaatgaa 4080
   ggccttcaga acttaacctc taatacatta accatacaca ttttaggatt ctagccatcc 4140
   ctttttccac cacacacaat tgttttcctg gttatctttg ttgtttcaca tggtcaggtg 4200
   acacaaagaa gttgttgcca tgccttaaga agcatgctaa gtcctgcagc agagcccatg     4260
   gatcggcaag aactgttcac catacagggg cttcctgcag tgtcatatat gcttgtggtc     4320
   ctgagttaga atgtctaact tgagtactaa ttgagcagaa aatgtcaacc ctcttggaat     4380
   agagtaacat ctgggtcttg tgggaaccat ttaatgtgct tcccctcata gcttatcaaa     4440
   ttgctccctt gaaacactac tgtatcctgc atccatttct aatgcagcca tccattcatt     4500
   atgtagattt atgttatcca ccgattttcc agaaaggcaa ataacctggg ccagaggggt     4560
   ttctcccatg taactcactg tccacctggg acttccacta agcattaagc caaagaggct     4620
   gtaaacactg gcaagataca accactctgt tctgatgaat aggccatttg ctccacatga     4680
   ctgaatgctg gaacaaatat acagaaaaaa aattctggga caagagtgtc aaaccaatga     4740
   tccccaaagt tccaggtgga aacgggttgt gatggtgtag caccttgaaa gggtgcctca     4800
   tttccataaa gagttgatca caaaagcagt gacccttctg ctggggctct tctcatcctc     4860
   caaagactta gtgtctgagc agtgctggct tggggtcaca accttctaca ccaaatcccc     4920
   aaactgccta gctctaaagc cagtcatgat gtcaccctgt taaatccccc acaccagtcc     4980
   ctgtcaggca tgcctgccca aggtctaaaa atctctgtct caggaagttc tttgaaatag     5040
   gaatccgaga acaaacagaa gtacatcagc tgttccaggc aatagactga tacaacataa     5100
<210> 30
   <211> 5000
   <212> DNA
   <213> Artificial Sequence
<220>
   <223> Nucleic acid probe
<400> 30
   agcatcaaac aattggaaac cttgtcattg ctggtactta catctgggca attggaaatg      60
   atgatgtgac tggcatggtg gctctacgat gtactaacca acactgattt atacatgcag     120
   gaatccctcc ccttcactaa tctttcaaac ctccacccag agttgcatca aacaagggcc     180
   caagtacctg ccaaatgcct atgtgttgtt gaatgtgtat ttgctgagta taggagaaag     240
   gaagttttcc atgcttctct gcagctcctt cccagaagct aagagaattg ggctaatacc     300
   ctgaaggctg attccagcaa ccattgtcct tgagaattat tcctgcctga attagtcctc     360
   tatactcaaa ctccttttgg gatcttggta tctcacagtg tgtatgtggg ctcatgagca     420
   gctaggctat aaaagaggct cagccttgca ctgttgctcc cctgcagaaa agctcggatg     480
   ttatttctct ccattttctt attaatgctg cattaggttt ctcaacatcc ctgcttccat     540
   tgacaaagga gcaatttcac gctgtgctct gattagaagc actggagatg tccaacgagc     600
   tatgctctat tcttactcta cccagatcat tacccaatag tctagtctgg gccacactta     660
   cagggatata tatacaagta ttggttattt agtaactctc aagtattaag gatagtgtca     720
   agtcagttca agaagcttat gtgaagggcc taccattagc ccagcatgtc aaagttgaga 780
   agtgcattaa ttcattcact tgtgctgctg tctacacaaa aaggttgaaa agggctgaaa 840
   aagacaagtg gcctatttct ttcttaccca agtccaggcc aaatgctttg cttatcaaca 900
   atcaccttgt cttccgtgcg ttgactaact agaaagaacc tcctggtttc tcctgagaga 960
   ttgctttctt tttgttgtgg tcaccatcac catgacactg attccatcct attccttaaa 1020
   tattaggtga gcagctactc agtgtaatgc tcagtatagt actccatggt cttttaaaac 1080
   tttgcaacct ggcagaggag ctgtgaatga gaaacaaggc aaagtcttgt tctccttctc 1140
   cagtgtgttc ccctcaatgc tctaagagca ttatttgtta atggaacatc atgtttgggt 1200
   aaggaggatg actaaaggta cttcagtggt tcagaaagag gtagaagatt tacctgttgg 1260
   ctactgcctt gttagcctac tagatgcaga tgctgtttgt tttttcccta tgtcctttct 1320
   gtattttctg ttaacaccag atgcacacca gagatgttct aagcaggtta aaagtggctt 1380
   gggattatat ctgattagca ttccacttag cacttgaaat acacaaatcc tgaacagaaa 1440
   aggttcacat tgcgtagtcc cctgcctgaa aaataatcat atccagtttt cactggagtt 1500
   gccactaata taaatgtgta ctgatgctgt ccgaaaatgg atggtctacc tcacatggct 1560
   ttcatgatct gtaagataat ccttactgtt cctatcattg gttttgctct tcttgtacct 1620
   tgtaacatga taccatctgg cttttactag agtgtcgttt atggccgtgg tggaagctct 1680
   tagtctaaca ttgggtcaag ttctactctt cttgggaaaa ttctttcagc tagttctcct 1740
   ctcccctctt gccagtggtt gggggaagca ggacacctct tctccccaag gggaaatgga 1800
   atagagaaag gaaactcccc ataccagaaa tggaaaaaag atgtgaaagc cttgtggtaa 1860
   gcacacagac tgactctgca attttacact tagaaattaa tcttcattct gtggaacttc 1920
   cttctggtta gtatcttctt ccctgggcac catcaggctc agcatgaatt atagtagggt 1980
   actgtgaaac ataataaatg tggtgcaatt ggcattattg ttaaaggcag acttctccaa 2040
   gaatcaacat aactccgtct agcatgctct ctgggtgcag cattgatatt ggcatgcaca 2100
   caggtcctcc ctgttcaatc atgttgtaaa caccagtgct taagcagtag tgaagttatt 2160
   gcctggctaa acagaaactc tcactcttca ataattgatt taagtcatca tctgtattat 2220
   aggtaatcag aaagacccca ggcccacaat ttcttctcca atggccctgc ttaggtggta 2280
   aatggggtaa agtcaagaga gtccctgtac taatagctct ctcttctcct gttatagttt 2340
   tctgccaatg tgttccagcc cccaggaaag cctcacggga ttgcctggag agggtgatgc 2400
   tattctcctg tctggtattg ctttctgttc tgtctttctt tcacccatta gacctgtttt 2460
   agagcctctc atttctattg ttttgtccaa gaagcttgtt tttgtttctt ggatacctct 2520
   cctaaattaa accatctcat agttctcaag cacattctat tggtgttgct cagagttttg 2580
   ccaaacatag ggatgagtgt tggtttccac tccctcctac ttccttgtcc acctggagaa 2640
   cagagttgga aatgaggatt cttgaccaca gtggccctgg gagatctaac cactggtatc 2700
   aatttagttg ccagtcagga tagccagtgt aaataaatcg cgttgtttgt gtctcagaaa 2760
   cagaaacgtg tattgctgca tttggttcat tacattcact aatatgcctt tgagtagggc 2820
   catctctgct cccaggaatc ctcccaatgt cactgaggca actctaataa ccaaatatga 2880
   tacccctaag attgggcaaa aaaagattgt gataaaccct atagaaatcc tgaaactttc 2940
   ttcctgggtt gaccaagaga agtttaatag agaagtcatc atttgagttc gcagtaaagt 3000
   tgaaaggtcg aaagagattt ccaactgaat ttaaccccat agggctttgg catcctagta 3060
   aaacttgaaa cacagacttt gtgaatgctt cttctctcag gtctctgctc tggtctttgc 3120
   ctggcagcag aactctgggt ctaggctgca ctgacagctg ggcactcaga accaaagtga 3180
   ggcatgtgag aaactttgga atgacccatt tcagaggttt tttcaaagca taagatgaat 3240
   agcccaccag aggtcccacg agaaaattca aaggacaaga tgtcattgta tctgattata 3300
   gtagttacac tcatggatgg ccaaagctgt aattattaaa ctgaagagtg ccaggggact 3360
   tggcttttcc tccactcctt atgttccaca gaggggaggg agaatcccat aacaatgcca 3420
   aacagcatga gaactctatg cagaaagaat tgctatgagc tcgatggagg aaacacttgc 3480
   agtgtgtttt aggtgatggc tataaatacc taaggggtat gttgatgctt gtgtctacat 3540
   gttaaagacg accataatca cccaaatggt atcatgaaca agatgcttgg aaaatcccag 3600
   gaatgatttg tgtttgtgct gaacaagact ttgccagcta aatggtctgg cagaagcact 3660
   acgcatatat atagataacc aagcaagaaa tgctttcaat ttcagtccac tcccaaggga 3720
   aaactagcac ttaatcctac cctgccacct gctggcccat gatagaccaa aaataactat 3780
   ctggaagttc caatttctca aaatagttat ttgccatttt tacgtaacaa gaggacatgg 3840
   atgggttcac cttttagcat ctttaggcca agttattctg atcttgtgga ctctcttaaa 3900
   ggagatcttt tgatctgaga ccatgtagtt ctgttcttgt aacgtcttga aagcatgtca 3960
   ccaccatctt ggttcagtta cttctccaca aaagtaagag ttcctgagtg tggaaattct 4020
   cttgttcacc tcctgtcctc actcccttcc aataagccat gtcttgtttg gaggatgggc 4080
   atagagttga gagtagtgat actcaatttt ccaatctcct ttgctacagc taagctctcc 4140
   cacccacatc cggtgttttc ctggaggctg taggtaaatg tgtcaaaaaa ttcaattcat 4200
   ttttaaggca aattcaattt gattccgttc cagtttcctt gctgttgcag ctagagtctg 4260
   aggggcccag aggaaaagga aaacccagag gggcataaga ttattcctga ggaagtacaa 4320
   ccaccatgga tggaaatcag aagtcactaa ctagctgttt acttctgggc aaatcttttg 4380
   gctcgttggc ccgaaaagtg tttacacaca tactcacgaa cgcttagcaa agttgaacct 4440
   ggagcaaatg tgttaatagc ccttaaaggg tcagtgtatg acgtcaccat agtaacaaca 4500
   caaacctctg caccgttctg gccactcttc tgcttgccac actgttccta aaaaaataat 4560
   agaaatgcgg ccgtacctaa gggtagggag actaatgctg tcatctatgc tccttcctgt 4620
   ggccacctca ttttctccac aggcttcatg tccccatcca agaattggcc tagaaaagct 4680
   ggaccgctgg tctgtttctg ttcccagatg agtatacctg taatgatcac tttgacctcc 4740
   tgacttcttc ccccaacctc cagactccac aggtctgtca attcctcttt tccctttccc 4800
   ttgtacataa aacttactac atagccatgg tacacaggtg gtgctcagta gcctcactgg 4860
   tgaactactg aacaatttga ttccgaaacc ttttaaaaca aaactgggga ttgaggctca 4920
   ctgagaaatg ttgagctagc tgtgttagtt cagcagctgt ttttactatt gctgaatgac 4980
   tctttgcatc tgggattcat 5000
<210> 31
   <211> 5000
   <212> DNA
   <213> Artificial Sequence
<220>
   <223> Nucleic acid probe
<400> 31
   taaagggtgt cagagacaat ctgagaggta aatgctacgc tatggactaa cagtaagggg      60
   aaaagaagat ggtaaatttg cagagaagat caagtttgat aggagcagtt gactggctgc     120
   ctcttgctgt ggttcctgtg aaaggcggct ttagtttgga tcttcagtgg ggtaaggggc     180
   tcttgttcag aatagaggct caaaaaacga tgatctctgt tacagcagct aaagtggatt     240
   ttccccaccc cagtaactca ccacatttcc ctattttatc ttcttagtag tacttgccag     300
   caggaaccct attgtgtttt cagatcctgt aacccatgtc tgcctagaga gagcctttgg     360
   tatgtgcctg cagagctgaa tgatagacaa ttttacattt cctcagcatt gtctgacagc     420
   cccaactgct cgagacgtgc ccaaaggtga ggaactccct agtacttcca ggttttcaca     480
   caaatctgaa tggctcaatg aaatctattg aagcccctga ctggctttac ttgcagaaat     540
   tccctttgca gaaaagagag gcttccagac actttgccta actaaataag aaaatggaca     600
   tgaaattaaa ctctatgaca agaaggcttt ttgcccttct aagtaccaga aacccaacta     660
   catgatgtcc aactacagaa agagtacaga cttaacttaa ctaaccttcc cccttcatct     720
   ccacgtgcac agtcatcgct ttggatcctc aaagcggttg tgttttggtt ttcttttttc     780
   actcagagtt tctcgttaag cggctgtttt agccctttaa agattccaaa tctggccttg     840
   ttccccatcc caaatggaag ctgctgacgc tggagactgt ggagagaaaa gggtggggcc     900
   gaagaaatgg acaggaggac tgaatgcaag atcaggagag agtctttcaa gacaggacta     960
   gaacaagatg ggactagaac gtgtctatag ccaaggagca gccgaggggc agggcaagat    1020
   caagagtttg gttctgtccg cagcatgggg cacatgtgtt ccagagaagc tggctggaaa 1080
   gtaacgctgt gggcaagaac agctgcccac ctgctatcat gtgatggcag tagaaagacg 1140
   accaatctct ttaaacacac tttccttcct ggttatggcc ttcaattcac tgtatgaggt 1200
   gaaaagggag cttagctgga caagggaagg atgccaaagg ctggggctta aggatcagat 1260
   tcagttgacc ctacccaagg cacaggaatt gttctatgga ctttctgctc tgctcttatg 1320
   acttttattc acaagcagct ttctaatgtc tcaaccttcc atggcaccca atactatttg 1380
   cttagatatt tggtggtgaa catgtgacat gagagatcgg ccgagccagg aaggagccca 1440
   tgttccttat ctcatataca cagaaaccaa gggccttgtc cccaggccca ggattgttta 1500
   tttctgatcg tgctcttggt accagttctc ctgagagtga gcttcactgt gctggatcac 1560
   aaatgtccgg tggaaagaca tctagctggg gccatctctc gagagaaggg agggtaacag 1620
   aggctcttct gatcctcctt gcttctgggg tctcttgctg ggcctctgag taggtctgca 1680
   agtagggctg ggcatcgggg ttgggagcca tagaggggat ccggttgcac tagcctgcat 1740
   cctcaggctg ctgaactgac cacggtgggc taaactggaa aagtttcttt gggcaggaaa 1800
   gtgaagcact ctcaggaaag ttattgcaca cagtggaatc atggctacgt ggctacctca 1860
   ggatttgtat tagtacactt agggctgctg taatgaccta ttcatggtac ccactggggg 1920
   agaatattag tgcaaatagt gcccaatgat ggataatcat ccagggattt gccttccagt 1980
   gataccacat aagggcatca caacaaggtc tcctccgaat gctgagtgcc ctggaaggca 2040
   gagtgaagct ctgaccccat tgtgttgtga ggtggagagt aaatgacagt ggtttccttg 2100
   ccttgggctt catccattct caccctcaga gctaactggg gctgactggg aatgagctaa 2160
   ttaggactgg cctttcccta tatgtgttcc ccagaaaagg ttttcacaag ggtctggaaa 2220
   ttggaatatt ttttccacgc taactctatg acctgcagtg tggctaacca atctagacca 2280
   aacatgcagc tctggattgt tataaaaaag gacagcatag ttgctagaac cctctagaga 2340
   tgtacagata ttagatagtt ttggcaggaa gtggtggcca aaatacatga aacaacaacc 2400
   aaacaatata tttcaggcat ctcaagtaat ccacaatctg gaggcaaaag tcaagactga 2460
   gttgacagag cacaaatctg tttcatttga ctacattaaa ggcctgggat tgttgaggga 2520
   tgcttatgca cagagaggaa ggtttgctaa tccaggagca ctgaatcatc atgatgtaaa 2580
   cttgcaggac ttcatggctt catatcacct tttctcattt tccagaagta tgctgagttc 2640
   cttggtcagc caaaataacc accctctcgc ccatatgaga ggatcttcct tgtccatttt 2700
   aatgcagttc cttagatgct cgtctggacg tgatgacgtg cccgccatat aagaaggtgc 2760
   aatcataggt atcacaggta gccagataga agacattttt gcaaccagac ccgacgacgt 2820
   gtgtcctggc aatttctctc caagaaacca ctagtgatga agcagtgctt taggtgcttg 2880
   ggggttgagc accttcctga aaaagaaggt gtccaggggt ctggccttgc tggaggactt 2940
   cgggggatgg tagcagcagc ataaataaca tagaagcatc aaataggaga ctaagacttc 3000
   caagaatcct tggagaaacc ctatcaagtc ctggcctact tgaagtcagt attgaggctt 3060
   tccctggcag tgaaactact gaggccaaga ttgctttccc aaacggcatc ccagatgacc 3120
   accctgttcc tgcagacaca catctgggct tctagccact gggggcacca gaggctcacc 3180
   gttttaactt tttctgagca tattcctttt cctgatatcc tgagcactaa gatctagctc 3240
   aaagttcagc cttcactttg acagccatta gagaaagtaa atgcacttca taaactaggg 3300
   ctctctccct actccaaacc attgcctacc cttccgctgg agttactaaa tctgcctatg 3360
   taaaactctc ctttggttcc tagaggataa aggattactt cagatttggt gccagacctc 3420
   tagtgaggag gccactagag taacctgggg gagaaatggg tggctcagcc actagaacca 3480
   actaagaaga ccacaattat aaaagaccac aacgttttag agctatcttc tagtggtaca 3540
   tctgcagttt tacctttagt aacagtgcca cattcttctt cattctagac cactgcattc 3600
   tgagccatca gtataaacat ttatttccct gaatgagatg aagccaaaga cctactgcct 3660
   taataatgga cagacagtgg aacagagcat gaaatagctg atcatttcat aaagccaatt 3720
   gcagcatctc tgtccatggg gcttcagggg aagagatgaa acctttgttg agattctacc 3780
   atgtgggtca ggcagggaca aatgactggc tgtctccatc gcaaacctga ataattgtag 3840
   ggctagaaat gagctatccc tttggctggg ctgcggcttg gggaagtaat tacctcagaa 3900
   agataagtat ttgacaaagt gttgcacagc cccttgttct aagaaaaaga gttcttggat 3960
   tatgtgttgt ggtgatgttg cacgtataca ctctctcttg aaaaaacaaa tgttttctca 4020
   ctatctgccc ttagggccac acctccttgt ctccatgtaa ccagggatga tggtgggtga 4080
   ataattaact gctgtttatt tctactattg tttccactca tccatatgaa cagcccagtc 4140
   cgctcccaac agtgtctccc tgccaggttg cattaataat gcaaattaat ataagtagta 4200
   actaccctag tatggacaaa tggacttttg caataagcag aatcaagaca gtgaacaatg 4260
   cctcgttgag ccaggaaaaa atgcaagtct ttgcatgtta gagtgattgg ttcaggtcac 4320
   agggcgttaa ctaaaggaca gtgattctta agaaccaggc aaagactcag ggtcatgaag 4380
   aggattcact tatctctgaa cattactaat caaccactac ttactctgaa attgaggtaa 4440
   cacagcctga ggcaacaaaa tgctctgaca attactgaaa cgtttatctc ctttcacaaa 4500
   aagaagggcg tttgggatgg ggcttagaat gtttataagc actgaagagg gaggcccatg 4560
   acatgagata tcgatgaaat aactgacaaa gtgacgttcc agtttggaat ccagactaca 4620
   gatagagtgt ttgcaacatg cagagtacca cttctgggat gagatggaaa gaaggcagga 4680
   ttggacctga agagagggaa tcataagtaa taccctagga aggaaaaaac agaaacgttc 4740
   cataaaagct gagccgtcag aaagcattga ctgggaagta tggaaacaca aaaagctaag 4800
   tatgttactc tcattatggg actaccagaa atctagcagt tcagatcagt gaggttgtaa      4860
   tccaggcact ttcatccccc agtggccgat tgcaaacaaa aacaagcaag ttttaaaccc      4920
   aagcctgcag tagcaaaatc ccagtaccaa agaagtgagg aaagatgtga gaagtcccac      4980
   cctcagtata gtttagttca                                                  5000
<210> 32
   <211> 4900
   <212> DNA
   <213> Artificial Sequence
<220>
   <223> Nucleic acid probe
<400> 32
   agtgtacaag ggcacctcgg ctgaaggaga ctctaggact ctgttctaaa tcaatggcct      60
   gacctctgga tagggagggg ttttggataa tgcagtgtgg cattaattct ccagagtaga     120
   aataagggtt cacactgatt ttcccagcca gtggcagctc taaaaaggag ggggcagttg      180
   agtagggtgc aacatctaca tcctggaaaa tgcaccatct cattagatcc aatcccactg     240
   ctttggaatt caaaccacaa actcccagac aaagctgtag atactaaaag cagttcaaat     300
   accaccacat ctccttcctg cggaagactg tttgctggca gcctattttt ctcttcattg     360
   ttgtggtaaa gggctccatc atttctcatc accagactgt aaaagctaca cattgaattt     420
   aaatggcctg gagttcaatt tcaagctctc cctttgaaaa gtgggaatgt actgtcttca     480
   gtttttacat ctacaatgag gttgaattaa tatgctgtgc taagcacttt gtagtggaca     540
   tgcagtatct gttctccctc tcccgacaga cttccctctg gagatcaaaa cagttccaag     600
   tacacagacg gttttagaat ctgtactttt aatgattggt gctgcttaga cggtcaaggc     660
   aaatttgagt acagccagaa tgccacataa acatagccac ttcatccagt tagtagctga     720
   cagttgctgg acagctttgg agaaataaaa cacagtggaa tgagtatatc tgaccagctc     780
   ttcctagagg aaacagatga aattatattg ggaaatctct ccttctagaa ccacattcca     840
   gaagcatgaa actacctggg aaaagtctaa gaaataataa ggtggtgaaa gaggctatga     900
   tggaaatgtc tgggacgacc tctagaatta acctagctca aacctctgtt cccagatcag     960
   tttcatcctg aaggcttccc tagggatgga tcatcaagtc ttgaaatggc aagttctgac    1020
   ctgaggtgcg acttcagact tctgcagtct gccttgtttc gactttagga ggtgagagga    1080
   aaattacttc caataatata ctccggagga agcctccgag attcctgtga aaatgggagg    1140
   tccacacgcc cattcgcgat gacagacaca gagagggctg cgcagccttg aacacagaac    1200
   aggggttttc ctatgcagaa tttttaaaac gcagcacccc ttggtcccgg cctcgacatt    1260
   atttctaggg atggattgac agaagcgaat cagtttgggg acggagagcc ccagtctccc    1320
   tgccgtggct ggtttccctc acatgctaaa tagactcacg tgggccccag gagtaatgcc    1380
   tgttagtgac tgcactgtgg ggctcctacc gtccgctact ttctgcttcc acacgggagg 1440
   ctgtcacctg tcatctcctt ccccacttag atcatccttc cgtggtagat tttacatctt 1500
   tgcaaaggag caaagtgcta ttttattttt caacagttag acctgttggg aagaggccag 1560
   tcagatgaaa ttacagtacc tctcacaatg actaaggagt aagtcagtca catcttcaat 1620
   gaaaagccag acactaaatg gccgtgaatt ggacattact atagagaggc agaagggcca 1680
   tagtctttcc tagctggaaa aaactggtta gctcctgaaa cagacttccc tgtcttggca 1740
   gattaacacc atccatgagc tcacttttcc ttctaatatc tgggattcta atatccattc 1800
   aagaaagaat gactacttca aaaaaagagt ctgtctgctt ccaagacatg ccaggaactc 1860
   gagaactgag aacaataatg taaagcttgt gtttcagagt aaagcagact gtcataaacc 1920
   aaggggattt ctgtgatccc tgcccaacct caagttccct tactcgaaat gcaagtaccc 1980
   tagtgtattt ctaagtactt gatgaatatc cccgggactg tcagaaacct gccttttgtg 2040
   ttgagaagga actgctatac ttctgttaaa gcaagtgtgt ctatggttgt agatatggtg 2100
   gaaataactt tgaaatacca agagttagtg agaagaagct ccagggatat gtttatggcc 2160
   aagagaactt tagtgtagca gaatagattt gcagagaatt gaaatgaccc aaggaggctt 2220
   cgatggagcc aaaatcatca gagggagagt ctcgggagaa agatgaatct ccagggcagg 2280
   ggccagggtt tgttggtctc gttttaagaa ctgaatttgc ctaggcatta gaaaaagtac 2340
   agaaagctcc ccagagcagt ctagagccca gcagtcgcag gcccctaggc catttgagtt 2400
   actattcctt ctgagtgtct gtcaatggag gttgcatgtg aaacaggaaa gaggtttgct 2460
   gaggctctat gtttatgttt gccttttctt caactggaga atttcttaag atgatgtata 2520
   atacctcccg caatgatagc ttccccgatg taacacctcc tgcaagttgt cctgagctat 2580
   tttaagctgt aatcagaata tttttttaca gctctgaata ccataggact gccagcattt 2640
   cgggccccag tagcaaatct agccctaata tagatgctgt atgggaataa gacactccaa 2700
   ttcaggcctt gtgaagcctt gttttatctt attctcagca tctaggttct aacccctact 2760
   gttctcccat tattgactgt cccttgccag gcctctctta cccaggtttt ttgggccata 2820
   atgatgcccc tgaggtttgt gctcccggaa tgccccagag ctggaaacta ggcagctttt 2880
   gcacataaaa ttactttatt atgaacttct taatttcata tcaaaatgcg tagatacctc 2940
   ctgaccccca ttcttgccct cctccatttc cctgttaaat cctgaccttt ctctgtagac 3000
   aaaatgctag ggtgagtcac cagtccttct ctcctaacaa aaaagcattg tactgtacag 3060
   tttgatttcg gctcttgtgt gcaacttgtg attgcaaact tgtactctac acaaagagcg 3120
   gaaaagctac cctttaagaa agagactcat cttcgcatgg cctgcagagg gcgctcttgc 3180
   atcatagcta taccatgcat agacactatt ttaatggtga atctggacag cactccaagg 3240
   gtacatttta acaggttgga agtaggcttt taacatatta tgggggggat caactgagta 3300
   tagccaaact gaccatcaaa ttgagagcca aaaagccatt aagttatgat tctggacaag 3360
   gctctgattg ccacagttca gaaaagttat ttgacatctt ttcttgtttc agagaaacca 3420
   tgatcctcct ccaggctggt tcttaatcag caccccctcc cttccctggg tgcctcccat 3480
   ttgtaggcag ccattattgg acggcagaaa agcagccctc aaagaaatgc taccatggga 3540
   tatggcctca agagaggagc ctcagcctga gatgagttgg ggggtggcct tgaagaaaag 3600
   tccattaggt agggggccca gatcactaat aatcagggcg atctggtgag gcagaagatg 3660
   ccagcaagaa atgccctgga gggcctgaag cagctgctga aagatactaa gtgactccgc 3720
   actagcactt gccacactag ataacctggt gagagaagct gctaattgta atagaggcag 3780
   aatgttatct gtactgttta ggctttgcag aaagctgtta ggaacagggt tgccaggtgt 3840
   cctgttttga actggacagc atggtatttt agcttctagt gtacgaaaat gcagtaaatg 3900
   tacactctac aagaagtttt tttccccagt ctctatgggg tggttatttc tgtaatccta 3960
   tttatggatg aagcagggga aatacagtca tcactggggc caatgatgga gtcaaaatgc 4020
   acatgcagca atctgacttg agctgccttg ctcttagtaa gggtgaacaa aaactaggag 4080
   agaggggctg ctgcagcccc cgcctataat ggaagcttta ttggaaaagg cagaaggtag 4140
   aaaaacatct ggaaggacaa aaagcccacc aggcaaccaa ccagtcaacc agtaacaatg 4200
   gttattttcc atttgctaat cagttggatc catttgagtc aaactgtgcc tttgtagact 4260
   tggttgcttc ttttggaagc ttctttcaaa gtttctttct cagcatgatt tccttgatcc 4320
   atggaagtta ttaagttgtt ggcagtccac ttcttgcttt tgtggagttg tatttttgtt 4380
   ctagctcttt tgttcacaga tagtcagact gagcagcctg tgggagaaag ctggctgcca 4440
   tcattctgcc actcactgct ggagggacaa caaggcagaa gcagtctggc gggccaagct 4500
   tttcccaggc tcccactgca gaccttcaaa atccatgtcc cccaagattc tgtatattta 4560
   aactggtgtc cagagcagtt tctatgacct caaaaggttg ttttttaatg atatcacaat 4620
   gtccaggtac tgcacagatg agactgaagt tccacgttct aaagccagaa gctgcatctg 4680
   aaatgttgca tagaaaactg ttcttttcct atatttatgc cactttttct agcttttccc 4740
   tcctggttcc aaaaacatca tctctagcct gcctagtatc agaggcactc cctgcccagt 4800
   actttgctac aaatactcca gtaaactggg cctgagcact gctttgggct tccagactgt 4860
   aggcttcctc tatctcccag gcataaatag actcttacct 4900
<210> 33
   <211> 5000
   <212> DNA
   <213> Artificial Sequence
<220>
   <223> Nucleic acid probe
<400> 33
   ataataataa aaaagttcca ttgtatctag ggaggcttac cagacccctt cccctatgca 60
   cagttgctcc aggctttgtt attagatgtt cctcactctg aaggaaattt cagtgtgaca 120
   agaatgttga caatccctgt gctatctgta ctaggatcta gttccactgt gctcttccag 180
   ggaaggatgt agctgtgtat catctttttc tagtggcagt ttcccagtcg ctgttctgtg 240
   tattaggacc acgggaggag agaaaaggtt attcttcctt ctccattttt ccacacttaa 300
   atttatggta ggttttgaat accgtttctc caaaataacc tatttcctcc ccaaacccaa 360
   acttcctcaa ctgggatcga actcaatcct acagcgtgtt ctgagattct atactcgcct 420
   cacttctccc aagatcgtaa attctggagg agccattcct tcatcatcca tgtttgcatc 480
   cttcacaccg ttcactggtt cgggggaggg cgccctaacc gagaagctgc ttaatacaaa 540
   gagctccagg ctcctggcgg ttcaccaggt ctaaacagcc gggctttatt tgtgggggcg 600
   aatactgttc tttttcttct ttctttgcag tagcctctag ttcgttagtc aaaacgtgaa 660
   aaaaaagacc tgctttgccc tgggaaatag taaccctgcc aaaagcagaa tttcatttac 720
   ataggcactc cttctagtag aacttgatgg agctgttaac cggctatact tccttcttgc 780
   acaatgggag cgtccctatc aatacgtttt ttttcctttc tcccagctgg cttactaaac 840
   ctccctggga agtcctttcc taaaaccggg ctgttctgtt cattctatac atttatgata 900
   ctaattttat gaaggataaa actggtgctt gaacacaggt actcaaaagc aggttctgtg 960
   ctttcccctt cactagagag cctgttaaac ctagtttgta ggaaaaggaa aaagtttttc 1020
   ttagtgctct gaactagcga ggtaatgagt gacctggttt atatttctga gaatttcacc 1080
   ttagtctgac aaccctttgt tccttcaaaa ctttggaggc aacaaagatg tctaccatgg 1140
   tttcttggat caggtattag tcacttgtgg aagtggtcac atagttttgt cttagagaaa 1200
   tttgcctgaa aattcttctt ggcccctgtc gttgtatcct caattttctt catagtttct 1260
   ctttgccttt ggtgcccttt ttctcctcct catcccaccc ttatataaca taccatttca 1320
   tatttgtggg cccagagtaa agagctcatt ctgttacaga cactgaatag ccttgttttt 1380
   gtcagtagtc agctctgttt ggaaagccca cacaaaggcc ttgtgtatag gatgccctac 1440
   tattaaagga gcatgttcta gtctcagtgg attaaatttg ctttggtgtg cattccctta 1500
   aagatctcac atgccctatt tgttgtagtc tgtttgatga tccacgggtt ttgccttgct 1560
   cccacaactt ctgcaaaaaa tgcttagaag gtatcttaga cggaattcct tgtggagacc 1620
   agctccattc aagtgtccta catgccgtaa ggaaacttca gctactggaa ttaatagcct 1680
   gcaggttaat tactccctga ggaaaagtat aacaagatca agatctctcc caaaatgcca 1740
   gtatgcaaag gacacttggg gcagcctctc aacattttct gcctgactga tatgcagctg 1800
   tctgtgctac tcgtggggag cacaccaaac atgtcttctg ttctattgaa gatgcctatg 1860
   ctcaggaaag ggatgccttt gagtccctct tccagagctt gactttgaga ccatgaaact 1920
   tgctgttatg caagcatatg acccagagat caacaaactc aacaccatct tgcaggagca 1980
   acggatggcc tttaacattg gctaatcctt ctgcttggcc ttgtcattgt ctttggtcct 2040
   accatgttcc tagaatggtc attatttgat gacctggcaa cttggaaagg ctgtctttca 2100
   gaaatgggaa gactaccttt tttctggcag ctatgtgttg ttttgttcga aagatggcaa 2160
   gtgtacaacc aaaccaatcc acttgcttac agaaagaatg ttgctttttc attttgtata 2220
   ctgcaagttc ccaggcaact cgaatttgca aacacagcca tggatacact atttacctta 2280
   cagtagtttc ctgggaatct aaggttttct acgataaagc agtttcctgc ttctcgtttg 2340
   gcacgcatgt tagatggcag agaccaagaa ttcaagatgg ttggtggcca gatttttgta 2400
   tcttgtggag gtacatttcc taagccggaa cactcaagct tttttcaggg tgtttggctc 2460
   ttgcagcaaa acaattgaga tgctaacagg gaggattaca agcgccgacc tggaatggta 2520
   actttttccc tcctcagatg accttacttc cactgcctcc acaaagacct tcttaccatg 2580
   acctggtttt ccagtgtggt attgccttat ttttaagaga ttgtttcagt ggttcacata 2640
   aaggctcgct cactggtttc tcttgagttc cttacacact atataagttg ttctttcagt 2700
   acaactcaaa atattgggat aggctgtcag tatgttaagg atagttgctc ctgagtcaat 2760
   tcttcactta ctccctctgt tgttcttggc tggatcctaa acaaatgtcc tcggcttact 2820
   gattgacact ttattaaagg aaggctttca tttggtcagc actagaacag tatcttctga 2880
   agacaaaact gaatgctata gctttgaaag gataaaaagc cctgaagtgc tcatcacgaa 2940
   tgaaacacca aaaccagaga ctatcatcat accagagcaa tctcagataa agaaatgaag 3000
   ttcatctgct gccatgccgt ctctgggcaa ccaggcccca actgtgctta agccataatg 3060
   cctgctgctc tctagacaac tccatgtact tggtgctttg ctcaatctct tatattctac 3120
   tgctattttt cctctgtgct agggaagttg actgtaatgg caagtttagc aacgtagaag 3180
   ctaccgtgag gaaatcttca ttaagacttg cctaaccaca caaggctttt aggtaggtag 3240
   tcttctgtac cttcagattt cacaccaaca cctcaaaatc cagaaatcat taaagcaaat 3300
   tgtttaaggt tccatgagtt cttttaagat gaggactgag actgctatag tggtctatgt 3360
   catactcccc catagtgctg ggcatctaac aagctcaagt tatttttggc catcttccgg 3420
   aatttcttgt taaatctcct ttgaaatgcc acagttgcac tcttggaaaa cttgtcttcc 3480
   tggttttcaa atttctgttt agaatgccag gtctttgtgc tgtactgttt cttgaaactg 3540
   gagactagca cacttccccc aattcaatta cttgctttcc atttgagaaa ttaaagccag 3600
   tccaaacaaa tcaataggct ataatgcctc cctattccct gctatatcaa gaccaaaatc 3660
   ctctaattgg ttttctaggc aaaggctcaa tagcctagtt aatacaaatt catgtaacct 3720
   ttgccttagg ttttatggca cagatcccag gttacttctg ggggaatgtt gcattcaatt     3780
   ttggcttaaa ttcctaccta aaatgagcta ttatctgaca ctctgctctg tacagacgtg     3840
   tttctgtttc cattttcagt tccttctcca catggtctaa gtaccagtag tttagtcagt     3900
   catttagatc actaatgacc ccccaaaaga taacaaaatt tcacaggcct gattattcat     3960
   acccctaccc acactttgct ggttttgctg aaaaaaaaaa ttttaaggta cctgagacct     4020
   gaggaatgaa tgttaatagc tcacacagaa tgagaagagc tctgatcagt gaagtagtaa     4080
   aaggtagagg gcactcctaa tccaactcta tcacttatac acaacacagc catgaacaaa     4140
   agcagcagcc attcttgata atccaatccc ctctgaaaca aacaatttct ggcagtagga     4200
   tttcatttgc attgcctttc ctttaacaac ttccagtggg ggtagttttc ctttttgtta     4260
   atcacgtctc acacagtcaa ggtgtcgatc tgacaaagag cacaattaac ttctctggga     4320
   caggaacagt ttaagcttct gtttacactg tcatttctcg ggcaaagtta ccaagctacc     4380
   taaatacttc tttagcataa ttttaaaaag gagttctctc tatggaaggc ctaatttgga     4440
   aatgtacttg ggtccctata caaagtcgtc cagaagccag atgatttccc agtgtagtcc     4500
   gccttggatt ttgaagtttt cttaaggaat aaactgtaga gaaaccatag ggccaaccag     4560
   atggatgatg aaatggaaaa aacagcaatt aaatactttt tactgaatgt atatgaggtt     4620
   accagcctat ttactagtca ccaaatgacc tgagttcaat aagacagcct ctgggctcaa     4680
   gcttattagc cttacaggct aaatgctgac aaaagtggtc ataacagagg gtttgacttc     4740
   aaatctgaac cataggcctg ttttggcagc aaacatcctt cttccaaaat agccagactt     4800
   cacatgagaa attctgcctt actaaaaaaa atgtacaggc attagttggg ggagaactgg     4860
   agtgatgaaa tggcataaat aaaggcaaag agaaagaaaa cagaaaacac tttcaaagcc     4920
   atcaatgcaa aatttgcaaa gtcctgatca tcacacaata cctattaacc ccccacttaa     4980
   acactgtttg tattctaagt                                                 5000
<210> 34
   <211> 4900
   <212> DNA
   <213> Artificial Sequence
<220>
   <223> Nucleic acid probe
<400> 34
   taataatctc ctcccagtgt ggtatggttt gatttttcct ttccacttat cttcgcctta 60
   ggggctgcca aagtaggttt tatactatgc cttcaaaaag cttacttcta ttctgcactt 120
   gaagctctaa ggaactgtgt atatacagtg cctcggccac agaatgacta tccttgctca 180
   atgacagtgg aataatacac aattattctc attattaggt actgtgttgg gcagataact 240
   ggttccctat tccatactta tctctccatt caaaatggac tagctgtgag gaaatcttta      300
   tcttcaatcc tttatcttag tccactggaa atatcatcat gactccatcc cttaaatctc      360
   tgcttttacg tttgctggct gaaagaaaag ccactttgcc tctgaagaca gaatcattca      420
   gtccttatcc tttacacaac aaccatagtt ccaagtctgg tggtttatat tctgggctgt      480
   gggcctttga tataccagaa ctactcctga ccttcaacta aacagactac cttcctgtct      540
   accctgaagg tatcactttg gtgttgctat ccactgattc acttctgcaa acttccctga      600
   ttgttaattc ctttgaagga aaagggctca aaattgtgat tctcagactt ggagattata      660
   gggcctgggt ataaaatact tccctaaata acttaccaag gaggttgcca ccgctgtatt      720
   ctgatcctta tacagcacag aacaatcaga ttataaacac ccagcagcat ttgtatgttt      780
   cagcccagtg cagcattctt agaaaaatca agggaatacc aaaatgaacc tccatatcct      840
   atctctggac tcccaatcca gctgtactga aatctaaaac tatgccatac ttacttccct      900
   caaacaaaga gaatagactg aaatttggcc caaggcactt acatgaaaac aaagctctga      960
   taacacagat tttcctcatg tgactgagga aataccaaga gcttagatcc aagatgacca     1020
   aagaggggaa aaaaagatgc aggaaaagta gatagtttgt gggtatgaac aatccctcct     1080
   ccaagttcat tttgggcagt aacctttctg ctaaagaatg tattctctat actgtgtatg     1140
   tggtacagtg acagttgggc tcttcccacc tgggtttgta aaaacggatg ggtgcaggct     1200
   ctccaatcaa gaaatgacac tttattttgc ttacaaaatg cctctagctc ccaagtgccc     1260
   catatctaat caccaccata tgtttgactt tagacactat cctttggcca ccattattgc     1320
   atattgatgt tattctgcac aacagctgac aagcagcatc gatctcccag cctctgctcc     1380
   cagctgtaag gggtaagaac agatttccat ctcctgcagt tccacagttg atccctgaac      1440
   acagaggtat ggtcttcaaa atgaccctaa tcttgccaat gttcagagaa gctctaactt     1500
   aatgaaaatc aatgctacca ggaagactga taccttacca gtcccctttc ccccacaata     1560
   ggtaaacata actttaaaaa ggggtgacct attatagcaa tcgtaaagtc gagaattata     1620
   gtttagcgtt caacattcaa accccccagg cccgcttcct tccagccaat cagggggtaa    1680
   caagtccaga gtttagggag ctccgttaga cacaatttga aaaacatcac attgcactct     1740
   tctattctcc tccgggaaac attctaagcg agggcgggtc ccccagtctt catagaatat     1800
   aagaaataaa tcaatcgtta catcaattcc acggggaaaa actctgtagg caggcagtcc     1860
   taaagaagcg tgtcaccgcg gttcccagga agcgttattc acaactgaac agaatagctg     1920
   ctgcagaaag catctccgga atatttgcaa cccagagccg tgcgccccac acgcaataca     1980
   ccggcaaagg gttaatctag gctaaggggg aaaaaaaaaa tcaccgaagg gaaagacaaa     2040
   taacaactct cgtccctcct cctcagcgtc aagaaaaggt ggggatcgca aaattaccgt     2100
   tcggaggaaa tagccacgag aaggaaatcg ttagctcgaa gccccatctt tcatctcacc     2160
   cgtgaacact cctccccttt gagaaaggac aacgctgcga caaaaatccc cagaaagaag 2220
   ctaaagtcca gcatctttcc taagagcccg ctacaacctc aaaagcctgc ccctgcccca 2280
   tcttggaagc gcgagccgcg ggacccatga ctccgggtcc tgaattttct gtcgacctta 2340
   agtttcttgt cacgagatga cggccatcac cgggtcgaga aaacggcgag aaaaggcggc 2400
   agaagaaaga actcaattac ttaggtgctc aattagggca gaagtgtcag cttgctttct 2460
   tcaatacagt caagagttaa tcatccctcc cccacccctt aatttaaaat ctggggtttt 2520
   gctcttggaa actgaaatgc aatcaccagc taatggatgc taaatatggg tagtgtgaag 2580
   aacagaggtc ttgtaatttc atctttcagt catcagagag caaagtagga aagacttaga 2640
   gaggcaaagt ccctctgcac ctaaggatac actattttta ctctaagggg gccatgaggg 2700
   aactgaggga gcataaatag gttaagtatt atccatgaca gtagggtggg agttaatgga 2760
   agctcattta tagcagttct acttcctgat ttatgggtat caaagtcact ctacctcaaa 2820
   cttttccaca aaagtatggt tagtagcaga gaggagtgaa caattgcctt aaaaacttgg 2880
   tcccaagcta gaattctttt tttacggttt agccaaggct atttatagta gtgtatgtca 2940
   aggagtagac tgattgatgt tcaccctctt caaatgtgag tgtcacagaa actgaaattc 3000
   tttcaagtaa gatttgttca agaataatgg gcttatgttg tcagttgtgg gttcaagcag 3060
   taattcactt tggactgctt ctattcaaca gtatgctgtc gactcctgcc acaacaatga 3120
   ctgaaaggtt caacaccagg aacccaagag ccaggacatc tactgaaggc tgttggttaa 3180
   gctgtatatg tttctggcca gaagaaactc taccttcaac caagttgtgg tgaagaggtt 3240
   atttatgagt tgcaccaaat ggccatctct gtctctttcc tggatgatct ggaagatgaa 3300
   aaactgaagg tgtgtgcact gcacatgagc agttgggcct gcagccaaat ccccaaggct 3360
   ggggacaaga ttctcacctt tgaccagctg accctggaca ctgtggcacc atcctgctct 3420
   ctgggcctca caagggccaa gaagtgtact ggcatttcag caaggccctg ggaacccagc 3480
   atagccacac taagccctgt tataggtaaa tgttcctccc aggagtggta aaatgctgtg 3540
   gctttgccaa ctaactccag cttctctcat gggttttatt gtggatgatt attgatttgt 3600
   cttgggtttg tccaatagtg atggctacat agtcttccta ttacctttta tctacaggca 3660
   ggcagtaggc cataatagta ttagaagccc ctccctttct aatagcacat tcaggatcat 3720
   ggcaaaatca gctaagtcat agttaacaca cactccttgt ctctacgtat gtggtgctaa 3780
   gtcatatttt tggtatagct gtataacatt gtccagagtg gagttttcat gttgcgagaa 3840
   agccatgtat aggccggagt acatgagtaa aaggaataac aactccccca tcccctgcca 3900
   tactttaaaa taatttcctc aaccctgcct tcattcaaaa tgtctggaga cagactggcc 3960
   cacttccaat tttctttcac gttatcatta ctgtgaagtc cacagtttgg ttggggttgt 4020
   tgctccttca gaaccgcatt atgtaatttt agtgtttact taagctcctt tgggctgcct 4080
   ccgtggtagc tatgagctca aagaaaaaag ttttcatcac cgtggaggta cagctactgt 4140
   gaatgtccta cctagtttgg gagagaaggg tatgtgtgca caggaggcct gacctggagg 4200
   gttggagatt aagtgtgctt ttgaagtgca aaaaacacca tcatggggac tcagtctatc 4260
   ccaacacctt tccttgaggg cttagagact gagcagggga agtccacacc ctaaaccaga 4320
   aatctgggta tctcttgctc ttgcccttta cagccaacca gttttcccat tccaccaaca 4380
   gaattccacc aatttaatta acttaggtca caggctgttt tacattccat cttcaagtct 4440
   ccaggacttc aaccagtgta cacctcaata tgacatttcc aaaactcctt tcatggctct 4500
   tatgtatttt acaaatcctg caactaacaa cgcagttctt gatagactgc gatctactgg 4560
   gaggactgct cggttttcac ctgaattatt gctcgtggtt aatcctccat ctactccaga 4620
   aaacgttcta actttttgag acgaactgtt gtaactagtc tcctggcgag gcctgctatg 4680
   tcttatacca caggctctaa aagcttattt cccttctcct aacgcatcta cctataaata 4740
   gcgctcaccg cgctgagtca ccaaaacccc gagtttcccc aaatagcgcg taacgactgc 4800
   gttcccttct aaaagtggag gctcggggga ggaaaaagaa agcccacagt gttgaaaggg 4860
   gcggtacgga ctgacctgtc aacggcggag gaggaagacc 4900
<210> 35
   <211> 5000
   <212> DNA
   <213> Artificial Sequence
<220>
   <223> Nucleic acid probe
<400> 35
   tttcagcaaa gctccgagga agttggtcct tttgttccac tatttataga ttgcgtcaac      60
   attgcgaaaa ggagaaggcg gagcggtggc ccctccccct aagccgctgt tcctgagctt     120
   catgtaaaca aggagggcgg taaacaccag aggaaggcct tacaacaata ccaagtgcaa     180
   gtagctcatt tgagtgttat gtttaacttt acagggtggg aataaaataa tttggcttct     240
   cagctcactg gcggaagaaa ctagagctga gctgggacga gggcaagaag cgcgtcgcac     300
   agtgctgaga gcgctttcgc tgggccagtc aagacagcaa taagtttgcc tctatttggg     360
   aaacctttcc aggcttgcct gccctctttt aagagggaga cctagcttcg cgccaagctg     420
   cagtgtcccc gacacactgt caggacacgc gcttttcgcc cctactttgt tcttgcgcag     480
   tcttcccagc tgcggagagg tgctagaagt tgtccggagc ccctcaaccc ccttggaaaa     540
   gcgaacgcgg atgttaatat accaactccg cagggaactt ttggcccggg tcttgagctg     600
   gcagaggcct cctgggattc taggcccaca gaatttttgc tccagtgaag ttggataagc     660
   actccgcctc cccgcaaaaa ccagagttct cggcttataa ttcccagcga accaacactg     720
   gcagattggc ctgcgagccg ctcgctctgt tgctctagca catttatgta gccaaacggt 780
   cgaagagccc catctttaag tgtgcttcct ctcactggca cctgacgcaa ggcgcagcca 840
   aggactatta cacctctggc tgctcggacg catcttcgca ctgcgccccc tcgctcttta 900
   tcaaaaacag ctgtaatttt caaagatgca cgatggagtt cgcttaggcg aacctcggga 960
   aggcagtctt ggtttcctgc taatgttttg ccggtaacag ctccgcccaa atttctctat 1020
   acgatgctga gaggcgagct acaggtgaaa tagactccta ggcacttctc ttagaaaaag 1080
   ccttctattt cctctttgaa cctaaaacgt tatgcgtctg ggcagtcccc aagagctatc 1140
   gaaaggccag ccttgctcac cccattggaa ccagcatcag aaacctcgat ttagagtcca 1200
   ctttctcggt gaaatgggaa gttgcctaaa cgatgcatct ggctttagat tttatgggga 1260
   atatgatctg gaaaagggaa cgcctgtttc agctggggca ctcgcctggg gtctctggca 1320
   gtggctgacg aggctttttg accaaaggag aaagtttgag gctgcagatc acatgtcctt 1380
   aaaggatctg ggagagggga cgaggcgaaa agaactatcc tgggggagca caagtgttcc 1440
   cgagactttg ccaaagtttc cagagtccgg aggtagcttg cctcccaccc ccgcccacca 1500
   gcgcttctcc agtcgacgtg gaaaaaaaaa tcgtctcaaa tagcacccct cggtctgcag 1560
   ccctttaaca ctggtaaagt agataacaag gaactctgct aactcagttt agaacgtccc 1620
   agagtttcca agtttgtcaa gcgactggga atcttggtcg ccgcgtggag agctgcgccc 1680
   atttcccctt ccagaagaaa tttttgatag tccggaaaag aaacatgaca aatagactca 1740
   ccatcaggct tgcgtcagag aaccattttc cccagactat agtgagattg tttttttcat 1800
   tttatgattt acctacctgc tgcagttgct gaatgtattc ttctcccctc cttttcccca 1860
   cttcatctgg ttgtcaactc ctttccctcc ccgcccccat cgctagtcta gcccagcgtt 1920
   tattaatggt caatataagc cacagtgttt gcatagccct cacaaaatgg gtcatgtttt 1980
   cggttccttt gatctgcagc cgtttgcttc tagcatttct gctacagcag gcttcgccgg 2040
   cagactgatc tttctagacc ttgtctcttt cagagcgcca attttatatt gaggctattc 2100
   ctttaaaaat caaatgagaa aatggagaaa gagtttgtag cactcacggg tggagaaaac 2160
   ccatctgcct gatccttttg tcccggtgcg gagtcgctgg cactactctt taaatagaag 2220
   agaattcctc cagtttgcct ccccttatag gctttcccgc ctcaaattgt tttaaatgct 2280
   cctcacagtc tttttgcccc ctcttggagc ccagctgccc tgctgagaga gcgggaggat 2340
   agtcgcacct aattagtacg cggctccggc ggagatgagt gcgtgggcac tgtgcgtgca 2400
   ttcaaagcgc cctgcttatg aaaacttggg gcatcaatat cgtctcatca ctcaggcgcc 2460
   cttagcgaat ctggacagat cgaggggcgc agtcttggct gtttagaaaa atacacctta 2520
   tgcgaacata tacagctatt ttctcgccct ccactccagt ggtctgcaag cccctggtgc 2580
   tagtgtgaat attcctgagt ttcccgcccg tgcagagcca agaacactga gcggaggtca 2640
   ctgtaagttc agagccgtgc atcctggtta gaattgggag aaaaaattct aagcccgccc 2700
   ccttctgtgg ggtcctggca tcagcgtgac caggacatgc tattattatt ggaaaccgag 2760
   tcttgaagtg gacccccagc ctccacccaa gccctgcggc gatcaggggc ggagggaatt 2820
   ctgtattcta gttgtggatc tttcgggtct ctgtcagtta acagaagcct ttggggtgtg 2880
   ggtccgggac gccgctcgga cgcccatggt ccagtggcag ggaagcctca ggctacggcc 2940
   tagatggagc attcacaagg gcgtttccag gccccagtgg cctctgagca ggggttaact 3000
   gtgggttaca ggcctggaaa agattcatcc cctgctcaca catgtgcctc tttcttggga 3060
   cgttgtgtgt tctccacact cctgggccgg tcaggagggg ttctaagaag acaggatgct 3120
   cagtctcaag gtgggacaag gccagagttc cttgcgacca aactgggatc ggaaatctgt 3180
   attttcctgg ctaaacttct ccctaggttt ccttctggtg tgaagatttg aaggattcac 3240
   ttttatcaag ggagctgatg tttccttgcc aggcaccaca tatatccctg tgctattaca 3300
   tatatgagac attcgaattc actggagaat agagagcagg ctttatgctc tcaggaagtt 3360
   gggttatagg gttggggcat ttagttcaag gtagcttggg tgtattgact gagcccttat 3420
   tcttatatgg cattcaaaat cctcctgcaa acacagtatt tctggggctc agaattgccc 3480
   tgaattaata actgtgctag actttccctg ggttgtgact aaaggcagct gtcattatca 3540
   tgtaggccat gtgaggtttg tgactggaga agggattatg gtcatgttat aatgttataa 3600
   tctgcttaca caggcattgg ggaaagagag ctagaccact tgattaaagt gaggtggcct 3660
   agagaaaaac agctttacag cctctgacaa tgttgacaag taaagaactg cagtagctga 3720
   agaagatgga tgaggaggag ggacgccctg acaaccacca gaacagccat gcattcagta 3780
   gactgagggg cctggcatca tttcctctag gactgtttcc atttgacata atcaggaaaa 3840
   gtacccacct gagttcagga aacttggtga aattcaggct gcattttagg gggtgagtgg 3900
   agacattgtg gacaatgcat ttgggttctt gtttcacaca ccagagcttc acttttcatc 3960
   tgcaacggca ttctgaccat ttttaatgag cattagttat tctcaatgag acattaccct 4020
   agaacctcta ccaccatgac taccagcact tctgaattga aactgtcttt ggctgtcaat 4080
   ttgctttggg gaccttttct cgcgtttgtt cattggcact gccagagcta gttacaactt 4140
   cccggtgcaa gcatgtgatg gccaggggaa ggtgtgggga ttcgtggctg tcctccccat 4200
   gtggtgcctg agacacagcg ggaatccgac gactctgaga acggactttc ccttttaaat 4260
   gctggaggct ttgaaatcgt cttcacaatg agggaggatt ttgctttgta tttaaggaca 4320
   caggttaagg ccgtattatg aaaggtatga tttgggtttg ggggagcagg atgttgttat 4380
   gcgtgagggg caaagtgatt atgaatgaca attgttactc tggagcatca ggcatgtaga 4440
   aagtataggg attgggctta atacaaattt agtggcataa cctggcagtc gcctatttga 4500
   gttctctgaa tctgtcagtc aattatgtgc atagttcgca actaggacgg gatgtcctgt      4560
   gaagaaaaga taggcattaa gcccctgtaa gtatgaggat aattgacccc tctgccatgt      4620
   ggtcccctcc ttccctccat ttggcctttt atccttggat ttgggtcaca acctttgggg      4680
   aagggtggaa acaaagcaga aagcagaaaa ttaaactttc tctctccctg cattctgcca      4740
   acccactctc tctttctcag tcacattccc tgtagggaag tccatttata gtttgaggta      4800
   taaatcaggc cattccattc acactactct gcactctgtg atctttcatg ctgcattact      4860
   gggagtgagg aggtattttc tagttaatat ctactgccct gatttgtatt ccaggaacta      4920
   gagtctgttt tgattgagcg tctaaaaatc cttgttaggg agggaggaga aaagtgccca      4980
   agatcagtgg gtagggcccc                                                  5000
<210> 36
   <211> 4900
   <212> DNA
   <213> Artificial Sequence
<220>
   <223> Nucleic acid probe
<400> 36
   accattttaa tatagcactc cactgtgggg gttcagttta tgacaatgga atacagccat      60
   aactagtcac aaaggaaccg agatcatgat ataatgggga tagatcagat gatcacaata     120
   caactggata aaaagatcac cttgtcactg cattcagttt gtgctgtaca gataggcctt     180
   ttattcctct gtcttaattt aaagacacat tttaaataca cacacaactc acagatgtat     240
   gcagggacat gcatacaact gttaaggcag cctgctttgg tcctgaagaa tataatttga     300
   cactcagatc tatacagcct aatttgtttt tacgaacact agcagagtac ttcaggttgg     360
   agtttttgcc taaagaatat aacaaatggg gagtaagttg agccagtatg acctatttta     420
   ttgcagtcta aagttgtact gaagggtgcc ttggaaaaca tgttctccaa atgttggtca     480
   agactgacat tcacagaatt tccaatgaca gcttcggaaa tatgatgact ctgcaagaaa     540
   tcaggggaaa ttcagaatgg ctgttaagac cactcctaag actatcttga ctatgtgaat     600
   gttttttctg tgtcatccca gcttgccatc tctagctcct gcttcacagg cctcgaacta      660
   cctctccagt ccacttcagt gccttaacag ctcagatttc tcatttaatg cttcattcac     720
   aaagattctt aaagaagcaa ccacagtgag gacgtgcttt ggcaggcaag gagaggactt     780
   gcactctgtc tcccactcca gcgtgtcaga ccatctcaga acaaggaatg tttgtctcag     840
   acattattat gccctatctc ttgatgaaag actgggatag agtcaaatca tgtgtcttgg     900
   atcaggtaaa gccaagtgta ttttagcctt taaactggga tatgcattcc ccacctgctc     960
   agactgaaaa ctgtcttgta ggattaagag gatggggagg tttgctggcc tggatttcga    1020
   gtttagtaga gtacattcag ctgcattgtg catttcagct ctatggaacc gttgtgcttc 1080
   gtccctgttt acctcatcct gtggtgggac catcccttat ttagtgaggc tgtttttttt 1140
   ccccccagac ccgagttgtt gttgcccatg ctgctgaatt cccaatatga ataaatcata 1200
   ctggcttcca gcagagagat tttgtggctt ctgtatcctg ccgctgcact tctcccttct 1260
   ccagcctcct ttctatttgg tggtgcagaa tcaccttggt cttgatcctg ctgcccttgc 1320
   atacacaaca ctcccattga cgctagtgaa gacttaagcg catgaaagga ttccaggatc 1380
   aaaaggttag ctctatgggg cttgtgaaaa agtgagaagg catctaattg aactatatcc 1440
   agaggagatc atggcagaga ccaacaggaa gaaaaacatt tccaaaccac agcgatattg 1500
   aaacatttct cttgcatttt cacataattg ggcttgacag tttcctgcca ttgtatagga 1560
   taacttcagt tgatggtgat catgtttttg cttaagggtc ggtgaaggga gcaaactttt 1620
   ccacttaaca tgtgcccatg gaaatcaggt acttcttttt tcagctacct tagcttctgt 1680
   tgggaatttg agggtgtttt gtggttaagt aggcttgctg aaatggatct agaaaagacc 1740
   caggtcagtc actggttgaa aaagggttga cagaaaatta aggagatatg actgagagca 1800
   gtttctcttt caggtcccca aatttctcct ctagccaatt cacattaagg tctgaaattt 1860
   cccccattgg tgtcattccg gccattgttt ccagaaggga ctttcaaaaa tgcaaatatg 1920
   ttcctgtgga gcatttgcac atttggtgag tttgtcagcc taaatgagca gttatctttg 1980
   gaactaagaa agaagggtct cggcaatggc agtgacagct cagtccagtt ctcacagtgt 2040
   gagaagaact tgggacatcc agctaatgga tctacaagag ttccaggtgg ccctgaagaa 2100
   attattagta ctgctcacat catgaggtat caggatgttt tttggcgtgg tagaccatct 2160
   attcagatga ggaagtagag tgcatccatt tatgaattta gctgccacag aagacagaaa 2220
   gtcatatata gattctccct ttcctcctca ttgcttcttc ccttctctca cccccatgcc 2280
   ccattcctta tctttttact caaaaaggtt aaatgacagc agtagcagtc acccctctta 2340
   tgcaagacat tctgggcttc atccaagata ggtggagcat tgctgtatca ctctcatgca 2400
   ccttactggg tcagatgtaa tcctggccca ctagccactc atgcctcttg cagggtcatg 2460
   aagctgctta aaatgtttgt tcggtaactt aattactgtt ggacagagag gcattctcaa 2520
   gctcctggcc catcctaccc aattcaggta gcaacaggag gggaaagagg cagttgtttg 2580
   tataaggcat gtaaatgaag tcctttggag ctgaaattaa tggatatttt cctttgaagg 2640
   gtcgactctc caggctatat ccaatacaat acagggacct cacactgtct ttttgtgagg 2700
   gcctttcctg aaactagaca tttaagcttt gatgagctga caaagggggg atgggcagac 2760
   aacaataacc ttgcagtcta ttttaggctc ttaaaaataa cacaaagtgc ccctttataa 2820
   gcatgtgtac aagataggaa actaccaaca tgaggggaaa aaaggccaaa aggctgatct 2880
   taattattga actgtgctgt ccgagtcttt gatatataga gaccctatta gacccatcaa 2940
   atgtcctctc agcccttgga ttatttctct tccaactctg ttttcctata atatgatcta 3000
   tgaggaaatg cgttgtcctt tcaaaatgaa ttttgatctg agggtagtag gtagagagtt 3060
   ggggtttccc ctgggattct tgtctttaat aaacccgaga cacaatggtg ttttctctag 3120
   atgtaaaaca aagtatgcag tgccttcatc agctgtgtag agtattgggc tgaaagtatt 3180
   ccaatttagt gtgtctgcca attcttcaat caaaaatatt gatgaacaaa gccctgggcc 3240
   aggtgctgga ttcccttgaa atgtaggagt ctgggaacta ggatttggca ttcacgggtg 3300
   aaagaaaatt gcagcttttg atgaatagcc tgataattat gtcccttagc ctgctgttgt 3360
   ggaggaatag gagtcatttt gaaatgggga atcagaagca aaaacagcca caagtcaact 3420
   gtcagctgag tgagtgcctg tctggacgtt atcctgcagg cagttaggaa gaaacattgt 3480
   ttgaagacgc ctgtcaccag ttctaaacta ttgagtcaaa taagctcact caggtattgt 3540
   cagaatgaac ccacctaatc tggttaacat tttggtgtct gggcaaggtc tacccaggat 3600
   ttatttgggt attgcgggag acctggcata gtcagcatcc agcagtgttt ccagagtggc 3660
   tggccttgct gcttctcctc acttgctcct caagggcaag agactggaca atggctggtt 3720
   ataagtagtt gaataaaatc cctgcagttc atccattctt ctcctaccag caggtgtcat 3780
   tatggaaata tgaaaatact tagaattact caaatatcag taccaagtcc acacccttaa 3840
   tcagagttgc tagggccctt catgacttgc tgatgatcac tctaactcaa ctcttgtacc 3900
   cttggaagct ggctacttca cccaacatga atataccaaa gaccatttgg taaatgacca 3960
   gctcaccaaa gctaattctg caaaaatcac ttgaccaaat cttaattact tttgcctctg 4020
   tccctattgc tcctgcttct gcaccttcct gtgccctggt gcaatggcta tgagaaattg 4080
   ctctaaaccc tgtgtaaagg tacatttgat gtaaatttgc atcacttcat cagaagtcca 4140
   gaacctgtgg gaatgggggt gcagagagtg aagaagcagt tttagactga catcgttggc 4200
   tggggcagac ggaccatctc tgccaggaac tctactcctc tgcctaaatg accatgcggc 4260
   cccccttcca agggaagtct ctaggtggac atcatgtgac cagaaattcc tcaacatttt 4320
   tcacagtgta tatgccctct ctagtttcca gtactgataa aagcttttcc ctgtttttgt 4380
   attccgcttg tcacttccct aacattacct gagatgagag aggcattatc gtctgctttc 4440
   agcaggacac tctgcttgga gccctggtct gggtactctt ggattttctg ctctcagcat 4500
   gacagaccct agcttcagtc ttgaggatgg ctttcagaaa atcacggctg gatttccttg 4560
   cctgtgatct tgtttttgaa ctgcctaaac ctgcactgtc acagaagacg aaatggatcc 4620
   tccatctgtc tcaccataca acccttgctg tatgtgtcaa cttgggttga aatcttatag 4680
   atgctgttgt cgatctctga tagcatgact ctccataaag aaaagcccat gcttactccc 4740
   aagatgaggc agtgagcctt aataaaaaag gtgctggaag taatccccct ttaagacagt 4800
   gcttctgaac cagattccta tataacttct ctcaaatgca gacatagata cctgcctctg      4860
   ccggtcttga tgctaggata gggctgatgg tcataggttg                            4900
<210> 37
   <211> 5000
   <212> DNA
   <213> Artificial Sequence
<220>
   <223> Nucleic acid probe
<400> 37
   actaaggtgt ttaaagtatg ctcatgtcac atctccttgt tctagcattg ttaggatgaa      60
   ttgcgaactc ttgactctga aactgggaag tgctctcttt tttcttaaat taacttctgg     120
   ggcaacatca actctggagg cactgtctcc tttctctacc ttaaggataa ttctgagact     180
   ctaaggacag aatggtttct tcagccaggt gcatttgctg tgaggctaaa catccaaaca     240
   gatttagagt gagcgacagt gggtattcag gtcaagtcca ccagtttgat gttccttctt     300
   acactgacct tccctggtgt atgcagtcct ctggaacatg ttctgtctag ctctggtggg     360
   ttttcttgtg agaataagcc acattgctcc agaattttgg tagtaaggtc actatgagga     420
   gaggtataca gttactgtta cagtatttac caaacagtgg gcccacagcc acactcctca     480
   cttctccttt tggtctctcc tgctgtgcct cattgctttc cagtctgagg atgacttcta     540
   ccccaaatta ttctttgagt tactcctagc aggatgagag caggacacag ggctctaggg     600
   cgggatgcta acatgttgta ctgatgttac ttctgtggca aaacttctca gagatcttgg     660
   gcccagtaac tggtccaaaa gaaagacttg agaggaagaa cctgccttgt actcacccct     720
   ttgggctaat gagattctca gctttttcca aacgctgact cttctctcat ctctaggtcc     780
   tttgggtaaa catctcttcc cctcctgaac agctctgact tgcttttttt ctgaatccag     840
   catcagtcct acaattagtc cattggccag gtgatggtca tgtgcctggt tagagtaggg     900
   tctcactttt cttctcagtc ctactgctcg ccccagcttt atgaaacatg agctatgtcc     960
   taagggaaga gcaagtgggc ctcaggtcct cagaaagcac ggtagcattc tgccttagat    1020
   atgagagaca cttcccaggc tcttcctgca tttccatctt atgctctggc aagcaaatgc    1080
   caatcttggc tcttttccac tattgtttga catgtgaatg caataaacct ctacccacat    1140
   aagctacctc cattgaagga ttccatactt ggcacagaat ttctaactga cacacactaa    1200
   tctcagtgcc tctggcatct ccttcttttg ctatgtacca gccctgtcac ctagtccctc    1260
   ccactattaa tcattaatga cttaagatgt taccacctcc tttacctgcc caacctcctt    1320
   taaacttgct tctcatagta ttcatccctt cccctgaatc cttgatcact tgtactgctc    1380
   atctagcact catttgtaca ttaaaaatga gacccagaga tcttgtgatt acacaagatt    1440
   ctgtagctaa gattacatcc ctgttactct gactgtcccc ctgccggccc ccaccaatcc    1500
   catcctagaa gaggaaggcc aggatcaaca tgctgatgga ttgactggcc ctggatagaa 1560
   gtggggtact ttctttttga acaagaagtg gggaaggtaa ggaacttgag caacatggaa 1620
   caaatttgga aatgctgctg taaggaaaca caatacagga atatgcaggg cagaactgag 1680
   gtttgagctt taaaacgtaa aagagaatgg agatgttgag agtgttgttg aagcgattga 1740
   ctaggatagg ctctgaatta ttgatgacca agagttcaga acttgttaca tataggtcta 1800
   gttgatggat ggtaaaggga gtgagaattt tttccagcca cttgtggaaa ccctagggtg 1860
   ttgagtcacc gtcttcaaga tggagggaga gagcatgtct gccagcagtg acatcacggt 1920
   cagaatgcac tcttgctgga tcaaacagcc ctgagaggaa gcatggggcc tcatttttat 1980
   gagaccaact gggaatagcc tgcatgggca ctgagccaaa gaaacagtct tatgaggatt 2040
   ttctcagcat ttcctctggc cacaggtatg gaaggaagaa catcaggaag gattgataaa 2100
   agttcaaaag aagagactac tgaggaaggc aagaacagca atattttgga aaagacaatg 2160
   aagagtgaga gggatggcct tgtgatgggg caggcctgga gcctctatgt ctctacatca 2220
   ctcaaaggtc tatctactcc aaactccagg accgggccag ttcagatgct catcctgcct 2280
   ctcaaatgtt ttggtttttg tttaacagtt gccaatactc cttcttgccc ttaggtttct 2340
   ggtctcctca aaattagctg tagagtggga cacatttacc tggttaccct tcacttttct 2400
   atacagtgat acagaatttc aggaaatgta gacttgtctg ttattcaggg acaggttggg 2460
   gatgtcttgg ctgccgcagg actgtatcga ccccgccacc gcctcctgcc ttgaaagtct 2520
   tccttaggca gaacccacat aggatcatga tggatttggg tggttgatgt acctgcttgg 2580
   tatagaagga tcaagggtta tctttgggag aaaacatgct cagatatact tctttgggag 2640
   aagacatgct gtccctcttg gctggagctg gagctccccc atcccgtgcc tctctttctt 2700
   agccccctct ttcaagctcc ctgtcctgct gacctatgct ttctcttggg aaggactgtc 2760
   cccatcctct ggagggacat cctgctgctg tatcccttct tatgtcttgc acacttgagc 2820
   tcatcaggct ttctggcagc taatggaata cccttccttg gggatggggg tgacctctgg 2880
   gccttaatgc ttggaagggg gcttgagctg gactctgagg agcactggcg gcagctctca 2940
   tgtcgtctct gaaaggctga gctagccctg tctggcagag gccttcactt cacagcactc 3000
   tcctgttctg tttggaattc atttccctta gtgtgacagt tgagataagc tatttggact 3060
   gcctccaggg agtgctccag aggtgggcag ggaggaggcc ttgggagctc ggctcagcca 3120
   tccctagtga aatacagtgg aaagcggcag ctggaatcag atccacaagg aatagcctga 3180
   gttctccaga ccaaagttat aagtcacagg cagggctatg gtagtcattc ctaggtatca 3240
   tcccagtggg acttgtgtac ctgggatccg atggtgggac tgctggctct ggagggctag 3300
   gacagcctaa tggcttcaca ggtccctgac tatgctttag atccctgaga gtggggacag 3360
   tcactcagag ccatcaagta acaccaaaat gcactttact ttctgaaaat ctagcaacga 3420
   tcccattctg aaggcccagg agttttgttt gtggatcaag gagaaatgaa gtggcagagg 3480
   gtgcattttg tttggaacat gaggcaaagg gaaatggagc tggagtgcaa agggagggga 3540
   aggcctgtca aaggcaacac atagtagagt gactgtcttt gaacagcttc gtgtcagacc 3600
   tcagggctta tacaacaaga accagttttc tccagattct gttccttagt tggaggccaa 3660
   atcaacagat gtcatatgtt tccatccttc cccctaaggt tgcgtgctaa ggttttatct 3720
   gatgatgact gcaagaatta tttccccatg ggtcagtccc cttggggtag ttgtataatt 3780
   acttgcctaa ttaatgtata agactatatt gagtgaatct aacaccatcc ctggtgctcc 3840
   aaaagcagcc atttcttctc acttgtactt ttgacccagg ggttcttggg ctcattcaca 3900
   taagcagact gcagagaaag gaagggcttt tcagtttact cgtcgcttct agttggctaa 3960
   gggcatctta tttgagtaaa tatgggtgtg gtcattaggc tttggaatct cttgatggac 4020
   acttgacatc tggaagattt ggttttccca attccatagt tcttgaacag ttggccccat 4080
   tttagactct gacataataa cttaaggatg accttttgct accaaaacag tttaatgccc 4140
   attctaccca gtcagccctg ttcttcccta ggttgctgac tttgattaaa ttcatgggtc 4200
   ggtttattaa aaggccacaa tggtgttatc tcacagaact ctctggaaga aagtccctag 4260
   gcctcaagga gaccaggaag aggacccgaa ggtcagaagg ttgcagagcc agccttctga 4320
   gcatccctga tcctgtaacc acatgtccac aagtagaaca aacaaggtgg ctagggtacc 4380
   ctctttccca agattggcca aatctaatct agaggcgtta gtaatgcaga aatgtcatat 4440
   agggatagac acaggggaga tgtaagggaa ctgtttttac tgccctgctt ctaacatgat 4500
   gagcaatcaa aaacacacaa gtttataggc ttcaggccaa catattttag tcagctgaaa 4560
   tcagggagac actgcctggt tggctggctg cctccggacc ttccccgtgg cagctgtgaa 4620
   catgtggatc ccttgtgcct gaggttctgc tggtagctcc tgcagcccgc cagactccag 4680
   gctcaaggct taggggtgtt gaacatggag ttgcttttct ttgcagttgg ttgtagctta 4740
   aaccatagtg cattgctgag tcgtgtgatg tgtgattcat caggcctcag gtgacaacgg 4800
   gagatccacg ggtgctctag atatagccat atttcagccc ctggatcctt tgttagtgcc 4860
   atattaggag aatgcctgtg gaaatggctt cttttctcca agtttttttg gccccagtgt 4920
   agaactagtg tctctgttct ctgctgtgac ctcacctgga aatgtctatt agtttatcat 4980
   gccatgctgg attaaagaaa 5000
<210> 38
   <211> 4900
   <212> DNA
   <213> Artificial Sequence
<220>
   <223> Nucleic acid probe
<400> 38
   gttgagtttt gtactgtaag ttccaccggt cagcttctca gccagacttg ggtaatttat 60
   gtccagggtg acaggaagca ctgaggaaag agcgttctca gcagggggaa tggcattggg 120
   atgttcccat gtctgaaggg gacaaggagt tgggaggaac tgacaaaaga ccactgaggc 180
   ctgaggtgtg gaggcaggtg cggagaaact ggccctagct cagctgccag tgaccagtga 240
   gatggattca gaatgttgtc ctagtgaggg tgctcagtga caacgcaggt cagaggcagg 300
   ctacaataca cacagctgct gcctggctgc gtgacaaagg tgtcttggca gaagcatttt 360
   ggctgtctga aatacagcct ttgcacaatc ctgagactat ttcattttct aacatccagg 420
   cacgctttta tggcatctgg agcattttgc tgaatgactt ggtagtgcac tgctgaccag 480
   acagcacatt tccagattta tgggctactt tatcccatgg ggaccaatcc tgtaaagtgt 540
   attcaacaga gatggcccta ggtgtttgct tttatggttg ttagccacat tcacactggg 600
   tcttaatatc agaaagaaaa ctggggagag tgtcaagaat tggcatgcac aaactgatag 660
   aattgccagt ggacgttctt attttctgag catttatgag acaaacattt ttatacattt 720
   gtactagaga tgcccagtat tgcttgctca ccctcagtcc ctgttgtctt taataatcgt 780
   gttatataca acacaatttc tggtcccttg acttcacatc cagaaatgtc tacagctcag 840
   actgaccagt taccaggttc tgtgtgcact ggcctacaca gatattatga gtccttcaga 900
   aaaggtcata ggtagactga tagctaaggc agccattttg gtcatgtgca aggtatcagt 960
   tggtaaaggg aaaaaaatga aactcacatg tagagagaga ggctttcata aaacaaaaca 1020
   acaactatgc aaaacctacc tgtgtcctct gtggttggat tgaaaaaaaa gggggtaggt 1080
   tgtgaaatta agctgaggac ttgcctgcaa acccccacgc tttttagcaa atattatttt 1140
   gaatgtcaga gtgcacagag ccgttctcta gggttgggct aaatttgact ctgtgggaga 1200
   ataaacctag aattccaaat gtggtgatcc caacagagta tgtgatgcaa gaccaatttt 1260
   ccacttgtgc cgggtgggtt tgttcaaatc tcacacagaa attctttgtg caaagcctgg 1320
   cggttgcatg aagaattttc tcctgaaatg ctcctctatg caatttactc ttggttcaag 1380
   tcgatgcttg tgatgctggg aaaaaccaag agacccagaa accctcattt aagcaaacag 1440
   cctttgaaac agaagcctga ctttagctat cacattcata ttcttatgcc aagagagatc 1500
   atattaaaac tttggtttct tttagggagg gagatgttac ccttgtccca gtggaagact 1560
   tgtcatgtga atttgctggt agcctccctg gggttgtgag ctttccttct ggttgattca 1620
   atcattcata tcctcaagaa aatgtgccaa tctggagctc aggagatcct gagaatatta 1680
   acaataacag caggctcaag actcgaatct tctgttccct ctgtcgagat gcctcgtctc 1740
   tctaggtctt gctgacttga atgtgtgtgg agagttcaaa gccaggaaag gatatgtttg 1800
   cctcactaca ggccaccctc aaagtgggcc agttcttagg gctcataggg gaactatgac 1860
   ccagtgtacc cctacagaaa agccactgtc tccatggctt tgtctctttt cattatgtcc 1920
   atcctctcta ctcctctgga ttacaagtgt cgtgagaagg ggggttgagg ctcctatttc 1980
   ctgtagggct tagattactg gaagccctta aattcttgat gtaggaactc tccctccaac 2040
   ccagagtgcc agggttttgc ttgccacctc ttctgatctg tccttacctt ctccgtgttt 2100
   ctcaagccct gctcccattg cattggaaca gtgtgtagct gtgcacactg tccacactta 2160
   agaaagggtg atgccagact caaaaagcct tggggaagag tgccatttct ggcctcttgg 2220
   aagcacctct gtaaatagtt ctgcttaggt gttcagacct gggtgggcaa gaggggtatt 2280
   cagtgatttg catggaccca atcaccttcc agtgccacta aatcatgttt tctgagcaga 2340
   gcagagtcac agctccctac tttaccctgt ggtattcagc aggccaaaga tttaaggaag 2400
   taccattcac tgggcactat taggtactag gtgtccgggg atatttctat tgctgttctt 2460
   agctctattg ttggtttact tggccagtgg ttgctagctt gtgggtgtaa aaaccagact 2520
   tctctctctg tgaaagttgc tgacatttta taccacatgc tggaccgtta aatcacaatt 2580
   tacgtggtca cttgtaaata gcacactttg acatgcttcc ctgttgtgtg cctctcccct 2640
   ttttaaattg cactggatac ttttgttctg gaatccttgg ctgtctaacc tgaaagcagt 2700
   gttggaggat tacagaattg atgctcaaag agaaggcttt gctttcattg cttaagaggg 2760
   tttcttggag aggttcagca agagaaacac aaaatgagat ccttacaaaa tatcacatat 2820
   gagaaaggtt gtagtctctt ggtccaaagg actactttca cctggagatg gttggagttg 2880
   tagtatctga gttagataag tgggccagga agaatcactg accaggaagg tcacacaagg 2940
   cctgagtcct ttctaacacc atctgtgcac tgtatttcag ggcctagtgg ctgcattgaa 3000
   aggaatggaa agtagttgct ggttatgttg agaaatatca ggaaagtgtg atgcctttct 3060
   cctgtattcc ctcttccctc cctcattttt tcactttatt gataaaaata atcccaaaag 3120
   atcagagagg ggttctatag gtgttaaacc actgtgttat gacttgaact acaaagccct 3180
   tgggggcatt caatttcctc ctctctccct ccaggccaga ggccatgatc tacatccaac 3240
   ttctctgcaa attcctgaac tctgactcga ttgtccctgt caatcctgcc tttgtgtctt 3300
   acctggcctc agtcctaatg aggctccttc aggatgcagc ctctgtgagc tgcttttggc 3360
   ttgtcatgta acagctctgg ctaggactgt gctgtgtagg agagaactcc acagtcagga 3420
   tctttgtgca ctcaggacgg gaagcatgga caccctggag agcatttgta ttaacatggg 3480
   catttaaaaa ggtcatacag cagaatagga aggcacactg aactctgcag ctggagtttc 3540
   ctcagatgta tttctgtttc cctagtctcg agagctgggc ttttgcaact cactgttata 3600
   ccaaagcaga ttgacttttt gagttttgta ttgcttccaa ctctctcaat gtcagaggcc 3660
   tcaaattaat tccaacatcc ccttgctgtt ttcccttaag aagctgtgat ggtgttgcat 3720
   tcactttgga ttcagtccat ttgtgtttat tggaaggccc cttgaatcag catttccact 3780
   gctctcccac atttgtcatg tggtgacacg tggacagaaa tccccctgca cgtagacaca 3840
   catatgcctg ccttgacttc tgattgacct tatcccttga acatcaggct taggccactt 3900
   aatggtggaa gaggaacttt gctccatgag ttgtgagtgt atgcacagca caggtgttca 3960
   gagtgatttt ccctccccca cgttcactac aaaaagtacc ggaaatgcca ccacaaacat 4020
   tccgagagca gcattgctgt gaactgcacc actctccttt tgttccattg aagatttcag 4080
   aacacatgct tctttgctct ttgaaatggc tttctgagta gtaatgggat tggaagagtt 4140
   cggagaattc catgtatcct atggtgatgt ctggagtgaa acatggtatg aatcaaattg 4200
   tcacagctca ctattatgga tgtcagcctc aacaaaacca aaagagcaag gcacataatc 4260
   gatcacaatg ccaagaccag tgactttcag atcagttatt ttgttacaca tcattgctca 4320
   caaggatttt gctcagtgtg acaaggaagt ggcagtttgc ttgctccttc cacactcttg 4380
   aaggcctatg gtgctctaaa ggtgactgtt gacctagtta gagcagcctc ccaagaatgc 4440
   aaaatgagac ctgggggaca cacatcctac tctgtatttt gcagctcatc agtaatttta 4500
   tgaggcaatt cagctcagat ggaagtggta atcttgcaag aaacccaatt tggagttgtc 4560
   gggcttagaa gtgattaggg ctctgagaaa tagatttttc tcaaatatga cattgattag 4620
   aatgccagtg tttgaggtga tgacaagaga gtgtttttgg aatagggact ggaatttcca 4680
   gcacacacat tgagttgatt ttatatgatt atattcccca gacccttgca tttaaataca 4740
   ccctactctt cttgcccagt ctgtccattg gcctttaagg aaataccaat cttgtaatca 4800
   atgatgtttc tcggcctctt caatggcatg tgggaattag tgtttcctgg gtgaaggtta 4860
   atttggggga agagtctaga taactcagtg gctttaggac 4900
<210> 39
   <211> 5000
   <212> DNA
   <213> Artificial Sequence
<220>
   <223> Nucleic acid probe
<400> 39
   tgttgtggga ctgccatcca agaacctgtt ctctgtatct atagcatgga gctgtcctcc        60
   agagtgctct ttagaaagag ctccaataat tgggaattat tgctatcaac aggatttcca       120
   agcaaaaagc ctggcactaa ttattgccct ctctttagtg tctttaactg ccagatctaa       180
   catgtgttaa ccttatacag tttggaaaag ctgtggggtg gaacaccttc ttactctttg       240
   ggagcaactg ggaaactaaa gagaaagaga tatgtcttgt cttgaaggaa acttgttgac       300
   ctaaatctgt gaactattgc aggtcactgc tgctgtgata ctttgggaat gaagtcccaa       360
   tgatacctct aaacagtcac agctgtttga gctctctcag atggagcgtt tggatactat 420
   gtttttgatg ttgtgcatgt gttcttgggg acttgacttc atgtcaataa aggacagcag 480
   catctcataa tgccactgcc aggcttgaag tggcaaatgt gagccagtgg tcatagcttg 540
   agctatctgt tttctgtatg cactgcatcc aaggctggct ttcaactcac taaagaaaat 600
   aaaattatga tgatcaactc tagtacttcc agcctgcaga aatcttggtc ctttgtatga 660
   catgaagttg ccaaaaagat acttagagtg gtttcatctt tgaatttcac agctttgcat 720
   cccgagtcag gaagctgtca tggcgttgtg ctggtacacc agtgcggatg ccagcatgtg 780
   catatgctga cacatatgct tgaatgttag acatctctca tctaaattca gagccaagga 840
   ggtccgtgta ggcaggcttt ccctaagctt cccttctggc tatgatttga aattaatcat 900
   atgagacttg acaaattttc atagcctaag gctacagtct ttattgccag agcagtaagc 960
   cacggaacat agtcattgtg gtaggaaaga tttttgtctg tgtagacacc tctaaacaag 1020
   taataacaaa ctgatattca agcgcaagtg gtccactctg actaattgtc cacctctcag 1080
   ggtttagaga catcccttgt ataaacatat tgtctcctta ttcgagtcga ctgtcttgtg 1140
   ggttatagca gtttttgcaa gtagattctg tcactaaaat gtgctggtgt caccagggcc 1200
   cttcccctgg gaagaggaat gtgagatggg gtacggccca ggcatgtggc atcccagcct 1260
   gcaactgtgc tgctgattgg gtgtccatct gatcggctaa catttttccc agcatgggca 1320
   aagccaccaa gctcattcat taccaggcca atcagccgta tccacatgta gcagcacacc 1380
   caccatcaac ttgtactaag agtcagaaac tgcctcgacg ttttgtggtg ctcctgacgt 1440
   ctgcacagcc ctacttgcac agtctttcct gatgtcatca cgtcttgcct ctgctactca 1500
   agtcctcaga gaactcccca ctcctctctt cactacagag gcataagacg caggccctga 1560
   gggattgaat gtctcggcac cacccagccg atcctcccac tcccagtctc cagggaagtg 1620
   aaagtttggg agtatcttca tccatgtgtc aaaaaatagt gaaagcctgc agaagcacaa 1680
   attcagcaaa agctcacttc tacattttca gaaatctcca acgtttggat catgccagga 1740
   tgttaacttt ttacaggccg aagctgaacg gcctgttatg agggagcccc aaaatatgta 1800
   tggttttgtt tggttgccct gttgggacct tcactgagtc ttacccaggg gttcttatag 1860
   tgtgtctgag gtgtgttccc caactggccc tgggtgcaga ctgaaaagca tatttaagcc 1920
   tgatggttta tcagcttggt tgactcccac ttctgctctc aagtgttagc agaggaaact 1980
   tggtggacat ttttttggag atttcttttt ccaaagaaag cggccattct gcacatctgg 2040
   agagggtgcc gtcatttgtt tggttcccag atgctttgca gtccacactt ctgggaagac 2100
   caagtcatca ttgccttcaa aggatttctt ttctcagcaa ggtgataacg ttcactgcca 2160
   tagcctcccc tgattaatgc tttaagattg attgctggtc atggcagtaa ataaaacaca 2220
   tgcctttcaa catccctttt tcccctcagc tcacagcttg ggagtttagg ttaacttttt 2280
   gttataacag gcgtttgaga ctccaactgc tgctaatatt acagcttaag aacaggctga 2340
   tagatcttgc atacgaaaca tctcactctg ggaaaatctt cacccttctc attcttagtt 2400
   agtggggacc cttccagctg aggcccagga cacctttgtt tgtcctagaa tactcttctt 2460
   tgattctgcc ctgaggctac atctggggca ctctaactgc cacctgagca cttggggaaa 2520
   gaacaaagta aaaagctagc atgaaggcag tttacatcaa ggtaaagctt ccagaatata 2580
   tttcaagtca gcctgagcaa tagtgtgccg gatgattctc tggacacatt gtaaaacacc 2640
   acaagcagtt agtgcaaggg tgtgaaacac agcacctttt ttgagttata tctggctgtt 2700
   taaaagcatg attttgggag gaaagagaga agaggggatc aaaatgtttt ccctcctttt 2760
   ttcagtggga gagcagagaa gaaatgttgg tcaagatgcc cccataagcc tacccatttc 2820
   ccgaagtgat ttgagatgga ttgttttcat ccaggccagt cttggaatag ggatgcccag 2880
   cagggcccag ggtctggcac tggagaaaga ctcacctgta tttgttcttc ctccactgag 2940
   gaacacctct gccttcggtt tattgctgtt ttctctggtt acaattttgg ttccattgaa 3000
   aagatcccag ctgtttccca gcttaaattt agcagggagg taaatggacc ctctgtgggt 3060
   ttagataagg tgtatggtca agcagatgtt gtttttcctt ttatggtgga aaagttaggc 3120
   taggtggaga gcattcagtg tccttcagtc atacatagta gttatcactt gctgatccca 3180
   ggaaaccggt aatgctttag ggctgcacgg atgcagggac ccatgctctt tgtgacttgt 3240
   gagactgata acaaatatac acgcgccatg tttagtggag ggttacgtgg tctttgtaag 3300
   gagaatctga gagtgaggac ccctctcagt ctcaggcttt ctggtctaac actgagtatt 3360
   agggagatct ctggtcattc tccaagcgca gcctctaact tgatactctc aaacaatgaa 3420
   aatgatactt catggttcct taagcatggc agagagaacc accagatcac ataatgcaac 3480
   tcatctaata ttcctattga aactgaccaa agccgaccat ttttgtgaag tttattctgg 3540
   tggagacagt gcttgcaggc cacatccact ctgttcttga agggagaaag aacaccagtg 3600
   catttttccc ggcatgcaca atgtcgccag tgcatatgtt gaggtctttg cgtttgaagc 3660
   gtggatgctg tttagtcctt actgcatata tttattatgt tctcttgttc attagttcca 3720
   gcacccagcc tgctgataga cacatagcat gagctccatg attcctcata ggaagattgg 3780
   agtactgtag aaattatagc ccaaacatag cttggaggag ctgtcatcta gtgggaaaaa 3840
   ttaatggtct acataccact gggtcctagt cctggcattg tactacaatc cttgcagctg 3900
   tactggacta gctatcagtc aatcatatgc ttataaagat tccaatccta cctggctcaa 3960
   gaatttcaga ggggacttac tgagttggac tggttttaat ggaccccttt gtcctccagt 4020
   acaggttcta tgagtttgca ggatcttaaa atttgtgtat gtaaggcctc gaagagctcg 4080
   gctgcctggt tctgtcctgt agattcaaat tatttcattc tagtttgatg gcagctttcc 4140
   cttcttctgt gacacatctt cttgctattg gaggtggctg gggtcttcct ggtttcttcc      4200
   acatccattt aaatgatgtt caaagaggaa tggtgggaca tctcaagtca aacaaacatc      4260
   ctgaactcag tggatgaagt ctccaacagg tttcactcca agtttcagcg aaagtggaag      4320
   cttcagaacc tacccctcca atttgctgaa gaaggtggga cagaaccaag gaaaggcaat      4380
   aaaaccgtga accacagaaa ctcccactct cactgtctcc atttcagaac ccagttcctt      4440
   ttccctcttt ccgttcattt cgagaagaca atgtcagaaa acagaatgac gtatgtgtcc      4500
   cactttttat tgactccctg ccatgagtag aagcttaagc tacttacaca acacccgtct      4560
   gtcccctctc ttccaaattt tggtaatcat tattttaatt gctaaggtta tgttttcttc      4620
   tctttatgtc tagcatcaca agctcttgac cctcaagaga cactatgctc actatacagg      4680
   tcaaaaggat aaatgctttt attcttgaat gaggagggcc actggagtgt cattgtctcc      4740
   taagcagcct taattcttcc caggaggttg actgcacttc ttgagagagg agtcctctag      4800
   ctctggcttt gttctcctct ttgccatgcc catgccaggc ttgccagcaa acagcctgca      4860
   gggctagtta tcttccttct gtgtggctct tctcaccctt agcaggagga taatagagtt      4920
   gcagtgcgta tgctaaacaa aagacgactt tcattccaag gaaatgagat agagacagca      4980
   cgagtcttac ttctgttgtt                                                  5000
<210> 40
   <211> 5000
   <212> DNA
   <213> Artificial Sequence
<220>
   <223> Nucleic acid probe
<400> 40
   agacgcatcc ttctcaaata catcctcacc ccctattctc attctggctg atctcctgtg      60
   tctctaggaa ctttgttcag atattctgct tcctcttatt tggagcctat gcatttttcc     120
   ctatgggcat tgatgaccca cactgagtag aatgagcaat gtgggtgttg gattgctggg     180
   caattcttat gctccctgga aatgccagca gcaaacatca tttccaaagc attcaaacca     240
   cctatgacta atcacaggat gaccaatgtt ccccttgtga cgtggtaatc aagtgcgttg     300
   cttcactctt gtgtgcaaat actcttcctt aaagacacat agagaaaagc tggccaagac     360
   ccaagaaatg gctgtagaaa gatattcatt ctcagggcat gaggaaaagt caaaggggga     420
   tggtcaggtt caagcatgag gcccttggag actgctggcc aggggtgctg cagagtcacc     480
   gtttctattg tgtaaacttt ttcaagggca gttactacaa gacaagtttt caggaccaac     540
   ttttcatctt tgagcggatg cacagtccgg actggttttc ttccgtaaga gagaaagagg     600
   tatacttgga agtgaacaga taactaaatt taatgagatg gtctgggaca cataggaacc     660
   ctcaaaatgt tactagaagg ttatggtgag cctagaatat cttttatgaa tagaacccct     720
   accaatagaa ccaagcacct gtgaaatcca ccctctgtac tgctcaccca gccttctcta       780
   cttgcaagtt gtcccaaaat cagccattag agagtaaaat tatgtggttt ggagttggtt       840
   tgccttttca agccccaagg gattataacc cttagttata gattaccttc aggtgtcagg       900
   agtgtctgag gttgtctttt aaagccccca cataagtttc tgatcatctc caagagttaa       960
   caagtggagt acgcctggag atattatgaa gtagacaatt atttgtttcc cgtcccacta      1020
   aggtaaatag ctcagtgcgt ttggtgcatt tatgattgtt gtgcagagga gtgcagctga      1080
   tgaattactt cctttcattt tctctcttga tcaaagacag gtttctttta tgagctggtt      1140
   tcttagagcc ttctcttgat tttgcaggaa aatgggggtg gctgccccca tccagtactc      1200
   tatgagcaca ttttcctcta aagctcttaa gtacttgtaa atcaaggctg gcattagggt      1260
   gtttggatac agagattatg gatagtaaat gtgaatttct tcacttggtt attggctatc      1320
   taattgttat tgagtttcct gtgagctggt agtaccttgc ttcatcagct taacccactg      1380
   ccaaaagttc tttatgcttc ctgcgttatt gttatcgata tgcccatgga gtgacatcta      1440
   tgttatcttc aggagcaccg cactaataat gacttgtgtg cactctgccc attagaattt      1500
   tgaatagatt acaggcatga aatattggtc tggattagtt gccttgatgg agaacacagt      1560
   cgtaggtggc caccccgaat ctctgccttc acacattgat gtttcaattg caggaactag      1620
   aatgtgtgta gtgaaggaaa aaccacagac tgaactttgg aaggagtccc atgcaggttt      1680
   gtaatcttca tgtagggcag atcccatggt gattctcgtg taatatgaag aaggaaagtt      1740
   gatgggtgtc ttctctaacg agaactctga ttagtggaag gttaaggggg tgatatcaaa      1800
   gtcacagtag gtgggcaaag gacttcagac cctggagaca caaaggtaac agctcctctt      1860
   tacactgcca ccgtctgctt tttagtagga aacctgggag tgccagtaat ggtttttaaa      1920
   ggctccatgg aaactcttaa cctttcacca ccttaaggca aagagggact gctctgtcct      1980
   ccttcatatg cagactctac attttggccc taatggagag aaaaccagca gtgcagaaat      2040
   tcctcaggtg cagcacggtc catgatagga gcccactgag taataaatgc cagaggaagt      2100
   ggaaggcaag atactagcca gctagcatgg tgtttttccc ctgttctctg acagaaggag      2160
   gacagccatg tgaggaagat aatcatggca gcaaaagttg ttacattaca gtaagaagta      2220
   catttgtatg gagttataca tgccacaggc ctgctctagg acacttggca atcagcaatc      2280
   gttcagacct gctttccttc tgatttcctt tcaagtagtt ccactctgtg taacaatgat      2340
   gcaaagcagg cggagagctg acctagggct ttcaagtggg gcagaggtaa gcaaagagat      2400
   aagcagtcgc ttagactcct aaatgcagtt cttctctctc gagaccacct tggattggag      2460
   aacacccaaa tgagcaggat tgcttttgct ttcaaaagag ttgaaaagct gagttacatg      2520
   agaaggtgac agcctcataa tggcccgtgt tccagcccca gcattgttac cccctacaat      2580
   gctgttgtag gagtgtaaag gcagtataaa caaccctgac agaaatcttg atttatcggt 2640
   tcttggggga ttcagcaaag gctttagggc atgaaaggca taaagaaaac ttggcaggaa 2700
   gccagatgta gcctttgctc ttatcagcca ctctttccca cccttgttgg taccatgaag 2760
   tttaggtagc atatgacgtc tcctaataga aatcactaca agtctggctg tttgcagagc 2820
   ttagataaat aagaataagg gcctacctag ctggatctag catttgcttg ctctcagaag 2880
   ttgcagtgac aggagaagaa cctgtcagac cgatctgggc atttctaacc actgtcctta 2940
   gcaacgttca caaagggccc cgtcttagac tcagaaaggc agctcacagc ccttaggggt 3000
   acccagttgg taagaaaagt aattaaacca acctgctgtg ctgagcaggc ctgcagcaat 3060
   gtaacactta gttatagcaa cagccataga taggaactca aaagatgaat aagaaagagc 3120
   tgtttctgat tcaaccttcc aattcctggg agaaatggat gaagtaggaa gtagagagag 3180
   ctgtagatta cagaaaggcc ctagtttagt ggtcttgtct cctatgacct ttagtcacct 3240
   cctgaaatca attatatcct caatgatggc tattactccg ttcccccttg aatatattac 3300
   cctggagcag tcgtaccact cagatggccc aacagcctca ccaatgtctt gcctgcatcc 3360
   tctctgttta aaaataggac tcccagagat tctaccttga aaaatatgta acatcattac 3420
   ctctgttaac accagtttgt cagcctctag acccactgtt cacagtggca tcaatcctgg 3480
   agaagatcca caccttagaa ctgggtttca cgtgatttat gttttctctg gtgagccccg 3540
   ctagtatgaa gacagtaggg ctttgctaga actggtgtgt tctcggggta gtaatggtct 3600
   gaatatctca ccaaaatgaa agaactacag gtgaaaaaaa taaacccagc cctatggttt 3660
   tcctttgctc actgttatgt ttcttgtagg aggcttgaag catctctaaa ctgcttgaga 3720
   ttggatttat tctctcatga ttctcggcac ctagctcagt acctgagatt tggtactttt 3780
   tttgggtgaa ttaacgagca atttgtcatg gtcctagaaa tgcttatgat taggatgggg 3840
   atcttgatag tagtctccat cacctttact tcatcccatg gttatgaaca gttgtatcca 3900
   ttcatatgga attctcatgg gtctggaata agcatttaag caaggccttc tactgaatat 3960
   ggctatcagg tgtggagttc aaaatgtgtg aaaaaccaag agctgaggac gtgaccatgt 4020
   gtctgatgac taagatggaa cccatgtagg agttgagtca gtaataacat gcctcttgct 4080
   gcagctcaag ttgcctgcaa gctaaaattg ttatttctgt gttaggcaaa atgttgggac 4140
   aggctaagca tgaccttgag tccttttgga tgtgctgtct tgtgaccctc ttcttggtgg 4200
   cctttgtttt cctggacagg gaaacctctt taacattgga cttcattgtc tgtaccccat 4260
   ttcctgttct tacagtggct gttcagtcag catgatggcc aagggtgtcc tagttgttcg 4320
   agaccctatc ctggactgct ctttgctcct atctatccaa cttctcaaag ttcagctttg 4380
   cttttccttt cctgccttgt tatcaatata taaacacaga cttaggagtg ggagatccag 4440
   atcagacatg tttacaggcc tgagcttttt ccgtgagttt ctggaacatt gtggtgcctg 4500
   caggctctaa ctgccaaatc tttttcagaa gtctttcagt tgttgcctcc aaacggagct 4560
   ctttgcagtc ctcgggcgga cgaatggacg gattcgagcg tcagaccctg gccagctcta 4620
   agggctaaag cagctctgag gtgtagcact tcacttccat cggttttaga ggccctacct 4680
   ggggagaaaa acggcagaaa aaaacaaacc caaactcaaa tccccaaacc ctcaaagcat 4740
   caagtgcttg aagcatttct aatatacctt ccgcatcaac cctcacctcc ttcttgtttc 4800
   caatgtactg tttctagttg ataaaggagg gaaatgtatg catccttgcc atgctcattt 4860
   gaaaacagac aacgtatcta aagatgtata gagtgattgg aaagcgatga agggagaaaa 4920
   agacactatt attggtccca tcgcctgtta gcaaatacag gggtctctgt ggccttgtct 4980
   tcttggtagc ctggtgtttc 5000
<210> 41
   <211> 5000
   <212> DNA
   <213> Artificial Sequence
<220>
   <223> Nucleic acid probe
<400> 41
   tctcagctca cctagtcatg ttcagaatgg acaaatccct cagaggaagc agacacggtt      60
   tctcgggacg gtgatccttt agagccacat gcacatgctt ttccagctac tttgttgtta     120
   tggagatcca gaacccaatt aaagcctgtc tatcatgttt gaggaaagtg cagtttgagt     180
   caaagcctag tccagtccaa tgggctgtac ttttctccag aattgtgtaa ttcctttggc     240
   ccattggggg tcagaaaaag aatggctaaa tgttactatc ccaagacact tggattgatt     300
   gaaagacctg ccaaactcga tgagtgaaca ggcagccctt cttcattcat gcatgcattc     360
   agtttttgaa tcaggtgaga ctttagatct cacgtgaaat cagtttgagg aaacaggaat     420
   cttcctcttg catgccctgc tccttccact tacacggaga ggcgctccat ccacgcacag     480
   tctttccact cccatggggg cacagtgggg tcctgtcatt tgcatacaga tattctcatc     540
   tgcatagaga ggttctctcc tgcgcagagg ggtcctgcca tttgcataga gatactctca     600
   agcgcagcgg gatccccatc gtgacctctc cctaccagat ccacttcacc aagacaccca     660
   agtacttcaa accaggaatg ccctttgacc tcatggtgag gaaagtgggg ttcagtctga     720
   tggggtaggc ccagtctgag aggggaggcc gagtatgaag atggattcca gcctgatggg     780
   gggaggcagg gccagtataa ctcacgaggc ctctttgtct ctccccagtt ctctatcgga     840
   tcttcaccgt caaccacaag ctgctacccg tgggccggac ggtcatggtc aacattgagg     900
   aatgccctgc ccacgcctag atcccagcca acagggagtt caagtcagaa aaggggcgca     960
   acaagttcgt gaccgtgcag gccaccttcg ggacccaagt cccctcgaag tctcctcttc    1020
   tgaactcctc ccctcttagt ctccatcact ttctaagttc cctcacctga gtccctcccc 1080
   ctttctgagc ccctcccatg cacacagcct gccaggcgct gactccagtg aggactggcg 1140
   tctcacatcc gtggaatgac aagcccactc ccgtgcccca ctccgacagg tactctatca 1200
   attgtgggtc tggaagtcca gagacttgga ggagttgctg acgctggttg gaaggttcag 1260
   ggtttggtgg gatgtgtggc cccctcgttg cccaggcttt ttgggggtgt ttggagactc 1320
   ggaggtttgg actttacaag atttgggagt ttgcagcttg gggacttgca attttcagtg 1380
   tgggtttaaa gattggctac actctaccca caaacagggc tggttctggg gtggaagcag 1440
   acatttgggg gtccaggtcc ctgtagaatt cagggtgcat ttgggtgttt gtggattcag 1500
   cctcaggtta ctcaccccat ggacatgttg gccccaggga ctgaaaagct taggaaatgg 1560
   tattgagaaa tctggggcag ccccaaaagg ggagaggcca gtccctcccc cattctactt 1620
   cccctccctc agcatggaag cctctggaag tggggctctg actcccagcc tacagagaga 1680
   ttcctaggaa gtgttcgact aggatattga cattagacag gtacaaaagc tctagaaatg 1740
   aggactttcc tcagtgatga cttttttcac caccaaagtc actcaggcat cctgacaagg 1800
   ttacaggaaa tcagagaaaa ctccccatta accccttcag tggggtttca gaaaccgcct 1860
   ctccagggat aagggggccc cacccacaga cccttctcct gcacatctcc cagaaaaaag 1920
   tcacgaggtt ctgaagaatt cccggtctca tctccctccc tccttccctc ccagtaggct 1980
   accatctgct ccagcctcca agtaataggg tggaagaagg ggatggcctt gctgtgggtt 2040
   ctggagaaaa attctaggaa agcagccacc tcagcctgga attagacgat gggatagggg 2100
   cccccaaata ttgggcagcc ctgtccttac cccgggacca cccctccctt ccagctatgt 2160
   gtacaataat gaccaatctg tttggctacg ccgttgtctt aaggcgccag cgactacgtg 2220
   ctgtggaagg agattttgtt cctggtggac ctcatctgct gtggtgccat cctgttcccc 2280
   gtagtctggt gaggaccctg acgccctccc ctgctccttc aggctgaagg gcgccctcct 2340
   cttcatcacc atcgccctga ttggctcagg ctgggccttc atcaagtacg tcctgtcgga 2400
   aactattcac cccttcagct gtgactgttc cctccatttt ttcagccccg attaccagtt 2460
   ttgagtccca cccaccgtcc tcattattca actttctgac ctccctgacc ctcccaccag 2520
   gtacagcgtc ttcaagatcc actggctcat ggcggccttg gccttcacca agagcatctc 2580
   tctcctcttc cacagcgtga ggtgatgatc cgggagaaga accccgatgg cttcctgtcg 2640
   gcagcggaga tgcccctttt caagctctac atggtcatgt ccgcctgctt cctggccgct 2700
   ccgcccagtt ccacgtggtg atcggctctc aggcggaaga aggccagtac agcctgaact 2760
   tccacaactg caacaattca gtgccaggaa aggagcatcc gagaagcaga gagagaaagc 2820
   cacttacttt ttagttttgc cttcaagtct tctgattgtt ggaaggaagg agagagggac 2880
   tccgcgggtg ttggtgtctc aaacagtagc agtttcctgg tcctgttcct catcaacacc 2940
   aaggatctgc agtgagttgg ggaccggggg ctcgctcaga tccttcgtct gcgtttccct 3000
   acccttcccc tgcagggagg aagtccctct caactttctc ttgtcatcct ctgggcctgt 3060
   cctaaataga ggggtaccag gaggcccttc tggaggtcct gagtggggtc ctctgggcac 3120
   ttaatgggat ttggggggtt ctgttgaaat attgctggga gctcaactgg aatgtaaaag 3180
   ttcttcggga aggtgaaggt gtccttgggg atactgggaa aggtagttta tttaatgctt 3240
   tgtggccctt gtctgctccc gctgcattct gggtaatgta gtcccggaca gttaagagtg 3300
   gaatctgggg gtggggctcc aattgcctca ctctggaata aaaggcttag cctaaggctg 3360
   gggtctcagc ctggggtcat aggtcatcct agggtcaaac ttaaccagaa ggattagcaa 3420
   cccggtaagg ggtcaggtcc aatctggagt cagggctagt ccagatcggt ggcctaaacc 3480
   agagattaag cctcagtgtg actgggactg atgtctctgc tgagacctgg ccccacctgc 3540
   tggtggcttc tgagctagaa aatgtctgat tgcagttggg caattgaacc cagtgagttc 3600
   gtcacctctc ccataaaagt ctgttcacac cttgtaaatc cctttgaggt gctggaatga 3660
   aagaggagag gaggcactgg ggattcccac tcccattctc ttccagttcg cgcctgcatc 3720
   tgcccatttt ccagtttaac ctggtttcat tctcagtact gcccctgaga ttccgactcc 3780
   gcccctcccc cagacatctg ctgaatctcc ctgcttccag ggatcccccg atgggtgact 3840
   caatttaccc cactgacccc actacacatc caaaagaaat cgcagaattg gaggaggggg 3900
   agttgtttgg cgtaagcctg ttccacttcg gtgcgttctt tcacgaactt tacatatctg 3960
   tccaacaggt ccagccccca ctgcgtgtgg cgctcgagca attcacctcc tggagaatct 4020
   gtacgaagga ctgttgctgg ctgaatctgg ggttggatga gaatcgggga acagggagtc 4080
   aggcccctcc tcccgagcag cccccatgcc tccgggccac agtcactgct acctctgcaa 4140
   actcacattc tccagctgtt taaagccatt ttccagctgc tgctgggccc gccgcccttc 4200
   taggtctgag aactgggagc actcacatcg aatatctggg gcatctgtga aaaatagaag 4260
   tgggcttggt ctcggttgaa gcgctgcagt tgagccgcat catgcccagc aggaagaaca 4320
   gctgactaaa agcacagctc actgttacta taggtttctg attccctttc aatctaggga 4380
   ctgggacatt gcagctgggg acgttcttgg gatccacagc atttgcagcc accttcatgc 4440
   cctccaagca cttggctatt atgggcacca cctccagctc ggcctccgac aggagcccat 4500
   cacttcactg tccttctccc ccaccatccc aagggtcccg gcccccacct tgttcttctt 4560
   gccaaaaggc cagcgcttgg tgcggctgcg acccgggcct gctcggggat cacaggtgtt 4620
   aagtcaagga gaggtatgag agtgtaagtg aagagggacc caaagtataa atcaaaggta 4680
   taaaccaagg gtgctaaggt tgtccactta gctttctggg ctgacagtct agaagacgca 4740
   tgcaggaatc aaggtctcag acaggtaaga ggccacagag gacaaaaata aataaacaaa 4800
   acccgtctga gatatttcac tcccttcccc agccatggtt cttgacagcg aagccatgca 4860
   cagagcaaag caggtctggg ttgggcaacc agggaagagg cctctggtca acctccttct 4920
   gaagttcacg actgcgttct tccaactgct gttgaagccg ttttcgctgc tgctctgggg 4980
   gcaagtggct aaaatcctcg 5000
<210> 42
   <211> 5000
   <212> DNA
   <213> Artificial Sequence
<220>
   <223> Nucleic acid probe
<400> 42
   tataatttgg ctgcctgttt gcaagtggca aacatcattc ctattatgta acagaactgg      60
   gaagaggatt ttgttgcctg gaaagagtga gtaaagattt tcagtttctc atccctgtct     120
   cccctatgtc tatatataca ggtagtcacg actataatgg acctttggaa aagaccagat     180
   ctgagctcaa cttccttcca cgcccctccc cagggtcttt cctgacctca tacttctgca     240
   cttccaattt cagccgttca atgttgctca gggtttcagc gatctggggc tccaagctgg     300
   cctcgaaatc ctcatcaaac tccgtgtaaa tgggggtgtc ctggctctct tctgagggag     360
   gctcttcaga gctgtttggg gataattcgg cagataaaga gctttaggcg aatttaaact     420
   gaacctagcc agagacactg cctcccatac cccgccaagc ccactctggc cgttctcacc     480
   ttcaaagtgg tagatggcca gtatccttct ccctacctga gtcacaagca gacacccact     540
   gacaatgaca taggccaact tccagaaagg ggcagggttg acccaaggtg tgcccaagat     600
   acccttcttc acacccggga cccccaggtt tttgcgaacc ccagaagaga ccaaaggagt     660
   cgtcccaggc tcctcgcgcc tcaggtggaa tcctgccctg gctccactcc aggaaggttc     720
   tcaccccaag caaacgctgc atccagcggt attgatagga acagggccgt ccacctcaaa     780
   acgggctgtc ctgcccccag ctgcgaggtg agcgttccct ctgcgctgga ctgcagactt     840
   cagagcaccc tgcaggttct aaccggatcc cccaaaccca gcctgtccca ggaggggagc     900
   ggtctggggc ttagcatccc ctgggggccc agaccaaatt taacaggact caatctagtc     960
   ctatgcccag tccctgcatc tgttctaggc ccggtccctt tgctcctccc caggcccagg    1020
   gcaagaccca accatgccta cataatcacg cttcacctga cttccccatt cccttctgca    1080
   ggccacaggc ctcctgggag tgtgtattcc cattgacccc tcttgtatga cctctagaag    1140
   cctccaccat gcccatatct gccttgcccc gaacgagcag tgacccggtg ttgctgaagg    1200
   aagtgcagag ctcagccctg gcaccctctg ttcccaagag ctccatgcag gtgccacagg    1260
   atggagaaga ccttgctggc caaccttggt accacggcct gcagctttag cgaggacacc    1320
   ctgatggatg gcccagctcg gatagagcct ctcaggtact cacagtctcc ctgcctcctt    1380
   gagaacaccc aacactcgca agccctcttt caactggagg atgagcaatt ccccagcata 1440
   ccggctctgg ttcacagtta tatgacaggc aggcgcccac tgtcccaggc cacaggggct 1500
   catctggtgg ggagcctctc ccacccttgc tgaggattcc tgggtccctg gatgagggac 1560
   ttagtgccaa ggtctattgg gacaggtggc agagggctgg gctaaagttt tctaaccatg 1620
   tgtaagcatg tagcatttct accgtcgagg tggttgaaat tcaaaactgc acccggatag 1680
   gttgttatac cccattattg cccctgactg ggatgttttt tcacccagag tcacctggca 1740
   cactccccag aattccttcc agactttact caatgagacc tttcctggtc acatgatcaa 1800
   gacctatggc caccctgtgt cctcaaacta accagaacct tgccattgac tcaacttcac 1860
   tctcactaag ggagacacaa gacaggaact tggggttggg cggacgtctc tccacaaaac 1920
   ccgccaccta aatggggctt ctttccggaa aatccgggag ggcggtctgg aggaagcgta 1980
   cccctcctta cgtcacagct ctagacccct tccctgaaca tgcccctacc ctctgactgt 2040
   accaatacca gaccggtgag ggataaggaa gaaactttct tcccctcttg agcctggagt 2100
   gccattacct tatcattagc tgaaatcatc ttcatgagat taggtctctc tgcccccatc 2160
   cctagcttcc accccagtag agtgtaagca ggccctcccc tctcagattg tgaccacccc 2220
   ctgggccagc cctggacctg taagcatatt ccctctcctt tgcaggccca gaggtggagc 2280
   agcacgtcag cccctcgact cacagtagag gcgcctgggg cttgagagag ttaaccccta 2340
   gtttgccagt tctcagccca gatgcgggcc aactcagggc ccgtgaaccc acacagggag 2400
   acagatgggg cctaacagat cccatgacta cttcctcatg ccctagaaac cagaagatta 2460
   agcaaatgta ggcaaagctg ggatgggctg tggccagagg tagctttagc taagacatac 2520
   acaagaatgg aagttcatcc ttaagtctat ccactttccc attagctgct attgaagcat 2580
   cacagagagc ttgactccct gttaggcacc cagaaaccca gagaggttgt tcctgtggca 2640
   gcttcctctg gcttcttgtc aagacctctt accttccctc acacgtcccc actacatacc 2700
   tctcagtata gctgtggctt ctcatgatgg tatcattctc tccctgggcc tcagatctca 2760
   aggggaccag ccgcttggag attcatccaa cctgacctcc catcatcttg gccccagcag 2820
   ctgcccacat cctaggtcta accagctccc atgtcctggt ggatctttcc aaccatctgt 2880
   tctttcctct ccagtctctt aagtgctgac cctcctagat gcccctcctg ttggccctag 2940
   caggcaaggt aagaggaggt tttccaatca gcccaaccca ttttgctgaa gttgctatca 3000
   gaatcatgtt caacgaagga taatgctagg accttggctt cagtgaccag aaagccaacc 3060
   cccctatttt ccatcgaact cagagtgatg agaatataaa ccaccccctc cctcaagagg 3120
   gcatgaactc agaaaagtat taactgtgtc cattatgtcc tgtgctgtaa ccccagcaat 3180
   cagtgtagcc cttatcactg taattcttgc cttcctggaa tatgagacct gcctcctctt 3240
   ttcatccacc tccacccttc ctcctcaatg aaaatgtgag ctggatccat gactcccaac 3300
   aaaccagcag gaccagtaga gctaagacgt gccaccctgt gccaggacat agtaaaccgt 3360
   tacgtatcat cagttctcag accaatagca atattgacag gattttgaag cctttgaagt 3420
   atttctccca cctctctctc gagtacacac tcaatagcaa gtcaggctga aggcagtaga 3480
   cccatatcct tggagtgctt gtgcccatat gcaaatcagc acccaggcgc actgcggtga 3540
   accctgaggt tcattcttcc ccatttgcca gccagcctcc catcaacaaa acccctgccc 3600
   ccctcctgac ctcagggaga tcagctgaac ccggagacag cttccttcca gtcatgtgat 3660
   gcctcaaaga ggggaaaggg ggcatgattc atcccctctc gtgtctgtct gatcctcaga 3720
   gacctcatgg atctccattc cagcttctct tgccgattct tacagtaatt atatgcaggg 3780
   agatgaataa aggactgctg cacttaatgt ctgttgtaac cctgtttctc acacttccat 3840
   ttgcagaatg tatcatcttc tcaattgctg ccacagtcta tgcaacccat tgcatatgga 3900
   ttcagcactc tctgaggttc cccagatcct gaagaattct aaccagcctt ccatccacca 3960
   gcttcccaca gcttgtaggc ttggcctctc ccacaacccc cctgacagtc cttagcttgc 4020
   ccagtgtaga cgtcaataga aattaactga aactcagtct ttgggcttca agtgtgcagc 4080
   tcaccttgcc aaaatcctgc tctgaggcgc tagatcccat gcctcttgga agcctaattc 4140
   aggattttct ctacctccta ttccccatac catcgttctc tcaaatgagc cccacccttt 4200
   ggctgtgtct ccccagactc tcaggcaagg ttgtgtgccc cagacaccag ggtatgacct 4260
   tatttcccgg agggcaaggc catgtccccc cacaccctcc gcgcccacct gctggatgga 4320
   gcccagcgtg tcagtgtact tctcctccgt ctgctggatc tcccgcaggc agcagcagcg 4380
   cttgtcatac tctgtcatct agaaatggag aggtgaggat acagaaaaag actgagcctg 4440
   ggataaagag ctacaaagga gagaatgaca cactgaggaa gaaagtagcg agaggagacc 4500
   aggttatccg cactagacag acccaagagg gctggggatc ggccggctca cctcaatgtt 4560
   gataaagatg atctcaatgt cttgaggttt caggaaccgt aagacagagt gaccccagag 4620
   cttcagtctg gccagctggg tctcacctct ccttgtattt gatgaagacc tggtagagat 4680
   tggctgcgcc aggggtgccc gatttgaata aatacgtgag cagctctcca ccccagagct 4740
   gtgggaattt ttctagaggc caaagcatca tatctgcccc caaggaaaca ggaaaaggac 4800
   gccccgtccc cacttttata gacacaggag agtaggtacg tggagcccac gcctagagat 4860
   gtgcctggca cctggagaag gaggtgatat ttgagaactc ccctgcgggg agggccttcc 4920
   aaacaatgct agctcaccag ctcctccatc gtactgctga ccctggaggc tgagacatga 4980
   ccccagtgac ccatccccga 5000
<210> 43
   <211> 5000
   <212> DNA
   <213> Artificial Sequence
<220>
   <223> Nucleic acid probe
<400> 43
   ctccaccgcc tcaccaggtt ctcaatggac agctggaaat tggtgatctg tcgcagtgtc      60
   tcgttgtctc gcttgacctc gttcacgcac tgagccaggt cgttccaccg aggtgatctt     120
   gagttccccg tcgatcttgg gccggccata gtgagccaga gactggtcct atgcagtagg     180
   cagggggtct agcaagggtc tcatctagga gcctggcacc atccctgccc aggctcctcc      240
   cagatctgtg ttgactccac ccatctgcct atctagaagc ctggctctgc ccctgcccag     300
   cgagcaggaa ggcatacctg gaaggatgga taacgcattc ctgtctggct cttcccaact     360
   gtccccatca gccctctgac cagttcctcc acatcctggt ggctaagctg ccgactgcgt     420
   ggaggacccc atagttccgg cgtcaaagcc ccaccttctt gttgtctcgg tctcctgaag     480
   agtcatcccg aacctggaag ggcaagccca tgacagtctc tatgccccta gcaccatgca     540
   tggatatcct tgtaaaaggg ctttctagga tatccaaata cacaaccata aacccaaaac     600
   tcctcccaga ttctcaccta agcagcatct gacaggcctt gcaggatgtg gtctcctcaa     660
   aggagaacat ctggaagtca tgcccgttgg cggtggcatt acctttacag acagggccaa     720
   atattctgct aaaaacataa agggacccct tgggttctct ttagatcagg agagtgacgg     780
   agaagcagaa gatcaaagta atcctgccca gttccaggat gtcctgggtc ctccccgcgc     840
   tgttgcccat tatttgtggt acacaggaag gaccacggga aagtcttacc ttcttcatag     900
   atgtggctgt taatatgatt atggagatct gtccctgggt caacatcaat agagatgaat     960
   tttcctcctg cccccacaca gcaacccctg ccaactcacc actctgcttg tccatctatc    1020
   tcaggatggc ctggagagcc cagagaggtg ataatgagta ttagcttcct gggaagatgg    1080
   cccgctcaga aaagccatgc tttctgccct aaacacccag gagacaaaaa caggaacaga    1140
   gaggttatag gatccataac cccacctgcc atctaaaccc ataaccctac acgtggatcc    1200
   gtgaagcctt gagttagaga aagacctgcc atgatgggca gccccctccc caactccggg    1260
   ggtgtctttc caagtctgtc catcctccca ggacccgccc acaggcaatg gaggccacag    1320
   tgctcccaca gaactcaatt tggtgtctca gaaggaagca aggggcaggc ccaccttcta    1380
   ggtccctgct ccttcctccc gcccccagcc cagagctaga ttcagaagtc ggtgctcagg    1440
   gatcacagaa tgcataacta attggctcgg aggttcctcc catacccagc ctggaacccc    1500
   agacatgggg agcgggcttc cacttctaca ggtcaccact aaggtttgac acatggattc    1560
   ggggggatgg ctcagccctt tgggtctggc tgggtgtctg attgccatat ggaggttgcc    1620
   tttccccctg aacccctttg attttttaag ttggtgacag cctggctcta acagcccttc    1680
   agcaccaagg taggccccgt agtctgggga ccaggcccat tccccaccaa gccccagtta 1740
   tggaggaagc aaccttgacc ccttcaggga ggagtaacta ctggaaacca aagacaagct 1800
   tcctaggttc tggaaagctg ggattgagcg gacattgacc cttaccgtaa gcccccggaa 1860
   agcctttttc tctgtgatcc ggtacagtcc ttctgctgtc ttcccattgt tcatcattaa 1920
   gagcataagt ttaaggtcac ttgcactgta gtgccactgg aggggggtca ccctgaatgt 1980
   gggaaaaggg ggtcccaagg acaaggcacc aaacagaagc aagaaccctc aaaaggcatt 2040
   tgagttgttg ctgaaatgat tatgcagcag attaggcaaa gacaagctat gggctctgcc 2100
   tgagacctcc tacctgctgg cctgctgatg gttctctttt caggctcctt gaaggggaac 2160
   tgcaaggtgg tgtccagaga cttgaagcaa tcctttagag aagggctcct cagtgctctt 2220
   ttctgcagag tattagatgc cctgtactgt aaggggacac aattgtactc ccagcatcat 2280
   cctcccgtct gaatttagaa agccttgctg tcccttcttg ttaaggatct tgatgatgtc 2340
   accctccttg agcgacagct ctgatcggtc tcgggcgcag aagtcatagc gggctttggc 2400
   tggcagaaac aagtggactt gaagacattt gaagtctatt ccactccttg gaatcggaat 2460
   tcaagcaggc aaagtgagca cagtggttta gtcccagctc ctttacatcc caatccttcc 2520
   tcaatgctcc aaaagcctct ctcaaagcct aattgtcctc cactgctcat taaactgacc 2580
   ctccaatgtt ttaacaaaac ccccttgagc cccgtctgct tgaggggacc caagaggaaa 2640
   tcggggtgtt ataaattaac catgtcaggg cacatcccgg gagcaccgcc agctggacgc 2700
   ctgtggattg gtgagggggc tttgtcagtg tggcacactc tctccccatc tctctcctag 2760
   caggtccttc tgaacgacac cagtccttct atccatcttc ctactttttc ccaaattaat 2820
   ttatagccca tgatccttcc ccagccctgt cagcttaggc cagcccatct ttttgcctcc 2880
   taagggttat gctagcagcc ggttgcagag agagctagga tataccaaga tccccacccg 2940
   gcactagagt gacggttaag agcgtggttc tgtgcttagg tgcataccga ctctctgcct 3000
   ggtgagggcc acccaggaac tccaagaaca agtccatgac ctcaacctca ccaacaccct 3060
   gaactagaca gcccaggtag ccaccttagg ccataggtag aatagttcaa gttgagaccc 3120
   ctagaaagca ctagaaatta aagacgtggg aagaatcctc agaccacaga aaacagctgt 3180
   aggtggagac ccttccacct ccttgcgtga atggtaccca tgaaatatga cagctcccta 3240
   tgtgacaatc tgcctcgcgc ctcacttcat tgcctggaat tccaccatta gggagtcatt 3300
   ccaaatcctg cagcttagag ctcaaacatc caaacctgac ttctgaggcc ccctaacagc 3360
   cttctctttg gaaacagtct aatcagagag tttgagacac agggagatgc agattacata 3420
   ggaccttatg gttatccccg tgcctgagct ctctgatgtt agttgtgctt ttgctgggtg 3480
   gactggaggc aaggaaagtg ttatccactc cctttgcaag ggacacctaa cgtctgcaag 3540
   cgttgctgtg cacagattct ccctctgcca atttctgcaa gggtttctgg ctgagctcca 3600
   tttcctaact gcaggctgct gataagaaat acatcttgga aacctttccc cagtcctgtt      3660
   tagagctcgg ggcacaggct ggtgaactta cacagtgggc attttatggt gcattctgca      3720
   tgaggggacc acgttattcg tcagtgcatg ttctacaaag caaccaaatt attttatgct      3780
   gcctgctaaa tggaggaatc gcggccctgc agagttgcgg gtgggggtgt aagaacagga      3840
   aggggaaaga tgtggaagag gaaatcaaca attctggcca agaagcttac aaaatctaac      3900
   tgtctagatg tagcagggga gtttgagaca gaagaaggat tcgacatttg gaggctgggc      3960
   taaagagttg gaaggttttg caatcaaacc cagatccctt aggttggaat tttatgtttg      4020
   tgttttgtag gtgggtattg gacaggatgg gttcacaggt gggcagatag ggaggaggct      4080
   ggggatacag ccaggagaga taacttgttg gcttctgact cttggtgtct tttgacttct      4140
   catttccctt ggttgagatg gttggtattg ttatcaggca tggatgatgt ccacacgccc      4200
   ggcagccact gatagcagct gtcacaaggt acaaaggagg tgcatagaac atccccgaat      4260
   tcttagaaag aaaagctcca cgtgtttttg ccttcccctt catggtctct ccctttttct      4320
   ttcccatcaa catctatctc ccatctctca acaccttctt tcattgcacc caataaagga      4380
   gcttccgctt cctgcctcca gagttgctca atgctgcctg gtcatcatag ccctttttgc      4440
   tgacttgctg caactaaaag acagcctaga aaatcctaat ttcccccttt cttcacaata      4500
   gaccatccat ggccctccca tttctcctga agtctatttc caatccaggc ctcccctagc      4560
   cccccattca gcctcacact caccccagaa gaggagcagg agaaaaatct atcctaacag      4620
   atggacttcc ctgagctatg gaattttgtc aaaggttccc tgaatgaagg tgtttgcagc      4680
   atccagtctg gacttatgat aggactcagc tcgaactgcc cctcctcaac cccagacttc      4740
   tcccttcaac ataactcccg accacattct ataacaactt ctttcatagc acttatcact      4800
   aaactttctc taccccttca ggcatctgca ttctctctgg ccaacttacc ctttgtgttt      4860
   ggtttccgtg tgggtcttat gtgcccttcc cagctgtgca gtggtctctt aatgctgtaa      4920
   gtttcccatc attctcttcc actagccctc tccctcacaa ctgaaaccca ccaaggtcag      4980
   agtaacaacc cggtcttgtg                                                 5000
<210> 44
   <211> 5000
   <212> DNA
   <213> Artificial Sequence
<220>
   <223> Nucleic acid probe
<400> 44
   caagccctgt tgatccccct tcccaaatat aatttgacaa ttacccattt aaccccacca 60
   ccttggctat gcctgatcca gatcttcctc tgaatttaac cctttgcatc gcactcctgg 120
   atccttgaag tgattttgcc cattgctgga caggaagcct tgtttgcaag cgcaatagta 180
   actgtccact gtattggtgc agggggatat aatgagggct tgaccactcc ttgcctttct 240
   caatggagtc accatcccat aaatgaatgt ggagaataac aatgcactag acacaggaag 300
   gtttccaaca gtgttattac aagttgcatg ctctgggcaa gctcttggat ctgcacattc 360
   atccacgtct gaagagtgga agagaaaacc caggtcaagg attattggct tcagatttgc 420
   agatgaaagt ctcaaactca accaagaccc accctaatcc cctcactgct gtcttttcaa 480
   acagacttgg acctgacttc cattcatcaa tatctgtggg gtacagaatg ggtgtggagc 540
   caagttggcc aatgagccaa caaaatcatg cttccctttg caaaatatgg agtcatgttt 600
   ctctctgctc acctctacat tccactcctt ggtctgtgaa attcaactgt ccattgcttg 660
   gtgcaaagcc agggtggcag gtgcaaaagt agctcccagg aaacagattc cattgccaag 720
   acatacaaag agattattac tttggcagct gaagttgcca tctttctggg agccttctgg 780
   attgggatga aagcctgcaa gacttaaaat ccatctatta ccctaataca caagggggaa 840
   aatgaacaaa ccacaactac acactacaac atgaaggacg tgtttactac aacacacaat 900
   gatctgctta ttatcgggta tcacatcttc cttacatttg aagagaaccc ctaggaagaa 960
   aacaacagca accaaaccaa acttcaacct cagcaaaata aatctacaaa ttagcccata 1020
   aagcccatca tatgaggtgg ctctgaattc ctgagtctct agaattctca gggaaactaa 1080
   aactcattac ttgatcagaa catgggtgtg ggactcccct ccccactgac atagcagaaa 1140
   catctcaact aggaccatct ccaggaaaca tagtactcag ttcgacaact ggcaaaatct 1200
   tcattaatga aataacagct gtcccttttc tgattaagaa taggctggga gtccatccaa 1260
   aggagaatta aaaggggcat ccactcgggc tcctttttct gatatttgct gagggtttcc 1320
   aaaaagatgc cagtgtcatg ctttccacac tctccaggaa gactgtggcc agggaggacg 1380
   tctcttcctt ggtgaattta cttggccgtc tgggcatcca ttaggtagga acttcgtagg 1440
   tttatgtgaa tgatactgga actactacca ggagtggggt gctaatgtaa aagatctaca 1500
   cagaaagtca gtcctgaatt tggtttctgt ccaaaaaaag gtacacctcc atcatggcga 1560
   gcaaaggaaa agaatagaaa gttcaattca tcataggaaa ggaaaggaga acttttgatg 1620
   ggaataagtg accctctcag ctgcacacgt ccctggaaat tccagatctc gcaggtctcc 1680
   ttgctcctcc ttcagaacac tcccctttaa atatggacgg tctccaggga tacgtcctat 1740
   gccgcttcct gtcccaacca gacagtacca acccccaggc ttcatctgcc ccttggatgt 1800
   tagacaagaa tctcagtaga gacctcttca caaacctgaa tgttctccag agtgtagatg 1860
   attggatctg agaagccgtc tttcttctct ccagtcatta ctcattcctc tagcggtagc 1920
   ttctgttaat tggcttgaat tgctggaaat tgtggaagct taccggcctc aatagcttcc 1980
   cgtaaaaaat gagagttaag agatttgcca tctgattaca gctgcagatg gtatatgtct 2040
   cagaagcttc caggatcaca cagccaaagg atgtccatct tccacccttc acatcagtgc 2100
   cacctgctcc aatttctagc ttcaggacta gacgggccac tttacccatt ctgtgaaatg 2160
   ggtctaaaac cacttaactt aaagagccca gcacactgta gcctgagaat gggatcaaaa 2220
   tccccccagg cgggaagaag agttatctaa aaccctggct cgggcaaaat tagcttgggg 2280
   gaattttctc cctctaacac tggtggggac acagcccgag cgatgtagac cttgttgtca 2340
   gtcttgtgta taccggcgag gaagagagtc ttcgccaaga ggagacacac gcagaggtgc 2400
   taaccaaagg cacagatgtg cagcatcttg atgttgcgag agctgaagta attcaccacc 2460
   ttcaggtttc tgaccatcaa gaacagtatc acagcctcca actgggaggt gagactcaga 2520
   tgatgctcag gatggtcccg gttgcagttc agtttccttt agatcactgc gcttcctctt 2580
   ttagaccttc ttcctgggca tgtgcaaact ggccccaaga aactccagat gaaccctgtc 2640
   tctgtattca gccagcagct gtggagagaa ataggaggag tcagcgcttg tctcagagtc 2700
   gtacctgtgg attcagattc ctcaattgtg gaacacccga tcttcatctt atcccccttg 2760
   gctaccaagt ccaacgtcac attctcttca ctgcattctt aagacataag ggaatgagcc 2820
   accatgagtg agagtcagaa gaatcatcat ctggcagggc tgtcaagtct ggatggaggt 2880
   tgggaggcaa agagatgggt cattcaatct ccaaggtttt agattcttga ttttaggaaa      2940
   tacccttagg gcagctacag cattctggtt cattactgct gtctaattta tggcctctga      3000
   ggatctgagg tgtcaggctc agggagtgga catcaccctg agtgcaatgg agaagctata 3060
   gaacagtttc acacagaaga gcatcaggat caactttgag aaagagggct ttacctggtg 3120
   tcttttagcg ttgagacttc ggcattaaca ctggaaagcc tctgcctcag gatccacaag 3180
   gtccaggtca ggagaaggga aggagcagcc caggatgaag agctgggcaa aggccttgaa 3240
   ggtcagtaac ctggggagaa gatgtacagc atgtaaggct ctgggaggtg gctattgtcc 3300
   agctgggctt cgtcttccca gtgatccacc tcttgtattc ttctcgtacc tggagaagga 3360
   gacacatcat ccagatgcag ggaaatggtc cagtgacagg ctgctaatat atacatataa 3420
   atccccagtc ttcaactcca cacttcatcc atgagctaac tagggtgggc accctcagct 3480
   cccattcagt acccaacacg atccatgata tgggcaattt ttccagtggg gtgattcctg 3540
   tggctcattc tgaaatcact ggacttaacc tctatgacat tcggtttcct cataatgaaa 3600
   cttcttgcaa ggaaaatcat ttccctattt aattaagcca ctggatctaa ggtgcttgca 3660
   tccaaaaata tgactcactg ggatctgtat gtggtgggat gcaagaaagg actttaaccc 3720
   tgtgagagag aaaagaattt cctcagagtc tgcagcagaa ttaatacaaa cgtgattcca 3780
   agggtcatgt ctacagggac gatttctcat cagtgcatac aaccacagga agaccgcccc 3840
   caacattctt ccccagaggg ttctggggct ggtgggatcc tcatttccat gttaagctga 3900
   caggcgacaa atcatttatt gagaggttct ctgtatcagg cgtatgatag gctctggagg 3960
   ggcatgctta gaaccatgca ccaacaaggg caggagaaaa cacaactatt tttaagtgtt 4020
   tgtgccaaag tagcttatct gtctatgtgc cgtcttggtt ccctgggact ctcattacag 4080
   ttttactgac catttcttta ttggttacct gaaggacctc aggaaaccaa agcactttcc 4140
   aaaggttgtt gtcaccaagt gcttcaaccc tccagggagt gctagaattc tgctacatca 4200
   aagacatcaa ttgagttttc agggaagtta ttctaagaga agatgcaaag ggtccaagtc 4260
   aagaagaaga catttctctc tggctttaaa gacaagctct ttatcataga tgaactcttg 4320
   ttcacaaaca tgtgtcccac ctcccaagtt aggctgccaa gaataacaaa gacagagcct 4380
   ccatcaactg agtttaatat atatagagct tcttaaggtg actatatttc agaccaacat 4440
   gtaacaccgt tgtacagaca gacaaagcct taggcaaatc ttctctcagg aaaaaacttt 4500
   aaaccaagag tgatcacttc acgacacaga atatcagaat agtgttctgt tgctgtgcag 4560
   tagatagaca cactctcttt cctggatata ttcatttaac ctgggcagct gagaaaccct 4620
   tgtgccgaat tcctagtcat gttgcgattg ctctctggtc catggcagtt gaagaaagac 4680
   agaataaata agccatgtat gagtagtgta catccactcc acctgctggc tttgaagtta 4740
   tgggattcct ttggaaaagg gaaaatgaag ggatattcac atggttgcaa aatgtgtgca 4800
   accaaaaaca aaaatgcatt atcatcctag cttaggggtg tattgtcact gcgcaggctc 4860
   ttttgtccaa gacactgaca ttttccccat tttctttgcc ttcttcttgc agctactgcc 4920
   accttgccag aaactcacta actggcatct ggattcagct catagttccc tttctggcct 4980
   ctctgctgta ttttatgctc 5000
<210> 45
   <211> 5000
   <212> DNA
   <213> Artificial Sequence
<220>
   <223> Nucleic acid probe
<400> 45
   aactcaattt attcaggtct gtacaacatg acttaccctt ctacatggtg tgctgtttca 60
   caaatgatgt cacgtgtgtt tgtaaatcct gcctccctga aagaacaaga catttgggag 120
   aattcttaga tccagagtcc agtagtgtgg cacgtgcaat gaagtgtcgg aaggatgcat 180
   tttaaagatg gcgggcggga gtctacagcg cccctgtggt cacacataga ttggacaaat 240
   gccactattt ctagctttcc tgtgaaaagt ctaggctcat tcacctattt tggcttttta 300
   gttgtaatgt cagatattga gctttggacc taacagaaat cctagcttgt catctctggg 360
   caagttagaa gttttagtta atttctctgt caccttttct tccttctgag tgctttattg 420
   gaatccgaca tctcatgcct ctagaagctt tctggttcct tgatctgaaa gaaaagagaa 480
   attgcattag gttcctgtat tgggcatggc caagattata tggaaaacta gagagtaggg 540
   cttggtgtct ttatctttag cctcccattt taggatcctt tctcccaaac caagctctgg 600
   gacaaatttt cctggcattt tccctttgct gttgaataaa actaccagca gttaagacgc 660
   tagtggcaaa aggctatctt aacacgtcat taatttctct tcatcaacac ccttcaggga 720
   gtgttgctct ttgtggtaca ctgtctcctt aatcgccagg taaggctaat tattttgtct 780
   gtcatcagcc tagtcccaaa ctggatgtaa gaaaaatcac caagctagat agcagttgtg 840
   gaaaacctag gaaccaaatc aggaacagga aggactaatt agcaagttta tcacaaacca 900
   gaacagggca gagccacctc aaaggcgtct cacaggtcca ttctccaagc ctcttacttc 960
   aaatgcttaa gagacaaaat ctggttggct ggggggaaaa ttgagagact tgttccagat 1020
   ctgttagaag cccccatcca cttgagtccc tttgtctcct aagctccaga attaaccatt 1080
   gtgatatgga atcgcatatc ttaagtttgg aagcttttct gcctactgaa tgttagagat 1140
   tcaggctctc agcttccctt taaaaataag gtatgctacc tttgccatca taagccaaga 1200
   ttatcagacc agtctgacat ttcttaaatt tggtttcctc aattcttgag ggtcaaagtg 1260
   ccacccttct tggtgtttcc ccatcccccc atttctcaat agatgctgga tccttacccc 1320
   aagagtgggt aaggatcttg cattggtgag tcacgtgcat gaaaattaaa ggactgtcta 1380
   atttcagacc ctgtctcaag attccaagtc acttttttca tgcggttccc acagataaac 1440
   ctggtgttct acttccaagt tctgtggatt ttgagaagca aactttcctc cctcaataaa 1500
   ttcctgaaag tactgtctcc acttttcttt ccttggattc ctcctggctc acttgttgct 1560
   ttgatgtaat aatatcacct ctcctgggac ttcttttcaa aacctcttcc tcttgctggt 1620
   gaatagacag ttaccctgtt gtgctacact aggttaatgt cacactttgg ggtagagatt 1680
   aactatctcg tctatttcct tcaaggtggc caactacacc agcacgggca gattcaagaa 1740
   gaggttcatg taccctgtag gctacgggat cccagctgtg attattgctg tgtcagcaat 1800
   cacccctaac tcttctgttc cctccaggtg ctgtgctcca tcattgcagg gctgctgcac 1860
   ttcctctacc tggcttgctt cacctggatg ctcctggaag gacgagaaga ctcaaagata 1920
   agcaggtgag aattcctgga tagatgaatg tccaggacat caaaggacag aaagtgccaa 1980
   ctaggttgat cagcttctgc cacagtctcc acattctgga tggggacaca aaggcttaga 2040
   agtcacccca gcctagtcca cattttaatt gccaattata cttcaaacca gttcttcctt 2100
   gacaggagga agaaagttcc acacgtggtc tctccataat tctctccaga gtccttgagg 2160
   ccatgctctc agaatagagg ccactcagta aggagggtct acaacaaaag ttgaaggtaa 2220
   agataactgc agtgagatga aactagcagc agaaagaata gcaacagggc tatgaatggt 2280
   ctaagtggga gttaataaaa gcagtgatga tgtagagagg aaaagtatga gctccatttc 2340
   agttgtcctc ctatagaagt tggaataaga cctttacatt ctgaggggcc atttagcaaa     2400
   atcacccagg tggggctgac catctctctg ctgtgcctct tcctggccat cctcaccttc     2460
   ctcctgtgcc ggcccatcca gaacaccagc acctccctcc ccatctgtcc agctttgccg     2520
   tcctcgtggc tcttgccccc aaggtaccaa gattcattcc tgtgaacctg tgtatgtccg     2580
   agaatttgtg agttatcctc acccactttc atactattct ctttccagcc tggtgacaag     2640
   agaacaaaac atatctgtgt ctactgggag ggatcagagg gaggccgctg gtccacggag     2700
   tctataaata gcaaggaagc tggcgaagcc attcagaggg tcaactccag ggtgctgagc     2760
   tgaccagaga agggcagaat agatggagaa taaacccacc tcggtttgaa ggaaaaaatt     2820
   tccctctctg aacctgtgtt cctgactttt cgccataatc aggtgagaaa cacccagggc     2880
   tgggtctcca tgcagtacct ggccactaac agtatttctt ttccattttt ttcttcagag     2940
   agcactgcag ttgccctatc acttatcaat ctcttgggga tattctgaat gcatcctttt     3000
   atattacgta gacaaaaatg ctgcacccca agcgacacac ggtgtattga acagcattta     3060
   agggatttcc aagggcagtt ttagccattc attgcctgcc gactggctga tgcatgctct     3120
   gtttccatgt aatggtctga aaaggtaaca ctcaatggtg tcagattgcc ttgaacactt     3180
   ccttgagaag cgtttatgta acatggtgga ataaaatgat cttccgtaac cactaactag     3240
   gcattttgca gagagaagtc gggctgtcac atggggtgtg tattttgtga catacacaaa     3300
   caggactgac tttcttaatg ttttcagtct atgaaaccaa gaggtgcaat gagacaaggg     3360
   agaatgcttt tctggaagct ggaaataaca ccatggatat aatgcttagc tactcttgct     3420
   tataaagatt gcattctccc ctaaccaggg actccggtac cattaagtgt cctgtggaaa     3480
   ctgcttagct tgcctatcag gaggttgacc tcacaaaatg atcagctctt gtgtttatag     3540
   gtttacaggg attcttccca aatgcagaag ttctgttcct agtgactatt ttttttttgg     3600
   gtgctctgct gtcttcaaca ttcaccttca ctttgtgata caagagagtt gttgtactat     3660
   cacacccttt taccagccaa tgaaaagggg agaagagaag gaccctccat aaagaccata     3720
   aagacgtctg ggtcctggct ttgtcagggt ttctgaatga tgaaatctgt gggctctcag     3780
   gatcacagac cctctacacc actaattcaa agcgtggagt tgagcatctg gaatgcgagt     3840
   tttgcttctc caggaaaggg tcaaatttct gaatttgata taggtaagag aggctgagta     3900
   acacgcacct tgtagcacct taataaatat cgttgccttc tgggctcccc actccaacac     3960
   ttgtgcatat tccctatttc ttacatttca gtaagagtca tatctctttc aatgacatct     4020
   tttctttcac agagaacaat agtcaaggga cgacacagtc aaacgtggat atttgggtga     4080
   gtggggtgaa gcctggattt tataattcta ctgtgcaata ccttttaggc ttcttagagg     4140
   ggaattagga tgggctgcag aattattagg gaaagactca acttgagggt gactagaaga     4200
   caactttctg gctggtatta tagattatga tcatccggat tgttacggta agaacctcca     4260
   aatcctattt ccactatgaa aaatggtggg gacttggggg gcaacacttt ctcttgattc 4320
   acagatatta atgaatgtga aaccgggctg gcaaagtgca agtataaagc atattgtagg 4380
   aataaagttg gaggttacat agagcacctt ctgcctgtgg gtcccttgct tgaacctcag 4440
   ctctggtcca ttcagcacgg aaggctgatg tggttgggag aggcaagaca cataaacatt 4500
   tctgcccaaa acacataaac ccatagccca aataaaaccc gtacctcttc agcagactat 4560
   ttgacaaagt acagccattg gccaggtgtg cttgtccctg tgccaaaagg atgccaacag 4620
   aggcattcct aagatgacat tcttctgacc gcagcatcgg tacgtggtag atgcatactg 4680
   aatatttctg tttctctgca caggggaaag gaaattggga tctggaagct tcctttggag 4740
   gagggaagga gctttttcaa gagacagagc atggagtctc tggcactttc ttctgactct 4800
   gtagagaaga agctgaattt gctgctgagt tttctgaaat tgggaaacac agctgcaatc 4860
   agatgcactt ctcatacttc agaaggatgt gatgtccaaa aaagtcatct gcatttatag 4920
   gaaccctctc tggggtaggc taaagggtca cttcgagtat ctgctacatg gattgacacc 4980
   aaatgggtcc gcacttggag 5000
<210> 46
   <211> 5000
   <212> DNA
   <213> Artificial Sequence
<220>
   <223> Nucleic acid probe
<400> 46
   ctcatgcccc tggtgtcctc gcttgatttg cttcctcaaa tttggcattt tctccagcaa      60
   taactgcgtg tgcctgtcct agtagaaact ctcctcttga ggaccatcag aaattggatt     120
   tcatgaggtt tatttgttcc aggcattctg accacgtcct gcttcagaga gattgttccc     180
   ggcgtctcag tgctatgggg tccctataac caaatacgct aagatcatcc tcgcagggtg     240
   ttttccttga gagagttacc cagaggcaga attattgggt caaacgagag ggagagatta     300
   aagatcttca cttggctcaa gattcaaaag atagaagagg gtaaactgaa aagtctcctt     360
   gcctcggctt cccagccact tgctttcttt ctctcttttt ccctcgttaa ttctcattac     420
   cactgggtaa aaggtcagtc cttagcatgc aaagttgtga ttacaattgg caccagggaa     480
   gggacatgag agatctccat aggaggcagt aatggaaggg gtgagagaag catctgcagc     540
   aaaatttgac ttgcaagagg attccagtgc atgatgtgca gctgcctgct tgggaatagg     600
   aggtatccag aaagtaatca agtataactc caccaatggg acacaagatt cccgcaacag     660
   aaattgattt cagtgaagta gtattgtcta ctgcagagat tcctttattc atgaccaaag     720
   cagagggatt tacatgtcaa ctttttcaac cggctggtta tgatccacag gaggaggctg     780
   gtcctcttta gtgggtaacg aacaaggaaa acaagatcta aaaagatgag cgagtttcct 840
   aagtctgtag ggtgaattaa cagcttttcc atacacctga cacccataat gtacacatca 900
   tgttatgtga tactctgaaa cctgctggga tgccttatca cgcacaaaga agggggcaat 960
   ggtgtggaaa ggggcttaag gtgaactaga atgatcaaga aagaaaaata ttctggttct 1020
   ggaaacattt tcaaggaggc ctcattacat gtccggttac tttatttctt gaaagctgct 1080
   atgtcggctt ctatctcaca ccggagctca agataaagag cgacgtactt cctcttctgt 1140
   cagttctgaa aagctaccag actcagtgag aggcaaggac ttttcatctt tagggtaagt 1200
   catttcccca ttcattttct gaggcacttt gatatggtcc accatgataa tgtaccactt 1260
   aaattacggc agtggaaaga gaactaatag accctgcatt ctatcagtgt aattctcttc 1320
   aatggtacca tcccaaaaac caagaatatg gatgagtcat tttcccaagt tggtcaactg 1380
   gaataggaaa tgaagcaata atcgcgtgca gaattggact atgagcacac agaatgctca 1440
   gaatttcagg gacttccttt gggtttaggc atttcagttt tacatgcaga caattttgtc 1500
   agttgttgtt tgccatatgg accaatccat gttccaatca gctgtggttg ggaggtagag 1560
   ttaaataata ccatgtctga caacaagagt gcaaataacc cagattctcc tatccaacgg 1620
   cttctgcttt tgttgtttct taatttgttc attgcagctc atgagtcacg ctgctgtctg 1680
   tatccctttc tgggacttat gtttgggaaa tgaccaggcc atatcaaacg ttcctagacc 1740
   gaacatttgc tccttgggtt ttgaaatggg ctggtttctc tgacggtgac agtgtactgt 1800
   aagtgcctca tgatccagaa catacaggga aacctcttaa tgcacagggc cggttgcaga 1860
   aggatggaga acaggtgttt ctcacaattg cccaaagtga tgatttatct ctgaaagtgt 1920
   attacctagc aggagattgg ggtcactgat tgggtttata atgtttgttg aatgaataca 1980
   tctgggaaag accgtgtgtc aaacccgagg tgtggggcgc catcatcaca gtgaagctcc 2040
   tgccttcagc agatcctact acttgccgcc attctccccc atcacatgaa aaaaactcct 2100
   atatacaccg aataaataat gatatttgcc tgctctggga aacagggaga agagaaattg 2160
   gattgtctgg ggagccaacg ggagactact tgtttgtaaa atttcctgga cacgtatgag 2220
   tattatctcc cagactagat tgcttagctc tgtggaggtg tgcacctcaa agcaggttct 2280
   tgcaaaaacc cacttccccc tgcagttttt attgagtctc ctttctccct ctggtggaga 2340
   caaatgagac cacagcggta aaagactgtg gcaagtgtag cttgatgttt atagtaagaa 2400
   gccacgtggg agagaaacaa ggcttggcag tcaacaattc atcactctgg ttgccaactt 2460
   ccaaccacaa gatgagtgag taacactatc ttagattatc ttattaaaag catttgaaag 2520
   gcaacagtgt aaggagtgga tggttttgtg gcgagaaatt gggtttcgta aggcaaagaa 2580
   ggagaccttt atatgtcatt ctccaatctt ggttgaagat actgacgtgg tacctggcat 2640
   gacggcaaca gttttgttct gtgtctggca gttgtattta gggatagtct gtctccctgc 2700
   actctgttat aactctcatg gatatgtaag ctcagtgtcc acctttccat tcgatgagat 2760
   gtaagagagt ggcattaggg gagacccaaa tatttctctg ctggggcccc tctctggact 2820
   cggactcacg gaatgaagca cctctcgcgg agaccgaaag attctcccag acgctgcaag 2880
   cgcggctctt ctcacaagac tggtgggtcg ccccctcttc cctcccaagt ccaaaccagg 2940
   catagcattt cctcacccag ttattcctct caaatccctt ctgcagccct agggtcccga 3000
   tttctacaca gtggagtctt gcaacttgca ggttctacac ggtacctacc tgatcataca 3060
   gaagtccacg gatcagtgag gataggtgaa acttgaggct cttaggaagc caagttcaca 3120
   gtttggagca tgtggacaaa taaggggcca gagtgcacgg gaaaggttgt attttgaaag 3180
   gtattataaa gagtggtgag ctgaattcac cagattccta tccagccatc tggtccctaa 3240
   aatgggacaa gaagcttaaa gctgttttca tctgacttgc ttaactcatt tggctggaga 3300
   gccattcccc acaggccagt tgttcgctgg tacttggagc caagcagaga tgggaggctc 3360
   tttcccattc ctgcctgcag cctcacagcc cccaccctcc accccctccc atcccatgct 3420
   gtgggtcagg acctttcaag cctggactct gagaactgcc aggaccccat gactcaccgg 3480
   cagttggggg caggacgaca caggaatcca cacctgccct gtgtccactc acacatctgg 3540
   aggcttcccc acctgcctgc gatcgaccag gtccccatgc atacacagtc acccacaaag 3600
   ggccgtgggg ctccaggcca ctgaaaggca tcaagtccaa ccttctggct gaaggaatca 3660
   tgatttggaa aaaggaacat ccgactcacc tttagccagc tggagaaaag acacatgatg 3720
   tagggggact gtgcagcaga caagctctcc catgaaactg aaatcagttc tgaaggtgaa 3780
   gaggtgtgac ttcaggagca tgctcacctg gacaggtacc tgagagaatt cctctctctt 3840
   ctgtcatcac tttatcttct tgctccagct gggagatcag cctaggttta tcaacttggt 3900
   cataggagac ttaaatttca gaaaaagaga tgtctttttt tctcccctga ggcccaagta 3960
   aagtatgaga tgacagaaag ttggaagtca tgaaggtaca ccattgtgga ttctcttatg 4020
   aacagcaagg taagatctac tgctgtagga tttcccacat tcactgcagc catagggtct 4080
   ttctccagta tgaatcttcc tgactgagtg cggaactcac gaaaacactg gttacatttg 4140
   tacggttttt ctccagtgtg aattctcaag tgcggtctga ggtatgagga attgctgaag 4200
   tattgtgagt tgtcatgtga ctccctgaag ccatcactga aggctttcct acagctactg 4260
   cattacctgg gcagctcccc agtagatact ttttttttgg aatatgactg tttgtgtcac 4320
   aggcttttca acactgtgta tactcaacat attcctctgt tgtatgatct ccgaaatgtg 4380
   acttaaggaa tgagtgtccc accttttgtt ctaagttact gcgtggccac tcagttaaga 4440
   gatgaggcat cctaacattg attacgttcc tagggcttcc tctaaatgaa ttctctcatg 4500
   atgaaaagaa aatagatgag aataagccag acggtgtctc acccagtgcg ttattcctac 4560
   ttcttttagc cagcagagtg ggataaagcc tgtctaagct gcagaactcg aggctggatg 4620
   ttgcccctca ctgacctcct gtcagctcct ggaatgcagc cctctcctcg cttgaacaac 4680
   aacttgcagt tctcttcctt tcggggagta tcagtgcatg gacaccaccc cagggtagct 4740
   gagcaactga tagaattcct gggcttggat atgaactttc catccaggtg atgggcatga 4800
   caaaggaagc tactgttggg ataatgtcat ttggtcgtat tcccatcaga aaccttaagc 4860
   tttggccaca accaggactc ttcatataaa atggtgatgg ctggggtatg agtttgtcac 4920
   ttatgttgta gaatctgggg ggctgcctgt tgagattctg accattgctt gttttgggtg 4980
   tcattgctgg ggatacttat 5000
<210> 47
   <211> 5000
   <212> DNA
   <213> Artificial Sequence
<220>
   <223> Nucleic acid probe
<400> 47
   ctgtagacag ccctttccac ctgaacaagt ctcagagatg caattgatgc ttatcacact 60
   cctttacttg caccttccag attcccttta cccttgacta tgaggggcat ctgaaggggt 120
   catttcaggg acttctgtta tagttttgga cgtgactgag gggtgcttgc tggagtagtt 180
   tcgcgatgac ggaggtcttc tttgccagct tgtcaggact accatcgtct tccatgttct 240
   gagactggag tacccaacat tcccctagca ggggacactt ggcttgatag ggctctatca 300
   ggatagcagc taccttgggt ttctatattt gcttatttgt ccctctctac tcggtgactc 360
   attccacttg gaagaaactt tggactggag ttccctgccc aaaagagctt gtggatttag 420
   ggtggtgcaa tactgccatc ttcagggcat attttgaaat aaaaaaagaa cggcactcta 480
   gatgtgtcag ccgtgggaaa cgattatgaa acttccattg ctcactgtaa tctgtgatca 540
   gaaagggagt ggctcccttg tctgtggagt tgaagagatt tcccttgttc aaagtgagta 600
   caagataatt agcctgccat actgcagaca agtctccttg tctcatattg atcttttttt 660
   cttatgggct tcttcccagc tgagtcaact ttacattcca tgcctgtcct ttcatccaag 720
   gctcctatcc agtggtaaga ccctgacagg ctgagaaagc aaggcacagg ctttggtttc 780
   atagaccaca gcctcacaac gtgctggcta ctacccttgg atctctgcct ccttcttcca 840
   cccttctaga agaaagaaat acttctcttt cttctctttc cctcttccct gctctctttc 900
   gacccccaaa atattatatt tgtcttagtg gctcaagtca gctcccggaa gccctctgcc 960
   tcattttgaa ccatggctct cctatgatta gttgtacgaa tcaatgtgag tctcttggaa 1020
   aatggaaaat gtttttcttc caaggaaagc tcatcaagga aggcatcagt ctcagggagg 1080
   gtggacggcc gtgtggtctc caaagattaa atgaggtaac attgatctct catgctccct 1140
   tcaggatgga cattcccata tccccaagtc cctccaagtt ccctgttctc caagaaattg 1200
   aagttgcgtc tgaattcttc acattcctcc aaactctggc ccagagttcc gcagtatggg 1260
   gttgttaaat tagcctgaag acacacaaag caactaactg tgtggtgtcc cggtaaggac 1320
   ctgtcgtgtg gtcgggggat gtggctatga gttgggctgt gttaaggaaa acctcttgga 1380
   atatgtaggt gccttttttt aatgggagtg aggggagggg acacaatggc tttttgagct 1440
   tacttttacc ttctgatact agtcaaggtc cagaaccagc caccagccaa atttctatct 1500
   gctaatcatt tctctccttc acagtgggtg gaagtttagg gttaaatgtc ctttgaatgt 1560
   cacctggtga gtccttgaca ccttaggctc ttcagaaaca tagcagggca ggtagtccac 1620
   caagaaatgt gcttattgga ttgggaggtg tttatttgta gtctgctgta acacgtgtga 1680
   aagagcagga gcgtcatcag ccaagcacat cactttctct ctgcatcagt gatgtcaaat 1740
   acgcatcaga aaatgttcag gttttaggag ctgtcctagg tgctgtttca tcattggaag 1800
   ccagtttcat ttatttgact ctactgccac agtgaaaaga gcaaacagct attgggttgc 1860
   aaacctcctt tgacattagg aaatgttgac tttgtaacaa tgcagctttg ataacccatg 1920
   tgagagtcta ggcaggacca gtggggccca aatcttgctg ctcttgtact tttaggcact 1980
   gcccttgcag actcaccttt ttcaggctgg tgatgcacct tacacacaaa cacctaatgc 2040
   aatgtctttt taaattctcc aagtgggatg ggagcatgtg agggaaattc caatccaaaa 2100
   tatctgatat atggggtaac ccagcaaggt gtaccttcct ttggtgagag atggctgccg 2160
   gggcaaagac gggctttgat tcagagcaag cattcccacc atgtgattca atcatggaca 2220
   tctttgagat ttcagaattc tacctggaac cggtctgaat cagggaacgt gtgtatcagc 2280
   tgattcgaat gccagggacc taagctggta gctacctagc tctgagcctg ccttcaagaa 2340
   cacctgtgtt catcctctga ttctctgtgt gtacctcttg tggcgtttcc tctcccgggt 2400
   ggcacaagtg gcctctttgt ttggtcttaa aggcatccat ttctgggaat gaagccatgt 2460
   tcgctgctaa cacttttgga tgttgtgagg ccacgtggag ctctcgacag attcttcttg 2520
   tgtcagaagt ctagcctcag gtttctaccc tcccttcaca ttggtggcca agggaggagc 2580
   atttcatttg gagtgattat ggataagggt ctgtggtccc ggcgtgtgct cacgtgtgta 2640
   tgcacgtgtg tgtgtccatt agacacggct gatgtgtgtg caaagtatcc atgcggagtt 2700
   tatctttgga aggttgcaca aaacacctat agcggtagtt atcttgggag aagggaactg 2760
   gagacctggg gttgggggag agagacctgt ttgcctgtta ttgcatagct tgtctttaca 2820
   tgcatgcttg catttgcata ttgcattgtg gtacacacac ttgagttccc atatgtgcta 2880
   catgtacgct tacgtttgca gaggtgttcg ttgaaagggt attgcccttt acacgtgtgc 2940
   ttggttttgc ctttcctatg tctacacgct caccgtgttt gcatgctgta tgttacaggt 3000
   cctatctgtc tgctgtccgg gcaggtatgt ctgctctcct agaccagggc tgagcacgct 3060
   gcatccaggc cacagggtgc tgtgtgtgac atagacacca tttccacaat cttccattgc 3120
   tttttagact cttccatttc ttgctttttc ctggcacctt tgtaacccag gaccacatag 3180
   aggtccacct gataccacct ttctcaaatc ccatcctctc ccacttcgcc tggaaccaga 3240
   ctctatcact gtctcatcac tttctggaat gtttcattgc tttccagaat ctttcatcac 3300
   aacctgtctt ccatcccctt ggcactttgc agaaacagta gtctcaggtg ggaagtagca 3360
   tcattccata gcaagggtct gaaatcagac aagaaggatg gagtgaagga tgggttcccc 3420
   agggaggcca agaaaagccc tcttattcat ttgagcttgc caaactgccc ttgctgcaga 3480
   aacctcatta ctgtgtgcat gattctgctg gtaagggctg ccatttgcct tggggagtct 3540
   tgtatgaaac acctttctgc agagtcccat gagaatctca agctaacgtg cctcgttttc 3600
   ggaaaatcag gatgtggaag agcagcagag aggtttatgg acaagggagg gaaggaacag 3660
   ggtggcccac ccattccagg agtggatgtg atttttgatg tgtgctttgc tgccttggcg 3720
   tcatttctga gcaggatcct ggctgtgagc tccctgcgag gggtggacac tcccagatgt 3780
   gcaaagctca gccaccctcc tggtcttatt cccaagagcc tcctgtctct tctcataagt 3840
   tctggaatta tctctagtta cgtgcttatc acagggtatg tttcccactt tgcaaatctc 3900
   actctccacc cccctcctaa atgttccagc ccggttctct gcatgcacta ttagcttgga 3960
   actgtgtcga aaacttttca gagttttggg ctcgatgctt catcagcccg gaatctcttt 4020
   ttttgtgctg gagtgcctga tctgattgta gctgtttgag gcatctggaa ttcatggccg 4080
   ggtttagaac aacttggaag caaggagggt ctgcagtgga aacgggcatt gttcactctg 4140
   cagaatctgt attcttgctg tcaacaatgc cccgaacttc ctctggaatc tgctcctcac 4200
   ttggggattc gtgatgggaa ttcctaatag tgacccttgg gtcccaggaa caggagacac 4260
   gggccagtca aaggcatggg aagaagctag gagaagttag ttgtagtggt tccaaacctc 4320
   atttctttgc agtgccaaac ccagaggaag acagagtgca tcccagcatt cccagctaga 4380
   ctgaggtcat gtgacatcca gaaaaaggaa tccccctgta tccagactag cttatgatag 4440
   aggaaaagct tcttagctcc cagagttgaa caactgaaga gcttctcaca tggctggatc 4500
   catatgcctc taatccatat tcatgagtta ctttcctctg ccttttgatg tctctgtcca 4560
   caatagcctt tggaattatg atttgtagtt gctagataga agagattaca gcaaaatgtc 4620
   tgatttcctg gaactttgag agggagtcct cctataacca ggtggacctc cctctacccc 4680
   tcctgggacc acatctactc attttgattc cagtgcttct ctcatcaggt actgtgctat 4740
   tagtacttaa agccttgata cctgacttcg caggaagatg ggtcagaaat gccaatctac 4800
   gctgctgctc cggggtccga gtgtcatggg tgggacatca aggctgacca aggacagatt 4860
   ctccaccctc taagcagtgc atatctgtat tttaccttat cggtggcttc ccaagaccaa 4920
   tggacagaat ccaaactcct catcgggagg gctcggccag ttctcattct catttcattc 4980
   cggtgccccc tggccgtggt 5000
<210> 48
   <211> 5000
   <212> DNA
   <213> Artificial Sequence
<220>
   <223> Nucleic acid probe
<400> 48
   ggtcattcct ggcagtctgt attgtaatcc atgttcccca ttgctgcacc ctcctgcgct      60
   ctgatctttc ttcttaatca agccttttat tctccagtgt agatgtattt tttcctattt     120
   aaactgatga tgtcgatcag tagcttgggc caatgagcaa atgccatttg gcagggcttc     180
   ctgaaaagct agactcttac tttctcagta gttactcatg aagcaccaac gtcttggtta     240
   ttttcacgag acccatttgt gcctgtatag aaattatctg aatcttgcta atacggacat     300
   cccaggcacc tgtgtggcct gatctgccct ctagtggcca tttgcaatac taactgtagc     360
   caagaagggt ctaaaaatgt acgggcttgg tccaaagcag agcgagtggt ccagggtcaa     420
   agccagggtg cccttactcg gacacatgtg gcctccaagt gtcagagccc agtggtctgt     480
   ctaatgaagt tccctctgtc gtgctggaga gcagatgctt caccaattca gaaatccaat     540
   gccttcactc tgaaatgaaa tctgggcatg aatgtgggga gaaaccttca ctaacacact     600
   ccgacccagc agctcctaca ccatgaatgg aggttgatct ggaaacagaa tattttcatg     660
   aaagggcgac agggtatgaa caaaagaaca ccgtgtcgct gtttgacatc ccagggccac     720
   ctcagcaggc cgtctctggg gagaattttc tctgatttct tccccttccc ttgctggacc     780
   cctgcacctg ctggggaaga ggaaaaatgg acaaaactca gcatttgcct ttcttacctc     840
   tcctgagctg gtctccacgt gagttccaaa acaaactccg ttagacgatt aacatttttt     900
   agagctgtgt ccgtccatct cagtggctgt accagaggca taactgtgct tgcaccgtgg     960
   gtcacaagaa ccctgggatt tgacttcggg gcttaagaca ccattcacag gaaggtactt    1020
   gatacacgat ttagagctta ttgtctatcc tcgaggcatt aaggggagga gagaaaatgg    1080
   aatttccttt gctcagcagt taatctcagg aaaaccacag tgtaccatgt ttcatgcagt    1140
   ggattctagg aagaggtagc aggtatcagg aatgatacct gtgcctgaac aaatatccct    1200
   tgatttatct gctctgatgc tttatataac tccagggcga gtccagtcat cagctccgct    1260
   gtaagctgtt ggccctgcat tcaagtaacc agaaccaccc cccacgcaag acttctgtgc    1320
   aaagctagaa acacaactaa tggttattga caaagtcagg gaaggatttt ggccccactg    1380
   agatccttta gttgtaagga catacataca catttttaaa catcgtaaca caagcacgca    1440
   ctgaagactt gtgtcaaaac cagatttttc ccatgtcttg gcaccatttc cctctgggat 1500
   ttgagatgtc agggttgaca gttcctttct ctttggctgt tcccgagtta ttttttcttg 1560
   tggcattaga ttgttgggtg agtaacatgt gaccctatgg cttaggttcc tttttacaat 1620
   ctcaccagtc gatggcatgc aaagtcaata gtgtctgctt ttatcaaatg cctaaaatgg 1680
   aagcagacag ctttcccata ttagatgcgt ctctccgatg tctaatgatc tgcagggtct 1740
   ctaaactcac ttggcatagg tttatttgca ctggagttgc acctccttcc aggtcagtct 1800
   ttggccaaaa caggctactt tgctcttccc tgaactcagc ttaggtgcct ctgtgttatc 1860
   agtatttccc ctatttgcag aaattggttt ctgcaaaacc aaaagctttt ttatagtcat 1920
   tcttgagaaa caaaagtggg agcagcccaa ccttggtgcc catcctccaa gggtacaggc 1980
   tggaattttt gctaaatttg atgtactgaa tactcacagt gagtaaggga aagtccttac 2040
   cttgatggat ttttgcgcat aagtaaatcg acagatattg taaggtcagg ttgtggtctg 2100
   gcgtgctcct acagcacttt taggatggtc gttcagtggc tcccccatgg atagaaccat 2160
   gctgggagtc tgcctcaaaa cctgaaatga acagctcagt cgataatgct atgagtccca 2220
   ttgctgtcac gcacagggta gatcatggtc ctgcctccat gggatatggt gtgaatccca 2280
   tcactgtggg gtccagcagg ggtcatgtta cagccgtagt tgcatcacat cccacacaga 2340
   acagcaaatg tcctgtctcc ttggagtagc aggttgaatt ctgccacgtt ggggtaactg 2400
   aaggtttgtc tggcctgggc ctgtttagct acggctgatg gtttagggga gcctcttgtg 2460
   tctttgatgg tcagtggggg tctctctcca taagtctctt cttggcctct ctcacttctt 2520
   cctattaagc tgaggtctca aatttgtcta cacctccatc tatttcacca tcatgtctgg 2580
   tctgacccag tccccttgaa aaatagggcc agatgttttc ttccattctg gtggcttatc 2640
   tgggaaggag ttcttgatgt tttcagagca gagttctggg aacccatgtt aaaaggcctt 2700
   ataaaaggca tttttccctc accttaacaa tggcctgaga aatatagcaa cctcctttga 2760
   ttttctcact gatgatgcgg gagatgctaa aacagaaacc cagaaacaga cacacatcaa 2820
   gaggccttga gctgacctgc ttacgtaaag tatcttcctc ttcagacttg agaattagcc 2880
   aagcgagagt gttggggtgg gatttcctac gcacatcgca gactttctgt tcgtactgtg 2940
   agcttcttcc atgagctatt ttggggccgt aactgagttg caaaaatctg tttcctggtt 3000
   tctccaggag gaagagactc gcttctgcat tattctcagg gctttgctgt tctctgaagg 3060
   acagtataat caaaccattt cctgccgttt gggtgagaaa aaaatcctcc acgtggcctg 3120
   tcctcactga tgtagcttca tcattgtcac ataaaggata taccaaggtc tttcgtgtct 3180
   tccctgacct gtagcctggt atgccgcttg ttaaaactgt gtgcaaaccc agggttaatt 3240
   ggctatgagt gaggtctctg ctctcagatg ctacttttgc accctgtttt ggtcctgggc 3300
   gatcattgtt gtctgccttg tatattgctt ccttcaattg aatttcagcc ctcaggttcc 3360
   cacagacaga tcagtgttaa tgatgttgat aattatgata gattgcaaag tctcgcagcc 3420
   tctggccttg tttgcaatgg ttggataatt tttcttccca ttgtctaggc cggaaggact 3480
   tatgtgtttg agaggtttgt gcttgtgatt ttcatgtgga gcttttcaca ttctgtctaa 3540
   caatccgagt tcagaggaac cagtatgggg agcaagagta tgtggaaacg tccagcacca 3600
   ctcatcttgc atgacacgtg gctttcataa tgtcttcact ttgcggtctc tctcaccaga 3660
   agaagctgct gttttgtggc tagttcatct gtgtcatgcc gtgagggagt gacagccccc 3720
   ggacggtttg gcattttcct gcaagtctgc catcttgtct ggcagacata gcctgggctg 3780
   ctgactgtcc ctaccccaaa ttctcagcag tgtatagtga aaaagccacc gcagctgaaa 3840
   gtttcaggaa gatgatagtt gcttctgtgt gctcacccag gagcttttgg gaggtttagg 3900
   atcaaattgc acttgcctct tggtgcaaga ttcgttcctt tgctttgaac tgcctccctg 3960
   aagcaaattg gtgtttctgt gactccctct gtctagggaa aaacaaaaaa gtgtcagagt 4020
   gactggattt ttgtgcgcaa gtcatttaac aatgcagcgc tgcgtctcct atttctctgg 4080
   cttttaacct tgctttggac tgcggctccc gaagcgggaa ggccctctgg tactctgttc 4140
   cctttctagc ctctatatgc gccgtatgtc tccaaagagc tgcgcagttc tattccaaag 4200
   cttgatgagc ccttgcaagc tcggtttctc cttttcccta gctttttcca aagttccatc 4260
   cgtggcagac tcgacctgcg gggtctgcag ccacgagggg atggggacac acaagttttc 4320
   aaatgcatgc ggaatactgg ctggaggtta ccccaaaaac gtagcctgtg gattttaaga 4380
   atgtatttat gagtatggtt tctcaacttc ctcatccatc taccataagc cagaaaaatg 4440
   acccccaaat gtgaatccga tggtgttctt tctgcctgac atctttcttt ggcttctgct 4500
   cccagattgt ccacatgtga gcacctgtct ttttggtttg ctgtttctct cgtgagttgt 4560
   atccagttgc ccaaggtgga aagttgggcg tcatccagca ctacatgaat gagaataaaa 4620
   acttccaaaa tgcccccaac aaacaggggt ttttgcacct tgccatgagt agctttctgg 4680
   agcaagccgg agtggttaga actctgccag ctcgtgcatg tagaagctcc cgggaaaact 4740
   gtcaacctgt gcacattcaa gaccgccata tacttgttat tgaaaagcca tcagtcatgt 4800
   catggctggc tgtccatccg ttccttagtt tgattgcatg gcagacatga ggaaggaagg 4860
   tgagaactga gctagaaagt tggcatttgg tgctctggtt gcataggtgc cagggctcag 4920
   tagcataaat ccaggtagaa ctgggaagtt ccaggtatct gggctgtttg ctctggtgga 4980
   caaagagcag ctgcaaggtg 5000
<210> 49
   <211> 5000
   <212> DNA
   <213> Artificial Sequence
<220>
   <223> Nucleic acid probe
<400> 49
   ggtcacaatt tgtaacgaaa tgtgcttgca tgcccagtgc taaagatttc tctctgtcta 60
   ccctctaggc tgtggaaagg tctgttctaa ccattagctt cttttaggaa cccgcagaat 120
   caggaggtaa gctaaaagtc gtctcccttt tgtcacgatg gaatgtgaaa taggtgcatt 180
   ttccttctga cttatgattc tgttactgtg gatgagctct tctagaaact tccaagcagt 240
   ttcaaagggg atttagatgt catcaggaag tgtggttgac aacgtgatat ttgggcttta 300
   gagcccaaat tcactggtgg ttcaagttcc caatcattct cggcattcta taatcacagg 360
   cctcagagag taatgctccc aatggccctc aaattatggt agtgggcggt tcctcctggc 420
   agccacagaa tagttacgtg gcatgaggtc agtagcatct cagagggtgt tgttgtcttt 480
   gacttagatg agttccaaga acacggccag agggaagata ggctgaggaa acagatagga 540
   aggaagttca agggggggat ttctatgtga gaaccaagaa acacctttga tctttagatt 600
   ttgattgcta agacacccac gacattgctc ttggaattgg agtagggtgg atgaaatagt 660
   attcaaagat gggggtggtg caaatgttct gtatttcaac aatctctgtt tcttctgtgg 720
   tcacgttcat ttggaattta cttattcaac ccacaaccaa tagacatttc cagccctttg 780
   ttagtataaa cagaacttct gcttggtgga atgcacgtcc ttttcaaata ccggctcctt 840
   tggaaaacac agaggatgga gtgaaatcct tacctggatt gtactggcag tgggatgaca 900
   gccggccctg accctgcagc tttcagtctg tgggagtaga gtcagctgtt gagcagttta 960
   ctctgtccct gtgtcaatgg gattgtgcat gttttggttc agtcctgccc cagacaaacc 1020
   gatattccct ccaaagctct atttggcaga agaagtgttt tcgttccctc ttgcctgtgg 1080
   tatctcccct gataagcgtt tctaagcctc acattttaaa cagagctgaa aaaggactct 1140
   aattgctggc ctgggatcct tgtgtgctgt aggaattcac tgagggggcg gggctggaga 1200
   tggcttgata aataaaggac ctgcaagaga gaatttatct tgagcattcc ttgggggaga 1260
   gaagcaaagg ggaagtgaat gctgcataat aattctttgc acttttcctg tctgaagtct 1320
   ttctctgctg gacgtgagtt gttgaaggtg ttgcttttct ttgtagacaa ttagaggcgc 1380
   tcatgcactg ggctatggac catccttaaa aggcatttat tgtgaagctg gttcatagac 1440
   ttctcgccca ggttgctaac agccaacctg tctgggtttt gggaagttaa agtgtctgta 1500
   ggcggcctag ccgtcttgtc ttgaagtttc tcttgccttc catcatgggt ttatttgttc 1560
   agcaaatgtt tatggagggc cagcacaggg gggaataaga cagagaatgt catttttcca 1620
   tcctgggtaa acagggtaga gaagaatgag gacctcaggc caggttagag agacctcgct 1680
   tggtgacctt gggaaggctt cttttccctc aggtgagacc atgagattag gaacctgttt 1740
   caattcccgt cggaatttcc caacagagct ggggctttgt gattcgaata tgttcagaga 1800
   cctgcatttt cgaacccaga gcccggggac atcccccgtg gttccctgtt accctctgga 1860
   tgtctgtcct gctactatgc acctctcccc aactcctggt tggaacgtgt ttatttcttt 1920
   gctcaccgtt ttgcaatctc tcttcccccg ccacaaggca gggactcttc tatctccgtc 1980
   accatcccat cctcccacag gtccctgcaa cagacacccc tgcagtttta tcctgaatcc 2040
   tgggtaattt ccccaggtcc tgggaagcct aataaagccc ctactcagat gttggattgc 2100
   taactgagct ttctattctc acaaactgaa agaggtcttg ctaatccaag gcatgacaat 2160
   cagagcaaca tgtgaagccc aaagagctgc agagctcccc attactcatc tctggagagg 2220
   aggcattcag atttgcctac tcagcattcc tggagataga accagatttt gcatgggtta 2280
   gaatcatatt ttgcttggga gatcatgtgc ctggtggata gtacatgctt gatagctgta 2340
   aacagttggt gctcaatata tcgtggcttg tggatcttcc cagctgcttc agaaccccaa 2400
   acataggctc agagtgtctt ctagctttca acccacccca gactctgact tagcctttcc 2460
   caaatggtgc tgtgcagaaa aatgaaaaaa ttgctaggag ctatctgagt aaggaaacag 2520
   gttcagagtc aggattaaag accaagacag cttgaggtca tcagaggcca ggaatttcca 2580
   tacaggctgc ggtccaaagt agctggcctc cctgtccctg gagtaagcaa acttccttat 2640
   acctatagct tcctctaagg aagcgtagtt acctacagcc tggccctccc caaggacaca 2700
   tttctttaac tcatttctgt caatggttac ctccaaaaat gctcccttgt gtctttatgc 2760
   tgggcttctt tctctagata cttccatttc ttggtttggg gcaaaagccc acctgctact 2820
   ggttagccag gaccatcaca gtgtgcccag gtaaccctgc gtttcacccc aaagcagaat 2880
   tttacaccag agcctggaca ttccagtcta ttctggtggc ttagaggagg gaagatggga 2940
   aattttgggt ttaatagagt aatatccatc aggttgaggg ctttgaagca cctgggaata 3000
   cccatggcgg aaagctcggc cacacaccct gatgtctcga cacctttgag ttgcccatga 3060
   atgggttttc agagcttccc gtgtttactt cgctgcacgc agcttcatcc aaacactctt 3120
   tgctgtattt ataaccacgg tgcatttcct cgggacgcac atcttacggc ttcttggccc 3180
   cttcgcccag ggtgcactgc ttctttcctc tgttccccca cccccacctc tgatgcagtt 3240
   tggaccaagt tttcttcagc cagctaattt agagacaaga acctggatat ttattgggcg 3300
   tcggggaagg ggaacattag cgtttctccc tgaggaatca tcttacacct ataacggcaa 3360
   ctggcccaca gtaacattga tggagcaagt gtcttcttgt ctgggctggg gagtcaggac 3420
   atagccacag ggaagaaaac agaaaggtcc ttccctcttg gaagcccctc ttgaaacacc 3480
   taattggtga catggtacac gtggtttgac actgccatgc taccgacaaa aaccacgtcc 3540
   ctagacttgg gggaccaagg aatctggagg aatatgatga tagtcattta atgaaggtgg 3600
   agggattgaa gtgtcggaaa tgctttgctg ctgtcattgc atttctttac ccagcatttc 3660
   aattgtgctt tgctttgctt ttctcacttg aaattgtggc ctaaaggaaa ttagcatcat 3720
   aagtctcctc tggcatatat ttccagcatc caagtggagc tgctgagatc ttgctcaaga 3780
   gtagcaaaat tggcaggagg tagaaacatt catgtttgcc atcccaagag caaaagtcat 3840
   ggaatgcctg atgcttagtg ttaagaggaa ggaattccac acagcctgtg gctagctttt 3900
   tgatgctacc ctggtttctg tgttgaaata tgaccctagt gtgaaggtcg cggtctcaga 3960
   ccatcttgtt tctggtatgt taagaaacag aaaaacacct cctgtgtcat cactaaagtt 4020
   agggtgcgtt tcagaaccac cctgtaccac agtgaagagg gacaaatggc cttagggagc 4080
   aggacccatg ggggccctct gggaatcttt ctctccctca ttgctacacc tgatcaatta 4140
   ctgagtccca tccctttaac tgcttacatg gccttcaaat ccatcttccc tacctaagct 4200
   gctttgttga aatctgtccc tagatagtct ctcttctgca cagacctctg aggtttttcc 4260
   atactgcaaa gggccttgct gcaggaggaa cagtctgacc aaacagcatt tgcagatgca 4320
   aattcaggat ccgaaattaa ttcttccccc ctctttgctg agctgccaga atgttccaag 4380
   gagatccctc ctttccagtt gtttctttca ttgatagggt ggttcccagc actcggggtc 4440
   agggcttgct tgtagaagtt tctgaaaagt tgcagtcatg tttcatttct ttctccccct 4500
   tgttttcatg atgggaggat atgaaaagca gtgttttgaa atctcctcct tagatcctga 4560
   tactcggaac tgtcgattct cccattttca ttttctcctg cgagctgcag gttgaaactt 4620
   gccttgcttt tagactgttg actgagtttg tagttaagca gataagtgcc aaaggagaag 4680
   tcagggctga ctgattgcaa caatcctggt ctcttcaaat gatttcaggg cttgtttggc 4740
   caggtcaagt ctgttctagg aggattttct ttccatcctt cctctgggaa atgaatgagt 4800
   cagtgaggaa tatggaggga atggagggca gtacaaaagt tctccggcct ggacccttgt 4860
   gattctttga aggctgagac gagagatgag actcatggct ttacagccag ccatttgaaa 4920
   aatgagactt ccccctaacc ttgtgtcaaa ctcagagacg ttgtcatctt ggctttgatg 4980
   tggtggagga gaggggcttc 5000
<210> 50
   <211> 5000
   <212> DNA
   <213> Artificial Sequence
<220>
   <223> Nucleic acid probe
<400> 50
   atctgtcccg cttcccaaaa cctcattttt ctctgcttta gcagtagccc aacactgaaa      60
   acagagtact gcttttttag aggagttctg atttggattc gactggctgt gacgtaaggc      120
   agtcagacct ctccttcgaa atgggccgtg attcatcttt atttttgtag ggctgtgtca      180
   cgttagaacc actacagcaa aagggagaat atagtcttta cctgttgatg gaagaggaag      240
   aatttgtggc cctgtttgcc atctgccaaa gaaccctgga gtgggaaaca gtggggctca 300
   ctggctcagc actgagtgtc tcagtcaaca catgaatgcc agtaatggga atctatttgg 360
   attcacttaa gccatgaggt gggggcagtg tgttaactgg atgtgtcagc tccttcctgg 420
   cttctcctct ctgggaacag agaacagtgc attgcagctt aagtggagta aaatgggtgt 480
   gggtttggag gaagtggatt tggcttggga aagttgatca caacaaagag gctattatag 540
   gtacatctaa tcgagaagag gaacaaggaa ggagtaggtg tgttgaccgg ggctcagagt 600
   gaatagtcca ggataatcca ttcttcagaa cttgttcttg ctaactcagt taagtgtcga 660
   atggcgtggg ttgtctactg tattttagag gacattagaa agactcagag ctagggtcac 720
   actcaggttc tacctcttgg actgacttta tttaacaagt accatttcag cactctctgc 780
   agtcagctgt tttcaaaggc tttaataggc tttattaccg agtgtttcca agggcttacc 840
   tggggaagga aaacattttt actcggaggt ctgataagga tgggctttaa agcgctggca 900
   agcccaggtc cttcagtgat ctgtttctct caatgcgcct gtgtcagata gaaccgtgca 960
   tttcccagta aaaggaggtg atgtgtgtgc tggagtttca gcgggtagag ttaaaatcca 1020
   tcgtaatgga aaggcggctt tccctttggc tgtctgctaa cttggtgact ggcctgacaa 1080
   gtttttcttt tccttcctcc aagctgatgg gtgggcagaa agtgttttgg tgggttcaag 1140
   gctgcgctga aagatggtag accagttgac cgtgtggtca cttggctgcc ccagagatga 1200
   gttttgtggg caagagtgaa gagggtgcct catgccggga agggaggctt ggaccagtgc 1260
   agggtcctta tgggctccaa tgccgtcatg aaggtcacac tttcactttg gagatgattg 1320
   gattttgtag agcaggaaag aacaagtacc ctagagagta tgtttctggg ctcccggctc 1380
   ctcttccggg ggttgaagat accaccctga tttttgggtg ccaacagcat gggatgctct 1440
   ggaaattctc agctctgggc attttgaggg gaccagaagc cacagggctt gcctgaccag 1500
   cccaccctat ctgggcagac tagcttccag gtgaatatct accagggctc agcctcattt 1560
   ctgactttgg ttttgaggca ggcagaggaa catgtatctt tggacaccct ctctccatta 1620
   tcctgcaatg gatttggggc aaagatacca ggagaggtcc aatgcacgaa gccttacatg 1680
   ggaagagtgg agacggggtc ctcttttccc ccagagtctt ttcagatact tctgtgaaca 1740
   tccgtagttg ctatttttgt cttccacact tggcagcttc tggaagcatc aggggaatgc 1800
   ttcccggggt accgggttcc tggcatgtgg tatatatctc agccatccat tagtagcagt 1860
   atccccaggg ggccaaaaaa tgattgtttt ttgaactgtt gaaggatgtc ctctctgttt 1920
   agaaacaagt gagctgcttc tagctgggac accaggggaa acagtgaaca ttcttttatt 1980
   ctattactga gttttccaaa gtgagtgagt ccttaaatag acctaaatag aatccttgga 2040
   attgtctgtg aggactccct tggagccccc agcatgagtc cttccatggg acatgttgga 2100
   atctgtttgt gtgtgtgact caacgggcgt ttcgtttctc tcccaaatag ctaactgggg 2160
   gcctcagaca gctgttgatt atagaaatct acctctttgc gcccaggttt cctgtccatg 2220
   aagccggctc tgcccctgat ccttctgatg catccaccgt gcgtctgctc acctgtcttg 2280
   ctttctgttc attttctctt ctatgttgac gcactggaga cagttcccct ttctccttct 2340
   gcacctgccg cgttactcaa tcccctgtaa tcccattgtc ccgactgcat catttcagag 2400
   aagtcagcca gcgaggtggg aggattgaca aaacaaatac atttagcttt ccttgcccca 2460
   tttctgaccg cgggcacctt tatccccgta aattatctcc ctgggaggga ccgccttcag 2520
   gagaatgaga actcagggca tttgggcaat ggagatcggg ctccctggga agccctcacc 2580
   acacccaggt aaaaggtaat ctgcctccca gcctgagctt ggtccctgca gggttcctgc 2640
   ttggttcaga ccccaggcag ccagtgttga ggggagggtg gagattaatc catagttgtg 2700
   ctgccttcat ttctaacatc ctctgggtat tttctgtccc agtggtaaac tgctctattg 2760
   tctttcacta atagggacac atacggcttt aatgaattaa cttaaggatc tctgacaccc 2820
   cagtgtgtcc aagctctaat ttctggaggt aataaatctg ggaatggatt ccatctggct 2880
   cagatgggag acagaacagg atttccatgt agctgacttg ccgggtccag aacctgcatt 2940
   tgatccagac gcctccattc cttctcgcag aggtagattt gaaagctatt cttgcaggca 3000
   ttgttccaaa gatgtcctcg gtacatcttc tgagactaat gacatgataa gtttctggaa 3060
   ggactatgat ctggatgatg agggtttcat cacagtcctc ttttctcaag ctcatcctcc 3120
   ctggtgggtc catgatccct ggaaattgtt ggtcttctcc ggccttttga aattgctgcc 3180
   ttatgaatgt ccagagttgg caccagcatc cgtacctgtt cccccatctt cccaatgccc 3240
   acgggaccca catccctgag tatcccaaag ccctcacaga ctcaatatgc tgccaactca 3300
   tctcttgttt ttcctcctgt cttctgtgtc ctgtctgctc ttgtctgtat aaatttcagg 3360
   actcctgaaa tagcacggaa gcccccttct aatcctgtgg tctactctca aggatgtatc 3420
   ttgttgtttc cttgcagaag atcagaaaag ggaggacctc atatgttccc tcctaattat 3480
   acccagttac acggtctcca aagtaggata tctcatcagg tttatataaa agcccctgat 3540
   gtatgttcag gaaaggcagg atgctatcat gagattctta tttcctttgc ctttgtggga 3600
   atcctgcggt gccacgtaca gactggctct aagctcctgt tggagttcaa agcggacttg 3660
   tgggaatgaa tgtttaattt cacatctggc attttcttcc tttgaatgca aatgcccttt 3720
   ttgttgtatt ttcatctgcg tggaaaccgc tgggttgata ccttttggaa agttcatgag 3780
   atgtccaaaa acccctggcc aactcgaatg aagtgagtga aatgaaacgc tatgtctttc 3840
   tctttacacc gcaagagatg ttaaagaaac gtgtgtcatt tgctgcactg aatatacgga 3900
   ttcttctgtt ttactgttct cttctccttc tacctgggct aagatgttgg ctctctcttc 3960
   ccagagggtc agtgtttcct ttaatcttct ctcagctcac tctggcttgc atctccgctg 4020
   ctttaccgat ggctctcgaa gttaatctct catctcccag ttgcgtctcc tgcagcctca 4080
   cctcacttga cccctctggg tgcagttatt cttattcttt gcttctctcc tggagacctg 4140
   cttcttgccc ttccccattc atgcatagag accacagggt gtgggctgag tccccagggc 4200
   gatggtgaac aaaatcctgt caaataaatg ggctgtgatg cctcttgaag gcttggggag 4260
   gggcaaagaa cttttcagat cattaatctc taatgtcaac tccctgttct tctgttcctt 4320
   gtagctttgg taattaattg tggatctgtg cctgggcttt cagggtcagt aaatggattt 4380
   accatttctt tgcacattta cagcatcttc cttgggcttg acaataggca cctttggggt 4440
   tgtaagatga agatggcgca atcatttaag gaatatagcc tgggtttccc actgtggggt 4500
   gttctgtttc cagatgtccg gaggcaaatt ctaaagcctt agcaacagca gcaattgtag 4560
   cagaagtagt ggcggttttt taccctattt attgtaattg ctgcgcccta ttcgaggtct 4620
   ccggcgtctt ctttggcgtc gtctttgccc tttcagaagc gtctgcacat ttttccaggt 4680
   gtcatttctc caacttgaac cctcccattg agttctggct ttcctatact gaaagcccct 4740
   tcctctcctg cagtaaggta ggtggaaccg ctgtcccgcc ttgttggtga atgtcgttgc 4800
   tcccaatcat tttctaagag ttttctatgt tgagcccagg taatatctgt gctgagactc 4860
   taacatgaag cctcaattct gatcatggcc ctagcagcat attttctacc tagattcggg 4920
   taactacctc tctcagccag tgggaaaggg tcttgtacat ttgcgaaaca aatccaagaa 4980
   gtgcttttga gaaatgagaa 5000
<210> 51
   <211> 5000
   <212> DNA
   <213> Artificial Sequence
<220>
   <223> Nucleic acid probe
<400> 51
   atgtttgagc catttggact tgattcaccg ggcatcatgg agccagtctc tatcttgagt        60
   aggacagcaa tctgttcaaa cagcgtttta aaaagactta aagtgctttg taatctgatg       120
   gcaattcaga gctggcactg actttggggc tgatgccaga gatgggatta gagttgaaat       180
   catgaaaagg acaatttctc gaaaaagaaa caccgaagta ataaatgata tgtcaaaaga       240
   cagcggggaa agcaggagtg atgtgtcagg gagccaggga aagggacagt cgactgtgac       300
   acagctctta tctgttgagg atgatacata ctggtccatg tgcttttcac agggtgagca       360
   aaaaggattt tgatctcaac taagcgcaat gaaaagctac atgatgctgg ttctctatgg       420
   ccacatacct ttggtggctc tgtggtcatc ttgatgctga cctgcaagat tgagataggg       480
   aagtctgggt gggggatgga gtggcagttt gccaggaaca cccagtgtcc tgcaaggaga       540
   tgccagggtc agtgggaatc atttcctaag ccctgcctgt cctcatgctt tccacctggt 600
   gtggggtcca caccagggga caggatgaac acgagtgggg atcgtggggt tgaatcctcc 660
   agcacctgcc agaagcacag gtatagcatc aggcagaagg ccgagccacc gaccgacttc 720
   atattcagca caggcggctc gatgaagcgg gagccaaggt tggtgatgat gaaggaggtc 780
   acgcagaagg ccacgcggtc acacagacgc ctggtacctg gcagcatcgc cttctccggg 840
   gcagcattgg tataccacag gtgccagtca cgagggtact gctcaaagga gttcatgagt 900
   cagctactac atggattgtc catttggccc tcccgatcca agacctggtg ggagaagggg 960
   aggaagaaag ggattgactt gggaatactt caaaagaaga gccctctgct ggttgcctgg 1020
   taataatgtc tccaacctaa tgactggttg gtagcctcac cttcacagaa gcacaaacta 1080
   tttactcatt cattcactca actgatgctg tgggcctgac cctctgggtc tggagtcctt 1140
   gtcacacttg ccctcatccc caaccttgac catcccatcc tgggatgtgc tctctgccca 1200
   ctccaagatt ttggcacaca tatgaaaact gaatagtagt ttgtggcgtt cgaaaagggt 1260
   acggcaggtg tacctgggag aaagggacag ggaagaggga tggggcatgt tgggggcacc 1320
   ctcagacctg tagacagcgt aggtgtggta gtcattcagg tagtcaatgc ggtcctccag 1380
   cttattgctg cggtggcttt ctgctcagtc acctctgtgg ctgtgatctt ggaggtatgc 1440
   agcgtgttca ccagctgcac atcatccagc agggagccgg tggcctcatt cagcagccta 1500
   ggctgtcact ccttcgacct tcaccttcct catctctttc caccttgaaa gacccctcat 1560
   cctcagctgc ccaacacaag cttatccctt ttctcaatcc atggtggtct tggctgaggt 1620
   ctctgggctg tagtgggggt tggagagctt ggtggtgatg tagaaacgga aattggtatt 1680
   atattccacc tccttatcgc cttggggact tgtcctgacc gattcgggct acagatttgt 1740
   tgagcatggg gttcagtgtg gggtccagat attcctgcac gttctgaagt agcaccgggt 1800
   tctaggattc gcaggtaatc gctcatctgc aggtcgatga tcttcaggcc ctagagagca 1860
   agggggaagg tgaaggaaca ctacgggaga ggtggggcct ccggactttg gtaggattgc 1920
   acaggaagtt atcgatggcg aaagaagggg agcaaggaac ctgaagctcc cagatctggg 1980
   gagagaaaag ccccatgctt ggtctcggag cctacttgtc atgcctgagt cccatctcac 2040
   cttcccgatc cagatttggt tgacaatctc atcccggtag ttggtcagga agggtcccat 2100
   aggttcaaag aggatctttc cctcccctaa acctgtgtgt cctgacaact ctagcctggt 2160
   tctaattact tttgagttct ctatctctaa tggcaatgtt gtgcttccca gccctgccca 2220
   aacactgaag cagatcccta ataatgcaaa aagcctttcc ttccattgct ctcaccaaag 2280
   gtgtgcaggg ggaggggaag ctaaaaggga tgggtctctc ggaagtggcg agcagagtga 2340
   aaggacacca ggtagtacag ctgatctcac ctggactgtc tcctcccatc tggccttctc 2400
   tctcttcaga cttcttgcga agctcctcct tctgtgccag cttctcatca tactgtttct 2460
   ttagcatctc cagtttctca gctacctggg agagattcga ggtggcaagt gaaggccaga 2520
   cagcacataa atgactgcca ctccatgcca gaggccatac ctaaggtgga accttgaaag 2580
   aggaagaaaa agaaagaaat tgggcagagc tgtgtatact agagaaaccc ctctcaagtc 2640
   attgccacct gtggactctg tggaggttgg aatgaatata tatatattca ctgaaatctg 2700
   gtgcctggtt acagactgta tcaccgtcct tttcatcatg aggaatatgg ggtgggggat 2760
   tacacatagc atttacacga tacatgttcc ctttcccgct gtggaaatag ttccactact 2820
   agaaagtaga agtagctctg ccttaactga gtaattctgg taagtttcat cctttcggag 2880
   cctcgccaag gtggctgcag acataaatca aaatccaaaa ggaacgaaag tgcacccggt 2940
   taaggggaag tcttcctgcc attcctgagc ctggtcccag gccctgccct agtttcccgt 3000
   agctatcaaa aattcaaaag gcctaagggc atgaggatat gaaagatctt cctcctaacc 3060
   ctgttcccca ctcagccagg ttccttcttt agaggcagcc aaaccaagat tttctctgtg 3120
   tatcctcatt acctcaaatg ctattgggtt tcttctacgg tctttggcaa tggtaataac 3180
   cagttttttg gcaacgtaat gagacttcat tctgaactta atacagcctc tgaggtcctg 3240
   aggaacaaca tccttttcca gggagaaaca gcccaggtta agaaaactga tctacccaga 3300
   ccctcagctg agatggagca gaacaggaag ttggggagga tgacttgggg actttaactt 3360
   cagaaggttg aagtctagat ttttaactca caaaggcaag taatttggaa tgcaaaatgg 3420
   gaaaaaatgg gaatgttcag acttggggat acagtcgcct taaagtatat taagattctg 3480
   ttctgaccca tttcagagcc atggctaaag ctatttactc aaaatttgtt ctgatctgca 3540
   atgtagttgg gctctgagcc tcttttcaga cctgagcctc agtaccatgc ataagctttc 3600
   aggagaaatg tttgttccct gtctgctttc tcttttcact taggggttct caagtgtggg 3660
   tgtggaccct ccaccaaaat acctaacact gggtgcacta ccactcctaa ttatttagcc 3720
   caggatatga ttcatctagg tgaaaaccct ttccaaacag attgccacac ctgtgattct 3780
   tcgctcttgg gaaaagacag tcgcctgggt tgcccagact cctcacggta actaacccac 3840
   ttggcctctg ggagagcccg tccccacaca acatatgcac agacaccctc acgttgttct 3900
   gtcaatcttt ggaagggctc ctgggtcgca gtgcctttga gcctttataa ccctctgcaa 3960
   ttgacttagc tttctgagcc agaggcagcc cagtctaaac tcctccttgt ccagcagttt 4020
   cacctccact actcacgcat agccacattt ttcctgggct cagttcctaa ggccaaatta 4080
   tctgacctgg cccctagttc ctcagcgagc gcggcttgct tctcccgaag ctgagccaag 4140
   gcagcgttca ttcggattcg cttgggctcc accacccgat atagccgccc atacagctgt 4200
   aagaatggaa cacagagtcc cggttcccat tttctgattc ttgttcccct ctgggggcct 4260
   gagggcattc ctgcccttgg atgcagcaat acattcttct gacagaacct agctgaggtg 4320
   ccacctcttc ttcccacttc cccagagtct ttgaggacaa agaagactgg cccattccct 4380
   tccccggggc ctcgcgttcc gcgggcagtg cctcttctag atctttctgg gcattgtcag 4440
   ccagtgcctg acacttgatt tcctcaactg caatcttttc actgttggct gttacggcct 4500
   ccgcttctgc tgcacaatga tgaccaggta ctcctcacac tgcttctgga actcagccac 4560
   cttcttcttg gcatcctcca gctccaacga catcacttgc atcttctggg aatactgagc 4620
   tactgaccag tgcatagtga caaagatctg ggccaccttc ctgtggatct gaggccagtg 4680
   acacaggatg aaaaggcaga ctcacattct cctgagttcc caggtctact cctatgaggc 4740
   acttctcagc cacctcgagc agggcctctt ggggccactc tgagaaccag ttgatggttg 4800
   tgtaaattcc gtgcacagac acagcctcca ggtactggct tcccaacacg catccactca 4860
   ctttctaaga acctaaaatg ccccatcccc ttgctcctgt aaaaggatgg acgactccca 4920
   tgtccaccaa aggaacttcc tgaaatcagg cacagcctaa ggaaaggaga tgggtgaggc 4980
   gggcagagga gcctgctgtt 5000
<210> 52
   <211> 5000
   <212> DNA
   <213> Artificial Sequence
<220>
   <223> Nucleic acid probe
<400> 52
   gtcctctagg aaggactcat cagctatttg ggtgtccaca aaaatgaagg acgtggtctt      60
   gagctccacc ccagcctggc gatacagacg cttgatatct cccagccgta ccatctcgga     120
   actcctgctt ccgataatgt ttggtgacct cgatctggaa ggtggtgtag tcgcagatgg     180
   atgaagccag gcgggccaga tgaagcagag gaaccaagag gtatgagaag aagttggtgc     240
   ccaaggccca ggaggcaatt caacttttaa cctccacctt ctctgttaaa cacacactgc     300
   acttgatttg ggagacctgg gaagggaccc ctatttcctt agacataccc catcactgag     360
   atgatgggga aggggatagg cgtggcaggt gctcactgtg tccttcagga aatcccctga     420
   gccatgggaa gaggggatca gagtcacaaa tgcctggttt gcctcccccc acagggctcc     480
   acacttcaga ccccatgccc ggtcaaagaa ggagccgagc ttgtcgctta tgatgcccat     540
   gaaggcttct gtgtctgccg catcaaccag ccggtcagag aagactctgt gggcagggaa     600
   tccagagccg tgtgatgctg gacttggtat catggaagtc cttgttggct ctaagcatgc     660
   cctggaacac ctgggaagag gactgactgt tgggaccagg gcacttcagc atgggggtga     720
   cttttggtgc gcatgctagt gagtgttaag gcttagtttg agaagagaaa gcctccgcgg     780
   aggctgggcc aagcagaagg cttcacctct tcctcaaagt cctgaagctt ctgattgatc     840
   atggtgccga atatgcggat gatctgggac ttctagaaac agaccctggc tcactctcca     900
   aataccttcc ctgttcccct gggatccctt ggccccacat acttctccag ttctccttcc      960
   cactccatag gatcaataaa atgtcataga caggaaaaaa accttcctta gtctacttgg     1020
   gcttcactgt gttctctgcc cattgggact acaaggcttc cttgtccaca gccaggaagg     1080
   tacgcttcca acccgtagct ctctcaaaca cacatgaata tagcactagg cgagtttttg     1140
   gccctgagtg ccagatatac acacaaacaa ccccaggagc caggccgcga tggccgctga     1200
   gcctgaagaa cagactctag cgaggcttac tcgaatgtac ttgatggtct gcttcgtacg     1260
   gtcataccag aagccatagt caatccagag gcggatcagc gtggtctggt gaaaaggtac     1320
   cgaatgaatg tgtgggtccc ctgagagaaa tacgggctta gagaggaggg tcaggcaggc     1380
   acttggatcc caagggagcc gttggccacc aagctgctca ccaggtagtt gtagcgaaca     1440
   gtgtcgacgg tgggcaccat gatcttatag aagggggcgc tagggaagag caggggccgg     1500
   ctcttaggga gcttgtcctc aaatgatgtc caactccgta ttttggggtc cacaaaatac     1560
   tcatataccg tgtcctaagg agggcatgta ggtcaggtta cacacacaga ccagatcatg     1620
   ctgaacacaa atgtcatctc taccatggtg acatagttct cgccgtcagc tgggttcacc     1680
   tggatggaga acgagaagag tatctacaac ctgctgaaag acagacaaac ctgaaatttc     1740
   ttatagctca gcagttgccc tgagcttcct cactgctaac tcatgaagat gcagccatca     1800
   agcacatggg gccatcccac ctctctcagg cctcttggag accacagaca gcaaactcgg     1860
   catccttcaa accatccctg gcaaagggat agtctgcttt ctgaaatgtt taaaaagtcc     1920
   tctcatcaga atagcctggg agcttcttct aggagaaatg gtgtcccagg gcctcatcta     1980
   cgccaaaact tatattttga aagtgaggag cggaatgagc tattttcagc tcccaaagca     2040
   tgggtgggaa attatagtga gatcccattg tgtacatatt caattcagaa cccctagtca     2100
   agtctctgct ccactcaagc ctggagttca acctgtctca gacacgccgt ttcccagaag     2160
   ttgttctggt gaggctgtga gcaccccctg cccctgcccg tccttgcagt tgtccttctt     2220
   aaaggccagc atcttgttga tgagcttttc gaacatgcgt tgaaggggct ccacctcagc     2280
   ctggagttga ataagaaggg cctcagttcc cttcatacct ttggcctctt ctccagccat     2340
   gactgaacat agggcttcca gcccaggtca gcgtagtcag tgtagaccat cccgcagcgg     2400
   cattgccagg tcctccactt caaacaggag agacacctaa ggatatacgt ttgtcagaac     2460
   ttcatctttc tctgcatccc ccaccttcag gaataataaa gttatcgtcc atgacggagt     2520
   tcatgttctc gatccacagt gtgtccacgg ggccatcgaa caggatccac ttctcgtcgg     2580
   gtttctcatc tgagtagatg cctgccccct taggagactc aggcgtctgc tctccacacc     2640
   ccacgatcct ctggatacct gcacatgccg tccgcatgac actggacaag atgccatctg     2700
   atggacaaaa acccctcttt atttccagac ccctcttagg tacgtgcatt tgtcccttct     2760
   ctcatcgctc caggccacca tggtcccagg tacagctctc ctggccccca ccctccattg 2820
   tcttcttact actcaagtct ctgcccactg actccactgt cccagccccg tactctaaca 2880
   atgttgaagt tagggtctcc gcgggagttc ttggtttcat acaactggaa aaccttggtg 2940
   agggtgaacg gcgtgctttg caggcccatg tctcgaatct cctgctcaac ggtctcccgc 3000
   agggaaaaca cagcaagcag aagaaaaagg atgaggcctt taaagggctt tcaaaatctc 3060
   cccggtgcca gagagaatac gttcaaactg ttaaaaatgg acaggggtgc atcaactgaa 3120
   gtgagcttgg cgatgttcat atctctcatt gagagcagca gaacctgaat gggaaaagac 3180
   agagactcag gcttgaggag ctccaaaggg cttcggggca cgtgccaaga acaatggata 3240
   caccagttag ccattagcaa agcagcgggc aacacgggga tgggacgagg tgggaaggtg 3300
   aagtcaggtc caaaggctgt gtccttgctc tacctcttca tcagtcagat ccggctgtag 3360
   gcggcgcttc ttgccagcat agcgcagaag ggaggtgagg gacagtcaga gacaagaagc 3420
   caggtcccag gacatgaagc agctaggagg cactctatgg attctcctga ggaaattgtg 3480
   tgaatgacgc cattgtcagt ccctaaattt ttctccccac acatcctact gacaatagtc 3540
   taagttcctt agaggataag ctgattgtga ggcccttggg gagctcctct gtccagggaa 3600
   gaaaaggggt tattggagta ccttgcagtt gccaaagccc tctccaaaga gaatgatttc 3660
   tgcaatgagg gtggagtcag gcaccaccat ggcaattggg ccatccctaa ctttctctcc 3720
   aactccaaga ccacatcctt tctcatctca gccccagccc ttcccagtcc actttggtct 3780
   tctctcttat ctctggtcct ctctccaggt tctgtgtgta tagctctcat cagtcctaat 3840
   agaaatcaca gctgttcctc tttgttcagt tcctaaaccc tttcctatcc ctgtgctgtc 3900
   cacacataag tggttattaa tccctttggg gaaaacagta agattgcttc cccttctccc 3960
   ggaaagttgt gtttatcctt ctctcccctt tgctatcttt cccaggagtc ccatccactc 4020
   cctgccacct accaggattc atggtaatga agatcccaca ggaccacacc agatttattt 4080
   caaagccatc aaaatggaaa gtgcaaggca gagtcactgg gagcgagagg tgccccaaga 4140
   ggctagaaag cgtgattaac gcacccttcc ttcaaatgtt tcaataggcg aggcggcctc 4200
   ttccatcaga tggggccaat ctggggtgat gctagaaaag gtgtgggttg gaaagagact 4260
   ggggtaggga acttgtgtgc actgtctact ctgctccagt actgacctgg gccagacctg 4320
   agtacattcg gcccatggac ttgtagtcca ggccctcaga gcagttgacc acaatgacat     4380
   atatgcccag ggccttgccc ctttggggga gccccctcgg tgcaggtgca atgccgtggt     4440
   cagtgtcatg taacacctga gaattgagga cagaactaga gtcagactcc tggaagcctc 4500
   cagacctgtc cgtcaggggg gtgatgacga gccggcccga gttacccaag tactcataat 4560
   tatactgaaa ttgcgtgttg gtctggcgga tgacacagtc agttggctga gccagtcaaa 4620
   ggaattgaca tccatgaggc cactcttgta aagcttctcc aacacatccc gggcatgaat 4680
   ttctatcgtc accagagcca cagcaggcac ttggtgacat cagccgtcca ctggatctga 4740
   ctggcagtga tcaccacctg gggatgtgca gaagccacgt cccaaagccc tgtccttggc 4800
   caagtcccag ctaattcaag aaccatgtgc ctctcccaac tgcacccaat ctgctctgaa 4860
   ggatgggaca tgggcctcag gtcaggagct aagggtccag gttgacccca gctcacctgg 4920
   ccagcccact ccttcaccca tttgtccctc ttgttgagga acttcctgag ggccaggtgg 4980
   cagttccgga gaaggtcccg 5000
<210> 53
   <211> 5000
   <212> DNA
   <213> Artificial Sequence
<220>
   <223> Nucleic acid probe
<400> 53
   cacagggaag gcacttatgg agcagcactt cagggctttg tacccagaga aaagcgggaa 60
   tggccaacag ctggcttgcg ttcctgagag aaatggaggc ttgggagggc agggtcccct 120
   ccaaagcttt tcatacccca acaccctccc ttcgccaccc agcatctcac tgtggccact 180
   tctactgacc ttctggattc gggactggcc cagaatctcc agcaggtcat cattggacaa 240
   gaagtagaag cgggggaaaa tatgtcgctt ggtctctaaa tacatatcca gagatttctg 300
   cgatgaatga catttgattc atttccactg ccctgggcgg atctggatgc cccaactctg 360
   tgttctttgc cagctcttga acagtggggc acagactgcc gctttctgaa gaaaggaggg 420
   acactagtga gtgtgaactc agaggtgacc ctcatgcctt caagcacagg gtctacaggc 480
   tgttggatcc tttgagtcaa tctgcacccc caccagaatt cttctgcaca ggatgcccat 540
   tctaggtgcc cgctgtcaaa cctgactccc tgattgtcta ccccagtctc ccccagtcga 600
   atgggaaatg ttaggagagc tgggggcatc agtcttttta ataaatgaag cacgcaacca 660
   tgaccaagca gagtgcctgc cgaggctgtg tcagggcagg gctccggaga gcattgttgt 720
   ccttgttcat cctgtccatg atggctttcc agttgctgtt gacctggtca aataaggtcg 780
   attcattggg cagctgcttg cggagggaat ctaaggtgag aggtaataac taagaatcag 840
   acagctgggt cctagatgaa gagtgacttt aaaggtagga gaatgaggct gataacaggg 900
   aaagcgtgat gccttcatgg tagacagagc tacctggtta tcttccagtg cctggaatac 960
   ttcttctgta cctctgagac acaaggggtc aaaatcatag gcccttatcc ttgtagggta 1020
   ctatgtcgag ctgagtcaca tcccaggtct tggcaatgtt ttgtaaagcc tgtggagaga 1080
   aattggggga cacagcagtc atccatccca agctccacaa tctgctccaa ggtgaagctt 1140
   tcagattcct gatcaaactc ccgctggatc tcatcccgga cctggtccca gtgcctgcag 1200
   gattctgcat agttaccaat tcatgattca actcattttg ctaaacaggc tcgaagatgc 1260
   aatcttaggc accaggggaa tacgaagatt gaggtgaaac tgaactgctc tatttttgag 1320
   cgagtggttt caataatttc ccagtttcgg tcctgagcaa aagttggggg catgagaaga 1380
   cccgacgacc tccctgggaa gccgtggtct ccatggtttc cgtctgcagg atcaggaacc 1440
   ggccagtctt ccactcattc cagttctcct cccagtctcg tgcgatctcc cagatttgct 1500
   acggacgccc aggcccttcg ctgctctcgc tctgtgaagc ttcccctcta gataatacag 1560
   cggcctcctc agtccacata tttttctcca ctctgttgcc cgctcacgtc tggcaaggac 1620
   agccatggct ctcctatctc tttgtgggca aatctcacct ctctcctcac gtgagagagg 1680
   gaagggaagg agagtaagag agaggtggat caaggagtac ctttgaattc gaaatcttcc 1740
   agaagtgtat gtgctttctt cttgaagtca tctgccgagt ggatcaggcc tgtcttgaat 1800
   acagccaagg gcctcccccg caatctgctg ctgatacggg tcccaggtaa accaaacacc 1860
   aaaggaactc acactgtcct cgactggcac ctcgtacttt gtgactgtct cctccttctg 1920
   cacgttatta gcaacttccg tgtagctgca agagccccag atcccagcta ggatgttgtt 1980
   ttctttgcag acataccctc tccaatctgc cccagccatg cagctgtgac tgactgtcat 2040
   tttcccagtc actggctata agatagcagt caaacttctt attctggcat tcaaagctct 2100
   ttctatttaa ctcccatctg ccttcttaga atcaaagggc tcacttcttt catcttccag 2160
   atctctggaa cttcttcttt ctcaccaggc cctgccatcc tcccctaccc acttcccaag 2220
   actctaggcc tgggctcctc ctgcagtgct gtgcctccta cagacagact ttcaatccac 2280
   cctcaccact caccgggcaa tgaggcgctg gtagcgatga atgaaggagt ccttgttgat 2340
   ctcccagatc tcccggtaca tgtcccaggt cttgaggtag ttctgcagca ggcttgcgtt 2400
   aaaggaccaa gaaagatgct tctcactgac ttccacccca acccaacttc tgcctgagcc 2460
   ctgcccactg ctccagacct tccactctaa aaccagcttc aaccctgtgc cttgtgccct 2520
   ccccgttgcc cactcaccca caactgtttg gatgggttca cgatgtaact tgcgcttggt 2580
   gagaatgtca gggaggtggc gaaggtcaca cacttcagca gcaggtctga aggccatagg 2640
   ataaagtcaa ggccaaggac caagtttagg aattaaggga aactgacaaa taagatgcaa 2700
   atgcggtcca gccgaatcat gtacagcatc cactgctgct gaatctgatg ggatggaggg 2760
   aggaaaaaga gaacttttca gggaagacag ggcacatcct actagagcac gatgtttgag 2820
   aagtacacac tggtaaaaaa gaaaaaaggg tcggggagca agagaatggc ctgatggaac 2880
   tgggggactg tggtttttcc catgatggtc accacatcct ggtgcaggtt catcaatttc 2940
   tgctgtacag ctgcccgatg ctctctttgg tcctcttcaa attccaggtc cctgtatacc 3000
   ggccccgtca tgggggagtt tcagctcggg aagagaccat ctggacctca ttctaccctc 3060
   ccacttgttc tccagctaga ctggatgaca cacaaaaaag ggcatgtata cggcactccg 3120
   tgatgaagaa ggcactggcc cccttcaagg cccagtgcag tttcttgagt cccgggtgga 3180
   tcttcttatc caggagccga cattcctaga gaactggccc agagctacaa tatatatggt 3240
   acaagtgacc agtggggtgg gaggggacct ggttggcttt actctccacc ctgcagctag 3300
   atgcgcaggt cctcggctcg ctcagctacg ttcaccacgt aatggggcgt ctcaaacagc 3360
   agccgctccc agtagtcaat ttccgcaaag agaatcagaa tttttaatat gctaaagcaa 3420
   agttcagaaa ttcagacacc tacctccaag gcaagtgtgg ctcaagtgtg atgataaatg 3480
   gcaactgacg cttggcctta attatcatgt ccttcaaact ggatgtcttg gatttgtcac 3540
   tgttgataaa catctgagca agcctacttg ggcatgtatg ctggccaaca ttgcaccaaa 3600
   gtttaatatg tcagtcaaca ggatgaattc agacaatacc ttcctgatgc tgagctgaaa 3660
   atcctgggag aaactttgaa tgatgttcag tctttaactg gagagagaag ctctgggaac 3720
   aaaaaacaga cctcagtatc tcgtctcctc ctctcaggaa attctactct gctccacaca 3780
   gggtgataat acagctcctt caggactaag agagccaggt aattccgagg aacatcagaa 3840
   catttggaca gtctgcttat ttcaagatct taactggagc ttgaacacaa agagcaaatc 3900
   taactgactt ggatttcttt actggtgatg agcccagact ttcaaaatcc ttcactgtga 3960
   tcgtgttctt tcccttccat tatttactgg attgaggcag aatgtaaagt atcctataac 4020
   atctctctgg aaaacaatcc ccaaaggaca gaaacaggca cctcttctat ctggaagacg 4080
   tgtttcgcca ttatttggat ccttgataag ctctactatc aagattatgc agaaggtatt 4140
   ccagacaaga gatgataata gcagacagag aagttgtctc ctggtttatc ctaactagag 4200
   aaaggacagc tggtgaactg tgtgccctca tatccataaa caccaagggc atctgtgcgg 4260
   ctcccctgac acaataactt tgggggtacc cagctcgatg tcttgcaggt tcaaccccat 4320
   tatgcgcttg caaatggcgt aactaggcta actctggcct agtttctcag tcttcagaat 4380
   cttatcgttg atttatgagg gtggctgtgc ttggccataa ctcacaaaac caggaagctc 4440
   agcaaagcca atctccaaca actctcacta ctcgaagcct tttctcaaac ctctcgacct 4500
   ttcctcatcc ctgcagctcc caacatccct gtcctcctca ctgatgcctc tgcctcaccc 4560
   ctctcacctg acactccgct ctgcttctcc atcctggttc ccttttgctg cccaggacaa 4620
   acactcacag tctcatctgt ccctgggact ccgtctccac acagcacttc tctggcaaag 4680
   cccctcctac atttggcccc ttttatgagt ggaatccaac actagtagcc acagcctcaa 4740
   agcatcttat gtggacagga agccccactc caagagtaga gctgttgact tagaaagcag 4800
   tatagtatga cagtttcata acaatgaagc tgtagaagaa ggcatggtct gacagcccca 4860
   ccaacagtca ggaaagggac agggtttggg tgacttagct gtatagggaa aaggggccag 4920
   gtggctcctg tacttgtcaa agttgacatc cagcatgccc gccttctcct ggctgattcg 4980
   cagcaatggg gtatccaacc 5000
<210> 54
   <211> 5000
   <212> DNA
   <213> Artificial Sequence
<220>
   <223> Nucleic acid probe
<400> 54
   tccactcttg gaaggttttt cgaaccagct catcaatggc ctggaccatc tgctgatagg 60
   tgtgcacact ctcctttcca gtcccaatac ggggcaggaa gcctgccacc ttcctccagg 120
   ggccaccatc tccctgagac ctacacatgc gcgcatgcgc acatacccca acaatatgca 180
   taaggcttgc tatggagatg tgataactta gaaggaaggt agagtcaaaa aggaaggtga 240
   taggaagaga ccaaggcgca atgagaaaag gagtcaaaat gaggaaagag aagcacagag 300
   cagctatctg caggctgcct aactctctac cagcaccagc tgcatgatgg caggtcacta 360
   cctcaacagc tttcaacctt tattcccaaa gggtgtgtgt ccatccccca tccctcctgg 420
   cggtctcact gacgacagaa tatggagaag gccaggcact tacggtcatg actctgtcga 480
   tgcgacgccg gaggatgtgc acgtagggct ccaggtctgg ccatttcttg ttccgttcac 540
   ggttcaccag ggccagctcg ctattgaaca gcatgtagag atccaccgcc ttcttgtcat 600
   gtcccgcacc aactcgaagg ctgaggtgat caggttctgg gtcatcacct ccaggtcctt 660
   gattccggca cggaacctgc accggtggag aggcatgtgg atctgccttc cctgcccacg 720
   cctggcatca gcctgtggtt ccctcactta ttgtagtctt catgccaaca ggtgttcttg 780
   acatccagga tacccccgcg gatttttatg aaagatgtcc tcaatctcca gcaagttccg 840
   tgttatctgt ggcccctggg caccaaagaa gcaaggaagg ggaccctgct tgccatcttc 900
   aagtgatact gacagtcaca tacctaaaaa agaagacagc atgagggttc ctgctttcct 960
   tccaaactcc aaagtcctaa gctatttggt ttagcttcaa agaccccgag aaacaaccct 1020
   cctgaatccc aaaggacaag actcaaggtg agaagaacca gacaggagac agagcatcta 1080
   agttctagga gcagattcat gccagggcaa tgagatacag tatctgtatc cagacagaaa 1140
   atttggccag atgtagaaac tcctgggtct cggaaaatac aaccaagagg ctaaacaaag 1200
   tttccaagcg tgacaacaga gaatgcagcc ttgcaggtcc tccttgctgg aagagacata 1260
   tccctcaaag atccggtcca gggagatggc gtggcagcat ctgctcctca agagccggtg 1320
   ctccttccta aatcccctct cccccactgc aatctccatc ttgaggaatg ggaagaagag 1380
   cttttccttc cacacagctc accaactcct cttctgaagc ccctcttcct gcctaagcca 1440
   cctccaccta gaaacagaaa gtccagccca ggctccaatg ctgagcaacc ctaaagcaga 1500
   agatcctctg ccgaatgcat tccccctgct tcagaaaacc cggccaccct cataaacccc 1560
   tccatcccac cctcagcata tgcacacctt tcggaagagc gctagagatg tccttgggct 1620
   tcatgaaagc caactcctgg taaggttcct tcaggattga caaaaaggtc aggtttgact 1680
   gtgcttgacg agagccatcc aaccatcttc aaccccattc tgacttgcct ctaagaggaa 1740
   tccctcccat cagccctaca cagtagcgat catttcaggt ctctgtgagc cttcggacca 1800
   aggactccgc actaagtgga tactctgttg ctgtcggatc aactggccgc atatcactct 1860
   cattcgtccc catagttgtc acccagttcc ctttcactgc cccttaatgc ccagctgaaa 1920
   ttttcaccta ggaagacagg tcttctatct gtattctaca ggccactctt acctcctgtg 1980
   tgacctagca ctaacataat caaagaaaaa aggtatttat cttgtaattc ctggactgaa 2040
   ccacatgagc tcatctttgg ctcccacgct gtctagtaat cctgttttgg gtaatcttat 2100
   cttgatgtat atttattggt taacttgatg tctctctcct tagggaccct ctattctact 2160
   attttatact ctcctgcaag tacaaggccg aaccctgggt ggctccctct ccttacttag 2220
   ctccctgtat catcccaacc attgcccact tcttgctgag cctgaatcct ttgattcgct 2280
   cacaaacctg gatctgctgt caagtaggac gacttggcaa ggtgcaggat ggattcaacg 2340
   tgcttcactc ccttcttcac cagctgctta ctgatgccag acaggtccat gcatcggttg 2400
   ctgtctccac agtctcctgg gcactgagca tctcctttat ctgccgggtc cagtggatca 2460
   tggaggctgc aggggagagt acaaaatctc cttcgtttct gccaggtgat gctagcagtg 2520
   ccagccactc actctctagc cgttgcacca gctctttgtc ctttatcacc atctcaggct 2580
   tcatgttcat ggcctctgca ttgtaccgag tgtctaagga aaggaaagtt tgtggaagtg 2640
   gacagagggg atgagttctt gctggccccg taggaatcac ggttgatgat cggtagggga 2700
   agaaattgcc ataaaataag gtcaaagatc tgtatcgcgc cttcaacact ctctaccacg 2760
   agacatgtga gtcctcccag tgtccccaca gcacctatat ctcctccaca caggtgctca 2820
   gacgaaggac ccaccacttc actagttgta cagttggggg acactaagga gaaactagtc 2880
   actccggtct tcccacttac gagaagcaaa atgatttcta atgctctcag gccagcctgt 2940
   gtttgcaaag atctgagggg caaagactcc accgagcagc cgaagcaggg ccgggatata 3000
   ctgatccttc tctaaatgga agtgccatga agtgcttcct gttcttttca cggatatgat 3060
   tccatatcca gagatgtggg gaggaaatgc agagagtatg gaggaagcac ttcagccaat 3120
   ggaaaaagca gaaaggagaa ggactggaag gaagagacaa ttgtctaggg ttttccattt 3180
   tggttgcata tatttctccc ttactaagat tttattacac cctctcaagt tcactccatg 3240
   taaccagggg agcaccattc tttgactcag tgcatatgtt tttcattggt ctcccatttt 3300
   acaagactta aggatgatga aggaactgag agccctcggc tctctgtagg accacctttg 3360
   atctgggaat gtagggtacg cacctgtaca ggcatgccca cctgggcaaa gtgttccaga 3420
   atggcatcat gctcctgtgt ccacactgca tccgccagtc ctgtcagcgc agctcgggaa 3480
   aggaagaggg gcttctacag aatcacaagt gaggacatgg gagatgagag aagccagaca 3540
   ggttcagaat ggttaggaga aactgaaaga tagctttcca gctttggaag aaacagaaaa 3600
   gtcaaggtca tcactctatt cttctcaccc aggtaactat ggtcatgact aggctttgat 3660
   gcaacttgga aataaagaag cagaggtcat ttagtgccat tatccaatta ggacaatatc 3720
   ttcccagctc attcaacttg ttcaggtgtt cacccgcatg atgaataagc tcatcacagt 3780
   ttgacccaag gtggggagac tgcgccttac ttaaagccct cctctgtcct ggcgaaggca 3840
   cagatgactt tgtcttttac tcagtgaact ggatttgtgg tgagtaggct aacagcagag 3900
   gacaagagag aattcctcat gtgccatcca caactgatcc tttgattcac cagttttggg 3960
   ggaggaatat ctgccacagg gaagacccaa gcctaccaca gttggggctc ataggaatca 4020
   gcccttctcc aaaccactgg cctgctgagc attcagttct gccacaccca ctctgactcc 4080
   cataaaaccc agcggctctg acctccccag aggattctcc atactgaacc tcactgtctt 4140
   ccctcccatc ttcccctact cctcctagca gcgagcaatc ccattttaac tgctttcacc 4200
   ggaacctaat gcaagttctt atggcccagg gacttttctc ccatgcccac gcttaacctc 4260
   tcaaccttta attttagaag gggattctat ttcacaagct cagggagatc ccagcaatag 4320
   caacaactac caaaaacctt ccaaccttac tggctccggc tcacccacag gaaaggaatc 4380
   cctcaggtca aagccacaag acaggctgag tctccatggt gatgggctta atgggagaaa 4440
   aacattattt gttttgccac ccagggtgaa aatgggaaaa aggaagagat caaaccctct 4500
   gtcacttagc ccgtttcctg cccagaagca acagcgcctg ctccccagcc ctaaaatgcc 4560
   tccaagcccc tctgactttc cccaccctcc attgctaagc agcttgcctg ggagcttcct 4620
   cggccactca atcgctgctt cttctcagct ttgctggaca tcgtgcaggc aaaacctaca 4680
   ggtatacagg agtggagagt aaccacaagg gctctgtact ccacaggggc ctgggaggcc 4740
   ttgctgtttt tccctaggat atcctactgt cttcggggca ctgggctaaa aatcaaagtc 4800
   agtgagtgag agaggatcaa ggaaggggtg cacatccccc atcctttgtc actgttcaat 4860
   cattcaagca aaccaagatg attttcctat gtacctcttt caacatctgt aaacagagaa 4920
   aaacagtatt tgccacctgg gtctatcgct ttgaagtcct gaggggcagg aacactgttt 4980
   tgtgtgtact gaggtctctt 5000
<210> 55
   <211> 5000
   <212> DNA
   <213> Artificial Sequence
<220>
   <223> Nucleic acid probe
<400> 55
   tctgtgctaa tggttaagat cagtcaggat catttatttg actcaaaatg ccctgagaac 60
   agattggaag aaagaaggac aaaagtcatc caatcataaa gcactctctt ttcattaggc 120
   tgcagcctaa cggcacctgt ggctctcctt cctcatcccc acaggctgcc acaggcattg 180
   atctcaggtt atgaatttca acagcaagga ggcttcctcc atcagtctcc agagaagcat 240
   caagctgcgc tttcctgcct caaactagag cctgaggcgc atgcaccgtg gggttcacca 300
   tttgctggat gaggagaaga gaagctggca cctcagttcc cccattttct gtttggcgtt 360
   cacacctaac agatctcatt atgccagcct ggcactacca tcctgaagag caagtggatt 420
   aaccacaccc tatcccagcg ctgacctcac cctcccacct accgtatctt tattcccaga 480
   agtgtcaacc ctttccctcc ggtaatcaaa catctcgctt tgctaattac tgctcaatcc 540
   gattaaacgc tgctgaagct catcaacaga ggtggaggta gggggaaggg acgaaggcgg 600
   gagcaaaggc tgaggagggg gaaagggagc cagaggctaa ggtctagaga ccccagtccc 660
   tagtggggaa cagcagattt ggggtgagct gggggccaga ccggctgtgc cctgggctgg 720
   tcccgtgaga accagagtag attactctac agcctgactc atccttctcc cccagcagcc 780
   ccccacaggt gatttgtagc ccaccgggca ctgaggctgc tgtttgctga gcacacagat 840
   tcctcccaac tcaccagctg ctaccccctg aagcagctca gtctcccccc gacatcaccc 900
   gaacacttcg attccctgtg ggaattgggg tacctggggc ctggtcccct cagaactccc 960
   ttcagaactc cactttccct gctacctttc aagtagctct tgtgacattt ctcccaggtc 1020
   tcaaaactct cccaagcccc atgtccacgg tggcccatat agccccttag gaagggccat 1080
   ttttcattct aattaaaatt tgccgttatt tggaaggagg gcaaagcctg agcttcactc 1140
   tacagctcag ccccaagaca gcctcaatcc tatgcggttt ggggatggca gggcgacagc 1200
   ttcctgctct ggactctgcc agcagctgtg ccacagcccc caaaaaagct cagagcatca 1260
   tccttgccct cccagagctt aaaatttaga cgaggaaaac tgtgtgtatt tgtccccaca 1320
   aagcaaatag gacggcctgt gttattctgc gtggaagtca ccagcatttt caaatagctc 1380
   cagacaccca agtcactaca tgtgcagtct gtgctgttcc tctccctggc tgtgcatctg 1440
   tccccaggtg tcttcctgtc tctcagcatg ttatgatgtc ttggctgctt tggcgaggtg 1500
   ttggacccct ccctttctca gtgggccctg ttgctgggct ctgggttgcc atagtgacgg 1560
   ttgccaagtc cctgaggctg atagccagcg atggggctgt cccacttcgg gcagctccag 1620
   tgtgggctca gaaggcaaga gtaaattcca gacagcccag tacctggcct cccagctttc 1680
   aactcccctc ctgcggtgcc tttgatccta attgcgtctt accctgtctc tggtgctttg 1740
   gcttagcaag acactcagac tgtccccatg gatgaattgg cgaccccact cctgcctgca 1800
   ccaaactagc cctatcccta cccacccccc ttggttggtt tcatgcaatc caaccctcag 1860
   ctgaaagcca ttctaaccaa gcctttgtcc caattcaagt ttttacagta aaactactga 1920
   ggtgacggca taccccgcca ccatggcaat atcaacttcc tgttccccag aaggagcgat 1980
   tagaaaaatc aggaaggagc agcaccagag aggtgaactt gacctctggt ggacaaagca 2040
   accatcttgg ctggtgattg aggtggccaa cctggtgccc aacaacagct ttgatctaat 2100
   aaacagtggc ctttgctaag taccactgtc agctagacca gccagtaggc caccattttg 2160
   gtcactaaga agggaactgt gttgagtctg aaaggagtca gcacaaggga agggagatct 2220
   tgggaagtgc cactgaagac tgggaaaagt ccttctcggt ccacctctaa cctcttacag 2280
   tcagtaagtt ccagcttcct cacctcccac ctcctgatcc cccagactaa gccacttgga 2340
   gttccagcca gaccacttcc tgtttcagaa gtgctacttc ctgtcctgga tgaaggcact 2400
   tcatatcttg ctattggcca ctggacaaaa cgggacactt cttctcatgg tagctgtcat 2460
   gacccaacca ccccaaatgg ctggagtaag tatgtagagg agaagggctg gattcaggat 2520
   agccacgtga gaggagccct cataccttgt agtagatgtt tggagggctc tgggggggcc 2580
   catcctgcac gatatacaca ccgaactctg ctcagaatcc aggtgtccag cccttcagct 2640
   tcctgccacg gagtcctccc cacttctctg acagcactga ttgaagcact gacttggcta 2700
   cccgggtccc atccgatgtg gctgggagag aaaagccaga ggaagaggtt ggcactccag 2760
   aatctagccc tgattctggc cccacaccct ggtgtgaaca tggccccaga gattagggct 2820
   atactcctgg aacttgatgg tgaagcggag atccaggtct gggcgatcac aagtgagaag 2880
   gaggtttggg gcagggggtg agatgtcaga ggcctggccc ccagaacttt ccttccctca 2940
   ccccacaacc ttccaggaca gcaattctca gctcacgctt gcccgccttt gtctgagtgg 3000
   tcctaagtta gccagctaat agcacagaag gcaaatgcct ggtttggcca cgaccatcat 3060
   tggggctgat gggaaccagg gtctaggaac ctgctgcctg tcccacactg ggtcttagac 3120
   ttctggggca agtgctctat tgctttggaa aggaaaggag ttgacttttc taaccacaac 3180
   caacacactt caccatctcc aacccctgca gccccttcct ggacatcaat agctgctgag 3240
   acagggtctc aggactgggt cccaggggac tgctacttga tatcttccca gactcccaag 3300
   ccaccccaca tccgaaagag cccccaggac accctaactc ccttttctct accctgtgaa 3360
   aacaagctta tgttctcaag ctacacacct gatactcaca ccaacagttc actgaccctc 3420
   tggctgtggg gaagagttgg gagacagact gtgaagagag tcaaggagcc ttggggaagt 3480
   tgtccccaga gcccccaaac ggaccaagtg ctgttgttac ctgggggtta gtgaaaaaca 3540
   agagtttagg ggatacactt gggtctttcc tagatggctg gggggagaag cggggctggg 3600
   caggaagggc tgtttccaat tatcctgggg gaagggagga gttgcctttt cgggtgtaag 3660
   agccctggct ttcagcacct ctgggcaggg gtgacattcc gctggcagta tttcttcaaa 3720
   gtccccccat tctcagtgtt tgggaaggtg ttgggggcag gagggaggag tcactgcttc 3780
   aaatgacaca ctcgtccccc gagcggagga gagtgctcct gctcccctca gagctgagta 3840
   atggtcccca tgctgctgga gaatggcaaa cagtgtggag tgtgggcgac aggcgccctg 3900
   ccgcccccac cttgggagtg agatttcaaa caggtttccc ccttcagttt accaagttgc 3960
   ctagcaacca accagtcaat ggcatgaagc cccaccccca agagatgggg agggtctatc 4020
   agtgccttca gtgaggacac ttgctccctc tgggattcac ctccctcccc aacccaagcc 4080
   ccaacatctc tggaattgaa tcgacccagg gaccacagtg ggtaaccaga ctgccggaga 4140
   tcccagcaca ggagctcagc atcctggaga caaggaacag gacctggagt cacccccaaa 4200
   agctgctttg ggctctcagg gtgtgacccc taccttgcgg gctcctgaga agaagcggtt 4260
   gccatcggga tgaaagcaga ggtgggtgat gcccccttgg ggagatgatg ccgctctggc 4320
   cctgcttttt tgctagagag ggaaggaaca aagtggggga gggggcaatg gctcctggca 4380
   ctaaccaggc taagcagagg gtcccagcac caacatgctt ggactcaaca tcccccccat 4440
   gctgtctgtc acaagcagga caaagacctc tgatgctccc cacatctatc ctcccaaagg 4500
   cttcatccct ctcaaatccc agttcttcct tggagagatg caagttgaag gcctacagga 4560
   aaacggaatc agctttatca acaattttct agaaagattt cagagacggg ggaatggctg 4620
   agtcactcgg agggaaaagg agtctgaggc gcgtgcctct agcagtgttg tggcagatgg 4680
   ggtgtatata tggagcacag agagcaccgg aaggctgtac agtgccaacg tcgtcagcgg 4740
   gagagagaat cagcataagc ctcaagggaa aagtttcaca aagacgaaag agcaattgtg 4800
   gacttgcaga aagttctgtg aaaaggtgga aaagaaagga gaaaacccga gaaactatat 4860
   tgggaagaag actcagtaca cacaagtata tggaaatacc cttgaaaaac agtcccgttt 4920
   gtgaacagga aacacttgga ctacactttt tccatctcca cgaaggactt aaatgtgcag 4980
   cattgatgaa gaggcagtta 5000
<210> 56
   <211> 5000
   <212> DNA
   <213> Artificial Sequence
<220>
   <223> Nucleic acid probe
<400> 56
   ttaccatttc tgcatattcc acctgctccc cctcatggta cagctctggg ggcaggttat       60
   aaattcgcaa gatgttatca gcactattgg tcaagatgca ctggactgtt ttacagtgaa      120
   aatctcgggg gtggtcagct ccctgccccg ttgttatcct taccacttac agcctttcaa      180
   gaagttctca ggttgggtgc ggagaagctg tagttccaag cgctgaaagg aagatggggg      240
   acaataaacc tgggtcgcca agcaaagggg gcagaggcct ggagaagtgg gtctcaggac      300
   gcaggaaggc ttgtattttc agaaagttct tgctcctcga ttcgaggact caactcacta 360
   ggggaaccaa actctgtttc caggggagtg gagagagaaa taacggttga gtctccaaag 420
   tcttcatact gcagaggaag cacagcggag attagcctca gccaggatgg cttcgaagtt 480
   ctcagggatc cgacgcagag acccgcggag ccgggacgtg aaaggttaga aggtttcccg 540
   ttcccatcaa gccctagggc tcctcgtggc tgctgggagt tgtagtctga acgcttctat 600
   caaaagcttt cttccttcca cccttcatat ttgacacaat gcaggattcc tccaaaatga 660
   tttccaccaa ttctgccctc acagctctgg cttgcagaat gattggggtt ttcccctccc 720
   atgtgctcaa gactggcgct aaaagttttg agcttctcaa aagtctagag ccaccgtcca 780
   gggagcaggt agctgctggg acacgcttcc ctggattggg taagctcctg actgaacttg 840
   atgagtcctc tctgagtcac gggctctcgg ctccgtgtat tttcagctcg ggaaaatcgc 900
   gatgatgggg atgttaggac catccgaact caaagttgaa cgcctaggca gaggagtgga 960
   gctttgggga accttgagcc ggcctaaagc gtacttcttt tcgaaacatt gagaactcat 1020
   agctgtatat tttagagccc atggcatcct agtgaaaact ggggctccat tccgaaatga 1080
   tcatttgggg gtgatccggg tccaggcagc ccccggctcc gctagatgga gaaaatccaa 1140
   ttgaaggctg tcagtcgtgg aagtgagaag tgctaaacca ggggtttgcc cgccaggccg 1200
   ggaggaccgt cgcaatctga gaggcccggc agccctgtta ttgtttggct ccacatttac 1260
   atttctgcct cttgcagcag catttccggt ttctttttgc tgctattctc cgcctgcatt 1320
   tctttttctg gattacttag ttatggcctt tgcaaaggca ggggtatttg ttttgatgca 1380
   aacctcaatc cctccccttc taccatggcg tgagacaggg agggaaagaa gtgtgcagaa 1440
   ggcaagcccg gaggtatttt caagaatgag tatatctcat cttcccggag gaaaaaaaaa 1500
   cgtttgataa tttgtcggaa aaacaatcta cctgttatct agctttgggc taggccattc 1560
   cagttccaga cgcaggctga acgtcgtgaa gcggaagggg cggaactcga attcatttct 1620
   cccgctgccc catctcttag ctcgcggttg tttcattccg cagtttcttc ccatgcacct 1680
   gccgcgtacc ggccactttg agccaagggc acctgcattt ctcttggctc cctggccatt 1740
   tggaaggcct agttcagcct ggcacatttg tatcctggcc cactgatgct ggtacccctg 1800
   acacggagat tttagttgct actgaagatt tgagagataa agacagggag acctgtctgt 1860
   agacctgtgt ccctccaagt gggattgaga ctttgggccc gacagcacct cctggcctgt 1920
   tgactgaata gatccctgaa ggaggtgtac ttgcattaat ggagtggggg tgggagcagt 1980
   accacagatc cgcactaaca aggtgcgatg atagttgcat aacttcgtgc aagatgctgg 2040
   tccacacagg ggctgcccct tgctctttct cgctctctta acctctcata taacaggctt 2100
   cccttgaatt tgggctttta tccatcccat cacaccctca gcatctctcc tggggatgca 2160
   gaacttttct ttttcttcat ccacgtgtat tccttggctt gctagtgggt tgcaggaggt 2220
   gcttacgcat gtttgtttct ttgctgccgt cttccagttg ctttatctgt tcacttgtgc 2280
   cctgactttc aactctgtct agcctctgct tgcctctgac ccctgggccc acctcttacc 2340
   gatttcttcc atactactac ccatccacct ctcatcacat ccccggcggg gaatctcctt 2400
   tcttaacctg tggcttctcc tccacctacc tggagctgga gcttaggctc cagaaaggac 2460
   aagggtggtt gggagtagat ggagcctggt tttttaaatg ggaaagtttc cagtctaaca 2520
   ctcaaaatgc cgttttcttc ttgactgttt tacctgcaat tggggcattt gccatcaggg 2580
   ggcagtgatg cctcaaagac cctggcccta ctcccctgcc ttcctaggtt ggaaagccat 2640
   aggattccat tctcatcctg ccttcatggt caaaggcagc tgaccccatc tcattgggtc 2700
   ttttagtctt cctccggttg aatcctataa ccacattctt gcctcagtgt atccacagaa 2760
   catccaaacc cagggacgag tgtggatact tctttgccat gccaccatct tgatttgaat 2820
   tcccgttgtc ccagccttag gcccttcaaa gcattggtca gggaaaaggg gcacagaccc 2880
   tctcactcat gtgatgtcat cttgcaatag gtgtgcgtca gaagcaccca ggacttccat 2940
   ttgctttgtc ccggggctcc actgaacaag ttggcctgca ctggtgtttt gttgtgggga     3000
   ccttttatta catggggtct agaacttgac ccccttgagg gtgcttgttc cctctccctg     3060
   ttggtcggtg ggttggtagt ttctacagtt gggcagctgg ggcccagcca aaccctgtct 3120
   gacaacctct tggtgaacct tagtacctaa aaggaaatct caccccatcc cacaccctgg 3180
   aggatttcat ctcttgtata tctcaccaag gattacggga tttaaatgtc tgatttagca 3240
   aggctgagcc tccagggtgg ccatctgctc catcagaaag tggcaggata cctgggttcc 3300
   taggtccttc ccagcatccc ctcatcctgg gcctcatgcc aggtagctga atgaattgaa 3360
   gctttaaact ctgccaggaa aacctttcaa agggcttctt ctctttctta ctagggaatg 3420
   ccaaacactc tccccaggag atccagaccc gcctctttca gagactttta acttaaacat 3480
   ctgtccctac ccagcaggca tttgacaacc atcctccacc ctttcaatcc caaccttttc 3540
   ctagattatg tccctagctc tgtttttatt tctgctgtgc tgcttcagat ccattctgac 3600
   tactatataa ctgctggaat gaggtggatg tgacatatgg agtggtagac aaaccaagga 3660
   aagcaagtaa caaatacagg agatgttctc atacaggaga gtctctcttg tatggagtta 3720
   tcccaagata gtgcgcacta aattagtcaa ggcgaagggg gtagatgctt ggacactgga 3780
   aggaagagat caaagctggg tgcaggaaaa catgggggcc cgaagggaag aaacacagat 3840
   ccagctagag aaaaagggaa gggtccacct gtgggtggag tgtggaggct gcagcattaa 3900
   tggaattttc aggctggcat gcagaggaaa aggaaggagt tagtgattag ttgataactt 3960
   gctaaaagaa gccagagaaa ggatggtggg ggcagatctg ccagaatggc cgcccctccc 4020
   ccagagctcc ctgggaagtg gaacttgaag gatattctgg gaaggcgaat gggggaaggg 4080
   agccatccat aaaaacaaag cttccagtcc ctcatttccc agctcacaga gtcaccagag     4140
   ggtgagaagc agaacgtgcc agcaaagagg gaaaaggcca cagaaccacc ttgtcctcaa     4200
   agacctgccc aggagccaga taaattcaaa gtcaccaaga tggcgactgt gtagacaggg     4260
   agaaagtatg agagatcaag ggacggtggc cagagcttag tagctctcac agctaaacca     4320
   gaactagaaa ggctcctaac tcctccgtgg ccatcttagc ccagctccct gtctgactga     4380
   tctgaggttg ggagaagttg ggaacatgac gaagttgccg agattctcag catcttcatc     4440
   tcgctgtggg ggtgcaggaa gatagggggt gagccagtta gggatggcag gcacccagga     4500
   tgtccctgtc atcattctag gaactcctga aggctgctaa tggcacgaaa gcctcctact     4560
   ccactcctac cccaagttct taccccatcc tcaggaatca aacagggtcc caaggacaac     4620
   gaactggtgg cagatggtgc taacagatca gcagctacca gacacctaga aggaactgac     4680
   gggtgggggc catatgggag cggagttcaa gctacagtgg agcccaaaag cccactctta     4740
   cccccttgtt tctatggtga tggggcctct aggagcaaag aagataggga gagacaggtg     4800
   gtctgcaggt ggtgtcccca gatccttcct ccattcaccc tggggaccaa ggtcatagat     4860
   acccggttca tcttgatgaa gacacagggc tgcccagtgc cagggcggca gacatcattc     4920
   ttttgggctt ggatagagtc gttgtaagct aaagaggtgg gagggagaga ctcaggggcc     4980
   agatgttcca cttccaaggt                                                 5000
<210> 57
   <211> 5000
   <212> DNA
   <213> Artificial Sequence
<220>
   <223> Nucleic acid probe
<400> 57
<210> 58
   <211> 5000
   <212> DNA
   <213> Artificial Sequence
<220>
   <223> Nucleic acid probe
<400> 58
   cagtagagag ttacagattc ttaaaatatc ggcgatgcat acgcgaagag ggtaaagctc 60
   agcgcgtgcg caccaagcac ctttttcctt gcggcgactc tgtgtccgtt gtctggtgga 120
   agacatacct tagaccttaa tcacatcttc cccacgatcc aagcacatgg agaattgtct 180
   ttcttccaca cggtcccttt cactttcatc ttatattccg gttcccaagc caaggagaaa 240
   tggtcagatc catttacgac gtttggcatc tttgacctca ggaaccctgg gttggatcag 300
   ccataaggtg gcatctttcg caagtctctc tccccaccac accttgatct caagacccac 360
   tgctctacca ttctactcag gaatggattt gaggtctcac tcatagtcat aatctctaaa 420
   aggatctctg aaaggaagct tatgaggttc ccttctgttt atcttaagat ttccacttct 480
   tgaagactct tggccaacta atcatctttc aaagaccctt ccttatttca cctccccctt 540
   tgcccaggat ttggttcctt agaactcctt ataataccac cttccttatc tgctctgaat 600
   tttcttgcct cccaagagtt tttctttgtg tgactgttgg tattctgaac ctcttggatt 660
   tgatgcctgg aattgtccta gagactctcc tgatttctgt ttcagttccc ctcaggcctt 720
   tttcccccac tgtagtcctc tctagtttct catgcccttg gaaatgtctt tctttgactc 780
   cttctctaag tcatattttt agtcattttt tactcactct tcatacttac ccatttttgc 840
   cttacttccc tcatcagatt tatttctctg cggtgactgt gggtacccat gtctaactta 900
   agctttttct cctaatattt ctgtattcct gtgtaatccc caggaatcac cttattccct 960
   gtctagctga aggaagctcc ctgctgatct ctttttgtca tttgttgaaa tagcttccta 1020
   aagtggccct ccctgtttac cttggtatct tttccttcat ctgggtcctt accaccccca 1080
   aattctgagt tccaagaaaa acgttttctg tcccacatat aagtgtccag cagaaagtct 1140
   ctctgaatct tttccctttc tcctgtgtgc tgtggcagtg atagacttga atagatgggg 1200
   gctgaaagcc aggtaatgtg gtaatgtgga tctgggcagg ttagcatttt ctgccctgtt 1260
   cctgagttaa aaaccacaaa tccctaaatt caggctaatc agtaccctac agatacttat 1320
   ccgttttgac cttgctgctg atttaatatt catgactcag caaacttggg atgggtcagg 1380
   gagtatttac actgcttctg gcttcagtac ttggttttta ctcagactgt tcctgctctt 1440
   ctcctagaac cccctccttt gctttgggag tctagaacta gaagacggtg aaggtgggga 1500
   aatataaggg ttgagtgaga tttggttgaa ctgctgagta atgtgagaga ttactccatt 1560
   ctgagctttg catttctgcc ttggaagtca tggtagctgg ggcaagtaga agtaaaagtg 1620
   cctcagaaga cttgaatgga gaacagtagg agttggcatt aaacctgaat gaacatgctc 1680
   ataactcagg ttctttttga aaaagaggcc aggaagtacc agtagtaatt aaagccttgg 1740
   gcagtagtaa ggctctaaga aagaacattc agtcaagaat gatcttgact gactgcagcc 1800
   gacatatccc taaagacgag aaaggtggct tgtatgaaga gtgaaatgca ggcttagatt 1860
   ttcccatcgt aaggaaatag caatctggta agtgtttgtg ttgtctagtg ttccgattat 1920
   tgtcttagga cccatgcatc agggcagcga gtctgctcag atctcttgct gtattgcgga 1980
   ggtaggccct tctctctcga tcatttcctt tctctgtttg actgcccata ttctttcccc 2040
   agtgcagcct ttcaggctgt ctcattacac cacttgtgtc cctatctaaa actcaagttt 2100
   ttttctaaaa tccattctag ggctttgtga aggatgtcca tgaagactct gtcaccatct 2160
   tctttgaaaa caagtaagac cttgggaata gagaatccag gagtggagaa gagggggcag 2220
   ctgagtccta tgaggcatgt aataggttgg gagagctgag ggtttgggtg ggaactcata 2280
   tggatcccag gagaggaaag ccatgtagcc tatggaaatg taaaatgcaa cagttcccct 2340
   tttatcactg ttgtgatcag ccaaactctc aggaacatcc ccagtttcag gcgatttgcc 2400
   tacaatgaaa ttgttaccct ggagcgactt cggccagtta atcccaatcc ccttgcaacc 2460
   aaaggcagct tcttcaaggt taccatggct gtgcccgagg tgggaatgag ggccctcttt 2520
   acagccctaa tctctggctt tatttctccc aggcatcact cccactttgc agttttctct 2580
   gttgttcccc tggccctcta gaaagccctg ggagccaact gcatctttct caacatcaca 2640
   aacagtgagc tcttcattct ggtgagttta ttctgcctga atttcacata agccttttga 2700
   tttcagaaat agagtatgta gctctcacct tatgcttgct ccttgctttc tctctaaacc 2760
   attttccttc agtaccctga tagtcagcct ttgtgggact tttctagtcc ctggctctcc 2820
   cttagtgttt attccaagcc agttttaatg actttcccac tggtttcctc ccagtcaacc 2880
   acagaagccc ctgtgaagcg tacttatgtc ccgcaatgaa gaagctacca agcacctaga 2940
   ggtaactttt agctcctttt ccttctgctg aagggtattg tcttcctccc ttcctaccct 3000
   aggctttttc tgggcaatta aacctgggtc agctatttta tctcagactc ctccagatgg 3060
   cagagctcta tcatggtata gtgcaattat tttcaaattt tttctgtgcc tgtgcaattt 3120
   ctgggtcatg ataatctttg acttcttcag ggatcctgct cagggcactg gcaggtaaaa 3180
   attcaccatt accttcagat gtctcctttc aggtattgtt tctatttggt gcttacaaca 3240
   tgggattcac tcctctaaaa cctttcctgt cttcctgccc ctgaacccag gggttcctct 3300
   agttatgcaa agggtcgtgt gcctgggact gtgagattct accaccatga ttcctacagt 3360
   gacttttcct ttcagacaag caagcagttg gcagcagcct tgatgggact ggcaattggg 3420
   actcacggtg ccaacatcca gcaggcccga aaagtacctg gggtgaccgc cattgagttg 3480
   ggtgaagaga cctgcacttt tggtgtgtat atacagttag aggtcagaat caagattctc 3540
   tacagtgatt ctgagctttt atccaggatt ggcacctccc tgtggactcc cagtttcaca 3600
   aaaaggctga attcctcatt tacctattcc aggcaaagtg attggaaaga acgggaaagt 3660
   gatccaggag attgtggata aatctggtgt ggtgagggtt gtgataatga caagaagaac 3720
   cccagggagg aggtatgggt acggggcaag tgggcataga gattggtttc cagaagtaaa 3780
   tgggattgtc ctctgagggc atccttccat ttcattttcc tttctgatcc atcgtttctg 3840
   ctctctgttc tctagggaat ggttcccttc atttttgttg gcacccgaga gaacatcagc 3900
   agaattttcc ttgtttagcc agtgtcttcc ttctccctgc attcctgaga ggttagatat 3960
   agagggacag tcagggtgca gaagcatgaa gcaaggcact gtttggggga tgtgatggat 4020
   cctgaggtgg gatgaatggc cgtgaataaa tcccattgtg ttcttcaggc cccagctcag 4080
   atgtgtctac agcttcagag tatgggccaa aaggaaagat gatgggcacc tgactgccaa 4140
   ctcctcctct tcttcctctg accagtgctg aaggatccag acagtaatcc ctacagccta 4200
   ccgccgtaac cgtggtaatc ggactgatgg ctctatcagt ggagaccgcc agccaggtca 4260
   gccaactctg gacaccagaa tccttcctca gagctgagag cccagtgact gtggctgact 4320
   atatctcacg agcagagtct cagagccgcc agaggccacc cctggaacgc actaaaccct 4380
   cagaagactc tctttcagga caaatctcct actgaattgc agaggcagct gtgtctgatg 4440
   gagtttagac cagtgtaaac tttgaatagg atagagaaga aagtgtggtt cacattttct 4500
   tagggaatgt ctcccacctg aatttctact caccgcttct tgcttttctg ttttctttcc 4560
   cactttcccc tcagggtgac tctgtcagca agcttcctaa ccctccctct tgtccaccct 4620
   acccccaggg taaggggagc ctctctcctt ccccatcaga ctggatgtgc ctttatcctc 4680
   taatgcccca atctctctct tatccctcct cccatcctgt accccccatt tctggggcct 4740
   catcactgtg gaagacgggg atagtaagag ataagtgggt gggaggcacg gggaaggttt 4800
   tgttttctgt tcctccacat ctgacttctc ttcattgtct cttgtcccaa agatggtctt 4860
   acttctggga attcctgaca ttcctaaact ccccaggccc aagtttgctg gtagagggag 4920
   ttgggccact cctataccct gagtcacagc acaggctagg cagggctgct cagagctgga 4980
   gctgttcctg tagaagctga 5000
<210> 59
   <211> 5000
   <212> DNA
   <213> Artificial Sequence
<220>
   <223> Nucleic acid probe
<400> 59
   acttggggag ttgtcctggg tttcctggct cccccctcat gacttggacg actgaagcta      60
   aaacttgcca taattcaata gcagtggaga aatataggtt tctcacttgg gaaaggggtt     120
   gaatcacggc ccaagcacct gctggaatga ctcaggaaac aaaaatggtc tccctcctcg     180
   gcctttcttg ccccaggggc tcctctcagc acaactcggg ccctgagggc acttcatccc     240
   cctgtgtttg atcccattgt ggaaagaatt ggctttaaat tgcctgtgct tcagggaggg     300
   cctatccgcg cctggcagct gtcggaactc cggagaccac aaaggggttt atggtttacc     360
   ccctccacgg aaggtcggtc tgatcgcagc tgcgggtcgg ggcctcagaa cacctcttca     420
   gtgccggggc accagacagc ggattttgca ttcgactctc cccaaagtgt gccgaacagg     480
   ttggaagcaa aggctccggt cgctctaccc tgtctgccca gaactggcca gactcacctc     540
   caacccaggg aagtgccctc taacctcttg tttctctctg cagctcttcc cactgcaaac     600
   atcctctcct tttctaagga tctggatcca tcaaccattt gtttccacta aaagaaaacc     660
   ctggctcaga aagtctgcaa gtgcagagga atggaggatg ggaaacggaa tgactccagt     720
   agctggctct actgcgcctt tttctgcttg tggggaggct ttgcattcca gtagaagagc     780
   agctgggcgg cgatgaggcc gcaagagaca gagccagggt tacccttcag catagctcaa     840
   gaaaggttcc cttatggaca aaggttcctc acggggcagc tttggccctt agctgggaga     900
   ctcttgggta agtccccagc acctttcaca gtctgtcctc tgtagtgcat gaccaggaag     960
   cagatggtga tcgtctggag catcaggaat aaggcttcac gatcacgctt ctctacagcc    1020
   cccacacggg actggctcat tctgaatcgt ggccattctc gggatgctca ccccttctca    1080
   agatcacctc cactggtgat tgctgccttg taacctcctt caccttaatc cccattagag    1140
   ttgtgggtat tacccttggg gtggaagggt gggcccctca cctgaatggg aagttgttag    1200
   cagcattaca gactggagac tcaacccttc agcactcttg gctcccagga ttttaaacac    1260
   ctggggcagc tttactaggg gaaagatgag aaaagtagga ccaatcttcc attccctata    1320
   aattaacacc caaacttggc ttgacctcca cggctctcca ttgcttcccc atataaagct 1380
   gagaaatgcc caagagctct ggcttgcttt tatgccctgg ccatccacaa cttctctagc 1440
   cttgaagcct tcccctcaag gaacttggtc tagtgggagt ctgactctca cgggtcatga 1500
   gacaggggca gactgacgct ccccaaacgg tctgacggtg acccttgtgg agctgtctcg 1560
   caatcagaaa aaggaaggga ggcaaattat tccgaaatca gctggaaaga aagcaatcag 1620
   ggtggcggag cctttggggg tgcaatgaag gcccgattca tcaggggctt tgaattaagt 1680
   ttaaccctgc tagcgatcta cgctacagca caaactccac aaagaagtaa ttccacccgg 1740
   tgacttcccc actggcacta atatggtgtc ctccctctaa ggagcccgag acacttccgc 1800
   gcagccgttt aagcggtccg tccgcctcgg ccgccatatt gcaaagctag cttccgccag 1860
   tctctccaga ccgccattga ctttcgttgc gcgtgcgcgc tcagggacca cgttttggac 1920
   gcgctgttgg aattgtaaag cttgccgaag ctgcacgcct gcgcggttag acctcccacg 1980
   cctttgttga cgcttccagt ggagccccca gtggaggtca gagtacccac tgctccttct 2040
   tgattcaacc ccttgtaggt gggataggag catgccaggt gctgcgaggg tttaatcggg 2100
   tcaggagcca ctggcgctgc ctttgggaat gaagttccac agccccccac tcattgcttt 2160
   ggctgtctgc tgcatttgaa actgtacgag ggtcaggaag aacaccagct gtgccaggct 2220
   ttggggggtg aacccatgag gcatcgtcac acactgacat tttcatccct cctcagcagc 2280
   tgcttccggc tccctgagac ggaaactggc agagtcttgt agaggatgga aagaaagctg 2340
   ccctgtagcc atgacaagct gggagagcga gatttggggg gttaagaaga gagatgagtg 2400
   gctgcaactg aagctctgcc ccaggggtgc ttggagatct cgaagggatg cattctgagc 2460
   cgagaatgtc cactctggag gagtgaggaa gggtcagatc acttacgtgc tgcgtggggg 2520
   aaggacacat tgtactccac cgccatgtag ctcaggtaga caaccttctg ctggaggtcc 2580
   tgtggggcga agaggaaggc cctcccctct catcccccag ttttagcttt acctctcctc 2640
   caccctgggt tgtgtacatg actcgaatct gaatctgggc ttggaggtgg acacagggct 2700
   agccagcctc atggctgaac cgcctcaccc aaagtccagc taggctgtct gcaccagttg 2760
   ctcaaccctg ctccctcagc cctctcttgg aaaggaggaa tagtcattag tttttgtaat 2820
   taagttaggg gactgttggc cctgcttcat cagggcaaag tcaacagtgt cacatgggtg 2880
   caaacggagt cctagggcag gcttggcagt tgtggcaagt agctatggga acagctgagg 2940
   tacaggcccc tcccttggtg gggaaatcgg tttccactcc tttcccctgg acctctgggc 3000
   cagcattgtc attcacagag ccgagaacat ggctttccag cgtctgagaa ctcagatggt 3060
   gacaagaaag caacagccca aaggtaacca tctgctgggg cgtccacctc gaccgattct 3120
   ggaaaacaag gaaggggatg aggaatatca ggagctttaa gcccagggag ggccaggtgt 3180
   gaaggggtag gggagtgttc agaaaacaaa tatgcagcag accaggtagg cacctgggtt 3240
   ccaggaaccc tggcttcaag taggacactt ctgacgggca gacccacaac aggctggcag 3300
   ccttccctag ccacacctaa aactgaagca gatgcataaa aaggctcaag aatccagtat 3360
   atggttcccc tggcacttac tatttttcaa gaatcattag gaggagccaa gtagtctgct 3420
   ctcagagtac aaagactgat cctttgccca ggaccttccc gccaggcaga caacagccct 3480
   gggaagtagg acactgccag gctcttcccc cagagagaaa ccagcatcct tgggaatgac 3540
   tctggcttac gctgaaagac tggaaagcgt gcacaagagc aaagtgaccc aggggattgg 3600
   agcaaccagt tttatagaac aggtgatgca tatccatgtc ctattgcact cctgccatag 3660
   gacccaagct acgaggcatg ttcactctga gaaaactgag cattatccac acccagaata 3720
   atcactgccc ctgccacaga acatttaaaa acatgctctg acatcagtgt tgcagcatga 3780
   cccaggaaaa agtccagcat ctgtaagatc acccccacac caagggatct cttagcccat 3840
   agtgaaggtg cttattcaga acacagtcaa acttgcccag ccagccaggc ttcacccaac 3900
   gcacccatgt cttcctccca aggtggtgat cagagaagac ttctctggtc cttggattat 3960
   actcatcatc cctaaatcag ccctgaagga gggaatagaa taaattagga gttgaatgtg 4020
   gtaaagagca taccctttgg gatattcaag tcctaggtta atgaggtcaa taagcaacca 4080
   atgaaccaag catttcatgg accagttatt aactcatcta gtattgcaca ccaagctgga 4140
   ctagaaacat ggctgtagat gcaaatataa gctggctaca ataccacctc tttaaaggaa 4200
   gctgagctgt agtctccgag caaacagaat ctggtgccta ctaacaaaag cctcagagct 4260
   tacaaccctc ctccctttag ggaaaccagc tgcttctaga aatcaatcaa gaggccactc 4320
   taactctata cggctgaaag ttgtaatggg ggaggacaga attccatttc tccagtctca 4380
   cctcgatgac gccttcgggc agccgggctc tgtcaggagt ggcctgcatg atgccatcaa 4440
   tctgctgtcc cagatccaac tcccaccagc tcccaaaaaa tacccaaagg aggagtttct 4500
   cctcggggtt aggctcagtc ctggccgtca agaaaaccag cacactgaac ggccggtttc 4560
   ttctccccga cccgtgtttt agccgcgacg aagtgactgg cagcctcatt ggctggaggg 4620
   cccgcctttc tcgccggtac ggaagtgaca tagaagagtc aggcctcgag gcctggcagc 4680
   catcttgggg ccctgcactc ttccctttac ttctgctccc ggtctccact ggactcaaat 4740
   gttatttgtt ttcccctgta gcagagatgg attcgacact gcctggacgt gtgccaggga 4800
   ctgagctaag catatccact aatttgaagc cccttaagga agaccaccac ttacgtatca 4860
   acaaaggatc ctttattgac cagagcagga ccgtggcatt aagagaggaa aggaactgga 4920
   gtttcttggg aagggactcc catgtctccc ttcccattta tgggcttggg gtctggggta 4980
   cgaggctcac acagtgagtt 5000
<210> 60
   <211> 5000
   <212> DNA
   <213> Artificial Sequence
<220>
   <223> Nucleic acid probe
<400> 60
   gggcatgtcc tgctgggtga agctgaatgg ctgagacagg gccagatgct tgcagtactg 60
   ggtgttaaca aagcaagcca tgagaaaccc cccgtgtcag agagaaccag tgaagccagt 120
   gttttggtgt tctaaaacag attacttcct gctccacaac tgactatgag ccaaatgagg 180
   ggatgcataa cagcctcccg cccatcatct gcttacctgc aacacaaaag caccacagtc 240
   actgtcatta ttctgcctgg ccacattcta gagaagagaa agaaagcagg cccttcacat 300
   accaacaccc tatacccctc cctcaggaaa ctcaccattt tgaagtaacc tttccagccc 360
   tggtggaaat ccagtcggtc tttctttacc gcctctgcct tcaaaatagg tgatggtgcg 420
   tcgcctcaca tcaacagaga tgagggacca atgcacctcc aggtggatgg ggattagcag 480
   tagctcctta ttgaagatgt agatagtgcc acaggtcagg tacaacaaac agaagtcttg 540
   ccttttgcca tgaaggttct agagaggaac agagacacag gtaagggaac caagtgaaag 600
   ctcttgattt tgaggactga aattaaggaa tgccggagcc aatcatcctg cctagctgta 660
   gaaatttcct ggcctttgaa tgggagggca gggaaaccag ggcattacac aagtggatgt 720
   gaattcaaaa ctcacgtttt tggtccacct tttcacccca tcataaccct ttggtacgga 780
   gtttatcata gaagaaacta tcttgtttta caatagttac aataggaacc catcccaatc 840
   cttggggctg gccatagcga aaaacactaa atactggcag aaaggaaaac aactgataaa 900
   gttcctctgg gtactgacct atctggttgg gcctaccttt tcagggactg tgtccatgac 960
   caggtctcca tacatgttca tcacctgggg agaagatggg gacaccctgc tccctgaatc 1020
   ccctctcagg cctgtttctc taccctttct cacctggtca ttgagccagt tctgtccata 1080
   caaggtcccc aagtcatcca taagccactc ggaagcccct caccatggca ttgcctggca 1140
   tccgctggta agactggatc agctgcaaca ccaggccctt cctgagtagg ccaagggtgg 1200
   agctggggca cacagaaata cctactactc cctggaatac aaattctaac tgactccctt 1260
   ttttctggct catgatgggt ccttctgagg acctgcgaaa ctctagcttc ctcctcccca 1320
   actacagccc tatggaagaa aaggcagcag tattctctcc acatccccaa ggccccagcc 1380
   cttctttcaa ggagggcttt tggaaaactc ctgctggaaa atgtcctcca gcttctctac 1440
   tacctcatca gtgctgaggg gtatgaggct gccatacgtt tgaaggaatt cgtccaagat 1500
   ccggctttct ccccaggcct ctctgcctct tctgccatgc ccaaatctga gccctgaaaa 1560
   agctcctggg ccacatggtc cccgatgctg cacacattgc agggcagggg aaatggaatg 1620
   ggcatgagag actggcctgg ggaaagcaca atgggatgca cacagttgac tcatgcatgc 1680
   aaataagacc gcaagaagga cccactccaa gaaatgggga ccccataagc atgagataaa 1740
   catgtccaag agattctaca gggatggaga gagagttcct actcatagac aggaagaaag 1800
   gcctgaaact gtttggctgg agttctccga aagggaaata ttctttcaca actgtaagat 1860
   gatgtactcc actactggga gagatcccat cccgtcactt ggatttcgtg tggacaggat 1920
   ttttgtaaac cacatctccc tttctcatct gcgctcaaga ccacgggata cctacttagg 1980
   gcatctgttt cctagctctg ggagccggca ctcttccatc ctactttcct taagcccagc 2040
   tctcaccatg gcaacaggct cctggtgctc caggctctta accctcccta acccctcccc 2100
   cacccctttc ccaccttacc cgagtcaggg tccagaggag actttggagt ccacctgagt 2160
   ccatcttcct ccatgggggg cccagagggc actggtggag tcatcttcca gtaccctgaa 2220
   aaacaaaaga tcagggtatc aaaatctaag catcacccca ccgcagatcc tgcctctctt 2280
   cctggccatg cctcctctgg cccagtccag agacaaggga aaatggctaa gcagcaggcc 2340
   tcagagatct tatttctaag atcaccaggc caaacccaat agcactgcca ggttctacgt 2400
   gcataatagc tacatctctg gagatagtga tggaaactgg ggtccgatag atggagagaa 2460
   ggacgtagga taaggcttct tgtggggtga agaggcagag tgaggaacgg ggagccaaac 2520
   ccaggaagac gcctctgttc ctgcacattc cctctccttt atatactcag ctcttggctg 2580
   tctccagtat gtacccaccc agctgggagc cactttttat aacacaatca cagtttcaca 2640
   aaccccagga aggttccatg tggagagagg ttaagtttcg cccttgcccg gggaattatg 2700
   tgccttctcc agctccaccg ttcctctgcc tcctgtaagg acccggcatt ctgcacttca 2760
   ctcctcagac ccgcctctgt ttaggtttcc aacctccctc acctgcgcta tagcagctgt 2820
   tgtaggcccg gtccgggtgg gagggcatac ttcttataca tcggaataga gtctcctgcc 2880
   ttccttggcc ttctcgagac tttggtatga atagaggggt caggaaccct cagtaggggg 2940
   ccaaagaggc cctctgtgaa gctcagagtg tggctggggt tacctggcta tacagcagat 3000
   cggacaccat atccttgggc ctgtaggcct ctcccacgcc taagagctgg ttgccacatc 3060
   acctctgtca catcggagtc atctggaggt gagagtgtgt ttccgggatc tctccccatt 3120
   ctcccaggct tccagggcat cggaactctg ctcatggaaa gagggtcaag gttaggttaa 3180
   gatgcgaggg tcccagccag ccctccaccc tccctcagcc tcaggagaat taggttccag 3240
   tccctctagg aaggacaggg ctgccagtga cacccaggag gaacaggcag tgcgcaggaa 3300
   ccgggtgtgt tggaataagg cctgcactac tccctgtgct gaaattggca agcaggactt 3360
   cctgtttccc gagaaaagct cttacataag gctctggacg cgcaggagga agtctgcctc 3420
   ctaggccaag gaggccgctt ctgctgtacc ttgggcggag gattgatttt tttcctgggc 3480
   agtgtgcctt cctacctctg ggcctaggcc cagtctcttt ggcttcctgc cgccaggtcc 3540
   agcccctctt agtgcctgaa gtggggtctt ctggacccat ccttgcccac agagtacagt     3600
   tgtaagataa gagtgggaat actgtattta tgtgggatgg aaaacacgtg aacaggccca     3660
   ggcagcctct agagggaggg gcaggtctgg agcaaagagc caactcaccc tcccagccct     3720
   gtagcttgtc ccccactctc ctggcaaacc cttcgccact ctcttactgc gttacccatg     3780
   cagccagatg gggcttacct gctggagctg ttgattctgg cttcctccca gccactcact     3840
   gtcccgtcca cacctgaaca cggatgctca gagataagga aacgtctctc tacaggactc     3900
   ggccagagac attcagggca gattgtcacc tgcagtcact tctggggaga agagtagaac     3960
   tgaaggagaa gcgtgagggg atttaatgtt ttacttcatg accatctgaa cagcaatagc     4020
   ccctcaaact catctcaacc aacactgagc tgacccccct catcccactc ttgctttact     4080
   gtctgttttc cacatttggc ctcacagtgg aaagacattc tggtgagggg gatggtatgt     4140
   gcaaagtcca gaagaggcac accgggagca ggggtgtcag cgcagcaaac atgaagacag     4200
   cagaccacga agagactgga agccatgtag agagccaata ccacaagaaa agtgaagttt     4260
   aggggctcag catggcagca caggtcagat ggagtggagg tagagggtgg ggtagaaatg     4320
   gaaaagatga gcagacacta gcagcattta gagaaaaaga tcatcagaac gtggaatatg     4380
   gaggagacag aagaaaaaag ctttcatggg gtcccacgcc tgggtcacag agagaaccaa     4440
   gaccttcatg aactggcttc tgtggttctg agactgatgg ccgggaaggg agtgggaggt     4500
   cccaggcctc tgactgccag gcctagtctc catcaggatc ccagtagttc ttctaactca     4560
   tggccaccct gccttcctgt ttggggtagg gtctgaagga taaatgcaaa taaagaattt     4620
   cacgtagtct gtaagggtag ggactgtgta tgctttgcag gcattgtatt tccagggtca     4680
   gcaaaagttt gttgaatgaa aggtaagatt cagcccagag ctggggctga gtatcagaag     4740
   gcgctgtatt ctggggattg gtggggcgtg tgccctggac actctctgct tggcctggct     4800
   attaaccctt tggccagtga tgggtgggaa atggggtatc tgggttctgg tctgcactgg     4860
   cttctggctt ttggcaaggc cttgagtggg ccccccagct tttctggaag aagacacaga     4920
   aactggtacc agtggcttcc tctgtggaag ggaaattggg tatgtggggt ctggtgagag     4980
   aattttcacc atacactcat                                                 5000
<210> 61
   <211> 79
   <212> DNA
   <213> Artificial Sequence
<220>
   <223> Nucleic acid probe
<400> 61
   aattcgttgg aaacgggata atttcagctg actaaacaga agcagtctca gaatcttctt 60
   tgtgatgttt gcattcaaa 79
<210> 62
   <211> 79
   <212> DNA
   <213> Artificial Sequence
<220>
   <223> nucleic acid probe
<400> 62
   cttcgttcga aacgggtata tcttcacatg ccatctagac agaagcatcc tcagaagctt 60
   ctctgtgatg actgcattc 79
<210> 63
   <211> 79
   <212> DNA
   <213> Artificial Sequence
<220>
   <223> nucleic acid probe
<400> 63
   tgaactctcc ttttgagagc gcagttttga aactctcttt ctgtggcatc tgcaagggga 60
   catgtagacc tctttgaag 79
<210> 64
   <211> 79
   <212> DNA
   <213> Artificial Sequence
<220>
   <223> nucleic acid probe
<400> 64
   tttcgttgga aacggaatca tcttcacata aaaactacac agatgcattc tcaggaactt 60
   tttggtgatg tttgtattc 79
<210> 65
   <211> 83
   <212> DNA
   <213> Artificial Sequence
<220>
   <223> nucleic acid probe
<400> 65
   cctatggtag taaagggaat agcttcatag aaaaactaga cagaagcatt ctcagaaaat 60
   actttgtgat gattgagttt aac 83
<210> 66
   <211> 87
   <212> DNA
   <213> Artificial Sequence
<220>
   <223> nucleic acid probe
<400> 66
   cacagagctg aacattcctt tggatggagc aggtttgaga cactcttttt gtacaatcta 60
   caagtggata tttggacctc tctgagg 87
<210> 67
   <211> 71
   <212> DNA
   <213> Artificial Sequence
<220>
   <223> nucleic acid probe
<400> 67
   gtttcacatt gcttttcata gagtagttct gaaacatgct tttcgtagtg tctacaagtg 60
   gacatttgga g 71
<210> 68
   <211> 58
   <212> DNA
   <213> Artificial Sequence
<220>
   <223> nucleic acid probe
<400> 68
   cctgtggtgg aaaacgaatt atcgtcacgt aaaaactaga gagaagcatt gtcagaaa 58
<210> 69
   <211> 65
   <212> DNA
   <213> Artificial Sequence
<220>
   <223> nucleic acid probe
<400> 69
   tgcattcaac tcacagagtt gaaggttcct tttcaaagag cagtttccaa tcactctttg 60
   tgtgg 65
<210> 70
   <211> 71
   <212> DNA
   <213> Artificial Sequence
<220>
   <223> nucleic acid probe
<400> 70
   cattcccttt gacagagcag tttggaaact ctctttgtgt agaatctgca agtggagata 60
   tggaccgctt t 71
<210> 71
   <211> 80
   <212> DNA
   <213> Artificial Sequence
<220>
   <223> nucleic acid probe
<400> 71
   cctatggtag taaaggaaat agcttcatat aaaagctaga cagtagcatt cacagaaaac 60
   tcttggtgac gactgagttt 80
<210> 72
   <211> 80
   <212> DNA
   <213> Artificial Sequence
<220>
   <223> nucleic acid probe
<400> 72
   atttcgttgg aaacgggata aaccgcacag aactaaacag aagcattctc agaaccttct 60
   tcgtgatgtt tgcattcaac 80
<210> 73
   <211> 80
   <212> DNA
   <213> Artificial Sequence
<220>
   <223> nucleic acid probe
<400> 73
   cgtagtaaag gaaataactt cctataaaaa gaagacagaa gctttctcag aaaattcttt        60
   gggatgattg agttgaactc                                                    80
<210> 74
   <211> 79
   <212> DNA
   <213> Artificial Sequence
<220>
   <223> nucleic acid probe
<400> 74
   acagagctga gcattccttg cgatgtagca gtttagaaac acactttctg cagaatctgc        60
   aattgcatat ttggacctt                                                     79

## Description

### BACKGROUND OF THE INVENTION

### Field

This disclosure relates to immunohistochemistry and *in situ* hybridization, particularly to the detection of CD79a protein, TP53 nucleic acid, and chromosome 17 centromere DNA in a single sample.

### Background

Many cancers are characterized by genetic changes that lead to aberrant control of cellular processes, or to uncontrolled growth and proliferation of cells. These genetic changes include gain or loss of function (for example, including amplification or deletion of all or a portion of a gene), gene rearrangement, and changes in sequence (for example, substitution, addition, or deletion or one or more bases). Such changes are known to occur in the genetic regions such as 19q12, and with regions associated with various genes, including ATM, DLEU2, AND TP53 genes. In addition to their well-known applicability to genetic abnormalities associated with cancer, abnormalities in these regions, and others such as 13q12 and INSR, have been associated with autism spectrum disorders, metabolism, motoneuron specification, and cardiovascular disease. Detection of genetic changes in these regions can provide diagnostic and prognostic information for patients and in some cases, inform treatment decisions.

Chronic lymphocytic leukemia ('CLL') is an indolent disease of the bone marrow, which begins to produce too many lymphocytes (white blood cells). It is the most common leukemia in the Western world, with approximately 25,000 (roughly 13 cases per 100,000 age 65+) new cases in the Western world diagnosed per year. Patients have variable courses, from indolent disease, to rapid progression with limited response to treatment and clinical studies have described CLL as a clinically heterogeneous disease. Because not all patients benefit from the current standard of care, there has been rising research interest to identify the molecular subgroups with prognostic and potential therapy-predictive signatures within a specific clinical stage of CLL progression. Abnormal cytogenetics are found in the majority of patients with CLL, and each subtype is associated with differentiated frequency, outcome, and suggested treatments.

Del 17p is associated with aggressive clinical course and short OS. Some CLL patients present originally with only 13q aberration, but then progress over time and years of treatments to carry the more aggressive aberrations. Until most recently, patients testing 17p-deleted were evaluated for novel agents or stem cell transplantation, with limited results. However, in the past year, 4 novel therapeutics have been FDA approved for treatment of CLL patients, and other trials are still awaiting final results. Obinutuzumab has shown improved results compared to the standard CD20 therapies in all subtypes, and idelalisib has proven effective for patients who have failed prior treatment (many of these patients progress to harbor more aggressive aberrations after treatment). Ibrutinib received updated approval for 17p-patients specifically, and each CLL clinical trial will need to show efficacy in this most aggressive subtype specifically. These new types of highly specific treatments meet an unmet medical need in targeting these specific CLL patients whose clinical course is most grim.

The current standard of care to accurately diagnose and subtype these patients is a fluorescence *in situ* hybridization (FISH) panel testing for the 4 aberrations (13q, trisomy 12, 17p, and 11q). Some laboratories have begun incorporating CGH array testing or next generation sequencing technology to analyze for these CLL subtypes, but all of these diagnostic technology options are very costly, require a large capital investment in platforms and lab-specific requirements. As a result, the standard FISH testing is typically centralized among a few Heme-specific academic or reference laboratories per region, and turnaround times for results may be as long as 3 weeks. Further, FISH does not incorporate the context of tissue for the reader/scorer.

Workers in the field did not believe it was possible to perform a gene-protein assay to co-detect TP53, chromosome 17, and a B cell marker (e.g., CD79a, etc.) using bright field microscopy. One of the reasons is because scoring a deleted entity (e.g., 17p) using bright field staining was thought to be not possible or at most would result in inaccurate scores. Another reason it was thought not to be possible to perform such a gene-protein assay was because the protease treatment used in *in situ* hybridization was thought to destroy tissue morphology, and thus also destroy the staining of the B cells. In other words, the *in situ* process would affect the proper reading of the slide.

US 2008/299555 A1 discloses a triple staining protocol for in situ co-detection of HER2 gene, chromosome 17 centromere DNA and HER2 protein in a tissue sample.

Shao et al. (Mod Pathol. 2011 Nov;24(11):1421-32) is concerned with the morphologic, molecular and cytogenetic analysis of chronic lymphocytic leukemia/small lymphocytic lymphoma (CLL/SLL) associated with histiocytic and dendritic cell sarcomas. Tissue samples were stained separately for various protein markers in chromogenic immunohistochemistry (IHC) assays, the markers being, amongst others, CD79a, CD5, CD23 and CD163. Further, genomic rearrangements were analyzed in fluorescent in situ hybridization assays (FISH) using a FISH probe for p53 and a control probe for chromosome 17 centromere. All other (protein) markers including CD79a were each detected separately in a single marker chromogenic IHC assay.

The "Chromoprobe Multiprobe-System CLL Panel" (http://www.genycell.es/images/productos/protocolos/pmp_.pdf) discusses a panel of various probes that can be applied simultaneously to a patient sample in a single FISH reaction. Amongst others, a probe carrying a fluorescence label to detect deletions in the TP53 gene is disclosed.

Almond et al. (J Med Genet. 2005 Sep;S82) summarizes the analysis of chromosome anomalities in samples from patients having aggressive phenotypes of chronic lymphotic leukaemia (CLL). The anomalities were detected in single marker FISH assays using a slide-based device designed to detect eight chromosome anomalities including, among others, deletions in the p53 genomic DNA.

Liu et al. (Br J Haematol. 2012 Apr;157(1):136-9) discloses an analysis of morphological, immunophenotypic and genetic features in samples of patient cases having diffuse large B-cell lymphoma (DLBCL) and chronic lymphocytic leukaemia (CLL). CD79a is one of several B-cell markers the expression of which is shown in a single marker chromogenic IHC assay with an H&E stained tissue sample of a bone marrow biopsy. Also disclosed is the analysis of TP53 protein expression based on the findings in a separate chromogenic IHC assay.

EP 2 636 757 A1 is concerned with compositions and methods for the detection of nucleic acid sequences associated with chromosomal aberrations. Various targets for nucleic probes are mentioned, including, amongst others, TP53 and certain sections of chromosome 17.

As such, prior to the present invention, workers in the field believed that a multiplex assay for co-staining a B cell protein marker (e.g., CD79a protein), TP53 DNA, and chromosome 17 centromere DNA would not be possible and at most would not result in accurate scoring.

### SUMMARY

In one aspect of the present disclosure is a multiplex method for co-detecting CD79a protein, TP53 genomic DNA, and chromosome 17 centromere DNA in a sample on a single slide, said method comprising staining the CD79a protein by contacting the sample with a CD79a protein-specific antibody and contacting the sample with a first chromogen component for the CD79a protein-specific antibody, the first chromogen component is adapted to emit or make visible a first color, wherein the presence of the first color indicates the presence of the CD79a protein; and staining TP53 genomic DNA and staining chromosome 17 centromere DNA by contacting the sample with a TP53 genomic DNA-specific nucleic acid probe and with a chromosome 17 centromere DNA-specific nucleic acid probe, and contacting the sample with a second chromogen component for the TP53 genomic DNA-specific nucleic acid probe and with a third chromogen component for the chromosome 17 centromere DNA-specific nucleic acid probe, the second chromogen component is adapted to emit or make visible a second color and the third chromogen component is adapted to emit or make visible a third color, wherein the presence of the second color indicates the presence of TP53 genomic DNA and the presence of the third color indicates the presence of chromosome 17 centromere DNA. In some embodiments, the sample is a blood sample. In some embodiments, the first chromogen component comprises fast red, the second chromogen component comprises silver, and the third chromogen component comprises a green chromogen component. In some embodiments, the method further comprises visualizing the colors using bright-field microscopy. In some embodiments, the method is automated. In some embodiments, the step of staining the CD79a protein is performed before the step of staining TP53 genomic DNA and staining chromosome 17 centromere DNA. In some embodiments, the sample is subjected to a protease treatment with proteinase K, pepsin, collagenase, dispase, or a combination thereof after the step of staining the CD79a protein but before the step of staining TP53 genomic DNA and staining chromosome 17 centromere DNA, optionally wherein the sample is subjected to a heat treatment after the step of staining the CD79a protein but before the protease treatment. In some embodiments, the CD79a protein-specific antibody is an anti-CD79a SP18 rabbit monoclonal antibody. In some embodiments, the CD79a protein-specific antibody comprises a first label comprising an enzyme, and the first chromogenic component comprises an inducing component comprising a substrate for the enzyme of the first label for inducing the first label to emit the first color, optionally wherein the first label comprises biotin and the inducing component comprises streptavidin conjugated to an enzyme. In some embodiments, the first chromogen component comprises a detectably labeled secondary antibody that specifically binds to the CD79a protein-specific antibody, and the detectably labeled secondary antibody comprises alkaline phosphatase and the first chromogen component further comprises fast red. In some embodiments, the TP53 DNA-specific nucleic acid probe comprises a nucleic acid molecule having at least 90%, at least 95% or at least 99% sequence identity with the sequence according to any one of SEQ ID NOs: 51-60; or a nucleic acid molecule having at least 90%, at least 95% or at least 99% sequence identity with at least 250 contiguous nucleotides of any one of SEQ ID NOs: 51-60; or a nucleic acid molecule consisting of the sequence according to any one of SEQ ID NOs: 51-60; or a nucleic acid molecule consisting of at least 250 contiguous nucleotides of any one of SEQ ID NOs: 51-60. In some embodiments, the TP53 DNA-specific nucleic acid probe comprises two or more portions, wherein the first portion comprises at least 250 contiguous nucleotides of a nucleic acid sequence with at least 90% sequence identity to one of SEQ ID NOs: 51-60; and the second portion comprises at least 250 contiguous nucleotides of a nucleic acid with at least 90% sequence identity to one of SEQ ID NOs: 51-60, wherein the first and second portions are different from one another, and wherein the TP53 DNA-specific nucleic acid probe is at least 500, at least 1000, or at least 5000 nucleotides in length. In some embodiments, the TP53 DNA-specific nucleic acid probe comprises at least two of the probes. In some embodiments, the TP53 DNA-specific nucleic acid probe comprises a detectable label, and wherein the second chromogen component comprises a primary antibody that specifically binds to the detectable label and a secondary antibody that specifically binds to the primary antibody, wherein the secondary antibody is conjugated to horseradish peroxidase and the second chromogen component further comprises hydrogen peroxide and silver. In some embodiments, the chromosome 17 centromere-DNA specific nucleic acid probe comprises a set of two or more single-stranded oligonucleotide control probes specific for X distinct monomers of an alpha satellite control region of chromosome 17, wherein X = 2-14, wherein each control probe comprises a sequence selected from the group consisting of SEQ ID NOs: 61-74; or a sequence selected from the group consisting of a truncated version of SEQ ID NOs: 61-74, the truncated version being at least 40 contiguous bp of said SEQ ID NOs:61-74; or a sequence selected from the group consisting of a sequence that has at least 70% sequence identity to one of SEQ ID NOs: 61-74, or complements thereof. In some embodiments, the step of contacting the sample with the chromosome 17 centromere-DNA specific nucleic acid probe comprises hybridizing the probe under conditions for a period of time less than about 3 hours. In some embodiments, the two or more single-stranded oligonucleotide control probes each comprise between 50 to 100 nucleotides. In some embodiments, the chromosome 17 centromere DNA-specific nucleic acid probe comprises a hapten, and the hapten comprises dinitrophenyl, digoxigenin, biotin, or fluorescein, and wherein the third chromogen component comprises a primary antibody that specifically binds to the hapten, and a secondary antibody that specifically binds to the primary antibody, wherein the secondary antibody is conjugated to horseradish peroxidase and the third chromogen component further comprises a green chromogen component as substrate for the horseradish peroxidase.

In another aspect of the present disclosure is a single slide comprising a sample of cells chromogenically stained for CD79a protein, TP53 DNA, and chromosome 17 DNA. In some embodiments, CD79a protein is stained with a first chromogen, TP53 DNA is stained with a second chromogen, and chromosome 17 is stained with a third chromogen. In some embodiments, the first chromogen comprises fast red, the second chromogen comprises silver, and the third chromogen comprises a green chromogen component. In some embodiments, more than 50% of the nuclei have enumerable signals for chromosome 17, and wherein each enumerable signal is a generally round shape, a round shape is a shape defined by a simple closed curve fitting within a first region, the first region is an area on and between an inner concentric circle and an outer concentric circle, the inner concentric circle having an inner radius (Rᵢₙ) and the outer concentric circle having a outer radius (Rₒᵤₜ) wherein Rᵢₙ is ≥ 50% of Rₒᵤₜ, and the simple closed curve has a radius Rₛᵢₘₚₗₑ wherein Rᵢₙ ≤ Rₛᵢₘₚₗₑ ≤ Rₒᵤₜ.

The foregoing and other features of the disclosure will become more apparent from the following detailed description, which proceeds with reference to the accompanying figures.

### BRIEF DESCRIPTION OF THE DRAWINGS

FIGS. 1 and 2 show dual ISH assays for 17p- (TP53/Chr17), 13q-(DLEU/13q), Trisomy 12, and 11q- (ATM/Chr11). Ovals indicate exemplary stained cells. Dashed arrows indicate red dots, which correspond to Chr17, 13q, Chr12, and 11q probes labeled with DIG. Solid arrows indicate black dots, which correspond with TP53, DLEU, and ATM probes labeled with DNP.
FIG. 3 is a Whole Blood Smear 17p- dual Chromogenic ISH assay. Solid arrows indicate black dots, which are TP53 nucleic acid probes labeled with DNP. Dashed arrows indicate red dots, which are Chr15 nucleic acid probes labeled with DIG.

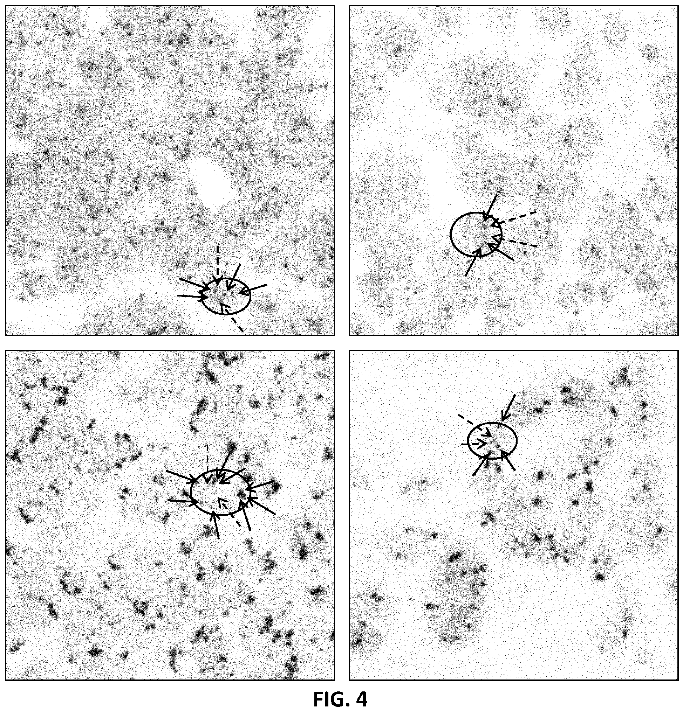

FIG. 4 shows a 19q12 Dual Chromogenic ISH assay. Ovals indicate exemplary stained cells. Dashed arrows indicate red dots, which correspond to Chr17, 13q, Chr12, and 11q probes labeled with DIG. Solid arrows indicate black dots, which correspond with 19q12 nucleic acid probes labeled with DNP. Dashed arrows indicate red spots, which correspond with INSR nucleic acid probes labeled with DIG.
FIG. 5 shows a round shape defined by a simple closed curve fitting within a first region. The first region is an area on and between an inner concentric circle and an outer concentric circle. The inner concentric circle has an inner radius (Rᵢₙ) and the outer concentric circle has an outer radius (Rₒᵤₜ). Rᵢₙ is ≥ 50% of Rₒᵤₜ. The simple closed curve has a radius Rₛᵢₘₚₗₑ wherein Rᵢₙ ≤ Rₛᵢₘₚₗₑ ≤ Rₒᵤₜ.
FIG. 6 shows a schematic representation of various steps used to stain CD79a, TP53 DNA, and centromere 17 DNA. The present invention is not limited to the markers, reagents, steps, or order of steps shown in FIG. 6.
FIG. 7 shows a gene-protein assay in normal bone marrow staining for CD79a (arrows pointing to regions of red stain), TP53 (arrows pointing to black dots), and chromosome 17 DNA (arrows pointing to diffuse blue/green dots).
FIG. 8 shows a gene-protein assay in a peripheral blood smear staining for CD79a (solid arrow, pointing to region of red stain), TP53 (dashed arrows pointing to black dots), and chromosome 17 DNA (dotted arrows pointing to diffuse blue/green dots). This sample has a homozygous 17p deletion.

### SEQUENCES

The nucleic acid sequences provided herein are shown using standard letter abbreviations for nucleotide bases. Only one strand of each nucleic acid sequence is shown, but the complementary strand is understood as included by any reference to the displayed strand. In the provided sequences:
SEQ ID NOs: 1-10 are nucleic acid sequences of probes including linked uniquely specific nucleic acid segments complementary to region 19q12 of the human genome.
SEQ ID NOs: 11-20 are nucleic acid sequences of probes including linked uniquely specific nucleic acid segments complementary to region 13q12 of the human genome.
SEQ ID NOs: 21-30 are nucleic acid sequences of probes including linked uniquely specific nucleic acid segments complementary to the human ATM gene.
SEQ ID NOs: 31-40 are nucleic acid sequences of probes including linked uniquely specific nucleic acid segments complementary to the human DLEU2 gene.
SEQ ID NOs: 41-50 are nucleic acid sequences of probes including linked uniquely specific nucleic acid segments complementary to the human INSR gene.
SEQ ID NOs: 51-60 are nucleic acid sequences of probes including linked uniquely specific nucleic acid segments complementary to the human TP53 gene.
SEQ ID NOs: 61-74 are nucleic acid sequences of probes to the human chromosome 17 centromere DNA.

### DETAILED DESCRIPTION

### I. Abbreviations

aCGH array comparative genomic hybridization
ATM ataxia telangiectasia mutated
AP alkaline phosphatase
CGH comparative genomic hybridization
CISH chromogenic *in situ* hybridization
DLEU2 deleted in lymphocytic leukemia 2 (non-protein coding)
DNP 2,4-dinitrophenyl
FISH fluorescent *in situ* hybridization
HRP horseradish peroxidase
INSR insulin receptor
ISH *in situ* hybridization
SISH silver *in situ* hybridization
TP53 tumor protein p53

### II. Terms

Unless otherwise explained, all technical and scientific terms used herein have the same meaning as commonly understood by one of ordinary skill in the art to which a disclosed invention belongs. The singular terms "a," "an," and "the" include plural referents unless context clearly indicates otherwise. Similarly, the word "or" is intended to include "and" unless the context clearly indicates otherwise. "Comprising" means "including." Hence "comprising A or B" means "including A" or "including B" or "including A and B."

Suitable methods and materials for the practice and/or testing of embodiments of the disclosure are described below. Such methods and materials are illustrative only and are not intended to be limiting. Other methods and materials similar or equivalent to those described herein can be used. For example, conventional methods well known in the art to which the disclosure pertains are described in various general and more specific references, including, for example, Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2000); Ausubel et al., Short Protocols in Molecular Biology: A Compendium of Methods from Current Protocols in Molecular Biology, 4th ed., Wiley & Sons, 1999; Harlow and Lane, Antibodies: A Laboratory Manual, Cold Spring Harbor Laboratory Press, 1990; and Harlow and Lane, Using Antibodies: A Laboratory Manual, Cold Spring Harbor Laboratory Press, 1999.

In case of conflict, the present specification, including explanations of terms, will control.

Although methods and materials similar or equivalent to those described herein can be used to practice or test the disclosed technology, suitable methods and materials are described below. The materials, methods, and examples are illustrative only and not intended to be limiting.

In order to facilitate review of the various embodiments of the disclosure, the following explanations of specific terms are provided:

**Antibody:** A polypeptide that includes at least a light chain or heavy chain immunoglobulin variable region and specifically binds an epitope of an antigen (such as CD79a protein). Antibodies include monoclonal antibodies, polyclonal antibodies, or fragments of antibodies. An antibody can be conjugated or otherwise labeled with a detectable label, such as an enzyme, hapten, or fluorophore.

**Detectable label:** A compound or composition that is conjugated directly or indirectly to another molecule (such as a nucleic acid probe) to facilitate detection of that molecule. Specific, non-limiting examples of labels include fluorescent and fluorogenic moieties, chromogenic moieties, haptens, affinity tags, and radioactive isotopes. The label can be directly detectable (e.g., optically detectable) or indirectly detectable (for example, via interaction with one or more additional molecules that are in turn detectable). Exemplary labels in the context of the probes disclosed herein are described below. Methods for labeling nucleic acids, and guidance in the choice of labels useful for various purposes, are discussed, e.g., in Sambrook and Russell, in Molecular Cloning: A Laboratory Manual, 3rd Ed., Cold Spring Harbor Laboratory Press (2001) and Ausubel et al., in Current Protocols in Molecular Biology, Greene Publishing Associates and Wiley-Intersciences (1987, and including updates).

Detectable labels may include chromogenic, fluorescent, phosphorescent and/or luminescent molecules, catalysts (such as enzymes) that convert one substance into another substance to provide a detectable signal (such as by converting a colorless substance into a colored substance or vice versa, or by producing a precipitate or increasing sample turbidity), haptens that can be detected through antibody-hapten binding interactions using additional detectably labeled antibody conjugates, and paramagnetic and magnetic molecules or materials. Particular examples of detectable labels include: enzymes, such as horseradish peroxidase, alkaline phosphatase, acid phosphatase, glucose oxidase, β-galactosidase or β-glucuronidase; fluorophores, such as fluoresceins, luminophores, coumarins, BODIPY dyes, resorufins, and rhodamines (many additional examples of fluorescent molecules can be found in The Handbook - A Guide to Fluorescent Probes and Labeling Technologies, Molecular Probes, Eugene, OR); nanoparticles, such as quantum dots (U.S. Patent Nos. 6,815,064, 6,682596 and 6,649,138); metal chelates, such as DOTA and DPTA chelates of radioactive or paramagnetic metal ions like Gd3+; and liposomes, for example, liposomes containing trapped fluorescent molecules. Where the detectable label includes an enzyme, a detectable substrate such as a chromogen, a fluorogenic compound, or a luminogenic compound is used in combination with the enzyme to generate a detectable signal (a wide variety of such compounds are commercially available, for example, from Life Technologies, Carlsbad, CA).

Alternatively, an enzyme can be used in a metallographic detection scheme. In some examples, metallographic detection methods include using an enzyme, such as alkaline phosphatase, in combination with a water-soluble metal ion and a redox-inactive substrate of the enzyme. The substrate is converted to a redox-active agent by the enzyme, and the redox-active agent reduces the metal ion, causing it to form a detectable precipitate (see, for example, U.S. Pat. Nos. 7,642,064; 7,632,652). In other examples, metallographic detection methods include using an oxido-reductase enzyme (such as horseradish peroxidase) along with a water soluble metal ion, an oxidizing agent and a reducing agent, again to form a detectable precipitate (see, for example, U.S. Patent No. 6,670,113). Haptens are small molecules that can be bound by antibodies. Exemplary haptens include dinitrophenyl (DNP), biotin, digoxigenin (DIG), and fluorescein. Additional haptens include oxazole, pyrazole, thiazole, nitroaryl, benzofuran, triperpene, urea, thiourea, rotenoid, coumarin and cyclolignan haptens, such as those disclosed in U.S. Pat. No. 7,695,929.

**Hybridization:** To form base pairs between complementary regions of two strands of DNA, RNA, or between DNA and RNA, thereby forming a duplex molecule. Hybridization conditions resulting in particular degrees of stringency will vary depending upon the nature of the hybridization method and the composition and length of the hybridizing nucleic acid sequences. Generally, the temperature of hybridization and the ionic strength (such as the Na+ concentration) of the hybridization buffer will determine the stringency of hybridization. The presence of a chemical which decreases hybridization (such as formamide) in the hybridization buffer will also determine the stringency (Sadhu et al., J. Biosci. 6:817-821, 1984). Calculations regarding hybridization conditions for attaining particular degrees of stringency are discussed in Sambrook et al., (1989) Molecular Cloning, second edition, Cold Spring Harbor Laboratory, Plainview, NY (chapters 9 and 11). Hybridization conditions for ISH are also discussed in Landegent et al., Hum. Genet. 77:366-370, 1987; Lichter et al., Hum. Genet. 80:224-234, 1988; and Pinkel et al., Proc. Natl. Acad. Sci. USA 85:9138-9142, 1988.

**Immunohistochemistry (IHC):** A method of determining the presence or distribution of an antigen in a sample by detecting interaction of the antigen with a specific binding agent, such as an antibody. A sample is contacted with an antibody under conditions permitting antibody-antigen binding. Antibody-antigen binding can be detected by means of a detectable label conjugated to the antibody (direct detection) or by means of a detectable label conjugated to a secondary antibody, which binds specifically to the primary antibody (e.g., indirect detection).

***In situ* hybridization (ISH):** A method of determining the presence or distribution of a nucleic acid in a sample using hybridization of a labeled nucleic acid probe to localize a specific DNA or RNA sequence in a portion or section of tissue (*in situ*), or, if the tissue is small enough (e.g., plant seeds, Drosophila embryos), in the entire tissue (whole mount ISH). DNA ISH can be used to determine the structure of chromosomes, such as for use in medical diagnostics to assess chromosomal integrity and/or to determine gene copy number in a sample. RNA ISH measures and localizes mRNAs and other transcripts within tissue sections or whole mounts.

For ISH, sample cells and tissues are usually treated to fix the target nucleic acids in place and to increase access of the probe to the target molecule. The detectably labeled probe hybridizes to the target sequence at elevated temperature, and then the excess probe is washed away. Solution parameters, such as temperature, salt and/or detergent concentration, can be manipulated to remove any non-identical interactions (e.g., so only exact sequence matches will remain bound). Then, the labeled probe is localized and potentially quantitated in the tissue using either autoradiography, fluorescence microscopy or immunohistochemistry, respectively. ISH can also use two or more probes, which are typically differently labeled to simultaneously detect two or more nucleic acids.

**Intron:** An intron is any nucleotide region or sequence within a gene that is removed by RNA splicing during generation of a final mature RNA product of a gene. The term intron may refer to both the DNA sequence within a gene or the corresponding sequence in unprocessed RNA transcripts. Introns are found in the genes of most organisms, and can be located in a wide range of genes, including those that generate proteins, ribosomal RNA (rRNA), and transfer RNA (tRNA). When proteins are generated from intron-containing genes, RNA splicing takes place as part of the RNA processing pathway that follows transcription and precedes translation. The length of intron sequences is highly variable, ranging from less than 100 base pairs to tens of thousands or even hundreds of thousands of base pairs.

**Isolated:** An "isolated" biological component (such as a nucleic acid molecule, protein, or cell) has been substantially separated or purified away from other biological components in a preparation, a cell of an organism, or the organism itself, in which the component occurs, such as other chromosomal and extra-chromosomal DNA and RNA, proteins and cells. Nucleic acid molecules and proteins that have been "isolated" include nucleic acid molecules and proteins purified by standard purification methods. The term also embraces nucleic acid molecules and proteins prepared by recombinant expression in a host cell as well as chemically synthesized nucleic acid molecules and proteins. In some examples, the nucleic acid probes disclosed herein are isolated nucleic acid probes.

### TP53 tumor protein p53

The protein encoded by this gene is a tumor suppressor protein containing transcriptional activation, DNA binding, and oligomerization domains. It responds to cellular stresses and regulates expression of target genes with the effect of inducing cell cycle arrest, apoptosis, senescence, DNA repair, or changes in metabolism. A variety of human cancers are associated with mutations in this gene. A TP53 probe may be used with a Chromosome 17 centromere probe.

### ATM ataxia-telangiectasia mutated gene

The protein encoded by this gene is a member of the phosphatidylinositol 3-kinase family. These proteins respond to DNA damage by phosphorylating substrates involved in DNA repair and/or cell cycle control. Reference is made to Savitsky et al. (Science 268: 1749-1753, 1995) which suggested that an increased risk of cancer may be associated with aberrations at this gene.

### DLEU2 deleted in lymphocytic leukemia 1 (non-protein coding)

The DLEU2 gene is located in region 13q14 and is a non-coding region originally identified as a potential tumor suppressor gene and that may be deleted in patients with B-cell chronic lymphocytic leukemia.

### INSR insulin receptor

The INSR gene encodes for an insulin receptor comprising a tetramer of two alpha and two beta subunits. The insulin receptor is a transmembrane receptor that is activated by insulin, IGF-I, IGF-II. The insulin receptor plays a key role in the regulation of glucose homeostasis, a functional process that under degenerate conditions may result in a range of clinical manifestations including diabetes and cancer.

### 19q12 amplicon

The 19q12 locus is amplified in many cancer types including ovarian, breast and colon. Several genes are encoded within the region including CCNE1 and URI, which have been implicated as potential "drivers" of cancer cell survival. Each of these targets may provide valuable prognostic/predictive information for patient care.

### 13q12 region

The 13q12 region was selected as a highly conserved and uniquely distinct region of chromosome 13 that can serve as a surrogate for a centromere probe for chromosome 13. Chromosome 13 is known to being highly repetitive with other centromeres and thus a probe specific to a typically unamplified region near the centromere of chromosome 13 is desirable.

**Probe:** A nucleic acid molecule that is capable of hybridizing with a target nucleic acid molecule (e.g., genomic target nucleic acid molecule) and, when hybridized to the target, is capable of being detected either directly or indirectly. Thus probes permit the detection, and in some examples quantification, of a target nucleic acid molecule. In particular examples, a probe includes at least two segments complementary to uniquely specific nucleic acid sequences of a target nucleic acid molecule and are thus capable of specifically hybridizing to at least a portion of the target nucleic acid molecule. Generally, once at least one segment or portion of a segment has (and remains) hybridized to the target nucleic acid molecule other portions of the probe may (but need not) be physically constrained from hybridizing to those other portions' cognate binding sites in the target (e.g., such other portions are too far distant from their cognate binding sites); however, other nucleic acid molecules present in the probe can bind to one another, thus amplifying signal from the probe. A probe can be referred to as a "labeled nucleic acid probe," indicating that the probe is coupled directly or indirectly to a detectable moiety or "label," which renders the probe detectable.

**Sample:** A specimen containing DNA (for example, genomic DNA), RNA (including mRNA), protein, or combinations thereof, obtained from a subject. Examples include, but are not limited to, chromosomal preparations, peripheral blood, urine, saliva, tissue biopsy, fine needle aspirate, surgical specimen, bone marrow, amniocentesis samples, and autopsy material. In one example, a sample includes genomic DNA. In some examples, the sample is a cytogenetic preparation, for example which can be placed on microscope slides. In particular examples, samples are used directly, or can be manipulated prior to use, for example, by fixing (e.g., using formalin).

**Sequence identity:** The identity (or similarity) between two or more nucleic acid sequences is expressed in terms of the identity or similarity between the sequences. Sequence identity can be measured in terms of percentage identity; the higher the percentage, the more identical the sequences are. Sequence similarity can be measured in terms of percentage similarity (which takes into account conservative amino acid substitutions); the higher the percentage, the more similar the sequences are.

Methods of alignment of sequences for comparison are well known in the art. Various programs and alignment algorithms are described in: Smith & Waterman, Adv. Appl. Math. 2:482, 1981; Needleman & Wunsch, J. Mol. Biol. 48:443, 1970; Pearson & Lipman, Proc. Natl. Acad. Sci. USA 85:2444, 1988; Higgins & Sharp, Gene, 73:237-44, 1988; Higgins & Sharp, CABIOS 5:151-3, 1989; Corpet et al., Nuc. Acids Res. 16:10881-90, 1988; Huang et al. Computer Appls. in the Biosciences 8:155-65, 1992; and Pearson et al., Meth. Mol. Bio. 24:307-31, 1994. Altschul et al., J. Mol. Biol. 215:403-10, 1990, presents a detailed consideration of sequence alignment methods and homology calculations.

The NCBI Basic Local Alignment Search Tool (BLAST) (Altschul et al., J. Mol. Biol. 215:403-10, 1990) is available from several sources, including the National Center for Biotechnology and on the Internet, for use in connection with the sequence analysis programs blastp, blastn, blastx, tblastn and tblastx. Additional information can be found at the NCBI web site.

BLASTN may be used to compare nucleic acid sequences, while BLASTP may be used to compare amino acid sequences. If the two compared sequences share homology, then the designated output file will present those regions of homology as aligned sequences. If the two compared sequences do not share homology, then the designated output file will not present aligned sequences.

The BLAST-like alignment tool (BLAT) may also be used to compare nucleic acid sequences (Kent, Genome Res. 12:656-664, 2002). BLAT is available from several sources, including Kent Informatics (Santa Cruz, CA) and on the Internet (genome.ucsc.edu).

Once aligned, the number of matches is determined by counting the number of positions where an identical nucleotide or amino acid residue is presented in both sequences. The percent sequence identity is determined by dividing the number of matches either by the length of the sequence set forth in the identified sequence, or by an articulated length (such as 100 consecutive nucleotides or amino acid residues from a sequence set forth in an identified sequence), followed by multiplying the resulting value by 100. For example, a nucleic acid sequence that has 1166 matches when aligned with a test sequence having 1554 nucleotides is 75.0 percent identical to the test sequence (1166÷1554*100=75.0). The percent sequence identity value is rounded to the nearest tenth. For example, 75.11, 75.12, 75.13, and 75.14 are rounded down to 75.1, while 75.15, 75.16, 75.17, 75.18, and 75.19 are rounded up to 75.2. The length value will always be an integer. In another example, a target sequence containing a 20-nucleotide region that aligns with 15 consecutive nucleotides from an identified sequence as follows contains a region that shares 75 percent sequence identity to that identified sequence (that is, 15÷20*100=75).

**Subject:** Any multi-cellular vertebrate organism, such as human or non-human mammals (e.g., veterinary subjects).

**Target nucleic acid sequence or molecule:** A defined region or particular portion of a nucleic acid molecule, for example a portion of a genome (such as a gene or a region of mammalian genomic DNA containing a gene of interest). In an example where the target nucleic acid sequence is a target genomic sequence, such a target can be defined by its position on a chromosome (e.g., in a normal cell), for example, according to cytogenetic nomenclature by reference to a particular location on a chromosome; by reference to its location on a genetic map; by reference to a hypothetical or assembled contig; by its specific sequence or function; by its gene or protein name; or by any other means that uniquely identifies it from among other genetic sequences of a genome. In some examples, the target nucleic acid sequence is mammalian genomic sequence (for example human genomic sequence).

In some examples, alterations of a target nucleic acid sequence (e.g., genomic nucleic acid sequence) are "associated with" a disease or condition. In some examples, detection of the target nucleic acid sequence can be used to infer the status of a sample with respect to the disease or condition. For example, the target nucleic acid sequence can exist in two (or more) distinguishable forms, such that a first form correlates with absence of a disease or condition and a second (or different) form correlates with the presence of the disease or condition. The two different forms can be qualitatively distinguishable, such as by polynucleotide polymorphisms, and/or the two different forms can be quantitatively distinguishable, such as by the number of copies of the target nucleic acid sequence that are present in a cell.

**Topoisomerase II alpha (TOP2A):** DNA topoisomerases (EC 5.99.1.3) are enzymes that control and alter the topologic states of DNA in both prokaryotes and eukaryotes. Topoisomerase II from eukaryotic cells catalyzes the relaxation of supercoiled DNA molecules, catenation, decatenation, knotting, and unknotting of circular DNA. The reaction catalyzed by topoisomerase II likely involves the crossing-over of two DNA segments. The gene encoding topoisomerase II in humans is present at cytogenetic location: 17q21.2 and has genomic coordinates (GRCh37): 17:38,544,772 - 38,574,201. Tsai-Pflugfelder et al. determined the entire coding sequence of the human TOP2 gene as early as 1988 (Proc. Natl. Acad. Sci. USA 85:7177-7181, 1988). Lang et al. reported the complete structures of the human INSR and TOP2B genes in 1998 (Gene 221:255-266, 1998). The human INSR gene spans approximately 30 kb and contains 35 exons.

**Uniquely specific sequence:** A nucleic acid sequence (for example, a sequence of at least of at least 20 bp (such as at least 20 bp, 30 bp, 40 bp, 50 bp, 60 bp, 70 bp, 80 bp, 90 bp, 100 bp, or more) that is present only one time in a haploid genome of an organism. In a particular example, a uniquely specific nucleic acid sequence is a nucleic acid sequence from a target nucleic acid that has 100% sequence identity with the target nucleic acid and has no significant identity to any other nucleic acid sequences present in the specific haploid genome that includes the target nucleic acid.

**Vector:** Any nucleic acid that acts as a carrier for other ("foreign") nucleic acid sequences that are not native to the vector. When introduced into an appropriate host cell a vector may replicate itself (and, thereby, the foreign nucleic acid sequence) or express at least a portion of the foreign nucleic acid sequence. In one context, a vector is a linear or circular nucleic acid into which a nucleic acid sequence of interest is introduced (for example, cloned) for the purpose of replication (e.g., production) and/or manipulation using standard recombinant nucleic acid techniques (e.g., restriction digestion). A vector can include nucleic acid sequences that permit it to replicate in a host cell, such as an origin of replication. A vector can also include one or more selectable marker genes and other genetic elements known in the art. Common vectors include, for example, plasmids, cosmids, phage, phagemids, artificial chromosomes (e.g., BAC, PAC, HAC, YAC), and hybrids that incorporate features of more than one of these types of vectors. Typically, a vector includes one or more unique restriction sites (and in some cases a multi-cloning site) to facilitate insertion of a target nucleic acid sequence.

### III. Multiplex Gene-Protein Assays

Disclosed herein are methods for co-detecting multiple target molecules, such as at least one gene and at least one protein (e.g., one protein and two genes, etc.), in a single sample. In particular embodiments, the methods of the present invention disclose multiplex gene-protein assays for detecting CD79a protein, TP53 genomic DNA, and chromosome 17 centromere DNA.

In some embodiments of the methods, a sample is stained for CD79a protein by contacting the sample with a CD79a protein-specific antibody, and then the sample is contacted with a means (e.g., a first chromogen component) for detecting the CD79a protein-specific antibody. In one embodiment, the sample is stained for CD79a protein by contacting the sample with an antibody specific for CD79a, and then the sample is contacted with a first chromogen component for visualizing the CD79a protein, e.g., the first chromogen component is adapted to emit or make visible a first color corresponding to the CD79a protein. In some embodiments of the methods, the sample is stained for TP53 DNA and chromosome 17 DNA by contacting the sample with a TP53 DNA-specific nucleic acid probe and a chromosome 17 DNA-specific nucleic acid probe (e.g., chromosome 17 centromere probe). The sample may then be contacted with a second chromogen component for visualizing the TP53 DNA and a third chromogen component for visualizing chromosome 17 DNA. For example, the second chromogen component is adapted to emit or make visible a second color corresponding to TP53 DNA, and the third chromogen component is adapted to emit or make visible a third color corresponding to the chromosome 17 DNA.

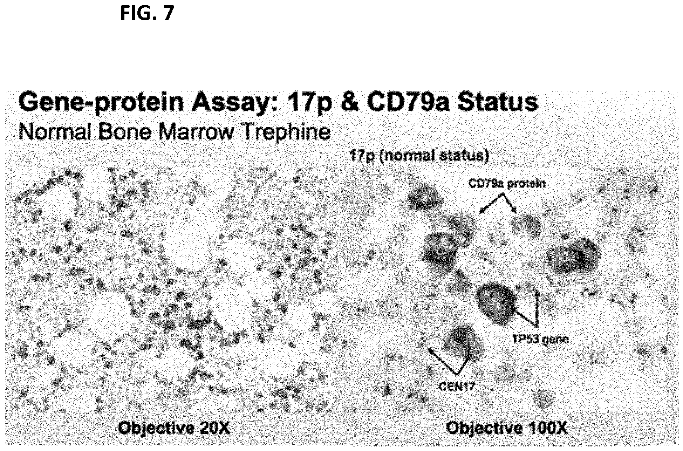

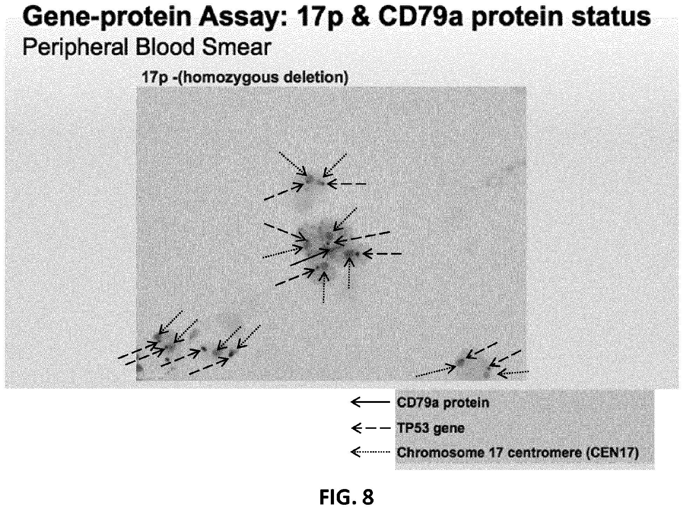

FIG. 7 shows a gene-protein assay of a normal bone marrow sample stained for CD79a (red), TP53 (black), and chromosome 17 (blue/green). FIG. 8 shows a gene-protein assay of a blood sample stained for CD79a (red), TP53 (black), and chromosome 17 (blue/green). That sample shows a homozygous 17p deletion.

Samples may be blood samples, however, the samples are not limited to blood. In some examples, the sample comprises tissue, bone marrow, or any other appropriate sample material. Samples are further discussed below.

The methods may utilize different detectable labels and/or detection systems for each of the targets such that each can be individually detected in a single sample. For example, in some embodiments, the CD79a protein is stained with a first chromogen (producing a first color), TP53 is stained with a second chromogen (producing a second color), and chromosome 17 is stained with a third chromogen (producing a third color). The proteins/DNA may be detected by the chromogens using additional reagents such as secondary antibodies specific for the primary antibodies. The first color is transparent enough to allow visualization of the second color and/or third color. In some examples, the first color blocks no more than 50%, no more than 40%, no more than 30%, no more than 20%, no more than 10%, no more than 8%, no more than 6%, no more than 4%, no more than 2%, or none of the intensity of the second color and/or the third color. Detection includes but is not limited to bright field microscopy.

In one embodiment, the first chromogen component comprises fast red, the second chromogen component comprises silver, and the third chromogen component comprises a green chromogen component.

In some embodiments, the step of staining the CD79a protein is performed before the step of staining DNA. For example, the step of staining the B CD79a protein is performed before the step of staining TP53 genomic DNA and staining chromosome 17 centromere DNA.

CD79a antibodies are known in the art and are commercially available. In one example, the sample is contacted with an anti-CD79a rabbit monoclonal antibody, such as the anti-CD79a SP18 rabbit monoclonal antibody (Ventana Medical Systems, Inc., Tucson, AZ, e.g., catalog number 790-4432). In some embodiments, the CD79a-specific antibody is detectably labeled (the label may be referred to as the first chromogen component), allowing for detection of CD79a protein in the sample. In some embodiments, after contacting the sample with the CD79a-specific antibody (the primary antibody), the sample is contacted with a detectably labeled secondary antibody against the primary antibody, such as a secondary antibody conjugated to an enzyme (e.g., alkaline phosphatase (AP), horseradish peroxidase (HRP), etc.) or a secondary antibody conjugated to a hapten that can be detected with a further reagent conjugated to an enzyme. The presence of the CD79a protein is detected by contacting the enzyme with a chromogen and/or substrate composition, which produced a colored precipitate (first color) in the vicinity of the CD79a-specific antibody.

In some examples, the sample is contacted with a CD79a-specific antibody; the sample is then contacted with an AP-conjugated secondary antibody that specifically binds the primary antibody, for example under conditions sufficient for the secondary antibody to specifically bind to the primary antibody. The sample is then contacted with a naphthol phosphate and Fast Red chromogen, which produces a red precipitate near the anti-CD79a antibody (and CD79a protein) that can be visually detected by light microscopy. In one example, the reagents (except for the anti- CD79a antibody) are included in a kit, such as the ULTRAVIEW Universal Alkaline Phosphatase Red Detection Kit (Ventana Medical Systems, Tucson, AZ, catalog number 760-501). One of ordinary skill in the art can select alternative detection reagents (such as alternative secondary antibodies, enzymes, substrates, and/or chromogens) including those that produce a different color precipitate for detection of the CD79a.

In some examples of the disclosed methods, the sample is contacted with a nucleic acid probe that specifically binds to TP53 genomic DNA. Methods of constructing TP53-specific nucleic acid probes are known to one of ordinary skill in the art, and TP53 -specific nucleic acid probes are disclosed herein (below). In one example, the sample is contacted with a hapten-labeled TP53 nucleic acid probe, for example under conditions specific for the probe to specifically bind to (hybridize with) TP53 genomic DNA in the sample. The sample is then contacted with an antibody that specifically binds to the hapten, for example, under conditions sufficient for the antibody to specifically bind to the hapten. The antibody may be conjugated to an enzyme (such as AP or HRP) or alternatively, the sample may be contacted with a second antibody that specifically binds the anti-hapten antibody, where the second antibody is conjugated to an enzyme. The presence of TP53 genomic DNA is detected by contacting the enzyme with a chromogen and/or substrate composition to produce a colored precipitate in the vicinity of the TP53 nucleic acid probe. In some examples, the number of TP53 DNA copies in the sample is scored by a slide reader by counting the number of areas of precipitate ("spots") in the nuclei of the tumor cells.

In one particular example, the method includes contacting the sample with a TP53 genomic DNA probe conjugated to dinitrophenyl (DNP), for example under conditions sufficient for the TP53 probe to specifically bind to TP53 genomic DNA in the sample. The sample is then contacted with an anti-hapten antibody that specifically binds DNP, for example under conditions sufficient for the anti-hapten antibody to specifically bind to the DNP. The sample is then contacted with an HRP-conjugated secondary antibody that specifically binds to the anti-hapten antibody, for example under conditions sufficient for the secondary antibody to specifically bind to the anti-DNP antibody. The sample is then contacted with silver acetate, hydroquinone, and hydrogen peroxide. The silver ions are reduced by hydroquinone to metallic silver ions, which can be visually detected by light microscopy as black spots. In one example, the reagents (except for the TP53 probe) are included in a kit, such as the ULTRAVIEW SISH DNP Detection Kit (Ventana Medical Systems, Tucson, AZ, catalog number 760-098). One of ordinary skill in the art can select alternative detection reagents (such as alternative haptens, antibodies, enzymes, substrates, and/or chromogens) including those that produce a different color precipitate for detection of TP53 genomic DNA.

Examples of TP53 probes are disclosed herein (see below). Briefly, in some embodiments, the TP53 DNA-specific nucleic acid probe comprises a nucleic acid molecule having at least 90% sequence identity with the sequence according to any one of SEQ ID NOs: 51-60; or a nucleic acid molecule having at least 90% sequence identity with at least 250 contiguous nucleotides of any one of SEQ ID NOs: 51-60. In some embodiments, the TP53 DNA-specific nucleic acid probe comprises a nucleic acid molecule having at least 95% sequence identity with the sequence according to any one of SEQ ID NOs: 51-60; or a nucleic acid molecule having at least 95% sequence identity with at least 250 contiguous nucleotides of any one of SEQ ID NOs: 51-60. In some embodiments, the TP53 DNA-specific nucleic acid probe comprises a nucleic acid molecule having at least 99% sequence identity to the sequence according to any one of SEQ ID NOs: 51-60; or a nucleic acid molecule having at least 99% sequence identity with at least 250 contiguous nucleotides of any one of SEQ ID NOs: 51-60. In some embodiments, the TP53 DNA-specific nucleic acid probe comprises a nucleic acid molecule consisting of the sequence according to any one of SEQ ID NOs: 51-60; or a nucleic acid molecule consisting of at least 250 contiguous nucleotides of any one of SEQ ID NOs: 51-60. In some embodiments, the TP53 DNA-specific nucleic acid probe comprises a nucleic acid molecule with at least 90% sequence identity to at least 400 contiguous of any one of SEQ ID NOs: 51-60, at least 500 contiguous nucleotides of any one of SEQ ID NOs: 51-60, at least 1000 contiguous nucleotides of any one of SEQ ID NOs: 51-60, at least 2500 contiguous nucleotides of any one of SEQ ID NOs: 51-60, etc. In some embodiments, the TP53 DNA-specific nucleic acid probe comprises two or more portions, wherein the first portion comprises at least 250 contiguous nucleotides of a nucleic acid sequence with at least 90% sequence identity to one of SEQ ID NOs: 51-60; and the second portion comprises at least 250 contiguous nucleotides of a nucleic acid with at least 90% sequence identity to one of SEQ ID NOs: 51-60, wherein the first and second portions are different from one another. In some embodiments, the TP53 DNA-specific nucleic acid probe is at least 500 nucleotides in length, at least 1000 nucleotides in length, at least 5000 nucleotides in length, etc. In some embodiments, the TP53 DNA-specific nucleic acid probe comprises at least two of the probes disclosed herein.

In some examples of the disclosed methods, the sample is contacted with a nucleic acid probe that specifically binds to chromosome 17 centromere DNA. Methods of constructing chromosome 17 centromere-specific nucleic acid probes are known to one of ordinary skill in the art. In addition, chromosome 17 centromere nucleic acid probes may also be commercially available. For example, a chromosome 17 centromere probe suitable for use in the disclosed methods includes the chromosome 17 centromere probe included in the INFORM HER2 Dual ISH Probe Cocktail (Ventana Medical Systems, Tucson, AZ, catalog number 780-4422). In one example, the sample is contacted with a hapten-labeled chromosome 17 centromere nucleic acid probe, for example under conditions specific for the probe to specifically bind to (hybridize with) chromosome 17 centromere genomic DNA in the sample. The sample is then contacted with an antibody that specifically binds to the hapten, for example, under conditions sufficient for the antibody to specifically bind to the hapten. The antibody may be conjugated to an enzyme (such as AP or HRP) or alternatively, the sample may be contacted with a second antibody that specifically binds the anti-hapten antibody, where the second antibody is conjugated to an enzyme. The presence of chromosome 17 centromere genomic DNA is detected by contacting the enzyme with a chromogen and/or substrate composition to produce a colored precipitate (third color) in the vicinity of the chromosome 17 centromere nucleic acid probe. In some examples, the gene copy number of chromosome 17 centromere DNA in the sample is scored by a slide reader by counting the number of areas of precipitate ("spots") in the nuclei of the tumor cells.

In a particular example, the method comprises contacting the sample with a chromosome 17 centromere DNA probe conjugated to digoxigenin (DIG), for example under conditions sufficient for the chromosome 17 centromere probe to specifically bind to chromosome 17 centromere DNA in the sample. The sample is then contacted with an anti-hapten antibody that specifically binds DIG, for example under conditions sufficient for the anti-DIG antibody to specifically bind to the DIG. The sample is then contacted with a HRP-conjugated secondary antibody that specifically binds to the anti-DIG antibody, for example under conditions sufficient for the secondary antibody to specifically bind to the anti-DIG antibody. The sample is then contacted with a green chromogen component, producing a green/blue precipitate which is deposited in the nuclei near the chromosome 17 centromere probe (and the chromosome 17 centromere DNA) and can be visually detected by light microscopy as green/blue spots. In one example, the green chromogen component is HRP-Green (42 Lifescience, Bremerhaven, Germany, catalog number S-99056). One of ordinary skill in the art can select alternative detection reagents (such as alternative haptens, antibodies, enzymes, substrates, and/or chromogens) including those that produce a different color precipitate for detection of chromosome 17 centromere DNA.

In some embodiments, the chromosome 17 centromere-specific nucleic acid probe comprises a set of two or more single-stranded oligonucleotide control probes. The chromosome 17 oligonucleotide control probes are specific for two or more (between 2 and 14, e.g., 2, 3, 4, 5, 6, 7, 8, 9, 10, 11, 12, 13, 14, ≥ 4, ≥ 6, ≥ 8, etc.) distinct monomers of the alpha satellite control region of chromosome 17. In some embodiments, the chromosome 17 oligonucleotide probes (control probes) each comprise between 50 to 100 nucleotides.

In some embodiments, the chromosome 17 oligonucleotide control probes (each control probe) may comprise one of SEQ ID NOs: 61-74 (or complements thereof) (see below in Table 1). In some embodiments, the chromosome 17 oligonucleotide control probes (each control probe) may comprise a truncated version of one of SEQ ID NOs: 61-74 (or complements thereof). The truncated version may be at least 30 contiguous base pairs of said sequence, at least 40 contiguous base pairs of said sequence, or at least 50 contiguous base pairs of said sequence. In some embodiments, the chromosome 17 oligonucleotide control probes (each control probe) may comprise a sequence that has at least 70%, at least 80%, at least 90%, or at least 95% sequence identity to one of SEQ ID NOs: 61-74 (or complements thereof).

**Table 1 - Single-stranded Oligonucleotide Probes for Chromosome 17**

| **Oligo name** | **Sequences** | **Length** |
|---|---|---|
| SEQ ID. NO. 61 | | 79 |
| SEQ ID. NO. 62 | | 79 |
| SEQ ID. NO. 63 | | 79 |
| SEQ ID. NO. 64 | | 79 |
| SEQ ID. NO. 65 | | 83 |
| SEQ ID. NO. 66 | | 87 |
| SEQ ID. NO. 67 | | 71 |
| SEQ ID. NO. 68 | | 58 |
| SEQ ID. NO. 69 | | 65 |
| SEQ ID. NO. 70 | | 71 |
| SEQ ID. NO. 71 | | 80 |
| SEQ ID. NO. 72 | | 80 |
| SEQ ID. NO. 73 | | 80 |
| SEQ ID. NO. 74 | | 80 |

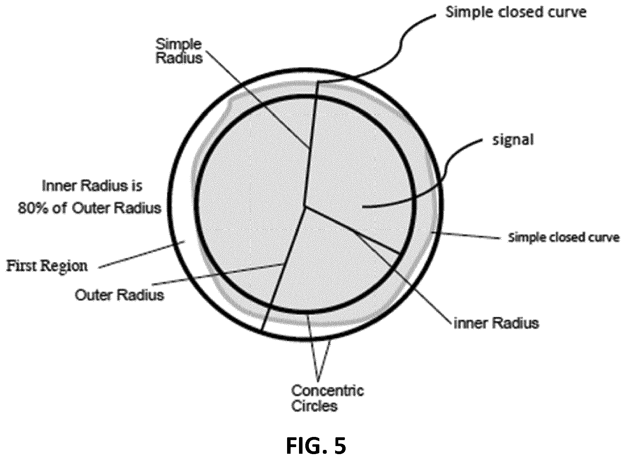

The chromosome 17 centromere oligonucleotide probes (control probes) can achieve an enumerable signal when hybridized to its DNA target. An enumerable signal has a generally round shape. In some examples, a round shape is a shape defined by a simple closed curve (see FIG. 5) fitting within a first region. The first region is an area on and between an inner concentric circle and an outer concentric circle. The inner concentric circle has an inner radius (Rᵢₙ) and the outer concentric circle has a outer radius (Rₒᵤₜ). Rᵢₙ is ≥ 50% of Rₒᵤₜ, and the simple closed curve has a radius Rₛᵢₘₚₗₑ wherein Rᵢₙ ≤ Rₛᵢₘₚₗₑ ≤ Rₒᵤₜ.

The chromosome 17 oligonucleotide control probes may be hybridized under conditions for a period of time less than about 3 hours, less than about 2 hours, 1 hour, or less than about an hour. The chromosome 17 centromere oligonucleotide probes (control probes) may achieve at least two enumerable signals per cell, e.g., with a staining intensity of ≥2 and staining coverage of ≥50% of the number of total nuclei within 3 hours of hybridization (or within 2 hours of hybridization, or within 1 hour of hybridization). The chromosome 17 centromere oligonucleotide probes may be configured to hybridize uniquely and specifically to a portion of the control region of human chromosome 17 so that other chromosomes or portions thereof are not evidently labeled without the influence of blocking DNA. In some examples, the chromosome 17 oligonucleotide control probes each comprise between 50 to 100 nucleotides.

The chromosome 17 oligonucleotide control probes may each comprise a detectable label, e.g., a hapten (e.g., dinitrophenyl, digoxigenin, biotin, or fluorescein, etc.). The labeled chromosome 17 oligonucleotide probes may be detected as previously described, e.g., with a secondary antibody directed to the hapten and/or with other detection components and reagents. For example, in a particular example, the method comprises contacting the sample with a chromosome 17 oligonucleotide control probes conjugated to digoxigenin (DIG), for example under conditions sufficient for the chromosome 17 oligonucleotide control probes to specifically bind to chromosome 17 centromere DNA in the sample. The sample is then contacted with an anti-hapten antibody that specifically binds DIG, for example under conditions sufficient for the anti-DIG antibody to specifically bind to the DIG. The sample is then contacted with a HRP-conjugated secondary antibody that specifically binds to the anti-DIG antibody, for example under conditions sufficient for the secondary antibody to specifically bind to the anti-DIG antibody. The sample is then contacted with a green chromogen component, producing a green/blue precipitate which is deposited in the nuclei near the chromosome 17 oligonucleotide control probes (and the chromosome 17 centromere DNA) and can be visually detected by light microscopy as green/blue spots. In one example, the green chromogen component comprises HRP-Green (42 Lifescience, Bremerhaven, Germany, catalog number S-99056). One of ordinary skill in the art can select alternative detection reagents (such as alternative haptens, antibodies, enzymes, substrates, and/or chromogens) including those that produce a different color precipitate for detection of chromosome 17 centromere DNA.

The disclosed methods are directed to detection of multiple protein and/or nucleic acid targets in a single sample. As a result, the detectable signal for each member of the assay must be individually distinguishable. Therefore, in some examples, the visual signal generated by the detection assay for each member of the assay is a different color. In one specific example, the methods result in a red staining for CD79a protein, black staining for TP53 DNA (for example, black spots in the nucleus, such as individually distinguishable black spots or clusters of black spots), and green/blue staining for chromosome 17 centromere DNA (for example, green/blue spots in the nucleus, such as individually distinguishable green/blue spots or clusters of green/blue spots). However, other combinations may be used.

The methods disclosed herein may also include steps for pretreatment of samples (e.g., blood samples) prior to or between the steps including contacting the sample with a CD79a-specific antibody, a TP53-specific nucleic acid probe, and a chromosome 17 centromere-specific nucleic acid probe. These steps are known to one of ordinary skill in the art and may include deparaffinization of a sample (such as a FFPE sample), cell conditioning, washes, and so on. One of skill in the art can make adjustments to these conditions (for example, minor adjustments to times and/or temperatures of incubations, wash steps, etc.).

In some embodiments, the sample is subjected to a protease treatment (proteinase K, pepsin, collagenase, dispase, a combination thereof, etc.) after the step of staining CD79a protein but before the step of staining TP53 genomic DNA and staining chromosome 17 centromere DNA. The protease treatment is effective to allow for hybridization of the nucleic acid probes to their respective DNA targets. The protease treatment does not eliminate the first color and tissue morphology is sufficiently maintained so as to allow for the detection of the first color. The sample may be subjected to a heat treatment after the step of staining the B cell marker but before the protease treatment.

The present invention also features a slide (single slide) comprising a sample of cells chromogenically stained for CD79a protein, TP53 DNA, and chromosome 17 DNA. The markers may each be stained with a different chromogen, e.g., CD79a protein is stained with a first chromogen, TP53 DNA is stained with a second chromogen, and chromosome 17 is stained with a third chromogen. In some embodiments, the first chromogen comprises fast red, the second chromogen comprises silver, and the third chromogen comprises a green chromogen component. In some embodiments, more than 50% of the nuclei have enumerable signals for chromosome 17. An enumerable signal may have a generally round shape. In some embodiments, a round shape is a shape defined by a simple closed curve fitting within a first region. The first region is an area on and between an inner concentric circle and an outer concentric circle. The inner concentric circle has an inner radius (Rᵢₙ) and the outer concentric circle has a outer radius (Rₒᵤₜ). Rᵢₙ is ≥ 50% of Rₒᵤₜ, and the simple closed curve has a radius Rₛᵢₘₚₗₑ wherein Rᵢₙ ≤ Rₛᵢₘₚₗₑ ≤ Rₒᵤₜ.

The disclosed methods can be automated. Systems for automated IHC and/or ISH are commercially available, such as the BENCHMARK ULTRA slide staining system, the BENCHMARK XT slide staining system, and the DISCOVERY XT slide staining system (Ventana Medical Systems, Tucson, AZ), BOND-MAX and BOND-III slide stainers (Leica Biosystems, Buffalo Grove, IL), and the IQ Kinetic slide stainer (Biocare Medical, Concord, CA). Ventana Medical Systems, Inc. is the assignee of a number of United States patents disclosing systems and methods for performing automated analyses, including U.S. Patent Nos. 5,650,327; 5,654,200; 6,296,809; 6,352,861; 6,582,962; 6,827,901 and 6,943,029.

Non-limiting examples of chromogens that may be used in the disclosed methods include (but are not limited to) those shown in Table 2. While not exhaustive, Table 2 provides insight into the varieties of presently available chromogens. Further illustrative chromogens include those described in U.S. Pat. Publ. 2013/0260379 and U.S. Prov. Pat. App. No. 61/831,552, filed June 5, 2013.

**Table 2 - Examples of commercially available chromogen/substrate systems**

| **Abbr.** | **Name** | **Color** | **Enzyme** |
|---|---|---|---|
| DAB | 3,3'-diamino-benzidine + H₂O₂ | brown - black | peroxidase |
| AEC | 3-amino-9-ethyl-carbazole + H₂O₂ | red | peroxidase |
| CN | 4-chloro-1-naphthol +H₂O₂ | blue | peroxidase |
| BCIP/NBT | 5-bromo-4-chloro-3-indolyl-phosphate + nitroblue tetrazolium | indigo - black | alkaline phosphatase |
| FAST RED | 4-chloro-2-methylbenzenediazonium + 3-hydroxy-2-naphthoic acid 2,4-dimethylanilide phosphate | red | alkaline phosphatase |
| FAST BLUE | Naphthol AS-MX phosphate disodium salt + fast blue BB salt hemi(zinc chloride) salt | blue | alkaline phosphatase |
| FUCHSIN | Naphthol AS-BI + New Fuchsin | red | alkaline phosphatase |
| NBT | nitroblue tetrazolium + phenazine methosulfate | blue -purple | dehydrogenase |
| ALK GOLD† | 3-methyl-1-phenyl-1H-pyrazol-5-yl dihydrogen phosphate + fast blue BB | yellow - gold | alkaline phosphatase |

| | | | |
|---|---|---|---|
| †International Pat. Publ. No. WO 2012/024185 | | | |

### IV. Samples

Exemplary samples include, without limitation, blood smears, cytocentrifuge preparations, cytology smears, core biopsies, and/or fine-needle aspirates. In some examples, the samples include tissue sections (e.g., cryostat tissue sections and/or paraffin-embedded tissue sections). In particular embodiments, the samples include tumor cells, such as breast tumor cells or ovarian tumor cells. Methods of obtaining a biological sample from a subject are known in the art. For example, methods of obtaining breast tissue or breast cells are routine. Exemplary biological samples may be isolated from normal cells or tissues, or from neoplastic cells or tissues. A solid support can hold the biological sample and permit the convenient detection of components (e.g., proteins and/or nucleic acid molecules) in the sample. Exemplary supports include microscope slides (e.g., glass microscope slides or plastic microscope slides), cover slips (e.g., glass cover slips or plastic cover slips), tissue culture dishes, multi-well plates, membranes (e.g., nitrocellulose or polyvinylidene fluoride (PVDF)) or BIACORE™ chips.

The samples described herein can be prepared using any method now known or hereafter developed in the art. Generally, tissue samples are prepared by fixing and embedding the tissue in a medium. In other examples, samples include a cell suspension which is prepared as a monolayer on a solid support (such as a glass slide) for example by smearing or centrifuging cells onto the solid support. In further examples, fresh frozen (for example, unfixed) tissue sections may be used in the methods disclosed herein.

The process of fixing a sample can vary. Fixing a tissue sample preserves cells and tissue constituents in as close to a life-like state as possible and allows them to undergo preparative procedures without significant change. Fixation arrests the autolysis and bacterial decomposition processes that begin upon cell death, and stabilizes the cellular and tissue constituents so that they withstand the subsequent stages of tissue processing, such as for ISH or IHC.

Tissues can be fixed by any suitable process, including perfusion or by submersion in a fixative. Fixatives can be classified as cross-linking agents (such as aldehydes, e.g., formaldehyde, paraformaldehyde, and glutaraldehyde, as well as non-aldehyde cross-linking agents), oxidizing agents (e.g., metallic ions and complexes, such as osmium tetroxide and chromic acid), protein-denaturing agents (e.g., acetic acid, methanol, and ethanol), fixatives of unknown mechanism (e.g., mercuric chloride, acetone, and picric acid), combination reagents (e.g., Carnoy's fixative, methacarn, Bouin's fluid, B5 fixative, Rossman's fluid, and Gendre's fluid), microwaves, and miscellaneous fixatives (e.g., excluded volume fixation and vapor fixation). Additives may also be included in the fixative, such as buffers, detergents, tannic acid, phenol, metal salts (such as zinc chloride, zinc sulfate, and lithium salts), and lanthanum.

The most commonly used fixative in preparing samples is formaldehyde, generally in the form of a formalin solution (4% formaldehyde in a buffer solution, referred to as 10% buffered formalin). In one example, the fixative is 10% neutral buffered formalin.

In some examples an embedding medium is used. An embedding medium is an inert material in which tissues and/or cells are embedded to help preserve them for future analysis. Embedding also enables tissue samples to be sliced into thin sections. Embedding media include paraffin, celloidin, OCT™ compound, agar, plastics, or acrylics. Many embedding media are hydrophobic; therefore, the inert material may need to be removed prior to histological or cytological analysis, which utilizes primarily hydrophilic reagents. The term deparaffinization or dewaxing is broadly used herein to refer to the partial or complete removal of any type of embedding medium from a biological sample. For example, paraffin-embedded tissue sections are dewaxed by passage through organic solvents, such as toluene, xylene, limonene, or other suitable solvents.

### V. Detectable Labels and Methods of Labeling

The nucleic acid probes disclosed herein can include one or more labels, for example to permit detection of a target nucleic acid molecule. In various applications, such as *in situ* hybridization procedures, a nucleic acid probe includes a label (e.g., a detectable label). A "detectable label" is a molecule or material that can be used to produce a detectable signal that indicates the presence or concentration of the probe (particularly the bound or hybridized probe) in a sample. Thus, a labeled nucleic acid molecule provides an indicator of the presence or quantity (for example, gene copy number) of a target nucleic acid (to which the labeled uniquely specific nucleic acid molecule is bound or hybridized) in a sample. The disclosure is not limited to the use of particular labels, although examples are provided.

A label associated with one or more nucleic acid molecules (such as the disclosed probes) can be detected either directly or indirectly. A label can be detected by any known or yet to be discovered mechanism including absorption, emission and/or scattering of a photon (including radio frequency, microwave frequency, infrared frequency, visible frequency and ultra-violet frequency photons). Detectable labels include colored, fluorescent, phosphorescent and luminescent molecules and materials, catalysts (such as enzymes) that convert one substance into another substance to provide a detectable difference (such as by converting a colorless substance into a colored substance or vice versa, or by producing a precipitate or increasing sample turbidity), haptens that can be detected by antibody binding interactions, and paramagnetic and magnetic molecules or materials.

Particular examples of detectable labels include fluorescent molecules (or fluorochromes). Numerous fluorochromes are known to those of skill in the art, and can be selected, for example from Life Technologies, e.g., see, The Handbook - A Guide to Fluorescent Probes and Labeling Technologies. Examples of particular fluorophores that can be attached (for example, chemically conjugated) to a nucleic acid molecule (such as a uniquely specific binding region) are provided in U.S. Patent No. 5,866,366 to Nazarenko et al., such as 4-acetamido-4'-isothiocyanatostilbene-2,2'disulfonic acid, acridine and derivatives such as acridine and acridine isothiocyanate, 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide, Brilliant Yellow, coumarin and derivatives such as coumarin, 7-amino-4-methylcoumarin (AMC, Coumarin 120), 7-amino-4-trifluoromethylcouluarin (Coumarin 151); cyanosine; 4',6-diaminidino-2-phenylindole (DAPI); 5', 5"-dibromopyrogallol-sulfonephthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein, fluorescein isothiocyanate (FITC), and QFITC (XRITC); 2', 7'-difluorofluorescein (OREGON GREEN®); fluorescamine; IR144; IR1446; Malachite Green isothiocyanate; 4-methylumbelliferone; ortho-cresolphthalein; nitrotyrosine; pararosaniline; Phenol Red; B-phycoerythrin; o-phthaldialdehyde; pyrene and derivatives such as pyrene, pyrene butyrate and succinimidyl 1-pyrene butyrate; Reactive Red 4 (Cibacron Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, rhodamine green, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives.

Other suitable fluorophores include thiol-reactive europium chelates which emit at approximately 617 nm (Heyduk and Heyduk, Analyt. Biochem. 248:216-27, 1997; J. Biol. Chem. 274:3315-22, 1999), as well as GFP, Lissamine™, diethylaminocoumarin, fluorescein chlorotriazinyl, naphthofluorescein, 4,7-dichlororhodamine and xanthene (as described in U.S. Patent No. 5,800,996 to Lee et al.) and derivatives thereof. Other fluorophores known to those skilled in the art can also be used, for example those available from Life Technologies (Carlsbad, CA) and including the ALEXA FLUOR® series of dyes (for example, as described in U.S. Patent Nos. 5,696,157, 6,130,101 and 6, 716,979), the BODIPY series of dyes (dipyrrometheneboron difluoride dyes, for example as described in U.S. Patent Nos. 4,774,339, 5,187,288, 5,248,782, 5,274,113, 5,338,854, 5,451,663 and 5,433,896), Cascade Blue (an amine reactive derivative of the sulfonated pyrene described in U.S. Patent No. 5,132,432) and Marina Blue (U.S. Patent No. 5,830,912).

In addition to the fluorochromes described above, a fluorescent label can be a fluorescent nanoparticle, such as a semiconductor nanocrystal, e.g., a quantum dot (obtained, for example, from Life Technologies); see also, U.S. Patent Nos. 6,815,064; 6,682596; and 6,649,138). Semiconductor nanocrystals are microscopic particles having size-dependent optical and/or electrical properties. When semiconductor nanocrystals are illuminated with a primary energy source, a secondary emission of energy occurs of a frequency that corresponds to the bandgap of the semiconductor material used in the semiconductor nanocrystal. This emission can be detected as colored light of a specific wavelength or fluorescence. Semiconductor nanocrystals with different spectral characteristics are described in e.g., U.S. patent No. 6,602,671. Semiconductor nanocrystals that can be coupled to a variety of biological molecules (including dNTPs and/or nucleic acids) or substrates by techniques described in, for example, Bruchez et al., Science 281:2013-2016, 1998; Chan et al., Science 281:2016-2018, 1998; and U.S. Patent No. 6,274,323.

Formation of semiconductor nanocrystals of various compositions are disclosed in, e.g., U.S. Patent Nos. 6,927,069; 6,914,256; 6,855,202; 6,709,929; 6,689,338; 6,500,622; 6,306,736; 6,225,198; 6,207,392; 6,114,038; 6,048,616; 5,990,479; 5,690,807; 5,571,018; 5,505,928; 5,262,357 and in U.S. Patent Publication No. 2003/0165951 as well as PCT Publication No. WO 99/26299. Separate populations of semiconductor nanocrystals can be produced that are identifiable based on their different spectral characteristics. For example, semiconductor nanocrystals can be produced that emit light of different colors based on their composition, size or size and composition. For example, quantum dots that emit light at different wavelengths based on size (565 nm, 655 nm, 705 nm, or 800 nm emission wavelengths), which are suitable as fluorescent labels in the probes disclosed herein are available from Life Technologies (Carlsbad, CA).

Additional labels include, for example, radioisotopes (such as 3H), metal chelates such as DOTA and DPTA chelates of radioactive or paramagnetic metal ions like Gd3+, and liposomes.

Detectable labels that can be used with nucleic acid molecules (such as the disclosed probes) also include enzymes, for example horseradish peroxidase (HRP), alkaline phosphatase (AP), acid phosphatase, glucose oxidase, β-galactosidase, β-glucuronidase, or β-lactamase. Where the detectable label includes an enzyme, a chromogen, fluorogenic compound, or luminogenic compound can be used in combination with the enzyme to generate a detectable signal (numerous of such compounds are commercially available, for example, from Life Technologies). Particular examples of chromogenic compounds include diaminobenzidine (DAB), 4-nitrophenylphosphate (pNPP), fast red, fast blue, bromochloroindolyl phosphate (BCIP), nitro blue tetrazolium (NBT), BCIP/NBT, AP Orange, AP blue, tetramethylbenzidine (TMB), 2,2'-azino-di-[3-ethylbenzothiazoline sulphonate] (ABTS), o-dianisidine, 4-chloronaphthol (4-CN), nitrophenyl-β-D-galactopyranoside (ONPG), o-phenylenediamine (OPD), 5-bromo-4-chloro-3-indolyl-β-galactopyranoside (X-Gal), methylumbelliferyl-β-D-galactopyranoside (MU-Gal), p-nitrophenyl-α-D-galactopyranoside (PNP), 5-bromo-4-chloro-3-indolyl-β-D-glucuronide (X-Gluc), 3-amino-9-ethyl carbazol (AEC), fuchsin, iodonitrotetrazolium (INT), tetrazolium blue, and tetrazolium violet.

Alternatively, an enzyme can be used in a metallographic detection scheme. For example, silver *in situ* hybridization (SISH) procedures involve metallographic detection schemes for identification and localization of a hybridized genomic target nucleic acid sequence. Metallographic detection methods include using an enzyme, such as alkaline phosphatase, in combination with a water-soluble metal ion and a redox-inactive substrate of the enzyme. The substrate is converted to a redox-active agent by the enzyme, and the redox-active agent reduces the metal ion, causing it to form a detectable precipitate. (See, for example, U.S. Patent Application Publication No. 2005/0100976, PCT Publication No. 2005/003777 and U.S. Patent Application Publication No. 2004/0265922). Metallographic detection methods also include using an oxido-reductase enzyme (such as horseradish peroxidase) along with a water soluble metal ion, an oxidizing agent and a reducing agent, again to form a detectable precipitate. (See, for example, U.S. Patent No. 6,670,113).

In non-limiting examples, the disclosed nucleic acid probes are labeled with dNTPs covalently attached to hapten molecules (such as a nitro-aromatic compound (e.g., 2,4-dinitrophenyl (DNP)), biotin, fluorescein, digoxigenin, etc.). Additional haptens suitable for labeling the disclosed probes include nitropyrazole, 3-hydroxyquinoxaline, thiazolesulfonamide, nitrocinnamic acid, rotenone, 7-(diethylamino)coumarin-3-carboxylic acid, benzodiazepine, or benzofuran haptens (see, e.g., International Pat. Publ. No. WO 2012/003476). Methods for conjugating haptens and other labels to dNTPs (e.g., to facilitate incorporation into labeled probes) are well known in the art. For examples of procedures, see, e.g., U.S. Patent Nos. 5,258,507, 4,772,691, 5,328,824, and 4,711,955. Indeed, numerous labeled dNTPs are available commercially, for example from Life Technologies (Carlsbad, CA). A label can be directly or indirectly attached to a dNTP at any location on the dNTP, such as a phosphate (e.g., α, β or γ phosphate) or a sugar.

Detection of labeled nucleic acid molecules can be accomplished by contacting the hapten-labeled nucleic acid molecules bound to the genomic target nucleic acid with a primary anti-hapten antibody. In one example, the primary anti-hapten antibody (such as a mouse anti-hapten antibody) is directly labeled with an enzyme. In another example, a secondary anti-antibody (such as a goat anti-mouse IgG antibody) conjugated to an enzyme is used for signal amplification. In CISH a chromogenic substrate is added, for SISH, silver ions and other reagents as outlined in the referenced patents/applications are added.

In some examples, a probe is labeled by incorporating one or more labeled dNTPs using an enzymatic (polymerization) reaction. For example, the disclosed nucleic acid probes (for example, incorporated into a plasmid vector) can be labeled by nick translation (using, for example, biotin, DNP, digoxigenin, etc.) or by random primer extension with terminal transferase (e.g., 3' end tailing). In some examples, the nucleic probe is labeled by a modified nick translation reaction where the ratio of DNA polymerase I to deoxyribonuclease I (DNase I) is modified to produce greater than 100% of the starting material. In particular examples, the nick translation reaction includes DNA polymerase I to DNase I at a ratio of at least about 800:1, such as at least 2000:1, at least 4000:1, at least 8000:1, at least 10,000:1, at least 12,000:1, at least 16,000:1, such as about 800:1 to 24,000:1 and the reaction is carried out overnight (for example, for about 16-22 hours) at a substantially isothermal temperature, for example, at about 16°C to 25°C (such as room temperature). If the probe is included in a probe set (for example, multiple plasmids, such as 2, 3, 4, 5, 6, 7, 8, 9, 10, or more plasmids), the plasmids may be mixed in an equal molar ratio prior to performing the labeling reaction (such as nick translation or modified nick translation).

In other examples, chemical labeling procedures can also be employed. Numerous reagents (including hapten, fluorophore, and other labeled nucleotides) and other kits are commercially available for enzymatic labeling of nucleic acids, including the disclosed nucleic acid probes. As will be apparent to those of skill in the art, any of the labels and detection procedures disclosed above are applicable in the context of labeling a probe, e.g., for use in *in situ* hybridization reactions. For example, the Amersham MULTIPRIME® DNA labeling system, various specific reagents and kits available from Molecular Probes/Life Technologies, or any other similar reagents or kits can be used to label the nucleic acids disclosed herein. In particular examples, the disclosed probes can be directly or indirectly labeled with a hapten, a ligand, a fluorescent moiety (e.g., a fluorophore or a semiconductor nanocrystal), a chromogenic moiety, or a radioisotope. For example, for indirect labeling, the label can be attached to nucleic acid molecules via a linker (e.g., PEG or biotin). Additional methods that can be used to label probe nucleic acid molecules are provided in U.S. Application Pub. No. 2005/0158770.

### VI. In situ Hybridization

*In situ* hybridization (ISH) involves contacting a sample containing a target nucleic acid (e.g., a genomic target nucleic acid) in the context of a metaphase or interphase chromosome preparation (such as a cell or tissue sample mounted on a slide) with a labeled probe specifically hybridizable or specific for the target nucleic acid (for example, one or more of the probes disclosed herein). The slides are optionally pretreated, e.g., to remove paraffin or other materials that can interfere with uniform hybridization. The chromosome sample and the probe are both treated, for example by heating to denature the double stranded nucleic acids. The probe (formulated in a suitable hybridization buffer) and the sample are combined, under conditions and for sufficient time to permit hybridization to occur (typically to reach equilibrium). The chromosome preparation is washed to remove excess probe, and detection of specific labeling of the target is performed using standard techniques.

For example, a biotinylated probe can be detected using fluorescein-labeled avidin or avidin-alkaline phosphatase. For fluorochrome detection, the fluorochrome can be detected directly, or the samples can be incubated, for example, with fluorescein isothiocyanate (FITC)-conjugated avidin. Amplification of the FITC signal can be effected, if necessary, by incubation with biotin-conjugated goat anti-avidin antibodies, washing and a second incubation with FITC-conjugated avidin. For detection by enzyme activity, samples can be incubated, for example, with streptavidin, washed, incubated with biotin-conjugated alkaline phosphatase, washed again and pre-equilibrated (e.g., in alkaline phosphatase (AP) buffer). The enzyme reaction can be performed in, for example, AP buffer containing NBT/BCIP and stopped by incubation in 2 X SSC. For a general description of *in situ* hybridization procedures, see, e.g., U.S. Patent No. 4,888,278.

Numerous procedures for FISH, CISH, and SISH are known in the art. For example, procedures for performing FISH are described in U.S. Patent Nos. 5,447,841; 5,472,842; and 5,427,932; and for example, in Pinkel et al., Proc. Natl. Acad. Sci. 83:2934-2938, 1986; Pinkel et al., Proc. Natl. Acad. Sci. 85:9138-9142, 1988; and Lichter et al., Proc. Natl. Acad. Sci. 85:9664-9668, 1988. CISH is described in, e.g., Tanner et al., Am. J. Pathol. 157:1467-1472, 2000 and U.S. Patent No. 6,942,970. Additional detection methods are provided in U.S. Patent No. 6,280,929.

Numerous reagents and detection schemes can be employed in conjunction with FISH, CISH, and SISH procedures to improve sensitivity, resolution, or other desirable properties. As discussed above, probes labeled with fluorophores (including fluorescent dyes and quantum dots) can be directly optically detected when performing FISH. Alternatively, the probe can be labeled with a non-fluorescent molecule, such as a hapten (such as the following non-limiting examples: biotin, digoxigenin, DNP, and various oxazoles, pyrrazoles, thiazoles, nitroaryls, benzofurazans, triterpenes, ureas, thioureas, rotenones, coumarin, courmarin-based compounds, Podophyllotoxin, Podophyllotoxin-based compounds, and combinations thereof), ligand or other indirectly detectable moiety. Probes labeled with such non-fluorescent molecules (and the target nucleic acid sequences to which they bind) can then be detected by contacting the sample (e.g., the cell or tissue sample to which the probe is bound) with a labeled detection reagent, such as an antibody (or receptor, or other specific binding partner) specific for the chosen hapten or ligand. The detection reagent can be labeled with a fluorophore (e.g., quantum dot) or with another indirectly detectable moiety, or can be contacted with one or more additional specific binding agents (e.g., secondary or specific antibodies), which can in turn be labeled with a fluorophore. Optionally, the detectable label is attached directly to the antibody, receptor (or other specific binding agent). Alternatively, the detectable label is attached to the binding agent via a linker, such as a hydrazide thiol linker, a polyethylene glycol linker, or any other flexible attachment moiety with comparable reactivities. For example, a specific binding agent, such as an antibody, a receptor (or other anti-ligand), avidin, or the like can be covalently modified with a fluorophore (or other label) via a heterobifunctional polyalkyleneglycol linker such as a heterobifunctional polyethyleneglycol (PEG) linker. A heterobifunctional linker combines two different reactive groups selected, e.g., from a carbonyl-reactive group, an amine-reactive group, a thiol-reactive group and a photo-reactive group, the first of which attaches to the label and the second of which attaches to the specific binding agent.

In other examples, the probe, or specific binding agent (such as an antibody, e.g., a primary antibody, receptor or other binding agent) is labeled with an enzyme that is capable of converting a fluorogenic or chromogenic composition into a detectable fluorescent, colored or otherwise detectable signal (e.g., as in deposition of detectable metal particles in SISH). As indicated above, the enzyme can be attached directly or indirectly via a linker to the relevant probe or detection reagent. Examples of suitable reagents (e.g., binding reagents) and chemistries (e.g., linker and attachment chemistries) are described in U.S. Patent Application Publication Nos. 2006/0246524; 2006/0246523, and 2007/0117153.

In further examples, a signal amplification method is utilized, for example, to increase sensitivity of the probe. For example, CAtalyzed Reporter Deposition (CARD), also known as Tyramide Signal Amplification (TSA™) may be utilized. In one variation of this method a biotinylated nucleic acid probe detects the presence of a target by binding thereto. Next a streptavidin-peroxidase conjugate is added. The streptavidin binds to the biotin. A substrate of biotinylated tyramide (tyramine is 4-(2-aminoethyl)phenol) is used, which presumably becomes a free radical when interacting with the peroxidase enzyme. The phenolic radical then reacts quickly with the surrounding material, thus depositing or fixing biotin in the vicinity. This process is repeated by providing more substrate (biotinylated tyramide) and building up more localized biotin. Finally, the "amplified" biotin deposit is detected with streptavidin attached to a fluorescent molecule. Alternatively, the amplified biotin deposit can be detected with avidin-peroxidase complex, that is then fed 3,3'-diaminobenzidine to produce a brown color. It has been found that tyramide attached to fluorescent molecules also serve as substrates for the enzyme, thus simplifying the procedure by eliminating steps.

In other examples, the signal amplification method utilizes branched DNA (bDNA) signal amplification. In some examples, target-specific oligonucleotides (label extenders and capture extenders) are hybridized with high stringency to the target nucleic acid. Capture extenders are designed to hybridize to the target and to capture probes, which are attached to a microwell plate. Label extenders are designed to hybridize to contiguous regions on the target and to provide sequences for hybridization of a preamplifier oligonucleotide. Signal amplification then begins with preamplifier probes hybridizing to label extenders. The preamplifier forms a stable hybrid only if it hybridizes to two adjacent label extenders. Other regions on the preamplifier are designed to hybridize to multiple bDNA amplifier molecules that create a branched structure. Finally, alkaline phosphatase (AP)-labeled oligonucleotides, which are complementary to bDNA amplifier sequences, bind to the bDNA molecule by hybridization. The bDNA signal is the chemiluminescent product of the AP reaction See, e.g., Tsongalis, Microbiol. Inf. Dis. 126:448-453, 2006; U.S. Pat. No. 7,033,758.

In further examples, the signal amplification method utilizes polymerized antibodies. In some examples, the labeled probe is detected by using a primary antibody to the label (such as an anti-DIG or anti-DNP antibody). The primary antibody is detected by a polymerized secondary antibody (such as a polymerized HRP-conjugated secondary antibody or an AP-conjugated secondary antibody). The enzymatic reaction of AP or HRP leads to the formation of strong signals that can be visualized.

It will be appreciated by those of skill in the art that by appropriately selecting labeled probe-specific binding agent pairs, multiplex detection schemes can be produced to facilitate detection of multiple target nucleic acids (e.g., genomic target nucleic acids) in a single assay (e.g., on a single cell or tissue sample or on more than one cell or tissue sample). For example, a first probe that corresponds to a first target nucleic acid can be labeled with a first hapten, such as biotin, while a second probe that corresponds to a second target nucleic acid can be labeled with a second hapten, such as DNP. Following exposure of the sample to the probes, the bound probes can be detected by contacting the sample with a first specific binding agent (in this case avidin labeled with a first fluorophore, for example, a first spectrally distinct quantum dot, e.g., that emits at 585 nm) and a second specific binding agent (in this case an anti-DNP antibody, or antibody fragment, labeled with a second fluorophore (for example, a second spectrally distinct quantum dot, e.g., that emits at 705 nm). Additional probes/binding agent pairs can be added to the multiplex detection scheme using other spectrally distinct fluorophores. Numerous variations of direct, and indirect (one step, two step or more) can be envisioned, all of which are suitable in the context of the disclosed probes and assays.

Additional details regarding certain detection methods, e.g., as utilized in CISH and SISH procedures, can be found in Bourne, The Handbook of Immunoperoxidase Staining Methods, published by Dako Corporation, Santa Barbara, CA.

### VII. Blocking DNA

Genome-specific blocking DNA (such as human DNA, for example, total human placental DNA or Cot-1™ DNA) is usually included in a hybridization solution (such as for *in situ* hybridization) to suppress probe hybridization to repetitive DNA sequences or to counteract probe hybridization to highly homologous (frequently identical) off target sequences when a probe complementary to a human genomic target nucleic acid is utilized. In hybridization with standard probes, in the absence of genome-specific blocking DNA, an unacceptably high level of background staining (for example, non-specific binding, such as hybridization to non-target nucleic acid sequence) is usually present, even when a "repeat-free" probe is used. The disclosed nucleic acid probes exhibit reduced background staining, even in the absence of blocking DNA. In particular examples, the hybridization solution including the disclosed probes does not include genome-specific blocking DNA (for example, total human placental DNA or Cot-1™ DNA, if the probe is complementary to a human genomic target nucleic acid). This advantage is derived from the uniquely specific nature of the target sequences included in the nucleic acid probe; each labeled probe sequence binds only to the cognate uniquely specific genomic sequence. This results in dramatic increases in signal to noise ratios for ISH techniques.

In some examples the hybridization solution may contain carrier DNA from a different organism (for example, salmon sperm DNA or herring sperm DNA, if the genomic target nucleic acid is a human genomic target nucleic acid) to reduce non-specific binding of the probe to non-DNA materials (for example to reaction vessels or slides) with high net positive charge which can non-specifically bind to the negatively charged probe DNA.

The disclosure is further illustrated by the following non-limiting Examples.

### EXAMPLES

The following examples are provided to illustrate certain specific features of working embodiments and general protocols. The scope of the present invention is not limited to those features exemplified by the following examples.

### EXAMPLE 1: CLL Analysis

Abnormal cytogenetics are found in the majority of patients with CLL, and each subtype is associated with differentiated frequency, outcome, and suggested treatments (see Table 3).

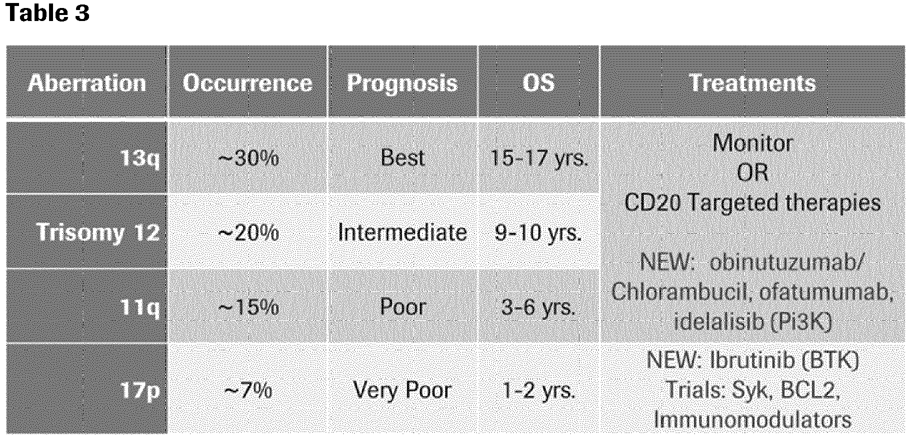

Example 1 demonstrates that probes of the present disclosure can detect such abnormalities via *in situ* hybridization (ISH) on formalin fixed paraffin embedded bone marrow/trephine samples.
**A. Preparation of Reagents:** DNA probes were labeled with either DNP or DIG using standard reaction conditions. Specificity of each probe was verified using CGH Target Metaphase spread. Functionality of each probe was determined by standard dual ISH using the U Dual Color ISH Open Probe procedure on standard cancer tissues supplied by Ventana Medical Systems, Inc.
**B. Reagents** (if commercially available from Ventana Medical Systems Inc., reagents are listed with the associated part number).
   - TP53/Chr17:
      ∘ TP53-DNP [30ug/ml] (mixture of 10 probes according to SEQ ID NOs: 51-60);
      ∘ ASR CEN-17-DIG [1.25 ug/ml] (Ventana Medical Systems, Inc., cat. #760-1224);
   - DLEU/13q12.11:
      ∘ DLEU-DNP [30ug/ml], (mixture of 10 probes according to SEQ ID NOs: 31-40);
      ∘ 13q12.11-DIG [30 ug/ml], (mixture of 10 probes according to SEQ ID NOs: 11-20);
      ∘ 1 mg/ml sonicated fsDNA;
   - ATM/13q12.11:
      ∘ ATM-DNP [30 ug/ml], (mixture of 10 probes according to SEQ ID NOs: 21-30)
      ∘ CEN11-DIG [4 ug/ml], 1 mg/ml placental DNA
   - Trisomy 12:
      ∘ ASR CEN-12-DNP [0.75 ug/ml] (Ventana Medical Systems, Inc., cat. #760-1221);
      ∘ ISH Probe Dispenser Card
**C. Detection Reagents**
   - ISH Protease 2 (780-4148)
   - ISH Protease 3 (780-4149)
   - HYB READY (780-4409)
   - ULTRAVIEW SISH DNP Detection Kit (760-098)
   - ULTRAVIEW Red ISH DIG Detection Kit (760-505)
   - Hematoxylin II (790-2208)
   - Bluing Reagent (760-2037)
**D. Bulk Reagents**
   - EZ PREP Concentrate (950-102)
   - *Ultra* LSC (650-210)
   - SSC Concentrate (950-110)
   - Reaction Buffer (950-300)
   - *Ultra* CC1 (950-224)
   - *Ultra* CC2 (950-223)
   - *ULTRA*VIEW SilverWash II (780-003)

### E. Tissue - Suggested Protocols

The following staining protocols were performed on formalin-fixed paraffin-embedded bone marrow trephine samples and blood clot using a BenchMark XT or BenchMark ULTRA automated slide staining platform:

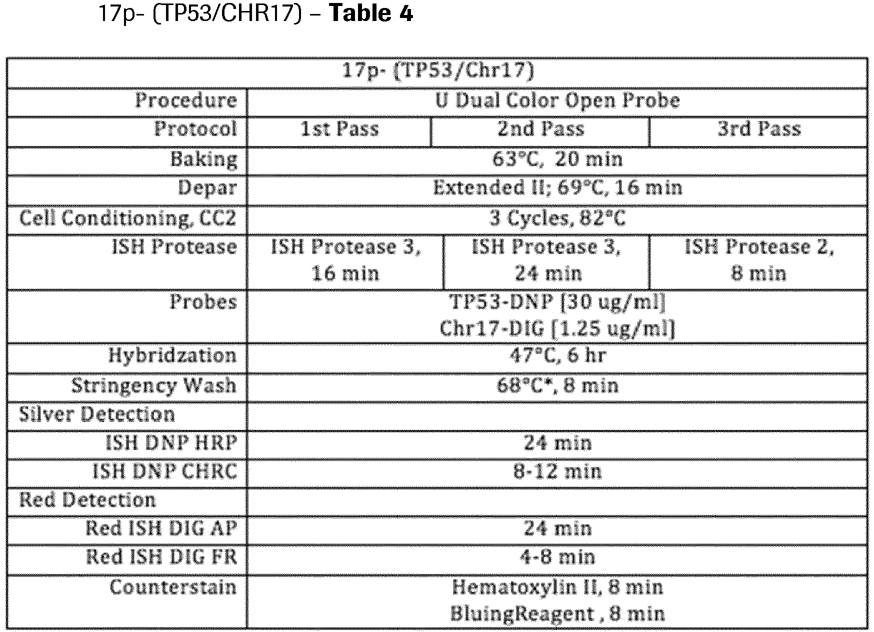

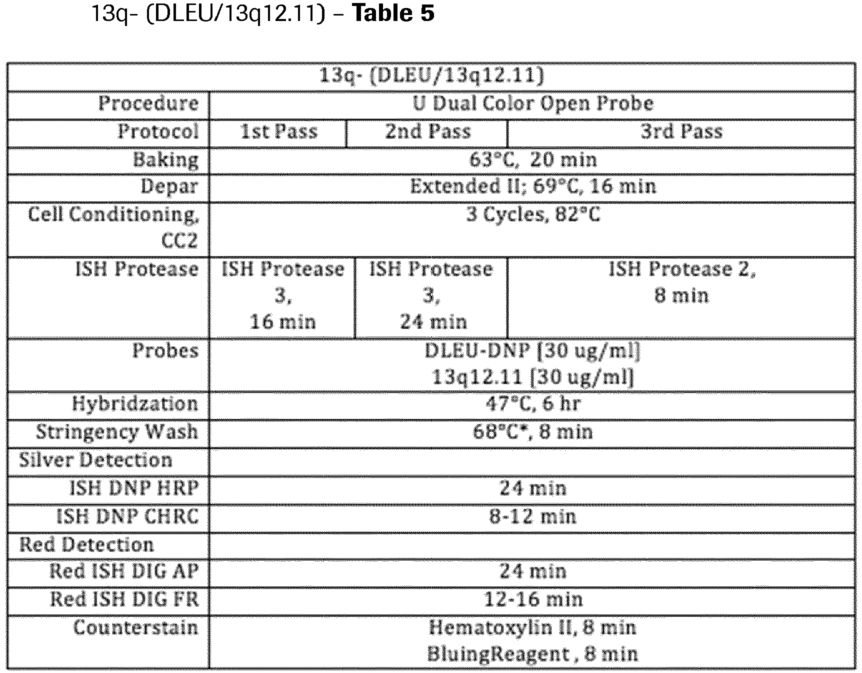

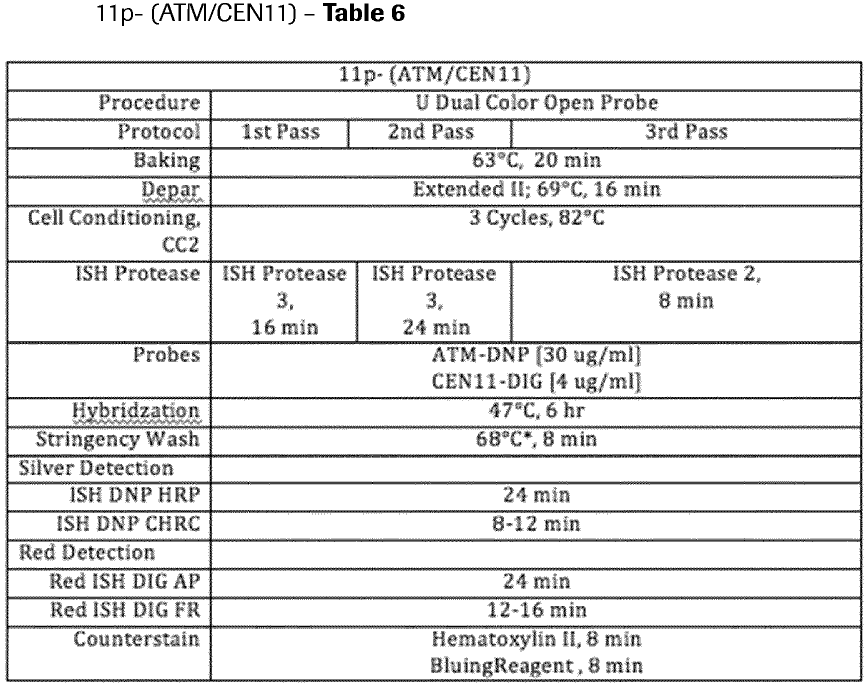

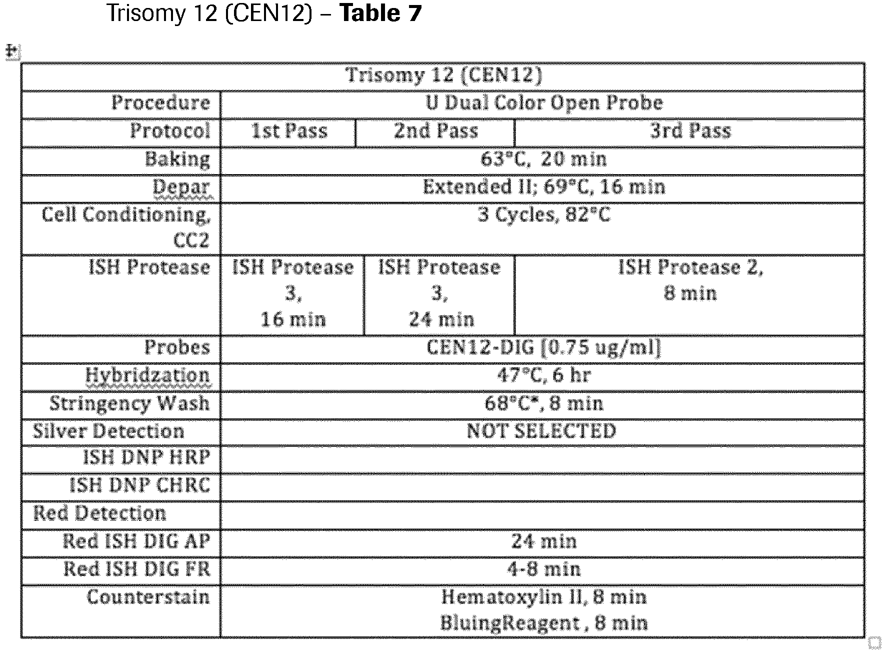

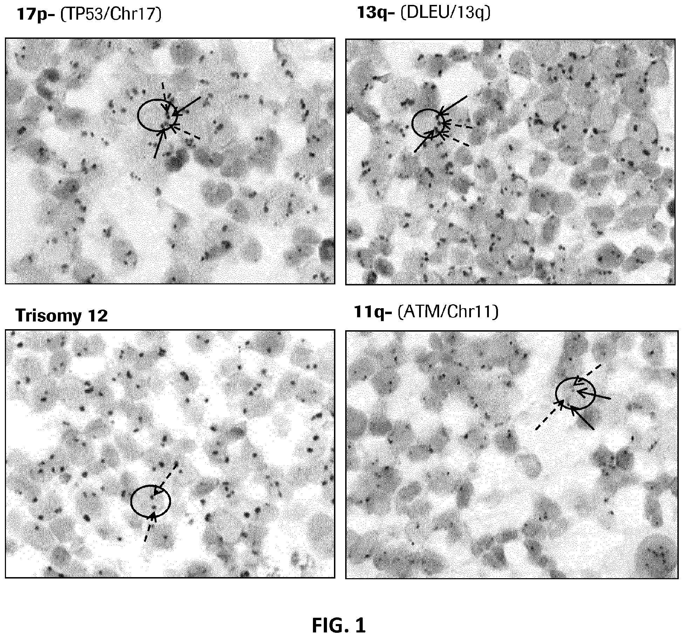

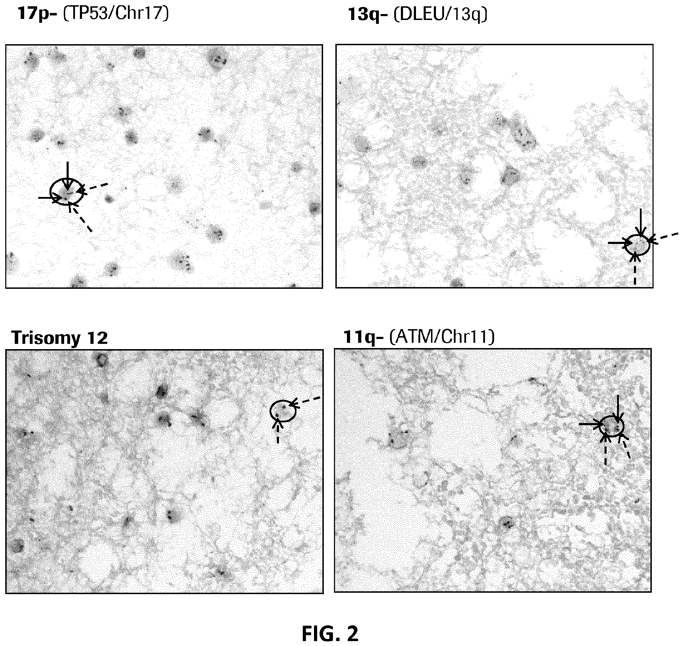

Exemplary stained slides are illustrated at Fig. 1 (bone marrow/trephine samples) and Fig. 2 (blood clot). Ovals indicate an exemplary cell. Dashed arrows indicate staining corresponding to the centromere/chromosome specific probe, which is red in color images. Solid arrows indicate staining corresponding to the gene-specific probe, which is black in color images.

### F. Blood - Suggested Protocols

The following staining protocols were performed on formalin-fixed whole blood smears using a BenchMark XT or BenchMark ULTRA automated slide staining platform:

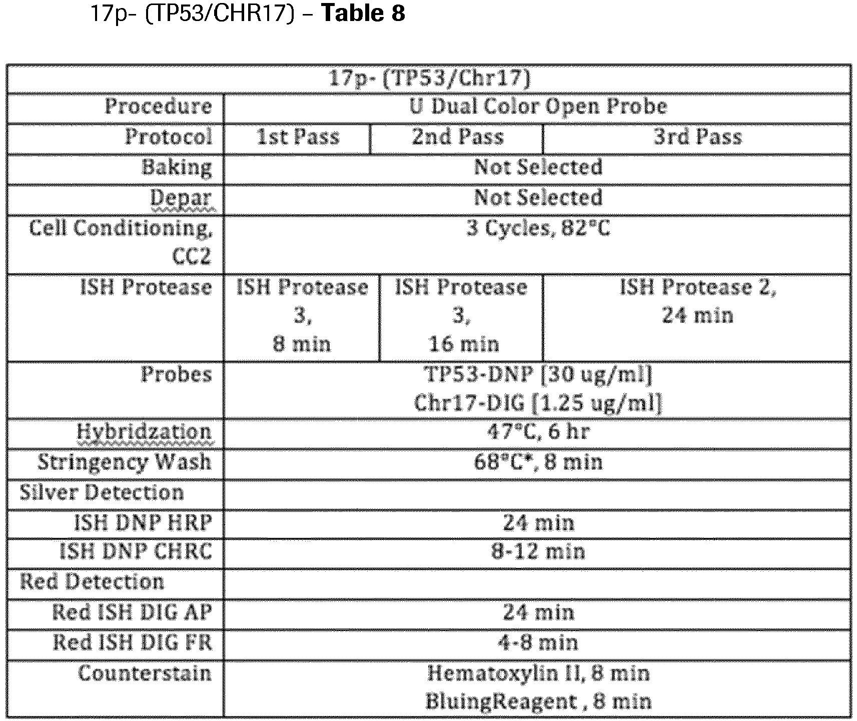

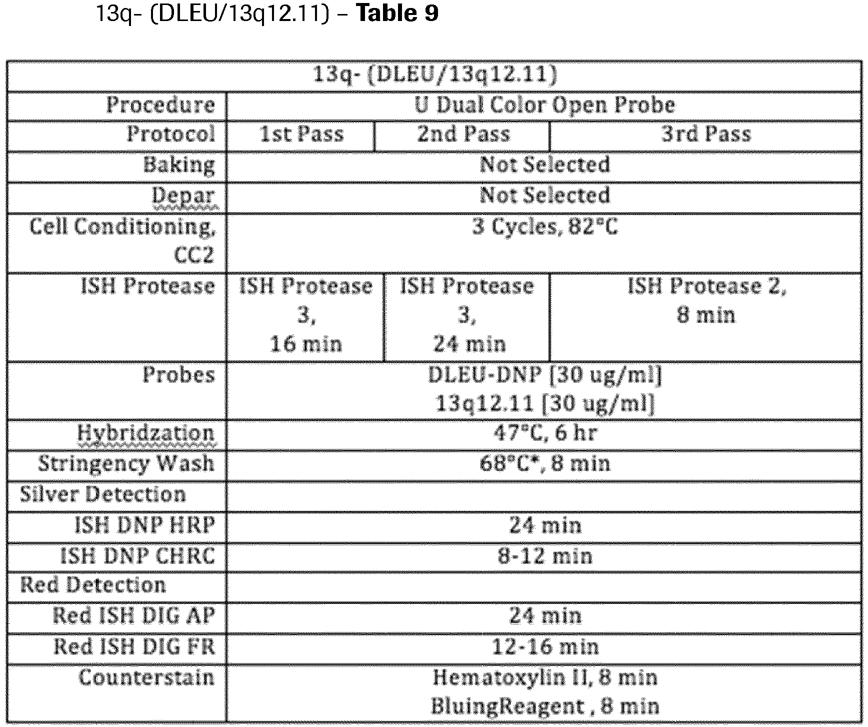

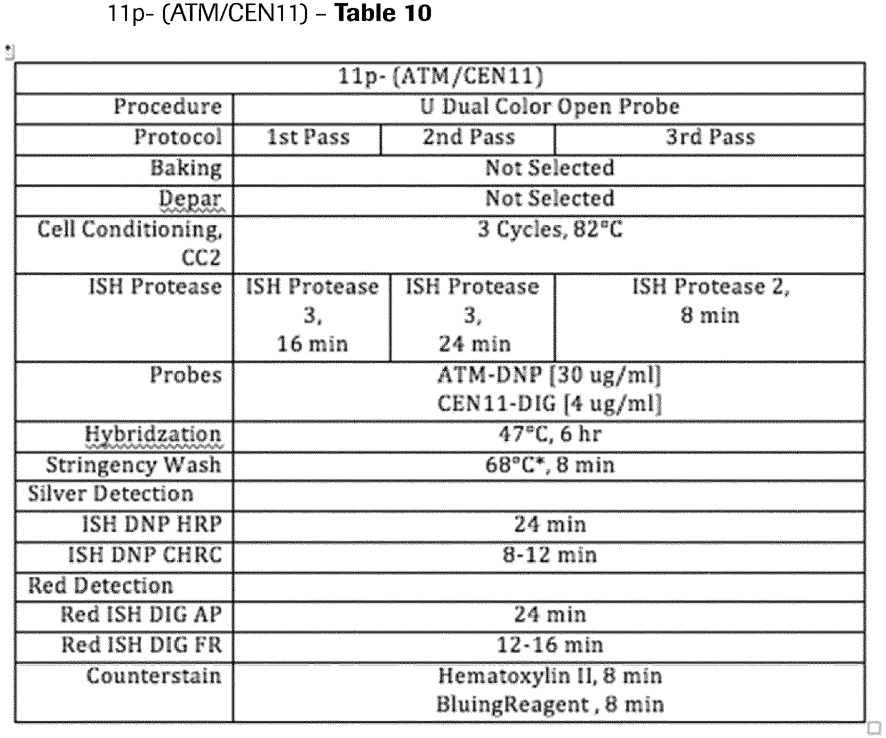

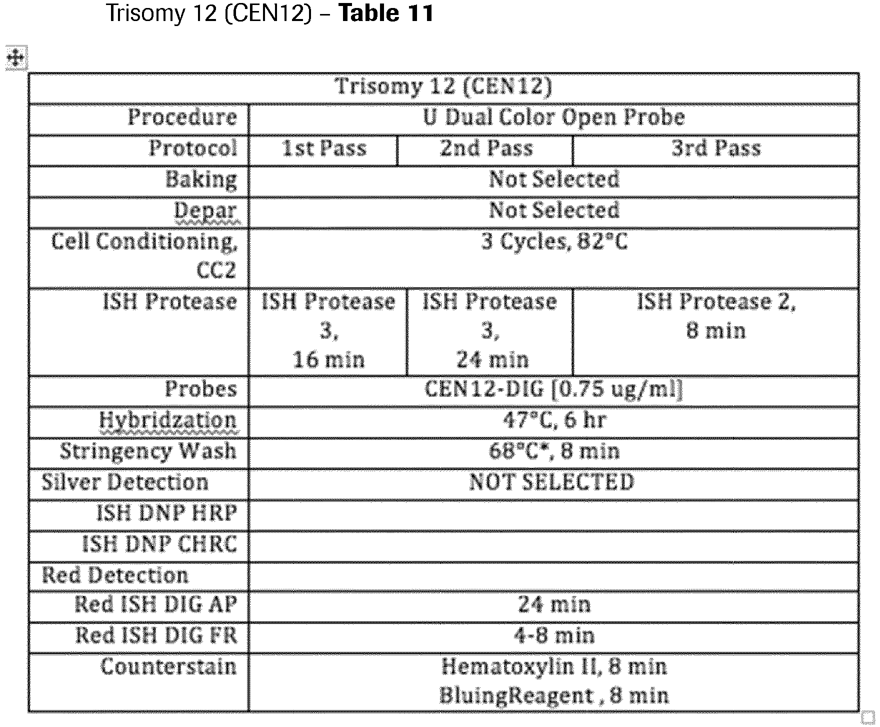

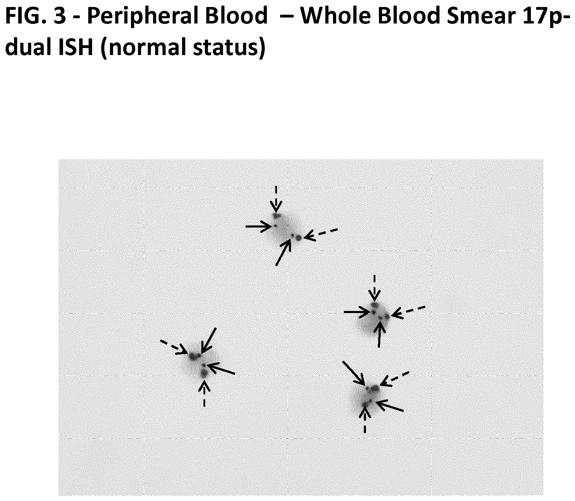

An exemplary slide stained for TP53 and chromosome 17 is illustrated at Fig. 3. Dashed arrows indicate staining corresponding to the chromosome 17 specific probe, which is red in color images. Solid arrows indicate staining corresponding to the TP53-specific probe, which is black in color images.

### G. Cell Fixation Method for Cell Preparation

- Prepare blood Sample as desired (whole blood smear/Cytospin/etc)
- Air-dry.
- Immerse slides in 10% NBF (Neutral Buffered Formalin); incubate for 20 min at room temperature.
- Rinse slides 3 times in PBS at room temperature.
- Rinse slides in ddH20 at room temperature.
- Air dry.
- Store slides at -20°C freezer in airtight container.

### H. Examining 19q12 amplification using Chromogenic ISH

Preparation of Materials: DNA probes were label with either DNP or DIG by Ventana Pilot Plant using standard reaction conditions as per Genomics Technology and Applied Research (GTAR). Specificity of CCNE1-DNP and INSR-DIG probes was verified using CGH Target Metaphase spread. Functionality of each probe was determined by standard dual ISH using the Dual Color ISH Open Probe procedure on standard cancer tissues supplied by Ventana TSM. These protocols are sufficient for both DNP and DIG signals but not deemed "robust".

### I. Dual ISH Scoring Criteria

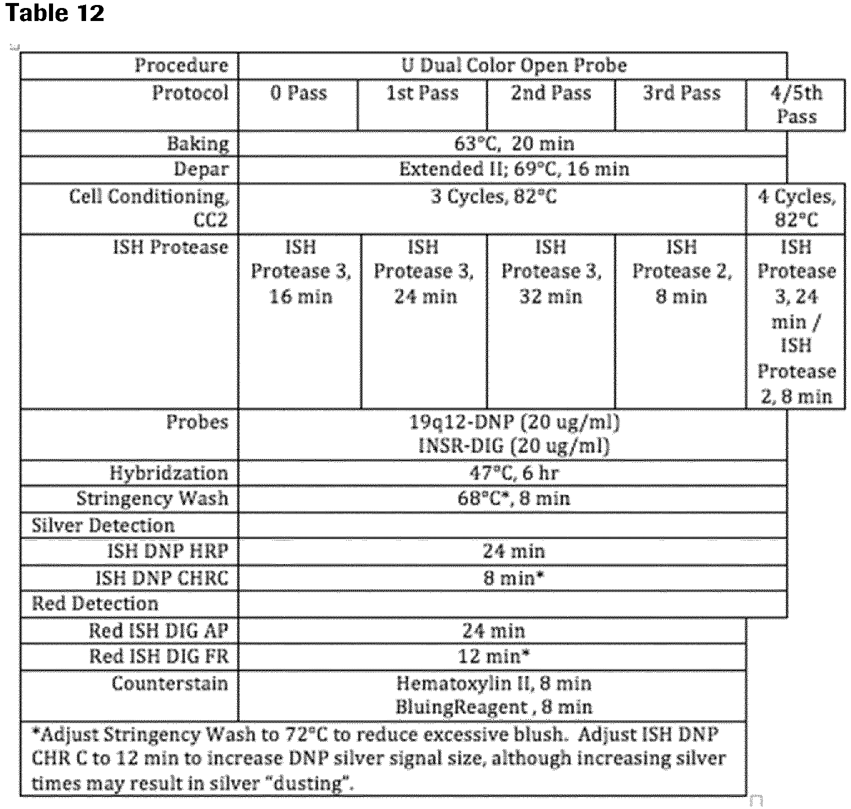

For a more detailed explanation of scoring, please refer to the INFORM HER2 DualISH PMA ULTRA Interpretation guide.)

Review the entire section/specimen looking for tumor regions with good staining (both red and black) that have the highest number of signals.

Select an area of interest and count 17 cells and classify signals with the most information (highest signals).

Select a second area of interest and count 17 cells with the most information (highest signals).

Select a third area of interest and count 16 cells with the most information (highest signals).

Total number of cells = 50

### EXAMPLE 2 - Multiplex Gene-Protein Assay for CD79a/TP53/CEN17

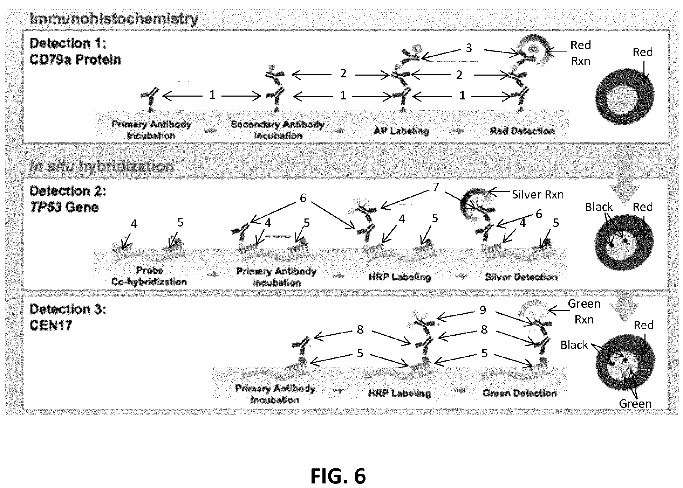

This example describes a multiplex gene-protein assay for detection of CD79a protein, TP53 DNA, and chromosome 17 centromere DNA in a single sample. The staining strategy consists of essentially three staining steps: (1) CD79a protein; (2) *TP53* Gene; and (3) CEN17. An overview of the staining strategy is illustrated at Fig. 6.

In the first detection, an antibody specific for CD79a **1** is contacted with the sample. A species-specific secondary antibody **2** conjugated to a first antibody-reactive group (such as a hapten) is then applied to the sample, which binds to the primary antibody **1.** A tertiary antibody **3** is then applied to the sample. The tertiary antibody **3** is specific for the antibody-reactive subunit of the secondary antibody **2.** The tertiary antibody **3** also is labeled with a detectable label. After reaction to visualize the detectable label, regions at which the primary antibody has bound appear as a first color. In the example illustrated in Fig. 6, the detectable label is alkaline phosphatase (AP), and the first color is red.

In the second detection, a nucleic acid probe specific for the *TP53* Gene (*TP53* Probe) **4** is hybridized to the sample. The *TP53* probe **4** is modified to contain a second antibody-reactive group that is distinct from the first antibody-reactive group (such as a different hapten). The *TP53* probe **4** may be hybridized by itself, or may be co-hybridized to the sample with a probe specific for the centromere region of chromosome 17 (CEN17 probe) **5** that is modified to contain a third antibody-reactive group that is distinct from the first and second antibody-reactive groups (such as a different hapten). Fig. 6 illustrates a co-hybridization reaction. A primary antibody specific for the second antibody-reactive group **6** is then reacted with the sample, which binds to the second antibody reactive group. A species specific secondary antibody **7** is then reacted with the sample, which binds to the primary antibody **6.** The secondary antibody **7** is conjugated to a detectable label. After reaction to visualize the detectable label, regions at which the primary antibody has bound appear as a second color that is visually distinct from the first color. In the example illustrated in Fig. 6, the detectable label is horseradish peroxidase (HRP), and the second color is silver/black.

In the third detection, a nucleic acid probe specific for the CEN17 probe **5** as described above is applied (if sequential hybridization is used) and/or detected. Fig. 6 illustrates a co-hybridization reaction, so a separate hybridization step is not necessary in that specific example. A primary antibody specific for the third antibody-reactive group **8** is then reacted with the sample, which binds to the third antibody reactive group. A species specific secondary antibody **9** is then reacted with the sample, which binds to the primary antibody **8.** The secondary antibody **7** is conjugated to a detectable label. After reaction to visualize the detectable label, regions at which the primary antibody has bound appear as a third color that is visually distinct from the first and second colors. In the example illustrated in Fig. 6, the detectable label is horseradish peroxidase (HRP), and the third color is green.

An example of this reaction scheme was run on blood smear glass slides. The blood smear glass slides were re-fixed in neutral buffered formalin at room temperature overnight. After fixation, the glass slides were rinsed in distilled water several times and air-dried completely. Prior to the staining protocol, the glass slides were soaked in 10% nonfat dry milk (Carnation) in Reaction Buffer (Ventana) for 10 minutes at room temperature. Blood smear glass slides were placed onto VENTANA BenchMark XT automated slide stainer for a TP53 gene-protein assay. Blood samples were heat pretreated with Cell Conditioning 1 (CC1, Ventana) for 48 minutes at 95°C. Then, after rinsing the slides with Reaction Buffer (Ventana), CONFIRM anti-CD79a (SP18) Rabbit Monoclonal Primary Antibody (Ventana, catalog# 790-4432) was applied for 28 minutes at 37°C followed by Reaction Buffer washing steps. A red chromogen was used for detecting CD79a protein as red staining. Blood samples were heat-pretreated with EZ Prep (Ventana) diluted Cell Conditioning 2 (CC2) for two cycles of an 8 minute incubation at 82°C. Then, the samples were digested with ISH-Protease 3 (Ventana) for 20 minutes at 37°C for completing the pretreatment for the ISH assay part of gene-protein assay. A cocktail of DNP-labeled TP53 and DIG-labeled CEN17 probes was applied onto glass slides with SSC diluted HybReady solution (Ventana). After denaturing step for 8 minutes at 80°C, the hybridization step was performed for 6 hours. A sequential stringency wash and ISH detection step was conducted for TP53 gene and CEN17 signal visualization. For the first stringency and TP53 ISH detection, three cycles of stringency wash steps were performed at 68°C using SSC followed by ultraView SISH DNP Detection Kit (Ventana, catalog# 760-098) and TP53 gene signal were visualized as black dots. For the second stringency and CEN17 ISH detection, three cycles of stringency wash steps were performed at 76°C using SSC and a HRP-Green signal detection protocol was applied for CEN17 ISH signal visualization. Anti-DIG antibody (from ultraView Red ISH DIG Detection Kit, Ventana, catalog# 760-505) was applied to glass slides for 12 minutes at 37°C followed by HRP-conjugated anti-mouse antibody (UltraMap, Ventana, catalog# 760-4313) incubation for 12 minutes at 37°C. CEN17 ISH detection was completed with HRP-Green detection (42 Lifescience). Finally the glass slides were counterstained with diluted Mayer's hematoxylin (1:4 in water) for 4 minutes. The glass slides were washed and air-dried followed by coverslipping.

As illustrated at Fig. 7 and Fig. 8, the protocol results in red staining of CD79a protein, black staining of the TP53 genomic DNA (which appears as dark black dots in the grayscale image of Fig. 7 & 8), and green/blue staining of chromosome 17 centromere DNA (which appear as more diffuse and lighter dots in the grayscale image of Fig. 7 & 8). Fig. 8 illustrates an example having a p17 homozygous deletion. CD79a protein staining is illustrated by solid arrows, TP53 gene staining by dashed arrows, and CEN17 staining by dotted arrows. All steps were performed on a BENCHMARK XT automated IHC/ISH staining instrument (Ventana Medical Systems, Tucson, AZ, Catalog #: N750-BMKXT-FS).

In view of the many possible embodiments to which the principles of the disclosure may be applied, it should be recognized that the illustrated embodiments are only examples and should not be taken as limiting the scope of the invention. Rather, the scope of the invention is defined by the following claims.

## Claims

1. A multiplex method for co-detecting CD79a protein, TP53 genomic DNA, and chromosome 17 centromere DNA in a sample on a single slide, said method comprising:
staining the CD79a protein by contacting the sample with a CD79a protein-specific antibody and contacting the sample with a first chromogen component for the CD79a protein-specific antibody, the first chromogen component is adapted to emit or make visible a first color, wherein the presence of the first color indicates the presence of the CD79a protein; and
staining TP53 genomic DNA and staining chromosome 17 centromere DNA by contacting the sample with a TP53 genomic DNA-specific nucleic acid probe and with a chromosome 17 centromere DNA-specific nucleic acid probe, and contacting the sample with a second chromogen component for the TP53 genomic DNA-specific nucleic acid probe and with a third chromogen component for the chromosome 17 centromere DNA-specific nucleic acid probe, the second chromogen component is adapted to emit or make visible a second color and the third chromogen component is adapted to emit or make visible a third color, wherein the presence of the second color indicates the presence of TP53 genomic DNA and the presence of the third color indicates the presence of chromosome 17 centromere DNA.

2. The method of claim 1, wherein the sample is a blood sample.

3. The method of claim 1 or 2, wherein the first chromogen component comprises fast red, the second chromogen component comprises silver, and the third chromogen component comprises a green chromogen component.

4. The method of any of claims 1 to 3 further comprising visualizing the colors using bright-field microscopy.

5. The method of any of claims 1 to 4, wherein the method is automated.

6. The method of any of claims 1 to 5, wherein the step of staining the CD79a protein is performed before the step of staining TP53 genomic DNA and staining chromosome 17 centromere DNA.

7. The method of any of claims 1 to 6, wherein the sample is subjected to a protease treatment with proteinase K, pepsin, collagenase, dispase, or a combination thereof after the step of staining the CD79a protein but before the step of staining TP53 genomic DNA and staining chromosome 17 centromere DNA, optionally wherein the sample is subjected to a heat treatment after the step of staining the CD79a protein but before the protease treatment.

8. The method of claim 1, wherein the CD79a protein-specific antibody is an anti-CD79a SP18 rabbit monoclonal antibody.

9. The method of claim 1, wherein the CD79a protein-specific antibody comprises a first label comprising an enzyme, and the first chromogenic component comprises an inducing component comprising a substrate for the enzyme of the first label for inducing the first label to emit the first color, optionally wherein the first label comprises biotin and the inducing component comprises streptavidin conjugated to an enzyme.

10. The method of claim 1, wherein the first chromogen component comprises a detectably labeled secondary antibody that specifically binds to the CD79a protein-specific antibody, and the detectably labeled secondary antibody comprises alkaline phosphatase and the first chromogen component further comprises fast red.

11. The method of any of claims 1 to 10, wherein the TP53 DNA-specific nucleic acid probe comprises:
(a) a nucleic acid molecule having at least 90%, at least 95% or at least 99% sequence identity with the sequence according to any one of SEQ ID NOs: 51-60; or
(b) a nucleic acid molecule having at least 90%, at least 95% or at least 99% sequence identity with at least 250 contiguous nucleotides of any one of SEQ ID NOs: 51-60; or
(c) a nucleic acid molecule consisting of the sequence according to any one of SEQ ID NOs: 51-60; or
(d) a nucleic acid molecule consisting of at least 250 contiguous nucleotides of any one of SEQ ID NOs: 51-60.

12. The method of any of claims 1 to 11, wherein the TP53 DNA-specific nucleic acid probe comprises two or more portions, wherein:
the first portion comprises at least 250 contiguous nucleotides of a nucleic acid sequence with at least 90% sequence identity to one of SEQ ID NOs: 51-60; and
the second portion comprises at least 250 contiguous nucleotides of a nucleic acid with at least 90% sequence identity to one of SEQ ID NOs: 51-60, wherein the first and second portions are different from one another, and wherein the TP53 DNA-specific nucleic acid probe is at least 500, at least 1000, or at least 5000 nucleotides in length.

13. The method of any of claims 1 to 12, wherein the TP53 DNA-specific nucleic acid probe comprises at least two of the probes of any one of claims 10 to 12.

14. The method of any of claims 1 to 13, wherein the TP53 DNA-specific nucleic acid probe comprises a detectable label, and wherein the second chromogen component comprises a primary antibody that specifically binds to the detectable label and a secondary antibody that specifically binds to the primary antibody, wherein the secondary antibody is conjugated to horseradish peroxidase and the second chromogen component further comprises hydrogen peroxide and silver.

15. The method of any of claims 1 to 14, wherein the chromosome 17 centromere-DNA specific nucleic acid probe comprises a set of two or more single-stranded oligonucleotide control probes specific for X distinct monomers of an alpha satellite control region of chromosome 17, wherein X = 2-14, wherein each control probe comprises:
▪ a sequence selected from the group consisting of SEQ ID NOs: 61-74; or
▪ a sequence selected from the group consisting of a truncated version of SEQ ID NOs: 61-74, the truncated version being at least 40 contiguous bp of said SEQ ID NOs:61-74; or
▪ a sequence selected from the group consisting of a sequence that has at least 70% sequence identity to one of SEQ ID NOs: 61-74, or
▪ complements thereof.

16. The method of any of claim 15, wherein the step of contacting the sample with the chromosome 17 centromere-DNA specific nucleic acid probe comprises hybridizing the probe under conditions for a period of time less than about 3 hours.

17. The method of claim 15, wherein the two or more single-stranded oligonucleotide control probes each comprise between 50 to 100 nucleotides.

18. The method of any of claims 1 to 17, wherein the chromosome 17 centromere DNA-specific nucleic acid probe comprises a hapten, and the hapten comprises dinitrophenyl, digoxigenin, biotin, or fluorescein, and wherein the third chromogen component comprises a primary antibody that specifically binds to the hapten, and a secondary antibody that specifically binds to the primary antibody, wherein the secondary antibody is conjugated to horseradish peroxidase and the third chromogen component further comprises a green chromogen component as substrate for the horseradish peroxidase.

19. A single slide comprising a sample of cells chromogenically stained for CD79a protein, TP53 DNA, and chromosome 17 DNA.

20. The slide of claim 19, wherein CD79a protein is stained with a first chromogen, TP53 DNA is stained with a second chromogen, and chromosome 17 is stained with a third chromogen.

21. The slide of claim 20, wherein the first chromogen comprises fast red, the second chromogen comprises silver, and the third chromogen comprises a green chromogen component.

22. The slide of any of claims 19 to 21, wherein more than 50% of the nuclei have enumerable signals for chromosome 17, and wherein each enumerable signal is a generally round shape, a round shape is a shape defined by a simple closed curve fitting within a first region, the first region is an area on and between an inner concentric circle and an outer concentric circle, the inner concentric circle having an inner radius (Rᵢₙ) and the outer concentric circle having a outer radius (Rₒᵤₜ) wherein Rᵢₙ is ≥ 50% of Rₒᵤₜ, and the simple closed curve has a radius Rₛᵢₘₚₗₑ wherein Rᵢₙ ≤ Rₛᵢₘₚₗₑ ≤ Rₒᵤₜ.

## Patentansprüche

1. Multiplex-Verfahren zum gemeinsamen Nachweis von CD79a-Protein, genomischer TP53-DNS und Chromosom-17-Centromer-DNS in einer Probe auf einem einzigen Objektträger, wobei das Verfahren Folgendes umfasst:
Färben des CD79a-Proteins durch Inkontaktbringen der Probe mit einem CD79a-Protein-spezifischen Antikörper und Inkontaktbringen der Probe mit einer ersten Chromogen-Komponente für den CD79a-Protein-spezifischen Antikörper, wobei die erste Chromogen-Komponente darauf angepasst ist, eine erste Farbe auszustrahlen oder sichtbar zu machen, wobei die Gegenwart der ersten Farbe auf die Gegenwart des CD79a-Proteins hinweist; und
Färben der genomischen TP53-DNS und Färben der Chromosom-17-Centromer-DNS durch Inkontaktbringen der Probe mit einer genomischen TP53-DNS-spezifischen Nucleinsäuresonde und mit einer Chromosom-17-Centromer-DNS-spezifischen Nucleinsäuresonde, und Inkontaktbringen der Probe mit einer zweiten Chromogen-Komponente für die genomische TP53-DNS-spezifische Nucleinsäuresonde und mit einer dritten Chromogen-Komponente für die Chromosom-17-Centromer-DNS-spezifische Nucleinsäuresonde, wobei die zweite Chromogen-Komponente darauf angepasst ist, eine zweite Farbe auszustrahlen oder sichtbar zu machen und die dritte Chromogen-Komponente darauf angepasst ist, eine dritte Farbe auszustrahlen oder sichtbar zu machen, wobei die Gegenwart der zweiten Farbe auf die Gegenwart der genomischen TP53-DNS hinweist und die Gegenwart der dritten Farbe auf die Gegenwart der Chromosom-17-Centromer-DNS hinweist.

2. Verfahren nach Anspruch 1, wobei die Probe eine Blutprobe ist.

3. Verfahren nach Anspruch 1 oder 2, wobei die erste Chromogen-Komponente Fast-Red umfasst, die zweite Chromogen-Komponente Silber umfasst, und die dritte Chromogen-Komponente eine Grün-Chromogen-Komponente umfasst.

4. Verfahren nach einem der Ansprüche 1 bis 3, ferner umfassend das Visualisieren der Farben unter Verwendung von Hellfeld-Mikroskopie.

5. Verfahren nach einem der Ansprüche 1 bis 4, wobei das Verfahren automatisiert ist.

6. Verfahren nach einem der Ansprüche 1 bis 5, wobei der Schritt des Färbens des CD79a-Proteins vor dem Schritt des Färbens der genomischen TP53-DNS und des Färbens der Chromosom-17-Centromer-DNS durchgeführt wird.

7. Verfahren nach einem der Ansprüche 1 bis 6, wobei die Probe einer Protease-Behandlung mit Proteinase K, Pepsin, Collagenase, Dispase oder einer Kombination davon, nach dem Schritt des Färbens des CD79a-Proteins, aber vor dem Schritt des Färbens der genomischen TP53-DNS und des Färbens der Chromosom-17-Centromer-DNS unterzogen wird, gegebenenfalls wobei die Probe einer Hitzebehandlung nach dem Schritt des Färbens des CD79a-Proteins, aber vor der Protease-Behandlung unterzogen wird.

8. Verfahren nach Anspruch 1, wobei der CD79a-Protein-spezifische Antikörper ein monoklonaler Anti-CD79a-SP18-Kaninchen-Antikörper ist.

9. Verfahren nach Anspruch 1, wobei der CD79a-Protein-spezifische Antikörper eine erste Markierung, umfassend ein Enzym, umfasst und die erste chromogene Komponente eine induzierende Komponente, umfassend ein Substrat für das Enzym der ersten Markierung zum Induzieren der ersten Markierung zur Ausstrahlung der ersten Farbe, umfasst, gegebenenfalls wobei die erste Markierung Biotin umfasst und die induzierende Komponente an ein Enzym konjugiertes Streptavidin umfasst.

10. Verfahren nach Anspruch 1, wobei die erste Chromogen-Komponente einen nachweisbar markierten Sekundärantikörper umfasst, der spezifisch an den CD79a-Protein-spezifischen Antikörper bindet, und der nachweisbar markierte Sekundärantikörper alkalische Phosphatase umfasst und die erste Chromogen-Komponente ferner Fast-Red umfasst.

11. Verfahren nach einem der Ansprüche 1 bis 10, wobei die TP53-DNS-spezifische Nucleinsäuresonde Folgendes umfasst:
(a) ein Nucleinsäuremolekül, das eine mindestens 90%ige, mindestens 95%ige oder mindestens 99%ige Sequenzidentität mit der Sequenz gemäß einer der SEQ ID NO: 51-60 aufweist; oder
(b) ein Nucleinsäuremolekül, das eine mindestens 90%ige, mindestens 95%ige oder mindestens 99%ige Sequenzidentität mit mindestens 250 aufeinander folgenden Nucleotiden von einer der SEQ ID NO: 51-60 aufweist; oder
(c) ein Nucleinsäuremolekül, bestehend aus der Sequenz nach einer der SEQ ID NO: 51-60; oder
(d) ein Nucleinsäuremolekül, bestehend aus mindestens 250 aufeinander folgenden Nucleotiden von einer der SEQ ID NO: 51-60.

12. Verfahren nach einem der Ansprüche 1 bis 11, wobei die TP53-DNS-spezifische Nucleinsäuresonde zwei oder mehr Abschnitte umfasst, wobei:
der erste Abschnitt mindestens 250 aufeinander folgende Nucleotide einer Nucleinsäuresequenz mit einer mindestens 90%igen Sequenzidentität, bezogen auf eine von SEQ ID NO: 51-60, umfasst; und
der zweite Abschnitt mindestens 250 aufeinander folgende Nucleotide einer Nucleinsäure mit einer mindestens 90%igen Sequenzidentität, bezogen auf eine von SEQ ID NO: 51-60, umfasst, wobei die ersten und zweiten Abschnitte unterschiedlich voneinander sind, und wobei die TP53-DNS-spezifische Nucleinsäuresonde mindestens 500, mindestens 1000 oder mindestens 5000 Nucleotide lang ist.

13. Verfahren nach einem der Ansprüche 1 bis 12, wobei die TP53-DNS-spezifische Nucleinsäuresonde mindestens zwei der Sonden nach einem der Ansprüche 10 bis 12 umfasst.

14. Verfahren nach einem der Ansprüche 1 bis 13, wobei die TP53-DNS-spezifische Nucleinsäuresonde eine nachweisbare Markierung umfasst, und wobei die zweite Chromogen-Komponente einen Primärantikörper, der spezifisch an die nachweisbare Markierung bindet, und einen Sekundärantikörper, der spezifisch an den Primärantikörper bindet, umfasst, wobei der Sekundärantikörper an Meerrettich-Peroxidase konjugiert ist, und die zweite Chromogen-Komponente ferner Wasserstoffperoxid und Silber umfasst.

15. Verfahren nach einem der Ansprüche 1 bis 14, wobei die Chromosom-17-Centromer-DNS-spezifische Nucleinsäuresonde einen Satz aus zwei oder mehr einzelsträngigen Oligonucleotid-Kontrollsonden umfasst, die für X unterschiedliche Monomere einer Alpha-Satelliten-Kontrollregion von Chromosom 17 spezifisch sind, wobei X = 2-14, wobei jede Kontrollsonde Folgendes umfasst:
• eine Sequenz, ausgewählt aus der Gruppe bestehend aus SEQ ID NO: 61-74; oder
• eine Sequenz, ausgewählt aus der Gruppe bestehend aus einer verkürzten Version von SEQ ID NO: 61-74, wobei die verkürzte Version mindestens 40 aufeinander folgende BP der SEQ ID NO: 61-74 sind; oder
• eine Sequenz, ausgewählt aus der Gruppe bestehend aus einer Sequenz, die eine mindestens 70%ige Sequenzidentität, bezogen auf eine von SEQ ID NO: 61-74, aufweist, oder
• Komplemente davon.

16. Verfahren nach Anspruch 15, wobei der Schritt des Inkontaktbringens der Probe mit der Chromosom-17-Centromer-DNS-spezifischen Nucleinsäuresonde das Hybridisieren der Sonde unter Bedingungen für eine Zeitspanne von weniger als etwa 3 Stunden umfasst.

17. Verfahren nach Anspruch 15, wobei die zwei oder mehr einzelsträngigen Oligonucleotid-Kontrollsonden jeweils zwischen 50 und 100 Nucleotide umfassen.

18. Verfahren nach einem der Ansprüche 1 bis 17, wobei die Chromosom-17-Centromer-DNS-spezifische Nucleinsäuresonde ein Hapten umfasst, und das Hapten Dinitrophenyl, Digoxigenin, Biotin oder Fluorescein umfasst, und wobei die dritte Chromogen-Komponente einen Primärantikörper, der spezifisch an das Hapten bindet, und einen Sekundärantikörper, der spezifisch an den Primärantikörper bindet, umfasst, wobei der Sekundärantikörper mit Meerrettich-Peroxidase konjugiert ist und die dritte Chromogen-Komponente ferner eine Grün-Chromogen-Komponente als Substrat für die Meerrettich-Peroxidase umfasst.

19. Einzelner Objektträger, umfassend eine Probe von auf CD79a-Protein, TP53-DNS, und Chromosom-17-DNS chromogenisch gefärbten Zellen.

20. Objektträger nach Anspruch 19, wobei CD79a-Protein mit einem ersten Chromogen gefärbt ist, TP53-DNS mit einem zweiten Chromogen gefärbt ist, und Chromosom 17 mit einem dritten Chromogen gefärbt ist.

21. Objektträger nach Anspruch 20, wobei das erste Chromogen Fast-Red umfasst, das zweite Chromogen Silber umfasst, und das dritte Chromogen eine Grün-Chromogen-Komponente umfasst.

22. Objektträger nach einem der Ansprüche 19 bis 21, wobei mehr als 50 % der Zellkerne zählbare Signale für Chromosom 17 aufweisen, und wobei jedes zählbare Signal eine im Allgemeinen runde Form aufweist, wobei eine runde Form eine Form ist, die durch eine einfache geschlossene Kurve, die innerhalb einer ersten Region passt, definiert wird, die erste Region ein Bereich auf und zwischen einem inneren konzentrischen Kreis und einem äußeren konzentrischen Kreis ist, der innere konzentrische Kreis einen inneren Radius (Rᵢₙ) aufweist und wobei der äußere konzentrische Kreis einen äußeren Radius (Rₒᵤₜ) aufweist, wobei Rᵢₙ ≥ 50 % von Rₒᵤₜ ist, und die einfache geschlossene Kurve einen Radius Rₛᵢₘₚₗₑ aufweist, wobei Rᵢₙ ≤ Rₛᵢₘₚₗₑ ≤ Rₒᵤₜ.

## Revendications

1. Procédé multiplexe pour la co-détection de la protéine CD79a, de l'ADN génomique de TP53 et de l'ADN du centromère du chromosome 17 dans un échantillon sur une lame unique, ledit procédé comprenant :
la coloration de la protéine CD79a par la mise en contact de l'échantillon avec un anticorps spécifique de la protéine CD79a et la mise en contact de l'échantillon avec un premier composant chromogène pour l'anticorps spécifique de la protéine CD79a, le premier composant chromogène étant adapté pour émettre ou rendre visible une première couleur, dans lequel la présence de la première couleur indique la présence de la protéine CD79a ; et
la coloration de l'ADN génomique de TP53 et la coloration de l'ADN du centromère du chromosome 17 par la mise en contact de l'échantillon avec une sonde d'acide nucléique spécifique de l'ADN génomique de TP53 et avec une sonde d'acide nucléique spécifique de l'ADN du centromère du chromosome 17, et la mise en contact de l'échantillon avec un deuxième composant chromogène pour la sonde d'acide nucléique spécifique de l'ADN génomique de TP53 et avec un troisième composant chromogène pour la sonde d'acide nucléique spécifique de l'ADN du centromère du chromosome 17, le deuxième composant chromogène étant adapté pour émettre ou rendre visible une deuxième couleur et le troisième composant chromogène étant adapté pour émettre ou rendre visible une troisième couleur, dans lequel la présence de la deuxième couleur indique la présence de l'ADN génomique de TP53 et la présence de la troisième couleur indiquant la présence de l'ADN du centromère du chromosome 17.

2. Procédé selon la revendication 1, dans lequel l'échantillon est un échantillon sanguin.

3. Procédé selon la revendication 1 ou 2, dans lequel le premier composant chromogène comprend du Fast Red, le deuxième composant chromogène comprend de l'argent et le troisième composant chromogène comprend un composant chromogène vert.

4. Procédé selon l'une quelconque des revendications 1 à 3 comprenant en outre la visualisation des couleurs grâce à la microscopie sur fond clair.

5. Procédé selon l'une quelconque des revendications 1 à 4, dans lequel le procédé est automatisé.

6. Procédé selon l'une quelconque des revendications 1 à 5, dans lequel l'étape de coloration de la protéine CD79a est réalisée avant l'étape de coloration de l'ADN génomique de TP53 et de coloration de l'ADN du centromère du chromosome 17.

7. Procédé selon l'une quelconque des revendications 1 à 6, dans lequel l'échantillon est soumis à un traitement par une protéase avec la protéinase K, la pepsine, la collagénase, la dispase ou une combinaison de celles-ci après l'étape de coloration de la protéine CD79a, mais avant l'étape de coloration de l'ADN génomique de TP53 et de coloration de l'ADN du centromère du chromosome 17, éventuellement dans lequel l'échantillon est soumis à un traitement thermique après l'étape de coloration de la protéine CD79a, mais avant le traitement par une protéase.

8. Procédé de la revendication 1, dans lequel l'anticorps spécifique de la protéine CD79a est un anticorps monoclonal de lapin SP18 anti-CD79a.

9. Procédé de la revendication 1, dans lequel l'anticorps spécifique de la protéine CD79a comprend un premier marqueur comprenant une enzyme, et le premier composant chromogène comprend un composant inducteur comprenant un substrat pour l'enzyme du premier marqueur pour induire le premier marqueur à émettre la première couleur, éventuellement dans lequel le premier marqueur comprend de la biotine et le composant inducteur comprenant de la streptavidine conjuguée à une enzyme.

10. Procédé de la revendication 1, dans lequel le premier composant chromogène comprend un anticorps secondaire marqué de manière détectable qui se lie spécifiquement à l'anticorps spécifique de la protéine CD79a et l'anticorps secondaire marqué de manière détectable comprend une phosphatase alcaline et le premier composant chromogène comprend en outre du Fast Red.

11. Procédé selon l'une quelconque des revendications 1 à 10, dans lequel la sonde d'acide nucléique spécifique de l'ADN de TP53 comprend :
(a) une molécule d'acide nucléique ayant au moins 90 %, au moins 95 % ou au moins 99% d'identité de séquence avec la séquence selon l'une quelconque des SEQ ID NO: 51-60 ; ou
(b) une molécule d'acide nucléique ayant au moins 90 %, au moins 95 % ou au moins 99% d'identité de séquence avec au moins 250 nucléotides contigus de l'une quelconque des SEQ ID NO: 51-60 ; ou
(c) une molécule d'acide nucléique constituée de la séquence selon l'une quelconque des SEQ ID NO: 51-60 ; ou
(d) une molécule d'acide nucléique constituée d'au moins 250 nucléotides contigus de l'une quelconque des SEQ ID NO: 51-60.

12. Procédé selon l'une quelconque des revendications 1 à 11, dans lequel la sonde d'acide nucléique spécifique de l'ADN de TP53 comprend deux portions ou plus, dans lequel
la première portion comprend au moins 250 nucléotides contigus d'une séquence d'acides nucléiques avec au moins 90 % d'identité de séquence avec l'une des SEQ ID NO: 51-60; et
la deuxième portion comprend au moins 250 nucléotides contigus d'un acide nucléique avec au moins 90 % d'identité de séquence avec l'une des SEQ ID NO: 51-60, dans lequel les première et deuxième portions sont différentes l'une de l'autre, et la longueur de la sonde d'acide nucléique spécifique de l'ADN de TP53 est d'au moins 500, d'au moins 1 000 ou d'au moins 5 000 nucléotides.

13. Procédé selon l'une quelconque des revendications 1 à 12, dans lequel la sonde d'acide nucléique spécifique de l'ADN de TP53 comprend au moins deux des sondes de l'une quelconque des revendications 10 à 12.

14. Procédé selon l'une quelconque des revendications 1 à 13, dans lequel la sonde d'acide nucléique spécifique de l'ADN de TP53 comprend un marqueur détectable, et dans lequel le deuxième composant chromogène comprend un anticorps primaire qui se lie spécifiquement au marqueur détectable et un anticorps secondaire qui se lie spécifiquement à l'anticorps primaire, dans lequel l'anticorps secondaire est conjugué à de la peroxydase de raifort et le deuxième composant chromogène comprend en outre du peroxyde d'hydrogène et de l'argent.

15. Procédé selon l'une quelconque des revendications 1 à 14, dans lequel la sonde d'acide nucléique spécifique de l'ADN du centromère du chromosome 17 comprend un ensemble de deux sondes de contrôle ou plus d'oligonucléotide monocaténaire spécifique de X monomères distincts d'une région de contrôle alpha-satellite du chromosome 17, où X = 2 à 14, dans lequel chaque sonde de contrôle comprend :
• une séquence choisie dans le groupe constitué par les SEQ ID NO: 61-74 ; ou
• une séquence choisie dans le groupe constitué par une version tronquée des SEQ ID NO: 61-74, la version tronquée comprenant au moins 40 pb contiguës desdites SEQ ID NO: 61-74 ; ou
• une séquence choisie dans le groupe constitué par une séquence qui a au moins 70 % d'identité de séquence avec l'une des SEQ ID NO: 61-74, ou
• des compléments de celle-ci.

16. Procédé selon la revendication 15, dans lequel l'étape de mise en contact de l'échantillon avec la sonde d'acide nucléique spécifique de l'ADN du centromère du chromosome 17 comprend l'hybridation de la sonde dans certaines conditions pendant un laps de temps inférieur à environ 3 heures.

17. Procédé selon la revendication 15, dans lequel les deux sondes de contrôle ou plus d'oligonucléotide monocaténaire spécifique comprennent chacune entre 50 et 100 nucléotides.

18. Procédé selon l'une quelconque des revendications 1 à 17, dans lequel la sonde d'acide nucléique spécifique de l'ADN du centromère du chromosome 17 comprend un haptène et l'haptène comprend du dinitrophényle, de la digoxigénine, de la biotine ou de la fluorescéine, et dans lequel le troisième composant chromogène comprend un anticorps primaire qui se lie spécifiquement à l'haptène et un anticorps secondaire qui se lie spécifiquement à l'anticorps primaire, dans lequel l'anticorps secondaire est conjugué à de la peroxydase de raifort et le troisième composant chromogène comprenant en outre un composant chromogène vert en tant que substrat pour la peroxydase de raifort.

19. Lame unique comprenant un échantillon de cellules colorées par des chromogènes de la protéine CD79a, de l'ADN de TP53 et de l'ADN du chromosome 17.

20. Lame selon la revendication 19, dans laquelle la protéine CD79a est colorée par un premier chromogène, l'ADN de TP53 est coloré par un deuxième chromogène et le chromosome 17 est coloré par un troisième chromogène.

21. Lame selon la revendication 20, dans laquelle le premier chromogène comprend du Fast Red, le deuxième chromogène comprend de l'argent et le troisième chromogène comprend un composant chromogène vert.

22. Lame selon l'une quelconque des revendications 19 à 21, dans laquelle plus de 50 % des noyaux présentent des signaux dénombrables pour le chromosome 17, et dans laquelle chaque signal dénombrable est généralement de forme ronde, une forme ronde étant définie par une courbe fermée simple contenue dans une première région, la première région étant une zone sur et entre un cercle concentrique intérieur et un cercle concentrique extérieur, le cercle concentrique intérieur ayant un rayon interne (Rᵢₙ) et le cercle concentrique extérieur ayant un rayon externe (Rₒᵤₜ), dans laquelle Rᵢₙ est ≥ 50 % de Rₒᵤₜ et la courbe fermée simple a un rayon Rₛᵢₘₚₗₑ où Rᵢₙ ≤ Rₛᵢₘₚₗₑ ≤ Rₒᵤₜ.
